# EUROPEAN PATENT APPLICATION

(11) **EP 2 292 658 A2**
(43) Date of publication of application: **09.03.2011**
(21) Application number: 10182001.7
(22) Date of filing: 25.10.2002
(51) Int. Cl.: C07K 16/08, C07K 16/12, A61K 39/02, A61K 39/12, C12N 15/82, C07K 14/705, C07K 14/32

(54) **Novel immunoadhesins for treating and preventing toxicity and pathogen-mediated diseases**

(30) Priority: 26.10.2001 US 47542
(62) Divisional of application: 02804821.3
(71) Applicant: PLANET BIOTECHNOLOGY, INC., Hayward, CA 94545 (US)
(72) Inventor: Larrick, James, William, Woodside, CA 94062 (US); Wycoff, Keith, Lynn, Palo Alto, CA 94303 (US)
(74) Representative: Carlsson, Carl Fredrik Munk

(57) **Abstract**

Immunoadhesins active against toxins and pathogens are described, with specific examples directed to immunoadhesins for thwarting pathogens such as anthrax and the common cold. The immunoadhesin-receptor ligand principle can be employed to counter virtually any pathogen, toxicant or toxin, including, e.g., natural and synthetic metabolic poisons.

SEQUENCE LISTING

<110> PLANET BIOTECHNOLOGY, INC.
      LARRICK, JAMES W.
      WYCOFF, KEITH L.

<120> NOVEL IMMUNOADHESINS FOR TREATING AND PREVENTING TOXICITY AND
      PATHOGEN-MEDIATED DISEASES

<130> 415142000449

<140> EP 02804821.3
<141> 2002-10-25

<150> PCT/US02/34197
<151> 2002-10-25

<150> US 10/047,542
<151> 2001-10-26

<150> PCT/US01/13932
<151> 2001-04-28

<150> 60/200,298
<151> 2000-04-28

<160> 101

<170> PatentIn Ver. 2.1

<210> 1
<211> 1596
<212> DNA
<213> Homo sapiens

<400> 1
atggctccca gcagcccccg gcccgcgctg cccgcactcc tggtcctgct cggggctctg 60
ttcccaggac ctggcaatgc ccagacatct gtgtccccct caaaagtcat cctgccccgg 120
ggaggctccg tgctggtgac atgcagcacc tcctgtgacc agcccaagtt gttgggcata 180
gagaccccgt tgcctaaaaa ggagttgctc ctgcctggga acaaccggaa ggtgtatgaa 240
ctgagcaatg tgcaagaaga tagccaacca atgtgctatt caaactgccc tgatgggcag 300
tcaacagcta aaaccttcct caccgtgtac tggactccag aacgggtgga actggcaccc 360
ctcccctctt ggcagccagt gggcaagaac cttaccctac gctgccaggt ggagggtggg 420
gcaccccggg ccaacctcac cgtggtgctg ctccgtgggg agaaggagct gaaacgggag 480
ccagctgtgg gggagcccgc tgaggtcacg accacggtgc tggtgaggag agatcaccat 540
ggagccaatt tctcgtgccg cactgaactg gacctgcggc cccaagggct ggagctgttt 600
gagaacacct cggcccccta ccagctccag acctttgtcc tgccagcgac tcccccacaa 660
cttgtcagcc cccgggtcct agaggtggac acgcagggga ccgtggtctg ttccctggac 720
gggctgttcc cagtctcgga ggcccaggtc cacctggcac tgggggacca gaggttgaac 780
cccacagtca cctatggcaa cgactccttc tcggccaagg cctcagtcag tgtgaccgca 840
gaggacgagg gcacccagcg gctgacgtgt gcagtaatac tggggaacca gagccaggag 900
acactgcaga cagtgaccat ctacagcttt ccggcgccca acgtgattct gacgaagcca 960
gaggtctcag aagggaccga ggtgacagtg aagtgtgagg cccaccctag agccaaggtg 1020
acgctgaatg gggttccagc ccagccactg ggcccgaggg cccagctcct gctgaaggcc 1080
accccagagg acaacgggcg cagcttctcc tgctctgcaa ccctggaggt ggccggccag 1140
cttatacaca agaaccagac ccgggagctt cgtgtcctgt atggcccccg actggacgag 1200
agggattgtc cgggaaactg gacgtggcca gaaaattccc agcagactcc aatgtgccag 1260
gcttggggga acccattgcc cgagctcaag tgtctaaagg atggcacttt cccactgccc 1320
atcggggaat cagtgactgt cactcgagat cttgagggca cctacctctg tcgggccagg 1380
agcactcaag gggaggtcac ccgcaaggtg accgtgaatg tgctctcccc ccggtatgag 1440
attgtcatca tcactgtggt agcagccgca gtcataatgg gcactgcagg cctcagcacg 1500
tacctctata accgccagcg gaagatcaag aaatacagac tacaacaggc ccaaaaaggg 1560
acccccatga aaccgaacac acaagccacg cctccc 1596

<210> 2
<211> 532
<212> PRT
<213> Homo sapiens

<400> 2
Met Ala Pro Ser Ser Pro Arg Pro Ala Leu Pro Ala Leu Leu Val Leu
  1               5                  10                  15

Leu Gly Ala Leu Phe Pro Gly Pro Gly Asn Ala Gln Thr Ser Val Ser
             20                  25                  30

Pro Ser Lys Val Ile Leu Pro Arg Gly Gly Ser Val Leu Val Thr Cys
         35                  40                  45

Ser Thr Ser Cys Asp Gln Pro Lys Leu Leu Gly Ile Glu Thr Pro Leu
     50                  55                  60

Pro Lys Lys Glu Leu Leu Leu Pro Gly Asn Asn Arg Lys Val Tyr Glu
 65                  70                  75                  80

Leu Ser Asn Val Gln Glu Asp Ser Gln Pro Met Cys Tyr Ser Asn Cys
                85                  90                  95

Pro Asp Gly Gln Ser Thr Ala Lys Thr Phe Leu Thr Val Tyr Trp Thr
            100                 105                 110

Pro Glu Arg Val Glu Leu Ala Pro Leu Pro Ser Trp Gln Pro Val Gly
        115                 120                 125

Lys Asn Leu Thr Leu Arg Cys Gln Val Glu Gly Gly Ala Pro Arg Ala
    130                 135                 140

Asn Leu Thr Val Val Leu Leu Arg Gly Glu Lys Glu Leu Lys Arg Glu
145                 150                 155                 160

Pro Ala Val Gly Glu Pro Ala Glu Val Thr Thr Thr Val Leu Val Arg
                165                 170                 175

Arg Asp His His Gly Ala Asn Phe Ser Cys Arg Thr Glu Leu Asp Leu
            180                 185                 190

Arg Pro Gln Gly Leu Glu Leu Phe Glu Asn Thr Ser Ala Pro Tyr Gln
        195                 200                 205

Leu Gln Thr Phe Val Leu Pro Ala Thr Pro Pro Gln Leu Val Ser Pro
    210                 215                 220

Arg Val Leu Glu Val Asp Thr Gln Gly Thr Val Val Cys Ser Leu Asp
225                 230                 235                 240

Gly Leu Phe Pro Val Ser Glu Ala Gln Val His Leu Ala Leu Gly Asp
                245                 250                 255

Gln Arg Leu Asn Pro Thr Val Thr Tyr Gly Asn Asp Ser Phe Ser Ala
            260                 265                 270

Lys Ala Ser Val Ser Val Thr Ala Glu Asp Glu Gly Thr Gln Arg Leu
        275                 280                 285

Thr Cys Ala Val Ile Leu Gly Asn Gln Ser Gln Glu Thr Leu Gln Thr
    290                 295                 300

Val Thr Ile Tyr Ser Phe Pro Ala Pro Asn Val Ile Leu Thr Lys Pro
305                 310                 315                 320

Glu Val Ser Glu Gly Thr Glu Val Thr Val Lys Cys Glu Ala His Pro
                325                 330                 335

Arg Ala Lys Val Thr Leu Asn Gly Val Pro Ala Gln Pro Leu Gly Pro
            340                 345                 350

Arg Ala Gln Leu Leu Leu Lys Ala Thr Pro Glu Asp Asn Gly Arg Ser
        355                 360                 365

Phe Ser Cys Ser Ala Thr Leu Glu Val Ala Gly Gln Leu Ile His Lys
    370                 375                 380

Asn Gln Thr Arg Glu Leu Arg Val Leu Tyr Gly Pro Arg Leu Asp Glu
385                 390                 395                 400

Arg Asp Cys Pro Gly Asn Trp Thr Trp Pro Glu Asn Ser Gln Gln Thr
                405                 410                 415

Pro Met Cys Gln Ala Trp Gly Asn Pro Leu Pro Glu Leu Lys Cys Leu
            420                 425                 430

Lys Asp Gly Thr Phe Pro Leu Pro Ile Gly Glu Ser Val Thr Val Thr
        435                 440                 445

Arg Asp Leu Glu Gly Thr Tyr Leu Cys Arg Ala Arg Ser Thr Gln Gly
    450                 455                 460

Glu Val Thr Arg Lys Val Thr Val Asn Val Leu Ser Pro Arg Tyr Glu
465                 470                 475                 480

Ile Val Ile Ile Thr Val Val Ala Ala Ala Val Ile Met Gly Thr Ala
                485                 490                 495

Gly Leu Ser Thr Tyr Leu Tyr Asn Arg Gln Arg Lys Ile Lys Lys Tyr
            500                 505                 510

Arg Leu Gln Gln Ala Gln Lys Gly Thr Pro Met Lys Pro Asn Thr Gln
        515                 520                 525

Ala Thr Pro Pro
    530

<210> 3
<211> 3003
<212> DNA
<213> Homo sapiens

<400> 3
gctataagga tcacgcgccc cagtcgacgc tgagctcctc tgctactcag agttgcaacc 60
tcagcctcgc tatggctccc agcagccccc ggcccgcgct gcccgcactc ctggtcctgc 120
tcggggctct gttcccagga cctggcaatg cccagacatc tgtgtccccc tcaaaagtca 180
tcctgccccg gggaggctcc gtgctggtga catgcagcac ctcctgtgac cagcccaagt 240
tgttgggcat agagaccccg ttgcctaaaa aggagttgct cctgcctggg aacaaccgga 300
aggtgtatga actgagcaat gtgcaagaag atagccaacc aatgtgctat tcaaactgcc 360
ctgatgggca gtcaacagct aaaaccttcc tcaccgtgta ctggactcca gaacgggtgg 420
aactggcacc cctcccctct tggcagccag tgggcaagaa ccttacccta cgctgccagg 480
tggagggtgg ggcaccccgg gccaacctca ccgtggtgct gctccgtggg gagaaggagc 540
tgaaacggga gccagctgtg ggggagcccg ctgaggtcac gaccacggtg ctggtgagga 600
gagatcacca tggagccaat ttctcgtgcc gcactgaact ggacctgcgg ccccaagggc 660
tggagctgtt tgagaacacc tcggccccct accagctcca gacctttgtc ctgccagcga 720
ctcccccaca acttgtcagc ccccgggtcc tagaggtgga cacgcagggg accgtggtct 780
gttccctgga cgggctgttc ccagtctcgg aggcccaggt ccacctggca ctgggggacc 840
agaggttgaa ccccacagtc acctatggca acgactcctt ctcggccaag gcctcagtca 900
gtgtgaccgc agaggacgag ggcacccagc ggctgacgtg tgcagtaata ctggggaacc 960
agagccagga gacactgcag acagtgacca tctacagctt tccggcgccc aacgtgattc 1020
tgacgaagcc agaggtctca gaagggaccg aggtgacagt gaagtgtgag gcccacccta 1080
gagccaaggt gacgctgaat ggggttccag cccagccact gggcccgagg gcccagctcc 1140
tgctgaaggc caccccagag gacaacgggc gcagcttctc ctgctctgca accctggagg 1200
tggccggcca gcttatacac aagaaccaga cccgggagct tcgtgtcctg tatggccccc 1260
gactggacga gagggattgt ccgggaaact ggacgtggcc agaaaattcc cagcagactc 1320
caatgtgcca ggcttggggg aacccattgc ccgagctcaa gtgtctaaag gatggcactt 1380
tcccactgcc catcggggaa tcagtgactg tcactcgaga tcttgagggc acctacctct 1440
gtcgggccag gagcactcaa ggggaggtca cccgcaaggt gaccgtgaat gtgctctccc 1500
cccggtatga gattgtcatc atcactgtgg tagcagccgc agtcataatg ggcactgcag 1560
gcctcagcac gtacctctat aaccgccagc ggaagatcaa gaaatacaga ctacaacagg 1620
cccaaaaagg gacccccatg aaaccgaaca cacaagccac gcctccctga acctatcccg 1680
ggacagggcc tcttcctcgg ccttcccata ttggtggcag tggtgccaca ctgaacagag 1740
tggaagacat atgccatgca gctacaccta ccggccctgg gacgccggag gacagggcat 1800
tgtcctcagt cagatacaac agcatttggg gccatggtac ctgcacacct aaaacactag 1860
gccacgcatc tgatctgtag tcacatgact aagccaagag gaaggagcaa gactcaagac 1920
atgattgatg gatgttaaag tctagcctga tgagagggga agtggtgggg gagacatagc 1980
cccaccatga ggacatacaa ctgggaaata ctgaaacttg ctgcctattg ggtatgctga 2040
ggccccacag acttacagaa gaagtggccc tccatagaca tgtgtagcat caaaacacaa 2100
aggcccacac ttcctgacgg atgccagctt gggcactgct gtctactgac cccaaccctt 2160
gatgatatgt atttattcat ttgttatttt accagctatt tattgagtgt cttttatgta 2220
ggctaaatga acataggtct ctggcctcac ggagctccca gtccatgtca cattcaaggt 2280
caccaggtac agttgtacag gttgtacact gcaggagagt gcctggcaaa aagatcaaat 2340
ggggctggga cttctcattg gccaacctgc ctttccccag aaggagtgat ttttctatcg 2400
gcacaaaagc actatatgga ctggtaatgg ttcacaggtt cagagattac ccagtgaggc 2460
cttattcctc ccttcccccc aaaactgaca cctttgttag ccacctcccc acccacatac 2520
atttctgcca gtgttcacaa tgacactcag cggtcatgtc tggacatgag tgcccaggga 2580
atatgcccaa gctatgcctt gtcctcttgt cctgtttgca tttcactggg agcttgcact 2640
attgcagctc cagtttcctg cagtgatcag ggtcctgcaa gcagtgggga agggggccaa 2700
ggtattggag gactccctcc cagctttgga agcctcatcc gcgtgtgtgt gtgtgtgtgt 2760
atgtgtagac aagctctcgc tctgtcaccc aggctggagt gcagtggtgc aatcatggtt 2820
cactgcagtc ttgacctttt gggctcaagt gatcctccca cctcagcctc ctgagtagct 2880
gggaccatag gctcacaaca ccacacctgg caaatttgat tttttttttt tttttcagag 2940
acggggtctc gcaacattgc ccagacttcc tttgtgttag ttaataaagc tttctcaact 3000
gcc 3003

<210> 4
<211> 6
<212> PRT
<213> Homo sapiens

<400> 4
Ser Glu Lys Asp Glu Leu
1               5

<210> 5
<211> 7
<212> PRT
<213> Homo sapiens

<400> 5
Arg Ser Glu Lys Asp Glu Leu
1               5

<210> 6
<211> 52
<212> DNA
<213> Artificial Sequence

<220>
<223> Description of Artificial Sequence: Cloning primer

<400> 6
tctgttccca ggaactagtt tggcacagac atctgtgtcc ccctcaaaag tc          52


<210> 7
<211> 38
<212> DNA
<213> Artificial Sequence

<220>
<223> Description of Artificial Sequence: Cloning primer

<400> 7
cataccgggg actagtcaca ttcacggtca cctcgcgg                          38


<210> 8
<211> 799
<212> PRT
<213> Homo sapiens

<400> 8
Gln Thr Ser Val Ser Pro Ser Lys Val Ile Leu Pro Arg Gly Gly Ser
1               5                   10                  15

Val Leu Val Thr Cys Ser Thr Ser Cys Asp Gln Pro Lys Leu Leu Gly
            20                  25                  30

Ile Glu Thr Pro Leu Pro Lys Lys Glu Leu Leu Leu Pro Gly Asn Asn
        35                  40                  45

Arg Lys Val Tyr Glu Leu Ser Asn Val Gln Glu Asp Ser Gln Pro Met
    50                  55                  60

Cys Tyr Ser Asn Cys Pro Asp Gly Gln Ser Thr Ala Lys Thr Phe Leu
65                  70                  75                  80

Thr Val Tyr Trp Thr Pro Glu Arg Val Glu Leu Ala Pro Leu Pro Ser
                85                  90                  95

Trp Gln Pro Val Gly Lys Asn Leu Thr Leu Arg Cys Gln Val Glu Gly
            100                 105                 110

Gly Ala Pro Arg Ala Asn Leu Thr Val Val Leu Leu Arg Gly Glu Lys
        115                 120                 125

Glu Leu Lys Arg Glu Pro Ala Val Gly Glu Pro Ala Glu Val Thr Thr
    130                 135                 140

Thr Val Leu Val Arg Arg Asp His His Gly Ala Asn Phe Ser Cys Arg
145                 150                 155                 160

Thr Glu Leu Asp Leu Arg Pro Gln Gly Leu Glu Leu Phe Glu Asn Thr
                165                 170                 175

Ser Ala Pro Tyr Gln Leu Gln Thr Phe Val Leu Pro Ala Thr Pro Pro
            180                 185                 190

Gln Leu Val Ser Pro Arg Val Leu Glu Val Asp Thr Gln Gly Thr Val
        195                 200                 205

Val Cys Ser Leu Asp Gly Leu Phe Pro Val Ser Glu Ala Gln Val His
    210                 215                 220

Leu Ala Leu Gly Asp Gln Arg Leu Asn Pro Thr Val Thr Tyr Gly Asn
225                 230                 235                 240

Asp Ser Phe Ser Ala Lys Ala Ser Val Ser Val Thr Ala Glu Asp Glu
                245                 250                 255

Gly Thr Gln Arg Leu Thr Cys Ala Val Ile Leu Gly Asn Gln Ser Gln
            260                 265                 270

Glu Thr Leu Gln Thr Val Thr Ile Tyr Ser Phe Pro Ala Pro Asn Val
        275                 280                 285

Ile Leu Thr Lys Pro Glu Val Ser Glu Gly Thr Glu Val Thr Val Lys
    290                 295                 300

Cys Glu Ala His Pro Arg Ala Lys Val Thr Leu Asn Gly Val Pro Ala
305                 310                 315                 320

Gln Pro Leu Gly Pro Arg Ala Gln Leu Leu Leu Lys Ala Thr Pro Glu

325                 330                 335

Asp Asn Gly Arg Ser Phe Ser Cys Ser Ala Thr Leu Glu Val Ala Gly
            340                 345                 350

Gln Leu Ile His Lys Asn Gln Thr Arg Glu Leu Arg Val Leu Tyr Gly
        355                 360                 365

Pro Arg Leu Asp Glu Arg Asp Cys Pro Gly Asn Trp Thr Trp Pro Glu
    370                 375                 380

Asn Ser Gln Gln Thr Pro Met Cys Gln Ala Trp Gly Asn Pro Leu Pro
385                 390                 395                 400

Glu Leu Lys Cys Leu Lys Asp Gly Thr Phe Pro Leu Pro Ile Gly Glu
                405                 410                 415

Ser Val Thr Val Thr Arg Asp Leu Glu Gly Thr Tyr Leu Cys Arg Ala
            420                 425                 430

Arg Ser Thr Gln Gly Glu Val Thr Arg Glu Val Thr Val Asn Val Thr
        435                 440                 445

Ser Gly Ser Ser Ala Ser Pro Thr Ser Pro Lys Val Phe Pro Leu Ser
    450                 455                 460

Leu Asp Ser Thr Pro Gln Asp Gly Asn Val Val Val Ala Cys Leu Val
465                 470                 475                 480

Gln Gly Phe Phe Pro Gln Glu Pro Leu Ser Val Thr Trp Ser Glu Ser
                485                 490                 495

Gly Gln Asn Val Thr Ala Arg Asn Phe Pro Pro Ser Gln Asp Ala Ser
            500                 505                 510

Gly Asp Leu Tyr Thr Thr Ser Ser Gln Leu Thr Leu Pro Ala Thr Gln
        515                 520                 525

Cys Pro Asp Gly Lys Ser Val Thr Cys His Val Lys His Tyr Thr Asn
    530                 535                 540

Ser Ser Gln Asp Val Thr Val Pro Cys Arg Val Pro Pro Pro Pro Pro
545                 550                 555                 560

Cys Cys His Pro Arg Leu Ser Leu His Arg Pro Ala Leu Glu Asp Leu
                565                 570                 575

Leu Leu Gly Ser Glu Ala Asn Leu Thr Cys Thr Leu Thr Gly Leu Arg
            580                 585                 590

Asp Ala Ser Gly Ala Thr Phe Thr Trp Thr Pro Ser Ser Gly Lys Ser
        595                 600                 605

Ala Val Gln Gly Pro Pro Glu Arg Asp Leu Cys Gly Cys Tyr Ser Val
    610                 615                 620

Ser Arg Val Leu Pro Gly Cys Ala Gln Pro Trp Asn His Gly Glu Thr
625                 630                 635                 640

Phe Thr Cys Thr Ala Ala His Pro Glu Leu Lys Thr Pro Leu Thr Ala
                645                 650                 655

Asn Ile Thr Lys Ser Gly Asn Thr Phe Arg Pro Glu Val His Leu Leu
            660                 665                 670

Pro Pro Pro Ser Glu Glu Leu Ala Leu Asn Glu Leu Val Thr Leu Thr
        675                 680                 685

Cys Leu Ala Arg Gly Phe Ser Pro Lys Asp Val Leu Val Arg Trp Leu
    690                 695                 700

Gln Gly Ser Gln Glu Leu Pro Arg Glu Lys Tyr Leu Thr Trp Ala Ser
705                 710                 715                 720

Arg Gln Glu Pro Ser Gln Gly Thr Thr Thr Tyr Ala Val Thr Ser Ile
                725                 730                 735

Leu Arg Val Ala Ala Glu Asp Trp Lys Lys Gly Glu Thr Phe Ser Cys
            740                 745                 750

Met Val Gly His Glu Ala Leu Pro Leu Ala Phe Thr Gln Lys Thr Ile
        755                 760                 765

Asp Arg Leu Ala Gly Lys Pro Thr His Ile Asn Val Ser Val Val Met
    770                 775                 780

Ala Glu Ala Asp Gly Thr Cys Tyr Arg Ser Glu Lys Asp Glu Leu
785                 790                 795

<210> 9
<211> 6313
<212> DNA
<213> Artificial Sequence

<220>
<223> Description of Artificial Sequence:
      Expression-type plasmid pSSPICAMHuA2

<400> 9
gaactcgagc agctgaagct tgcatgcctg caggtcgacg gtatcgataa ggatccctga 60
aagcgacgtt ggatgttaac atctacaaat tgccttttct tatcgaccat gtacgtaagc 120
gcttacgttt ttggtggacc cttgaggaaa ctggtagctg ttgtgggcct gtggtctcaa 180
gatggatcat taatttccac cttcacctac gatggggggc atcgcaccgg tgagtaatat 240
tgtacggcta agagcgaatt tggcctgtag gatccctgaa agcgacgttg gatgttaaca 300
tctacaaatt gccttttctt atcgaccatg tacgtaagcg cttacgtttt tggtggaccc 360
ttgaggaaac tggtagctgt tgtgggcctg tggtctcaag atggatcatt aatttccacc 420
ttcacctacg atggggggca tcgcaccggt gagtaatatt gtacggctaa gagcgaattt 480
ggcctgtagg atccctgaaa gcgacgttgg atgttaacat ctacaaattg ccttttctta 540
tcgaccatgt acgtaagcgc ttacgttttt ggtggaccct tgaggaaact ggtagctgtt 600
gtgggcctgt ggtctcaaga tggatcatta atttccacct tcacctacga tggggggcat 660
cgcaccggtg agtaatattg tacggctaag agcgaatttg gcctgtagga tccgcgagct 720
ggtcaatccc attgcttttg aagcagctca acattgatct ctttctcgag ggagattttt 780
caaatcagtg cgcaagacgt gacgtaagta tccgagtcag tttttatttt tctactaatt 840
tggtcgttta tttcggcgtg taggacatgg caaccgggcc tgaatttcgc gggtattctg 900
tttctattcc aactttttct tgatccgcag ccattaacga cttttgaata gatacgctga 960
cacgccaagc ctcgctagtc aaaagtgtac caaacaacgc tttacagcaa gaacggaatg 1020
cgcgtgacgc tcgcggtgac gccatttcgc cttttcagaa atggataaat agccttgctt 1080
cctattatat cttcccaaat taccaataca ttacactagc atctgaattt cataaccaat 1140
ctcgatacac caaatcgact ctagaggatc tatcgattcc cgggtaccat gggatctaaa 1200
ccttttttgt ctcttctttc attgtcattg cttttgttta catctactag tttggcacag 1260
acatctgtgt ccccctcaaa agtcatcctg ccccggggag gctccgtgct ggtgacatgc 1320
agcacctcct gtgaccagcc caagttgttg ggcatagaga ccccgttgcc taaaaaggag 1380
ttgctcctgc ctgggaacaa ccggaaggtg tatgaactga gcaatgtgca agaagatagc 1440
caaccaatgt gctattcaaa ctgccctgat gggcagtcaa cagctaaaac cttcctcacc 1500
gtgtactgga ctccagaacg ggtggaactg gcacccctcc cctcttggca gccagtgggc 1560
aagaacctta ccctacgctg ccaggtggag ggtggggcac cccgggccaa cctcaccgtg 1620
gtgctgctcc gtggggagaa ggagctgaaa cgggagccag ctgtggggga gcccgctgag 1680
gtcacgacca cggtgctggt gaggagagat caccatggag ccaatttctc gtgccgcact 1740
gaactggacc tgcggcccca agggctggag ctgtttgaga acacctcggc cccctaccag 1800
ctccagacct ttgtcctgcc agcgactccc ccacaacttg tcagcccccg ggtcctagag 1860
gtggacacgc aggggaccgt ggtctgttcc ctggacgggc tgttcccagt ctcggaggcc 1920
caggtccacc tggcactggg ggaccagagg ttgaacccca cagtcaccta tggcaacgac 1980
tccttctcgg ccaaggcctc agtcagtgtg accgcagagg acgagggcac ccagcggctg 2040
acgtgtgcag taatactggg gaaccagagc caggagacac tgcagacagt gaccatctac 2100
agctttccgg cgcccaacgt gattctgacg aagccagagg tctcagaagg gaccgaggtg 2160
acagtgaagt gtgaggccca ccctagagcc aaggtgacgc tgaatggggt tccagcccag 2220
ccactgggcc cgagggccca gctcctgctg aaggccaccc cagaggacaa cgggcgcagc 2280
ttctcctgct ctgcaaccct ggaggtggcc ggccagctta tacacaagaa ccagacccgg 2340
gagcttcgtg tcctgtatgg cccccgactg gacgagaggg attgtccggg aaactggacg 2400
tggccagaaa attcccagca gactccaatg tgccaggctt gggggaaccc attgcccgag 2460
ctcaagtgtc taaaggatgg cactttccca ctgcccatcg gggaatcagt gactgtcact 2520
cgagatcttg agggcaccta cctctgtcgg gccaggagca ctcaagggga ggtcacccgc 2580
gaggtgaccg tgaatgtgac tagtgggagc tcagcatccc cgaccagccc caaggtcttc 2640
ccgctgagcc tcgacagcac cccccaagat gggaacgtgg tcgtcgcatg cctggtccag 2700
ggcttcttcc cccaggagcc actcagtgtg acctggagcg aaagcggaca gaacgtgacc 2760
gccagaaact tcccacctag ccaggatgcc tccggggacc tgtacaccac gagcagccag 2820
ctgaccctgc cggccacaca gtgcccagac ggcaagtccg tgacatgcca cgtgaagcac 2880
tacacgaatt ccagccagga tgtgactgtg ccctgccgag ttcccccacc tcccccatgc 2940
tgccaccccc gactgtcgct gcaccgaccg gccctcgagg acctgctctt aggttcagaa 3000
gcgaacctca cgtgcacact gaccggcctg agagatgcct ctggtgccac cttcacctgg 3060
acgccctcaa gtgggaagag cgctgttcaa ggaccacctg agcgtgacct ctgtggctgc 3120
tacagcgtgt ccagagtact tcctggctgt gcccagccat ggaaccatgg ggagaccttc 3180
acctgcactg ctgcccaccc cgagttgaag accccactaa ccgccaacat cacaaaatcc 3240
ggaaacacat tccggcccga ggtccacctg ctgccgccgc cgtcggagga gctggccctg 3300
aacgagctgg tgacgctgac gtgcctggca cgtggcttca gccccaagga tgtgctggtt 3360
cgctggctgc aggggtcaca ggagctgccc cgcgagaagt acctgacttg ggcatcccgg 3420
caggagccca gccagggcac caccacctat gctgtgacca gcatactgcg cgtggcagcc 3480
gaggactgga agaaggggga gaccttctcc tgcatggtgg gccacgaggc cctgccgctg 3540
gccttcacac agaagaccat cgaccgcttg gcgggtaaac ccacccatat caatgtgtct 3600
gttgtcatgg cggaggcgga cggcacctgc tacagatctg aaaaggatga actttagaat 3660
tcgatatcaa gctaattccc gatcgttcaa acatttggca ataaagtttc ttaagattga 3720
atcctgttgc cggtcttgcg atgattatca tataatttct gttgaattac gttaagcatg 3780
taataattaa catgtaatgc atgacgttat ttatgagatg ggtttttatg attagagtcc 3840
cgcaattata catttaatac gcgatagaaa acaaaatata gcgcgcaaac taggataaat 3900
tatcgcgcgc ggtgtcatct atgttactag atcggggatc tgccggtctc cctatagtga 3960
gtcgtattaa tttcgataag ccaggttaac ctgcattaat gaatcggcca acgcgcgggg 4020
agaggcggtt tgcgtattgg gcgctcttcc gcttcctcgc tcactgactc gctgcgctcg 4080
gtcgttcggc tgcggcgagc ggtatcagct cactcaaagg cggtaatacg gttatccaca 4140
gaatcagggg ataacgcagg aaagaacatg tgagcaaaag gccagcaaaa ggccaggaac 4200
cgtaaaaagg ccgcgttgct ggcgtttttc cataggctcc gcccccctga cgagcatcac 4260
aaaaatcgac gctcaagtca gaggtggcga aacccgacag gactataaag ataccaggcg 4320

tttccccctg gaagctccct cgtgcgctct cctgttccga ccctgccgct taccggatac 4380
ctgtccgcct ttctcccttc gggaagcgtg gcgctttctc aatgctcacg ctgtaggtat 4440
ctcagttcgg tgtaggtcgt tcgctccaag ctgggctgtg tgcacgaacc ccccgttcag 4500
cccgaccgct gcgccttatc cggtaactat cgtcttgagt ccaacccggt aagacacgac 4560
ttatcgccac tggcagcagc cactggtaac aggattagca gagcgaggta tgtaggcggt 4620
gctacagagt tcttgaagtg gtggcctaac tacggctaca ctagaaggac agtatttggt 4680
atctgcgctc tgctgaagcc agttaccttc ggaaaaagag ttggtagctc ttgatccggc 4740
aaacaaacca ccgctggtag cggtggtttt tttgtttgca agcagcagat tacgcgcaga 4800
aaaaaaggat ctcaagaaga tcctttgatc ttttctacgg ggtctgacgc tcagtggaac 4860
gaaaactcac gttaagggat tttggtcatg agattatcaa aaaggatctt cacctagatc 4920
cttttaaatt aaaaatgaag ttttaaatca atctaaagta tatatgagta aacttggtct 4980
gacagttacc aatgcttaat cagtgaggca cctatctcag cgatctgtct atttcgttca 5040
tccatagttg cctgactccc cgtcgtgtag ataactacga tacgggaggg cttaccatct 5100
ggccccagtg ctgcaatgat accgcgagac ccacgctcac cggctccaga tttatcagca 5160
ataaaccagc cagccggaag ggccgagcgc agaagtggtc ctgcaacttt atccgcctcc 5220
atccagtcta ttaattgttg ccgggaagct agagtaagta gttcgccagt taatagtttg 5280
cgcaacgttg ttgccattgc tacaggcatc gtggtgtcac gctcgtcgtt tggtatggct 5340
tcattcagct ccggttccca acgatcaagg cgagttacat gatcccccat gttgtgcaaa 5400
aaagcggtta gctccttcgg tcctccgatc gttgtcagaa gtaagttggc cgcagtgtta 5460
tcactcatgg ttatggcagc actgcataat tctcttactg tcatgccatc cgtaagatgc 5520
ttttctgtga ctggtgagta ctcaaccaag tcattctgag aatagtgtat gcggcgaccg 5580
agttgctctt gcccggcgtc aatacgggat aataccgcgc cacatagcag aactttaaaa 5640
gtgctcatca ttggaaaacg ttcttcgggg cgaaaactct caaggatctt accgctgttg 5700
agatccagtt cgatgtaacc cactcgtgca cccaactgat cttcagcatc ttttactttc 5760
accagcgttt ctgggtgagc aaaaacagga aggcaaaatg ccgcaaaaaa gggaataagg 5820
gcgacacgga aatgttgaat actcatactc ttcctttttc aatattattg aagcatttat 5880
cagggttatt gtctcatgag cggatacata tttgaatgta tttagaaaaa taaacaaata 5940
ggggttccgc gcacatttcc ccgaaaagtg ccacctgacg tctaagaaac cattattatc 6000
atgacattaa cctataaaaa taggcgtatc acgaggccct ttcgtctcgc gcgtttcggt 6060
gatgacggtg aaaacctctg acacatgcag ctcccggaga cggtcacagc ttgtctgtaa 6120
gcggatgccg ggagcagaca agcccgtcag ggcgcgtcag cgggtgttgg cgggtgtcgg 6180
ggctggctta actatgcggc atcagagcag attgtactga gagtgcacca tatggacata 6240
ttgtcgttag aacgcggcta caattaatac ataaccttat gtatcataca catacgattt 6300
aggtgacact ata 6313

<210> 10
<211> 22
<212> PRT
<213> Phaseolus vulgaris

<400> 10
Met Ser Lys Pro Phe Leu Ser Leu Leu Ser Leu Ser Leu Leu Leu Phe
1               5                   10                  15

Thr Ser Thr Cys Leu Ala
            20

<210> 11
<211> 508
<212> DNA
<213> Artificial Sequence

<220>
<223> Description of Artificial Sequence: Protein coding
region of the plasmid pSHuJ

<400> 11
aggatctatc gattcccggg taccatggag aaccatttgc ttttctgggg agtcctggcg 60
gtttttatta aggctgttca tgtgaaagcc caagaagatg aaaggattgt tcttgttgac 120
aacaaatgta agtgtgcccg gattacttcc aggatcatcc gttcttccga agatcctaat 180
gaggacattg tggagagaaa catccgaatt attgttcctc tgaacaacag ggagaatatc 240
tctgatccca cctcaccatt gagaaccaga tttgtgtacc atttgtctga cctctgtaaa 300
aaatgtgatc ctacagaagt ggagctggat aatcagatag ttactgctac ccagagcaat 360
atctgtgatg aagacagtgc tacagagacc tgctacactt atgacagaaa caagtgctac 420
acagctgtgg tcccactcgt atatggtggt gagaccaaaa tggtggaaac agccttaacc 480
ccagatgcct gctatcctga ctgaattc 508

<210> 12
<211> 1845
<212> DNA
<213> Artificial Sequence

<220>
<223> Description of Artificial Sequence: Protein coding
region of plasmid pSHuSC

<400> 12
gtcgattccc gggtaccatg gtgctcttcg tgctcacctg cctgctggcg gtcttcccag 60
ccatctccac gaagagtccc atatttggtc ccgaggaggt gaatagtgtg gaaggtaact 120
cagtgtccat cacgtgctac tacccaccca cctctgtcaa ccggcacacc cggaagtact 180
ggtgccggca gggagctaga ggtggctgca taaccctcat ctcctcggag ggctacgtct 240
ccagcaaata tgcaggcagg gctaacctca ccaacttccc ggagaacggc acatttgtgg 300
tgaacattgc ccagctgagc caggatgact ccgggcgcta caagtgtggc ctgggcatca 360

atagccgagg cctgtccttt gatgtcagcc tggaggtcag ccagggtcct gggctcctaa 420
atgacactaa agtctacaca gtggacctgg gcagaacggt gaccatcaac tgccctttca 480
agactgagaa tgctcaaaag aggaagtcct tgtacaagca gataggcctg taccctgtgc 540
tggtcatcga ctccagtggt tatgtgaatc ccaactatac aggaagaata cgccttgata 600
ttcagggtac tggccagtta ctgttcagcg ttgtcatcaa ccaactcagg ctcagcgatg 660
ctgggcagta tctctgccag gctggggatg attccaatag taataagaag aatgctgacc 720
tccaagtgct aaagcccgag cccgagctgg tttatgaaga cctgaggggc tcagtgacct 780
tccactgtgc cctgggccct gaggtggcaa acgtggccaa atttctgtgc cgacagagca 840
gtggggaaaa ctgtgacgtg gtcgtcaaca ccctggggaa gagggcccca gcctttgagg 900
gcaggatcct gctcaacccc caggacaagg atggctcatt cagtgtggtg atcacaggcc 960
tgaggaagga ggatgcaggg cgctacctgt gtggagccca ttcggatggt cagctgcagg 1020
aaggctcgcc tatccaggcc tggcaactct tcgtcaatga ggagtccacg attccccgca 1080
gccccactgt ggtgaagggg gtggcaggaa gctctgtggc cgtgctctgc ccctacaacc 1140
gtaaggaaag caaaagcatc aagtactggt gtctctggga aggggcccag aatggccgct 1200
gccccctgct ggtggacagc gaggggtggg ttaaggccca gtacgagggc cgcctctccc 1260
tgctggagga gccaggcaac ggcaccttca ctgtcatcct caaccagctc accagccggg 1320
acgccggctt ctactggtgt ctgaccaacg gcgatactct ctggaggacc accgtggaga 1380
tcaagattat cgaaggagaa ccaaacctca aggttcccgg gaatgtcacg gctgtgctgg 1440
gagagactct caaggtcccc tgtcactttc catgcaaatt ctcctcgtac gagaaatact 1500
ggtgcaagtg gaataacacg ggctgccagg ccctgcccag ccaagacgaa ggccccagca 1560
aggccttcgt gaactgtgac gagaacagcc ggcttgtctc cctgaccctg aacctggtga 1620
ccagggctga tgagggctgg tactggtgtg gagtgaagca gggccacttc tatggagaga 1680
ctgcagccgt ctatgtggca gttgaagaga ggaaggcagc ggggtcccgc gatgtcagcc 1740
tagcgaaggc agacgctgct cctgatgaga aggtgctaga ctctggtttt cgggagattg 1800
agaacaaagc cattcaggat cccaggcttt ttgcagagtg aattc 1845

<210> 13
<211> 4465
<212> DNA
<213> Artificial Sequence

<220>
<223> Description of Artificial Sequence:
      Expression-type plasmid pBMSP-1

<220>
<221> modified_base
<222> (2150)
<223> a, c, t or g

<220>
<221> modified_base
<222> (2214)..(2215)
<223> a, c, t or g

<400> 13
ctggccggcg ccagatctgg ggaacctgtg gttggcatgc acatacaaat ggacgaacgg 60
ataaaccttt tcacgccctt ttaaatatcc gattattcta ataaacgctc ttttctctta 120
ggtttacccg ccaatatatc ctgtcaaaca ctgatagttt aaactgaagg cgggaaacga 180
caatctgatc atgagcggag aattaaggga gtcacgttat gacccccgcc gatgacgcgg 240
gacaagccgt tttacgtttg gaactgacag aaccgcaacg attgaaggag ccactcagcc 300
gatctgaatt aattcccgat ctagtaacat agatgacacc gcgcgcgata atttatccta 360
gtttgcgcgc tatattttgt tttctatcgc gtattaaatg tataattgcg ggactctaat 420
cataaaaacc catctcataa ataacgtcat gcattacatg ttaattatta catgcttaac 480
gtaattcaac agaaattata tgataatcat cgcaagaccg gcaacaggat tcaatcttaa 540
gaaactttat tgccaaatgt ttgaacgatc ggggaaattc gagctccacc gcggtggcgg 600
ccgctctaga actagtggat cccccgggct gcaggaattc gatcagatct gatcaagctt 660
atcgataccg tcgacctcga gggggggccc ggtaccccta gagtcgattt ggtgtatcga 720
gattggttat gaaattcaga tgctagtgta atgtattggt aatttgggaa gatataatag 780
gaagcaaggc tatttatcca tttctgaaaa ggcgaaatgg cgtcaccgcg agcgtcacgc 840
gcattccgtt cttgctgtaa agcgttgttt ggtacacttt tgactagcga ggcttggcgt 900
gtcagcgtat ctattcaaaa gtcgttaatg gctgcggatc aagaaaaagt tggaatagaa 960
acagaatacc cgcgaaattc aggcccggtt gccatgtcct acacgccgaa ataaacgacc 1020
aaattagtag aaaaataaaa actgactcgg atacttacgt cacgtcttgc gcactgattt 1080
gaaaaatctc cctcgatcga gaaagagatc aatgttgagc tgcttcaaaa gcaatgggat 1140
tgaccagctc gcggatccta caggccaaat tcgctcttag ccgtacaata ttactcaccg 1200
gtgcgatgcc ccccatcgta ggtgaaggtg gaaattaatg atccatcttg agaccacagg 1260
cccacaacag ctaccagttt cctcaagggt ccaccaaaaa cgtaagcgct tacgtacatg 1320
gtcgataaga aaaggcaatt tgtagatgtt aacatccaac gtcgctttca gggatcctac 1380
aggccaaatt cgctcttagc cgtacaatat tactcaccgg tgcgatgccc cccatcgtag 1440
gtgaaggtgg aaattaatga tccatcttga gaccacaggc ccacaacagc taccagtttc 1500
ctcaagggtc caccaaaaac gtaagcgctt acgtacatgg tcgataagaa aaggcaattt 1560
gtagatgtta acatccaacg tcgctttcag ggatcctaca ggccaaattc gctcttagcc 1620
gtacaatatt actcaccggt gcgatgcccc ccatcgtagg tgaaggtgga aattaatgat 1680
ccatcttgag accacaggcc cacaacagct accagtttcc tcaagggtcc accaaaaacg 1740
taagcgctta cgtacatggt cgataagaaa aggcaatttg tagatgttaa catccaacgt 1800
cgctttcagg gatccgcgag cttatcgcga taccgtcgaa tataataatt atatttgtaa 1860
gaatattatt ataataatat aaaatatata tatataaatt ataatatatt aattattgtt 1920
aattattaat aatatatata ttaatcattt agatatataa ttctatagcc ttagactcct 1980
catcaataga agactacgta taaaaataat cagataacat ctaaaacatg tagataaata 2040
atagttgttt catatccaac atgatgtcca gagcttcacg ctgccgcaag cactcagggc 2100
gcaagggctg ctaaaggaag cggaacacgt agaaagccag tccgcagaan cggtgctgac 2160
cccggatgaa tgtcagctac tgggctatct ggacaaggga aaacgcaagc gcannagaga 2220
aagcaggtag cttgcagtgg gcttacatgg cgatagctag actgggcggt tttatggaca 2280
gcaagcgaac cggaattgcc agctggggcg ccctctggta aggttgggaa gccctgcaaa 2340

gtaaactgga tggctttctt gccgccaagg atctgatggc gcaggggatc aagatcatga 2400
gcggagaatt aagggagtca cgttatgacc cccgccgatg acgcgggaca agccgtttta 2460
cgtttggaac tgacagaacc gcaacgttga aggagccact cagccgcggg tttctggagt 2520
ttaatgagct aagcacatac gtcagaaacc attattgcgc gttcaaaagt cgcctaaggt 2580
cactatcagc tagcaaatat ttcttgtcaa aaatgctcca ctgacgttcc ataaattccc 2640
ctcggtatcc aattagagtc tcatattcac tctcaatcca gatctggatc gtttcgcatg 2700
attgaacaag atggattgca cgcaggttct ccggccgctt gggtggagag gctattcggc 2760
tatgactggg cacaacagac aatcggctgc tctgatgccg ccgtgttccg gctgtcagcg 2820
caggggcgcc cggttctttt tgtcaagacc gacctgtccg gtgccctgaa tgaactgcag 2880
gacgaggcag cgcggctatc gtggctggcc acgacgggcg ttccttgcgc agctgtgctc 2940
gacgttgtca ctgaagcggg aagggactgg ctgctattgg gcgaagtgcc ggggcaggat 3000
ctcctgtcat ctcaccttgc tcctgccgag aaagtatcca tcatggctga tgcaatgcgg 3060
cggctgcata cgcttgatcc ggctacctgc ccattcgacc accaagcgaa acatcgcatc 3120
gagcgagcac gtactcggat ggaagccggt cttgtcgatc aggatgatct ggacgaagag 3180
catcaggggc tcgcgccagc cgaactgttc gccaggctca aggcgcgcat gcccgacggc 3240
gatgatctcg tcgtgaccca tggcgatgcc tgcttgccga atatcatggt ggaaaatggc 3300
cgcttttctg gattcatcga ctgtggccgg ctgggtgtgg cggaccgcta tcaggacata 3360
gcgttggcta cccgtgatat tgctgaagag cttggcggcg aatgggctga ccgcttcctc 3420
gtgctttacg gtatcgccgc tcccgattcg cagcgcatcg ccttctatcg ccttcttgac 3480
gagttcttct gagcgggact ctgaggatcc cccgatgagc taagctagct atatcatcaa 3540
tttatgtatt acacataata tcgcactcag tctttcatct acggcaatgt accagctgat 3600
ataatcagtt attgaaatat ttctgaattt aaacttgcat caataaattt atgtttttgc 3660
ttggactata atacctgact tgttatttta tcaataaata tttaaactat atttctttca 3720
agatgggaat taattcactg gccgtcgttt tacaacgtcg tgactgggaa aaccctggcg 3780
ttacccaact taatcgcctt gcagcacatc cccctttcgc cagctggcgt aatagcgaag 3840
aggcccgcac cgatcgccct tcccaacagt tgcgcagcct gaatggcgcc cgctcctttc 3900
gctttcttcc cttcctttct cgccacgttc gccggctttc cccgtcaagc tctaaatcgg 3960
gggctccctt tagggttccg atttagtgct ttacggcacc tcgaccccaa aaaacttgat 4020
ttgggtgatg gttcacgtag tgggccatcg ccctgataga cggtttttcg ccctttgacg 4080
ttggagtcca cgttctttaa tagtggactc ttgttccaaa ctggaacaac actcaaccct 4140
atctcgggct attcttttga tttataaggg attttgccga tttcggaacc accatcaaac 4200
aggattttcg cctgctgggg caaaccagcg tggaccgctt gctgcaactc tctcagggcc 4260
aggcggtgaa gggcaatcag ctgttgcccg tctcactggt gaaaagaaaa accaccccag 4320
tacattaaaa acgtccgcaa tgtgttatta agttgtctaa gcgtcaattt gtttacacca 4380
caatatatcc tgccaccagc cagccaacag ctccccgacc ggcagctcgg cacaaaatca 4440
ccactcgata caggcagccc atcag 4465

<210> 14
<211> 8074
<212> DNA
<213> Artificial Sequence

<220>
<223> Description of Artificial Sequence:
      Expression-type plasmid pBMSP-1spJSC

<220>
<221> modified_base
<222> (2315)
<223> a, c, t or g

<400> 14
ctgatgggct gcctgtatcg agtggtgatt ttgtgccgag ctgccggtcg gggagctgtt 60
ggctggctgg tggcaggata tattgtggtg taaacaaatt gacgcttaga caacttaata 120
acacattgcg gacgttttta atgtactggg gtggtttttc ttttcaccag tgagacgggc 180
aacagctgat tgcccttcac cgcctggccc tgagagagtt gcagcaagcg gtccacgctg 240
gtttgcccca gcaggcgaaa atcctgtttg atggtggttc cgaaatcggc aaaatccctt 300
ataaatcaaa agaatagccc gagatagggt tgagtgttgt tccagtttgg aacaagagtc 360
cactattaaa gaacgtggac tccaacgtca aagggcgaaa aaccgtctat cagggcgatg 420
gcccactacg tgaaccatca cccaaatcaa gttttttggg gtcgaggtgc cgtaaagcac 480
taaatcggaa ccctaaaggg agcccccgat ttagagcttg acggggaaag ccggcgaacg 540
tggcgagaaa ggaagggaag aaagcgaaag gagcgggcgc cattcaggct gcgcaactgt 600
tgggaagggc gatcggtgcg ggcctcttcg ctattacgcc agctggcgaa agggggatgt 660
gctgcaaggc gattaagttg ggtaacgcca gggttttccc agtcacgacg ttgtaaaacg 720
acggccagtg aattaattcc catcttgaaa gaaatatagt ttaaatattt attgataaaa 780
taacaagtca ggtattatag tccaagcaaa aacataaatt tattgatgca agtttaaatt 840
cagaaatatt tcaataactg attatatcag ctggtacatt gccgtagatg aaagactgag 900
tgcgatatta tgtgtaatac ataaattgat gatatagcta gcttagctca tcgggggatc 960
ctcagagtcc cgctcagaag aactcgtcaa gaaggcgata gaaggcgatg cgctgcgaat 1020
cgggagcggc gataccgtaa agcacgagga agcggtcagc ccattcgccg ccaagctctt 1080
cagcaatatc acgggtagcc aacgctatgt cctgatagcg gtccgccaca cccagccggc 1140
cacagtcgat gaatccagaa aagcggccat tttccaccat gatattcggc aagcaggcat 1200
cgccatgggt cacgacgaga tcatcgccgt cgggcatgcg cgccttgagc ctggcgaaca 1260
gttcggctgg cgcgagcccc tgatgctctt cgtccagatc atcctgatcg acaagaccgg 1320
cttccatccg agtacgtgct cgctcgatgc gatgtttcgc ttggtggtcg aatgggcagg 1380
tagccggatc aagcgtatgc agccgccgca ttgcatcagc catgatggat actttctcgg 1440
caggagcaag gtgagatgac aggagatcct gccccggcac ttcgcccaat agcagccagt 1500
cccttcccgc ttcagtgaca acgtcgagca cagctgcgca aggaacgccc gtcgtggcca 1560
gccacgatag ccgcgctgcc tcgtcctgca gttcattcag ggcaccggac aggtcggtct 1620
tgacaaaaag aaccgggcgc ccctgcgctg acagccggaa cacggcggca tcagagcagc 1680
cgattgtctg ttgtgcccag tcatagccga atagcctctc cacccaagcg gccggagaac 1740
ctgcgtgcaa tccatcttgt tcaatcatgc gaaacgatcc agatctggat tgagagtgaa 1800
tatgagactc taattggata ccgaggggaa tttatggaac gtcagtggag catttttgac 1860
aagaaatatt tgctagctga tagtgacctt aggcgacttt tgaacgcgca ataatggttt 1920
ctgacgtatg tgcttagctc attaaactcc agaaacccgc ggctgagtgg ctccttcaac 1980

gttgcggttc tgtcagttcc aaacgtaaaa cggcttgtcc cgcgtcatcg gcgggggtca 2040
taacgtgact cccttaattc tccgctcatg atcttgatcc cctgcgccat cagatccttg 2100
gcggcaagaa agccatccag tttactttgc agggcttccc aaccttacca gagggcgccc 2160
cagctggcaa ttccggttcg cttgctgtcc ataaaaccgc ccagtctagc tatcgccatg 2220
taagcccact gcaagctacc tgctttctct ttgcgcttgc gttttccctt gtccagatag 2280
cccagtagct gacattcatc cggggtcagc accgnttctg cggactggct ttctacgtgt 2340
tccgcttcct ttagcagccc ttgcgccctg agtgcttgcg gcagcgtgaa gctctggaca 2400
tcatgttgga tatgaaacaa ctattattta tctacatgtt ttagatgtta tctgattatt 2460
tttatacgta gtcttctatt gatgaggagt ctaaggctat agaattatat atctaaatga 2520
ttaatatata tattattaat aattaacaat aattaatata ttataattta tatatatata 2580
ttttatatta ttataataat attcttacaa atataattat tatattcgac ggtatcgcga 2640
taagctcgcg gatccctgaa agcgacgttg gatgttaaca tctacaaatt gccttttctt 2700
atcgaccatg tacgtaagcg cttacgtttt tggtggaccc ttgaggaaac tggtagctgt 2760
tgtgggcctg tggtctcaag atggatcatt aatttccacc ttcacctacg atggggggca 2820
tcgcaccggt gagtaatatt gtacggctaa gagcgaattt ggcctgtagg atccctgaaa 2880
gcgacgttgg atgttaacat ctacaaattg ccttttctta tcgaccatgt acgtaagcgc 2940
ttacgttttt ggtggaccct tgaggaaact ggtagctgtt gtgggcctgt ggtctcaaga 3000
tggatcatta atttccacct tcacctacga tggggggcat cgcaccggtg agtaatattg 3060
tacggctaag agcgaatttg gcctgtagga tccctgaaag cgacgttgga tgttaacatc 3120
tacaaattgc cttttcttat cgaccatgta cgtaagcgct tacgtttttg gtggaccctt 3180
gaggaaactg gtagctgttg tgggcctgtg gtctcaagat ggatcattaa tttccacctt 3240
cacctacgat ggggggcatc gcaccggtga gtaatattgt acggctaaga gcgaatttgg 3300
cctgtaggat ccgcgagctg gtcaatccca ttgcttttga agcagctcaa cattgatctc 3360
tttctcgatc gagggagatt tttcaaatca gtgcgcaaga cgtgacgtaa gtatccgagt 3420
cagtttttat ttttctacta atttggtcgt ttatttcggc gtgtaggaca tggcaaccgg 3480
gcctgaattt cgcgggtatt ctgtttctat tccaactttt tcttgatccg cagccattaa 3540
cgacttttga atagatacgc tgacacgcca agcctcgcta gtcaaaagtg taccaaacaa 3600
cgctttacag caagaacgga atgcgcgtga cgctcgcggt gacgccattt cgccttttca 3660
gaaatggata aatagccttg cttcctatta tatcttccca aattaccaat acattacact 3720
agcatctgaa tttcataacc aatctcgata caccaaatcg actctagggg taccatggtg 3780
ctcttcgtgc tcacctgcct gctggcggtc ttcccagcca tctccacgaa gagtcccata 3840
tttggtcccg aggaggtgaa tagtgtggaa ggtaactcag tgtccatcac gtgctactac 3900
ccacccacct ctgtcaaccg gcacacccgg aagtactggt gccggcaggg agctagaggt 3960
ggctgcataa ccctcatctc ctcggagggc tacgtctcca gcaaatatgc aggcagggct 4020
aacctcacca acttcccgga gaacggcaca tttgtggtga acattgccca gctgagccag 4080
gatgactccg ggcgctacaa gtgtggcctg ggcatcaata gccgaggcct gtcctttgat 4140
gtcagcctgg aggtcagcca gggtcctggg ctcctaaatg acactaaagt ctacacagtg 4200
gacctgggca gaacggtgac catcaactgc cctttcaaga ctgagaatgc tcaaaagagg 4260
aagtccttgt acaagcagat aggcctgtac cctgtgctgg tcatcgactc cagtggttat 4320
gtgaatccca actatacagg aagaatacgc cttgatattc agggtactgg ccagttactg 4380
ttcagcgttg tcatcaacca actcaggctc agcgatgctg ggcagtatct ctgccaggct 4440
ggggatgatt ccaatagtaa taagaagaat gctgacctcc aagtgctaaa gcccgagccc 4500
gagctggttt atgaagacct gaggggctca gtgaccttcc actgtgccct gggccctgag 4560
gtggcaaacg tggccaaatt tctgtgccga cagagcagtg gggaaaactg tgacgtggtc 4620
gtcaacaccc tggggaagag ggccccagcc tttgagggca ggatcctgct caacccccag 4680
gacaaggatg gctcattcag tgtggtgatc acaggcctga ggaaggagga tgcagggcgc 4740
tacctgtgtg gagcccattc ggatggtcag ctgcaggaag gctcgcctat ccaggcctgg 4800
caactcttcg tcaatgagga gtccacgatt ccccgcagcc ccactgtggt gaagggggtg 4860
gcaggaagct ctgtggccgt gctctgcccc tacaaccgta aggaaagcaa aagcatcaag 4920
tactggtgtc tctgggaagg ggcccagaat ggccgctgcc ccctgctggt ggacagcgag 4980
gggtgggtta aggcccagta cgagggccgc ctctccctgc tggaggagcc aggcaacggc 5040
accttcactg tcatcctcaa ccagctcacc agccgggacg ccggcttcta ctggtgtctg 5100
accaacggcg atactctctg gaggaccacc gtggagatca agattatcga aggagaacca 5160
aacctcaagg ttcccgggaa tgtcacggct gtgctgggag agactctcaa ggtcccctgt 5220
cactttccat gcaaattctc ctcgtacgag aaatactggt gcaagtggaa taacacgggc 5280
tgccaggccc tgcccagcca agacgaaggc cccagcaagg ccttcgtgaa ctgtgacgag 5340
aacagccggc ttgtctccct gaccctgaac ctggtgacca gggctgatga gggctggtac 5400
tggtgtggag tgaagcaggg ccacttctat ggagagactg cagccgtcta tgtggcagtt 5460
gaagagagga aggcagcggg gtcccgcgat gtcagcctag cgaaggcaga cgctgctcct 5520
gatgagaagg tgctagactc tggttttcgg gagattgaga acaaagccat tcaggatccc 5580
aggctttttg cagagtgaat tcccgatcgt tcaaacattt ggcaataaag tttcttaaga 5640
ttgaatcctg ttgccggtct tgcgatgatt atcatataat ttctgttgaa ttacgttaag 5700
catgtaataa ttaacatgta atgcatgacg ttatttatga gatgggtttt tatgattaga 5760
gtcccgcaat tatacattta atacgcgata gaaaacaaaa tatagcgcgc aaactaggat 5820
aaattatcgc gcgcggtgtc atctatgtta ctagatcggg gatccgtcga cggtatcgat 5880
aaggatccct gaaagcgacg ttggatgtta acatctacaa attgcctttt cttatcgacc 5940
atgtacgtaa gcgcttacgt ttttggtgga cccttgagga aactggtagc tgttgtgggc 6000
ctgtggtctc aagatggatc attaatttcc accttcacct acgatggggg gcatcgcacc 6060
ggtgagtaat attgtacggc taagagcgaa tttggcctgt aggatccctg aaagcgacgt 6120
tggatgttaa catctacaaa ttgccttttc ttatcgacca tgtacgtaag cgcttacgtt 6180
tttggtggac ccttgaggaa actggtagct gttgtgggcc tgtggtctca agatggatca 6240
ttaatttcca ccttcaccta cgatgggggg catcgcaccg gtgagtaata ttgtacggct 6300
aagagcgaat ttggcctgta ggatccctga aagcgacgtt ggatgttaac atctacaaat 6360
tgccttttct tatcgaccat gtacgtaagc gcttacgttt ttggtggacc cttgaggaaa 6420
ctggtagctg ttgtgggcct gtggtctcaa gatggatcat taatttccac cttcacctac 6480
gatggggggc atcgcaccgg tgagtaatat tgtacggcta agagcgaatt tggcctgtag 6540
gatccgcgag ctggtcaatc ccattgcttt tgaagcagct caacattgat ctctttctcg 6600
agggagattt ttcaaatcag tgcgcaagac gtgacgtaag tatccgagtc agtttttatt 6660
tttctactaa tttggtcgtt tatttcggcg tgtaggacat ggcaaccggg cctgaatttc 6720
gcgggtattc tgtttctatt ccaacttttt cttgatccgc agccattaac gacttttgaa 6780
tagatacgct gacacgccaa gcctcgctag tcaaaagtgt accaaacaac gctttacagc 6840
aagaacggaa tgcgcgtgac gctcgcggtg acgccatttc gccttttcag aaatggataa 6900
atagccttgc ttcctattat atcttcccaa attaccaata cattacacta gcatctgaat 6960
ttcataacca atctcgatac accaaatcga ctctagagga tctaaccatg ggatctaaac 7020
cttttttgtc tcttctttca ttgtcattgc ttttgtttac atctactagt ttggcacaag 7080
aagatgaaag gattgttctt gttgacaaca aatgtaagtg tgcccggatt acttccagga 7140

tcatccgttc ttccgaagat cctaatgagg acattgtgga gagaaacatc cgaattattg 7200
ttcctctgaa caacagggag aatatctctg atcccacctc accattgaga accagatttg 7260
tgtaccattt gtctgacctc tgtaaaaaat gtgatcctac agaagtggag ctggataatc 7320
agatagttac tgctacccag agcaatatct gtgatgaaga cagtgctaca gagacctgct 7380
acacttatga cagaaacaag tgctacacag ctgtggtccc actcgtatat ggtggtgaga 7440
ccaaaatggt ggaaacagcc ttaaccccag atgcctgcta tcctgactga gctcgaattt 7500
ccccgatcgt tcaaacattt ggcaataaag tttcttaaga ttgaatcctg ttgccggtct 7560
tgcgatgatt atcatataat ttctgttgaa ttacgttaag catgtaataa ttaacatgta 7620
atgcatgacg ttatttatga gatgggtttt tatgattaga gtcccgcaat tatacattta 7680
atacgcgata gaaaacaaaa tatagcgcgc aaactaggat aaattatcgc gcgcggtgtc 7740
atctatgtta ctagatcggg aattaattca gatcggctga gtggctcctt caatcgttgc 7800
ggttctgtca gttccaaacg taaaacggct tgtcccgcgt catcggcggg ggtcataacg 7860
tgactccctt aattctccgc tcatgatcag attgtcgttt cccgccttca gtttaaacta 7920
tcagtgtttg acaggatata ttggcgggta aacctaagag aaaagagcgt ttattagaat 7980
aatcggatat ttaaaagggc gtgaaaaggt ttatccgttc gtccatttgt atgtgcatgc 8040
caaccacagg ttccccagat ctggcgccgg ccag 8074

<210> 15
<211> 1062
<212> DNA
<213> Homo sapiens

<400> 15
gcatccccga ccagccccaa ggtcttcccg ctgagcctct gcagcaccca gccagatggg 60
aacgtggtca tcgcctgcct ggtccagggc ttcttccccc aggagccact cagtgtgacc 120
tggagcgaaa gcggacaggg cgtgaccgcc agaaacttcc cacccagcca ggatgcctcc 180
ggggacctgt acaccacgag cagccagctg accctgccgg ccacacagtg cctagccggc 240
aagtccgtga catgccacgt gaagcactac acgaatccca gccaggatgt gactgtgccc 300
tgcccagttc cctcaactcc acctacccca tctccctcaa ctccacctac cccatctccc 360
tcatgctgcc acccccgact gtcactgcac cgaccggccc tcgaggacct gctcttaggt 420
tcagaagcga acctcacgtg cacactgacc ggcctgagag atgcctcagg tgtcaccttc 480
acctggacgc cctcaagtgg gaagagcgct gttcaaggac cacctgagcg tgacctctgt 540
ggctgctaca gcgtgtccag tgtcctgccg ggctgtgccg agccatggaa ccatgggaag 600
accttcactt gcactgctgc ctaccccgag tccaagaccc cgctaaccgc caccctctca 660
aaatccggaa acacattccg gcccgaggtc cacctgctgc cgccgccgtc ggaggagctg 720
gccctgaacg agctggtgac gctgacgtgc ctggcacgcg gcttcagccc caaggacgtg 780
ctggttcgct ggctgcaggg gtcacaggag ctgccccgcg agaagtacct gacttgggca 840
tcccggcagg agcccagcca gggcaccacc accttcgctg tgaccagcat actgcgcgtg 900
gcagccgagg actggaagaa gggggacacc ttctcctgca tggtgggcca cgaggccctg 960
ccgctggcct tcacacagaa gaccatcgac cgcttggcgg gtaaacccac ccatgtcaat 1020
gtgtctgttg tcatggcgga ggtggacggc acctgctact ga 1062

<210> 16
<211> 353
<212> PRT
<213> Homo sapiens

<400> 16
Ala Ser Pro Thr Ser Pro Lys Val Phe Pro Leu Ser Leu Cys Ser Thr
1               5                   10                  15

Gln Pro Asp Gly Asn Val Val Ile Ala Cys Leu Val Gln Gly Phe Phe
            20                  25                  30

Pro Gln Glu Pro Leu Ser Val Thr Trp Ser Glu Ser Gly Gln Gly Val
        35                  40                  45

Thr Ala Arg Asn Phe Pro Pro Ser Gln Asp Ala Ser Gly Asp Leu Tyr
    50                  55                  60

Thr Thr Ser Ser Gln Leu Thr Leu Pro Ala Thr Gln Cys Leu Ala Gly
65                  70                  75                  80

Lys Ser Val Thr Cys His Val Lys His Tyr Thr Asn Pro Ser Gln Asp
                85                  90                  95

Val Thr Val Pro Cys Pro Val Pro Ser Thr Pro Pro Thr Pro Ser Pro
            100                 105                 110

Ser Thr Pro Pro Thr Pro Ser Pro Ser Cys Cys His Pro Arg Leu Ser
        115                 120                 125

Leu His Arg Pro Ala Leu Glu Asp Leu Leu Leu Gly Ser Glu Ala Asn
    130                 135                 140

Leu Thr Cys Thr Leu Thr Gly Leu Arg Asp Ala Ser Gly Val Thr Phe
145                 150                 155                 160

Thr Trp Thr Pro Ser Ser Gly Lys Ser Ala Val Gln Gly Pro Pro Glu
                165                 170                 175

Arg Asp Leu Cys Gly Cys Tyr Ser Val Ser Ser Val Leu Pro Gly Cys
            180                 185                 190

Ala Glu Pro Trp Asn His Gly Lys Thr Phe Thr Cys Thr Ala Ala Tyr
        195                 200                 205

Pro Glu Ser Lys Thr Pro Leu Thr Ala Thr Leu Ser Lys Ser Gly Asn
    210                 215                 220

Thr Phe Arg Pro Glu Val His Leu Leu Pro Pro Pro Ser Glu Glu Leu
225                 230                 235                 240

Ala Leu Asn Glu Leu Val Thr Leu Thr Cys Leu Ala Arg Gly Phe Ser
                245                 250                 255

Pro Lys Asp Val Leu Val Arg Trp Leu Gln Gly Ser Gln Glu Leu Pro
            260                 265                 270

Arg Glu Lys Tyr Leu Thr Trp Ala Ser Arg Gln Glu Pro Ser Gln Gly
        275                 280                 285

Thr Thr Thr Phe Ala Val Thr Ser Ile Leu Arg Val Ala Ala Glu Asp
    290                 295                 300

Trp Lys Lys Gly Asp Thr Phe Ser Cys Met Val Gly His Glu Ala Leu
305                 310                 315                 320

Pro Leu Ala Phe Thr Gln Lys Thr Ile Asp Arg Leu Ala Gly Lys Pro
                325                 330                 335

Thr His Val Asn Val Ser Val Val Met Ala Glu Val Asp Gly Thr Cys
            340                 345                 350

Tyr


<210> 17
<211> 1023
<212> DNA
<213> Homo sapiens

<400> 17
gcatccccga ccagccccaa ggtcttcccg ctgagcctcg acagcacccc ccaagatggg 60
aacgtggtcg tcgcatgcct ggtccagggc ttcttccccc aggagccact cagtgtgacc 120
tggagcgaaa gcggacagaa cgtgaccgcc agaaacttcc cacctagcca ggatgcctcc 180
ggggacctgt acaccacgag cagccagctg accctgccgg ccacacagtg cccagacggc 240
aagtccgtga catgccacgt gaagcactac acgaatccca gccaggatgt gactgtgccc 300
tgcccagttc ccccacctcc cccatgctgc cacccccgac tgtcgctgca ccgaccggcc 360
ctcgaggacc tgctcttagg ttcagaagcg aacctcacgt gcacactgac cggcctgaga 420
gatgcctctg gtgccacctt cacctggacg ccctcaagtg ggaagagcgc tgttcaagga 480
ccacctgagc gtgacctctg tggctgctac agcgtgtcca gtgtcctgcc tggctgtgcc 540
cagccatgga accatgggga gaccttcacc tgcactgctg cccaccccga gttgaagacc 600
ccactaaccg ccaacatcac aaaatccgga aacacattcc ggcccgaggt ccacctgctg 660
ccgccgccgt cggaggagct ggccctgaac gagctggtga cgctgacgtg cctggcacgt 720
ggcttcagcc ccaaggatgt gctggttcgc tggctgcagg ggtcacagga gctgccccgc 780
gagaagtacc tgacttgggc atcccggcag gagcccagcc agggcaccac caccttcgct 840
gtgaccagca tactgcgcgt ggcagccgag gactggaaga agggggacac cttctcctgc 900
atggtgggcc acgaggccct gccgctggcc ttcacacaga agaccatcga ccgcttggcg 960
ggtaaaccca cccatgtcaa tgtgtctgtt gtcatggcgg aggtggacgg cacctgctac 1020
tga                                                               1023


<210> 18
<211> 340
<212> PRT
<213> Homo sapiens

<400> 18
Ala Ser Pro Thr Ser Pro Lys Val Phe Pro Leu Ser Leu Asp Ser Thr
1               5                   10                  15

Pro Gln Asp Gly Asn Val Val Val Ala Cys Leu Val Gln Gly Phe Phe
            20                  25                  30

Pro Gln Glu Pro Leu Ser Val Thr Trp Ser Glu Ser Gly Gln Asn Val
        35                  40                  45

Thr Ala Arg Asn Phe Pro Pro Ser Gln Asp Ala Ser Gly Asp Leu Tyr
    50                  55                  60

Thr Thr Ser Ser Gln Leu Thr Leu Pro Ala Thr Gln Cys Pro Asp Gly
65                  70                  75                  80

Lys Ser Val Thr Cys His Val Lys His Tyr Thr Asn Pro Ser Gln Asp
                85                  90                  95

Val Thr Val Pro Cys Pro Val Pro Pro Pro Pro Pro Cys Cys His Pro
            100                 105                 110

Arg Leu Ser Leu His Arg Pro Ala Leu Glu Asp Leu Leu Leu Gly Ser
        115                 120                 125

Glu Ala Asn Leu Thr Cys Thr Leu Thr Gly Leu Arg Asp Ala Ser Gly
    130                 135                 140

Ala Thr Phe Thr Trp Thr Pro Ser Ser Gly Lys Ser Ala Val Gln Gly
145                 150                 155                 160

Pro Pro Glu Arg Asp Leu Cys Gly Cys Tyr Ser Val Ser Ser Val Leu
                165                 170                 175

Pro Gly Cys Ala Gln Pro Trp Asn His Gly Glu Thr Phe Thr Cys Thr
            180                 185                 190

Ala Ala His Pro Glu Leu Lys Thr Pro Leu Thr Ala Asn Ile Thr Lys
        195                 200                 205

Ser Gly Asn Thr Phe Arg Pro Glu Val His Leu Leu Pro Pro Pro Ser
    210                 215                 220

Glu Glu Leu Ala Leu Asn Glu Leu Val Thr Leu Thr Cys Leu Ala Arg
225                 230                 235                 240

Gly Phe Ser Pro Lys Asp Val Leu Val Arg Trp Leu Gln Gly Ser Gln
                245                 250                 255

Glu Leu Pro Arg Glu Lys Tyr Leu Thr Trp Ala Ser Arg Gln Glu Pro
            260                 265                 270

Ser Gln Gly Thr Thr Thr Phe Ala Val Thr Ser Ile Leu Arg Val Ala
        275                 280                 285

Ala Glu Asp Trp Lys Lys Gly Asp Thr Phe Ser Cys Met Val Gly His
    290                 295                 300

Glu Ala Leu Pro Leu Ala Phe Thr Gln Lys Thr Ile Asp Arg Leu Ala
305                 310                 315                 320

Gly Lys Pro Thr His Val Asn Val Ser Val Val Met Ala Glu Val Asp
                325                 330                 335

Gly Thr Cys Tyr
            340


<210> 19
<211> 993
<212> DNA
<213> Homo sapiens

<400> 19
gcctccacca agggcccatc ggtcttcccc ctggcaccct cctccaagag cacctctggg 60
ggcacagcgg ccctgggctg cctggtcaag gactacttcc ccgaaccggt gacggtgtcg 120
tggaactcag gcgccctgac cagcggcgtg cacaccttcc cggctgtcct acagtcctca 180
ggactctact ccctcagcag cgtggtgacc gtgccctcca gcagcttggg cacccagacc 240
tacatctgca acgtgaatca caagcccagc aacaccaagg tggacaagaa agttgagccc 300
aaatcttgtg acaaaactca cacatgccca ccgtgcccag cacctgaact cctgggggga 360
ccgtcagtct tcctcttccc cccaaaaccc aaggacaccc tcatgatctc ccggacccct 420
gaggtcacat gcgtggtggt ggacgtgagc cacgaagacc ctgaggtcaa gttcaactgg 480
tacgtggacg gcgtggaggt gcataatgcc aagacaaagc cgcgggagga gcagtacaac 540
agcacgtacc gggtggtcag cgtcctcacc gtcctgcacc aggactggct gaatggcaag 600
gagtacaagt gcaaggtctc caacaaagcc ctcccagccc ccatcgagaa aaccatctcc 660
aaagccaaag ggcagccccg agaaccacag gtgtacaccc tgcccccatc ccgggatgag 720
ctgaccaaga accaggtcag cctgacctgc ctggtcaaag gcttctatcc cagcgacatc 780
gccgtggagt gggagagcaa tgggcagccg gagaacaact acaagaccac gcctcccgtg 840
ctggactccg acggctcctt cttcctctac agcaagctca ccgtggacaa gagcaggtgg 900
cagcagggga acgtcttctc atgctccgtg atgcatgagg ctctgcacaa ccactacacg 960
cagaagagcc tctccctgtc tccgggtaaa tga                              993


<210> 20
<211> 330
<212> PRT
<213> Homo sapiens

<400> 20


<210> 21
<211> 978
<212> DNA
<213> Homo sapiens

<400> 21
gcctccacca agggcccatc ggtcttcccc ctggcgccct gctccaggag cacctccgag 60
agcacagccg ccctgggctg cctggtcaag gactacttcc ccgaaccggt gacggtgtcg 120
tggaactcag gcgctctgac cagcggcgtg cacaccttcc cagctgtcct acagtcctca 180
ggactctact ccctcagcag cgtggtgacc gtgccctcca gcaacttcgg cacccagacc 240
tacacctgca acgtagatca caagcccagc aacaccaagg tggacaagac agttgagcgc 300
aaatgttgtg tcgagtgccc accgtgccca gcaccacctg tggcaggacc gtcagtcttc 360
ctcttccccc caaaacccaa ggacaccctc atgatctccc ggacccctga ggtcacgtgc 420
gtggtggtgg acgtgagcca cgaagacccc gaggtccagt tcaactggta cgtggacggc 480
gtggaggtgc ataatgccaa gacaaagcca cgggaggagc agttcaacag cacgttccgt 540
gtggtcagcg tcctcaccgt tgtgcaccag gactggctga acggcaagga gtacaagtgc 600
aaggtctcca acaaaggcct cccagccccc atcgagaaaa ccatctccaa aaccaaaggg 660
cagccccgag aaccacaggt gtacaccctg cccccatccc gggaggagat gaccaagaac 720
caggtcagcc tgacctgcct ggtcaaaggc ttctacccca gcgacatcgc cgtggagtgg 780
gagagcaatg ggcagccgga gaacaactac aagaccacac ctcccatgct ggactccgac 840
ggctccttct tcctctacag caagctcacc gtggacaaga gcaggtggca gcaggggaac 900
gtcttctcat gctccgtgat gcatgaggct ctgcacaacc actacacgca gaagagcctc 960
tccctgtctc cgggtaaa                                                978


<210> 22
<211> 326
<212> PRT
<213> Homo sapiens

<400> 22

Ala Ser Thr Lys Gly Pro Ser Val Phe Pro Leu Ala Pro Cys Ser Arg
1               5                   10                  15

Ser Thr Ser Glu Ser Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Asn Phe Gly Thr Gln Thr
65                  70                  75                  80

Tyr Thr Cys Asn Val Asp His Lys Pro Ser Asn Thr Lys Val Asp Lys
                85                  90                  95

Thr Val Glu Arg Lys Cys Cys Val Glu Cys Pro Pro Cys Pro Ala Pro
            100                 105                 110

Pro Val Ala Gly Pro Ser Val Phe Leu Phe Pro Pro Lys Pro Lys Asp
        115                 120                 125

Thr Leu Met Ile Ser Arg Thr Pro Glu Val Thr Cys Val Val Val Asp
    130                 135                 140

Val Ser His Glu Asp Pro Glu Val Gln Phe Asn Trp Tyr Val Asp Gly
145                 150                 155                 160

Val Glu Val His Asn Ala Lys Thr Lys Pro Arg Glu Glu Gln Phe Asn
                165                 170                 175

Ser Thr Phe Arg Val Val Ser Val Leu Thr Val Val His Gln Asp Trp
            180                 185                 190

Leu Asn Gly Lys Glu Tyr Lys Cys Lys Val Ser Asn Lys Gly Leu Pro
        195                 200                 205

Ala Pro Ile Glu Lys Thr Ile Ser Lys Thr Lys Gly Gln Pro Arg Glu
    210                 215                 220

Pro Gln Val Tyr Thr Leu Pro Pro Ser Arg Glu Glu Met Thr Lys Asn
225                 230                 235                 240

Gln Val Ser Leu Thr Cys Leu Val Lys Gly Phe Tyr Pro Ser Asp Ile
                245                 250                 255

Ala Val Glu Trp Glu Ser Asn Gly Gln Pro Glu Asn Asn Tyr Lys Thr
            260                 265                 270

Thr Pro Pro Met Leu Asp Ser Asp Gly Ser Phe Phe Leu Tyr Ser Lys
        275                 280                 285

Leu Thr Val Asp Lys Ser Arg Trp Gln Gln Gly Asn Val Phe Ser Cys
    290                 295                 300

Ser Val Met His Glu Ala Leu His Asn His Tyr Thr Gln Lys Ser Leu
305                 310                 315                 320

Ser Leu Ser Pro Gly Lys
                325


<210> 23
<211> 1134
<212> DNA
<213> Homo sapiens

<400> 23
gcttccacca agggcccatc ggtcttcccc ctggcgccct gctccaggag cacctctggg 60
ggcacagcgg ccctgggctg cctggtcaag gactacttcc ccgaaccggt gacggtgtcg 120
tggaactcag gcgccctgac cagcggcgtg cacaccttcc cggctgtcct acagtcctca 180
ggactctact ccctcagcag cgtggtgacc gtgccctcca gcagcttggg cacccagacc 240
tacacctgca acgtgaatca caagcccagc aacaccaagg tggacaagag agttgagctc 300
aaaaccccac ttggtgacac aactcacaca tgcccacggt gcccagagcc caaatcttgt 360
gacacacctc ccccgtgccc acggtgccca gagcccaaat cttgtgacac acctccccca 420
tgcccacggt gcccagagcc caaatcttgt gacacacctc ccccgtgccc aaggtgccca 480
gcacctgaac tcctgggagg accgtcagtc ttcctcttcc ccccaaaacc caaggatacc 540
cttatgattt cccggacccc tgaggtcacg tgcgtggtgg tggacgtgag ccacgaagac 600
cccgaggtcc agttcaagtg gtacgtggac ggcgtggagg tgcataatgc caagacaaag 660
ccgcgggagg agcagtacaa cagcacgttc cgtgtggtca gcgtcctcac cgtcctgcac 720
caggactggc tgaacggcaa ggagtacaag tgcaaggtct ccaacaaagc cctcccagcc 780
cccatcgaga aaaccatctc caaaaccaaa ggacagcccc gagaaccaca ggtgtacacc 840
ctgcccccat cccgggagga gatgaccaag aaccaggtca gcctgacctg cctggtcaaa 900
ggcttctacc ccagcgacat cgccgtggag tgggagagca gcgggcagcc ggagaacaac 960

tacaacacca cgcctcccat gctggactcc gacggctcct tcttcctcta cagcaagctc 1020
accgtggaca agagcaggtg gcagcagggg aacatcttct catgctccgt gatgcatgag 1080
gctctgcaca accgcttcac gcagaagagc ctctccctgt ctccgggtaa atga 1134

<210> 24
<211> 377
<212> PRT
<213> Homo sapiens

<400> 24
Ala Ser Thr Lys Gly Pro Ser Val Phe Pro Leu Ala Pro Cys Ser Arg
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Thr Cys Asn Val Asn His Lys Pro Ser Asn Thr Lys Val Asp Lys
                85                  90                  95

Arg Val Glu Leu Lys Thr Pro Leu Gly Asp Thr Thr His Thr Cys Pro
            100                 105                 110

Arg Cys Pro Glu Pro Lys Ser Cys Asp Thr Pro Pro Pro Cys Pro Arg
        115                 120                 125

Cys Pro Glu Pro Lys Ser Cys Asp Thr Pro Pro Pro Cys Pro Arg Cys
    130                 135                 140

Pro Glu Pro Lys Ser Cys Asp Thr Pro Pro Pro Cys Pro Arg Cys Pro
145                 150                 155                 160

Ala Pro Glu Leu Leu Gly Gly Pro Ser Val Phe Leu Phe Pro Pro Lys
                165                 170                 175

Pro Lys Asp Thr Leu Met Ile Ser Arg Thr Pro Glu Val Thr Cys Val
            180                 185                 190

Val Val Asp Val Ser His Glu Asp Pro Glu Val Gln Phe Lys Trp Tyr
        195                 200                 205

Val Asp Gly Val Glu Val His Asn Ala Lys Thr Lys Pro Arg Glu Glu
    210                 215                 220

Gln Tyr Asn Ser Thr Phe Arg Val Val Ser Val Leu Thr Val Leu His
225                 230                 235                 240

Gln Asp Trp Leu Asn Gly Lys Glu Tyr Lys Cys Lys Val Ser Asn Lys
                245                 250                 255

Ala Leu Pro Ala Pro Ile Glu Lys Thr Ile Ser Lys Thr Lys Gly Gln
            260                 265                 270

Pro Arg Glu Pro Gln Val Tyr Thr Leu Pro Pro Ser Arg Glu Glu Met
        275                 280                 285

Thr Lys Asn Gln Val Ser Leu Thr Cys Leu Val Lys Gly Phe Tyr Pro
    290                 295                 300

Ser Asp Ile Ala Val Glu Trp Glu Ser Ser Gly Gln Pro Glu Asn Asn
305                 310                 315                 320

Tyr Asn Thr Thr Pro Pro Met Leu Asp Ser Asp Gly Ser Phe Phe Leu
                325                 330                 335

Tyr Ser Lys Leu Thr Val Asp Lys Ser Arg Trp Gln Gln Gly Asn Ile
            340                 345                 350

Phe Ser Cys Ser Val Met His Glu Ala Leu His Asn Arg Phe Thr Gln
        355                 360                 365

Lys Ser Leu Ser Leu Ser Pro Gly Lys
    370                 375

<210> 25
<211> 984

<212> DNA
<213> Homo sapiens

<400> 25
gcttccacca agggcccatc cgtcttcccc ctggcgccct gctccaggag cacctccgag 60
agcacagccg ccctgggctg cctggtcaag gactacttcc ccgaaccggt gacggtgtcg 120
tggaactcag gcgccctgac cagcggcgtg cacaccttcc cggctgtcct acagtcctca 180
ggactctact ccctcagcag cgtggtgacc gtgccctcca gcagcttggg cacgaagacc 240
tacacctgca acgtagatca caagcccagc aacaccaagg tggacaagag agttgagtcc 300
aaatatggtc ccccatgccc atcatgccca gcacctgagt tcctgggggg accatcagtc 360
ttcctgttcc ccccaaaacc caaggacact ctcatgatct cccggacccc tgaggtcacg 420
tgcgtggtgg tggacgtgag ccaggaagac cccgaggtcc agttcaactg gtacgtggat 480
ggcgtggagg tgcataatgc caagacaaag ccgcgggagg agcagttcaa cagcacgtac 540
cgtgtggtca gcgtcctcac cgtcctgcac caggactggc tgaacggcaa ggagtacaag 600
tgcaaggtct ccaacaaagg cctcccgtcc tccatcgaga aaaccatctc caaagccaaa 660
gggcagcccc gagagccaca ggtgtacacc ctgcccccat cccaggagga gatgaccaag 720
aaccaggtca gcctgacctg cctggtcaaa ggcttctacc ccagcgacat cgccgtggag 780
tgggagagca atgggcagcc ggagaacaac tacaagacca cgcctcccgt gctggactcc 840
gacggctcct tcttcctcta cagcaggcta accgtggaca agagcaggtg gcaggagggg 900
aatgtcttct catgctccgt gatgcatgag gctctgcaca accactacac acagaagagc 960
ctctccctgt ctctgggtaa atga 984

<210> 26
<211> 327
<212> PRT
<213> Homo sapiens

<400> 26
Ala Ser Thr Lys Gly Pro Ser Val Phe Pro Leu Ala Pro Cys Ser Arg
1               5                   10                  15

Ser Thr Ser Glu Ser Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Lys Thr
65                  70                  75                  80

Tyr Thr Cys Asn Val Asp His Lys Pro Ser Asn Thr Lys Val Asp Lys
                85                  90                  95

Arg Val Glu Ser Lys Tyr Gly Pro Pro Cys Pro Ser Cys Pro Ala Pro
            100                 105                 110

Glu Phe Leu Gly Gly Pro Ser Val Phe Leu Phe Pro Pro Lys Pro Lys
        115                 120                 125

Asp Thr Leu Met Ile Ser Arg Thr Pro Glu Val Thr Cys Val Val Val
    130                 135                 140

Asp Val Ser Gln Glu Asp Pro Glu Val Gln Phe Asn Trp Tyr Val Asp
145                 150                 155                 160

Gly Val Glu Val His Asn Ala Lys Thr Lys Pro Arg Glu Glu Gln Phe
                165                 170                 175

Asn Ser Thr Tyr Arg Val Val Ser Val Leu Thr Val Leu His Gln Asp
            180                 185                 190

Trp Leu Asn Gly Lys Glu Tyr Lys Cys Lys Val Ser Asn Lys Gly Leu
        195                 200                 205

Pro Ser Ser Ile Glu Lys Thr Ile Ser Lys Ala Lys Gly Gln Pro Arg
    210                 215                 220

Glu Pro Gln Val Tyr Thr Leu Pro Pro Ser Gln Glu Glu Met Thr Lys
225                 230                 235                 240

Asn Gln Val Ser Leu Thr Cys Leu Val Lys Gly Phe Tyr Pro Ser Asp
                245                 250                 255

Ile Ala Val Glu Trp Glu Ser Asn Gly Gln Pro Glu Asn Asn Tyr Lys
            260                 265                 270

Thr Thr Pro Pro Val Leu Asp Ser Asp Gly Ser Phe Phe Leu Tyr Ser
        275                 280                 285

Arg Leu Thr Val Asp Lys Ser Arg Trp Gln Glu Gly Asn Val Phe Ser
    290                 295                 300

Cys Ser Val Met His Glu Ala Leu His Asn His Tyr Thr Gln Lys Ser
305                 310                 315                 320

Leu Ser Leu Ser Leu Gly Lys
                325


<210> 27
<211> 300
<212> DNA
<213> Homo sapiens

<400> 27
taggctgcct gtgcccccca cctgcctgtc cacaacccag cctctggtac atccatgccc 60
tctgccctaa gcctcacctg cacttttcct tggatttcag agtctccaaa ggcacaggcc 120
tcctccgtgc ccactgcaca accccaagca gagggcagcc tcgccaaggc aaccacagcc 180
ccagccacca cccgtaacac aggtgagaag ccccttccct gcacactcca cccccaccca 240
cctgctcatt cctcagccgc ctcctccagg cagcccttca taactccttg tctgagtctc 300


<210> 28
<211> 383
<212> PRT
<213> Homo sapiens

<400> 28
Ala Pro Thr Lys Ala Pro Asp Val Phe Pro Ile Ile Ser Gly Cys Arg
1               5                   10                  15

His Pro Lys Asp Asn Ser Pro Val Val Leu Ala Cys Leu Ile Thr Gly
            20                  25                  30

Tyr His Pro Thr Ser Val Thr Val Thr Trp Tyr Met Gly Thr Gln Ser
        35                  40                  45

Gln Pro Gln Arg Thr Phe Pro Glu Ile Gln Arg Arg Asp Ser Tyr Tyr
    50                  55                  60

Met Thr Ser Ser Gln Leu Ser Thr Pro Leu Gln Gln Trp Arg Gln Gly
65                  70                  75                  80

Glu Tyr Lys Cys Val Val Gln His Thr Ala Ser Lys Ser Lys Lys Glu
                85                  90                  95

Ile Phe Arg Trp Pro Glu Ser Pro Lys Ala Gln Ala Ser Ser Val Pro
            100                 105                 110

Thr Ala Gln Pro Gln Ala Glu Gly Ser Leu Ala Lys Ala Thr Thr Ala
        115                 120                 125

Pro Ala Thr Thr Arg Asn Thr Gly Arg Gly Gly Glu Glu Lys Lys Lys
    130                 135                 140

Glu Lys Glu Lys Glu Glu Gln Glu Glu Arg Glu Thr Lys Thr Pro Glu
145                 150                 155                 160

Cys Pro Ser His Thr Gln Pro Leu Gly Val Tyr Leu Leu Thr Pro Ala
                165                 170                 175

Val Gln Asp Leu Trp Leu Arg Asp Lys Ala Thr Phe Thr Cys Phe Val
            180                 185                 190

Val Gly Ser Asp Leu Lys Asp Ala His Leu Thr Trp Glu Val Ala Gly
        195                 200                 205

Lys Val Pro Thr Gly Gly Val Glu Glu Gly Leu Leu Glu Arg His Ser
    210                 215                 220

Asn Gly Ser Gln Ser Gln His Ser Arg Leu Thr Leu Pro Arg Ser Leu
225                 230                 235                 240

Trp Asn Ala Gly Thr Ser Val Thr Cys Thr Leu Asn His Pro Ser Leu
                245                 250                 255

Pro Pro Gln Arg Leu Met Ala Leu Arg Glu Pro Ala Ala Gln Ala Pro
            260                 265                 270

Val Lys Leu Ser Leu Asn Leu Leu Ala Ser Ser Asp Pro Pro Glu Ala
        275                 280                 285

Ala Ser Trp Leu Leu Cys Glu Val Ser Gly Phe Ser Pro Pro Asn Ile
    290                 295                 300

Leu Leu Met Trp Leu Glu Asp Gln Arg Glu Val Asn Thr Ser Gly Phe
305                 310                 315                 320

Ala Pro Ala Arg Pro Pro Pro Gln Pro Gly Ser Thr Thr Phe Trp Ala
                325                 330                 335

Trp Ser Val Leu Arg Val Pro Ala Pro Pro Ser Pro Gln Pro Ala Thr
            340                 345                 350

Tyr Thr Cys Val Val Ser His Glu Asp Ser Arg Thr Leu Leu Asn Ala
        355                 360                 365

Ser Arg Ser Leu Glu Val Ser Tyr Val Thr Asp His Gly Pro Met
    370                 375                 380


<210> 29
<211> 300
<212> DNA
<213> Homo sapiens

<400> 29
gtcattagct ggatttagcc attccacaat gtacacatat ttcaaacatt gtgttgtata 60
tgataaacat gtataatttt tgtcaattaa aaatttttag gaagaggagg agaagagaag 120
aagaaggaga aggagaaaga ggaacaagaa gagagagaga caaagacacc aggttttttc 180
tgacccctgg gctatcaaaa cacctattgc ccaataacta gttggccgtt ggtgccctaa 240
actattgaag cgattgctgt tatgtggatg ggccccggac acttagaaac tcgtgacccc 300


<210> 30
<211> 429
<212> PRT
<213> Homo sapiens

<400> 30
Pro Thr Lys Ala Pro Asp Val Phe Pro Ile Ile Ser Gly Cys Arg His
1               5                   10                  15

Pro Lys Asp Asn Ser Pro Val Val Leu Ala Cys Leu Ile Thr Gly Tyr
            20                  25                  30

His Pro Thr Ser Val Thr Val Thr Trp Tyr Met Gly Thr Gln Ser Gln
        35                  40                  45

Pro Gln Arg Thr Phe Pro Glu Ile Gln Arg Arg Asp Ser Tyr Tyr Met
    50                  55                  60

Thr Ser Ser Gln Leu Ser Thr Pro Leu Gln Gln Trp Arg Gln Gly Glu
65                  70                  75                  80

Tyr Lys Cys Val Val Gln His Thr Ala Ser Lys Ser Lys Lys Glu Ile
                85                  90                  95

Phe Arg Trp Pro Glu Ser Pro Lys Ala Gln Ala Ser Ser Val Pro Thr
            100                 105                 110

Ala Gln Pro Gln Ala Glu Gly Ser Leu Ala Lys Ala Thr Thr Ala Pro
        115                 120                 125

Ala Thr Thr Arg Asn Thr Gly Arg Gly Gly Glu Glu Lys Lys Lys Glu
    130                 135                 140

Lys Glu Lys Glu Glu Gln Glu Glu Arg Glu Thr Lys Thr Pro Glu Cys
145                 150                 155                 160

Pro Ser His Thr Gln Pro Leu Gly Val Tyr Leu Leu Thr Pro Ala Val
                165                 170                 175

Gln Asp Leu Trp Leu Arg Asp Lys Ala Thr Phe Thr Cys Phe Val Val
            180                 185                 190

Gly Ser Asp Leu Lys Asp Ala His Leu Thr Trp Glu Val Ala Gly Lys
        195                 200                 205

Val Pro Thr Gly Gly Val Glu Glu Gly Leu Leu Glu Arg His Ser Asn
    210                 215                 220

Gly Ser Gln Ser Gln His Ser Arg Leu Thr Leu Pro Arg Ser Leu Trp
225                 230                 235                 240

Asn Ala Gly Thr Ser Val Thr Cys Thr Leu Asn His Pro Ser Leu Pro
                245                 250                 255

Pro Gln Arg Leu Met Ala Leu Arg Glu Pro Ala Ala Gln Ala Pro Val
            260                 265                 270

Lys Leu Ser Leu Asn Leu Leu Ala Ser Ser Asp Pro Pro Glu Ala Ala
        275                 280                 285

Ser Trp Leu Leu Cys Glu Val Ser Gly Phe Ser Pro Pro Asn Ile Leu
    290                 295                 300

Leu Met Trp Leu Glu Asp Gln Arg Glu Val Asn Thr Ser Gly Phe Ala
305                 310                 315                 320

Pro Ala Arg Pro Pro Pro Gln Pro Arg Ser Thr Thr Phe Trp Ala Trp
                325                 330                 335

Ser Val Leu Arg Val Pro Ala Pro Pro Ser Pro Gln Pro Ala Thr Tyr
            340                 345                 350

Thr Cys Val Val Ser His Glu Asp Ser Arg Thr Leu Leu Asn Ala Ser
        355                 360                 365

Arg Ser Leu Glu Val Ser Tyr Leu Ala Met Thr Pro Leu Ile Pro Gln
    370                 375                 380

Ser Lys Asp Glu Asn Ser Asp Asp Tyr Thr Thr Phe Asp Asp Val Gly
385                 390                 395                 400

Ser Leu Trp Thr Thr Leu Ser Thr Phe Val Ala Leu Phe Ile Leu Thr
                405                 410                 415

Leu Leu Tyr Ser Gly Ile Val Thr Phe Ile Lys Val Lys
            420                 425


<210> 31
<211> 500
<212> DNA
<213> Homo sapiens

<400> 31
gaagctgggg agaggagagc acagtggtta agtcagtccc tgcagcccaa ctgctcccga 60
aggtccggcc acagctgctc tcgtttgctc tcccctgcag agtgtccgag ccacacccag 120
cctcttggcg tctacctgct aacccctgca gtgcaggacc tgtggctccg ggacaaagcc 180
accttcacct gcttcgtggt gggcagtgac ctgaaggatg ctcacctgac ctgggaggtg 240
gctgggaagg tccccacagg gggcgtggag gaagggctgc tggagcggca cagcaacggc 300
tcccagagcc agcacagccg tctgaccctg cccaggtcct tgtggaacgc ggggacctcc 360
gtcacctgca cactgaacca tcccagcctc ccaccccaga ggttgatggc gctgagagaa 420
cccggtgagc ctggctccca ggtggggaga cgagggtgcc cacagcctgc tgacccctac 480
gcccgcccca gggccatgac                                              500


<210> 32
<211> 383
<212> PRT
<213> Homo sapiens

<400> 32
Pro Thr Lys Ala Pro Asp Val Phe Pro Ile Ile Ser Gly Cys Arg His
  1               5                  10                  15

Pro Lys Asp Asn Ser Pro Val Val Leu Ala Cys Leu Ile Thr Gly Tyr
            20                  25                  30

His Pro Thr Ser Val Thr Val Thr Trp Tyr Met Gly Thr Gln Ser Gln
        35                  40                  45

Pro Gln Arg Thr Phe Pro Glu Ile Gln Arg Arg Asp Ser Tyr Tyr Met
    50                  55                  60

Thr Ser Ser Gln Leu Ser Thr Pro Leu Gln Gln Trp Arg Gln Gly Glu
 65                  70                  75                  80

Tyr Lys Cys Val Val Gln His Thr Ala Ser Lys Ser Lys Lys Glu Ile
                 85                  90                  95

Phe Arg Trp Pro Glu Ser Pro Lys Ala Gln Ala Ser Ser Val Pro Thr
            100                 105                 110

Ala Gln Pro Gln Ala Glu Gly Ser Leu Ala Lys Ala Thr Thr Ala Pro
        115                 120                 125

Ala Thr Thr Arg Asn Thr Gly Arg Gly Gly Glu Glu Lys Lys Lys Glu
    130                 135                 140

Lys Glu Lys Glu Glu Gln Glu Glu Arg Glu Thr Lys Thr Pro Glu Cys
145                 150                 155                 160

Pro Ser His Thr Gln Pro Leu Gly Val Tyr Leu Leu Thr Pro Ala Val
                165                 170                 175

Gln Asp Leu Trp Leu Arg Asp Lys Ala Thr Phe Thr Cys Phe Val Val
            180                 185                 190

Gly Ser Asp Leu Lys Asp Ala His Leu Thr Trp Glu Val Ala Gly Lys
        195                 200                 205

Val Pro Thr Gly Gly Val Glu Glu Gly Leu Leu Glu Arg His Ser Asn
    210                 215                 220

Gly Ser Gln Ser Gln His Ser Arg Leu Thr Leu Pro Arg Ser Leu Trp
225                 230                 235                 240

Asn Ala Gly Thr Ser Val Thr Cys Thr Leu Asn His Pro Ser Leu Pro
                245                 250                 255

Pro Gln Arg Leu Met Ala Leu Arg Glu Pro Ala Ala Gln Ala Pro Val
            260                 265                 270

Lys Leu Ser Leu Asn Leu Leu Ala Ser Ser Asp Pro Pro Glu Ala Ala
        275                 280                 285

Ser Trp Leu Leu Cys Glu Val Ser Gly Phe Ser Pro Pro Asn Ile Leu
    290                 295                 300

Leu Met Trp Leu Glu Asp Gln Arg Glu Val Asn Thr Ser Gly Phe Ala
305                 310                 315                 320

Pro Ala Arg Pro Pro Pro Gln Pro Arg Ser Thr Thr Phe Trp Ala Trp
                325                 330                 335

Ser Val Leu Arg Val Pro Ala Pro Pro Ser Pro Gln Pro Ala Thr Tyr
            340                 345                 350

Thr Cys Val Val Ser His Glu Asp Ser Arg Thr Leu Leu Asn Ala Ser
        355                 360                 365

Arg Ser Leu Glu Val Ser Tyr Val Thr Asp His Gly Pro Met Lys
    370                 375                 380


<210> 33
<211> 500
<212> DNA
<213> Homo sapiens

<400> 33
ccacaggaaa ggagaaggga ggcaccacac cctggccggc cccacttctc tcccagtgcc 60
cccgtggcca gagcctgaca gcccccccac ctccccgcag ctgcgcaggc acccgtcaag 120
ctttctctga acctgctggc ctcgtctgac cctcccgagg cggcctcgtg gctcctgtgt 180
gaggtgtctg gcttctcgcc ccccaacatc ctcctgatgt ggctggagga ccagcgtgag 240
gtgaacactt ctgggtttgc ccccgcacgc ccccctccac agcccaggag caccacgttc 300
tgggcctgga gtgtgctgcg tgtcccagcc ccgcccagcc ctcagccagc cacctacacg 360
tgtgtggtca gccacgagga ctcccggact ctgctcaacg ccagccggag cctagaagtc 420
agctgtgagt cacccccagg ccagggttgg gacggggact ctgagggggg ccataaggag 480
ctggaatcca tactaggcag                                              500


<210> 34

<400> 34
000

<210> 35
<211> 26
<212> PRT
<213> Homo sapiens

<400> 35
Pro Thr Lys Ala Pro Asp Val Phe Pro Ile Ile Ser Gly Cys Arg His
1               5                   10                  15

Pro Lys Asp Asn Ser Pro Val Val Leu Ala
            20                  25


<210> 36
<211> 100
<212> DNA
<213> Homo sapiens

<400> 36
gacacgccga ttttttgtta ttagatgtaa cagaccatgg ccccatgaaa tgatcccgga 60
ccagatccgt ccgcacccgc cactcagcag ctctggccga                        100


<210> 37

<400> 37

000

<210> 38
<211> 200
<212> DNA
<213> Homo sapiens

<400> 38
cgctcggccc ccgttcctcc ccagacctgg ccatgacccc cctgatccct cagagcaagg 60
atgagaacag cgatgactac acgacctttg atgatgtggg cagcctgtgg accaccctgt 120
ccacgtttgt ggccctcttc atcctcaccc tcctctacag cggcattgtc actttcatca 180
aggtcagggg agcggccagg 200

<210> 39

<400> 39
000

<210> 40
<211> 100
<212> DNA
<213> Homo sapiens

<400> 40
tcaggcttct agcccctgtc tgaccccagg ggctgtcttt caggtgaagt agccccagaa 60
gagcaggacg ccctgtacct gcagagaagg gaagcagcct 100

<210> 41

<400> 41
000

<210> 42
<211> 495
<212> DNA
<213> Homo sapiens

<400> 42
tttccctgcc tcccgtcacc ctgccgccag ggcctctgcc ctgccctgcc ccttgtcctc 60
aggtttccag cctcagactc ccactgtgtc tgtcttccag cacccaccaa ggctccggat 120
gtgttcccca tcatatcagg gtgcagacac ccaaaggata acagccctgt ggtcctggca 180
tgcttgataa ctgggtacca cccaacgtcc gtgactgtca cctggtacat ggggacacag 240
agccagcccc agagaacctt ccctgagata caaagacggg acagctacta catgacaagc 300
agccagctct ccacccccct ccagcagtgg cgccaaggcg agtacaaatg cgtggtccag 360
cacaccgcca gcaagagtaa gaaggagatc ttccgctggc caggtaggtc gcaccggaga 420
tcacccagaa gggcccccca ggacccccag caccttccac tcagggcctg accacaaaga 480
cagaagcaag ggctg 495

<210> 43

<400> 43
000

<210> 44
<211> 1920
<212> DNA
<213> Homo sapiens

<400> 44
ggatccctgc cacggggtcc ccagctcccc catccaggcc ccccaggctg atgggcgctg 60
gcctgaggct ggcactgact aggttctgtc ctcacagcct ccacacagag cccatccgtc 120
ttccccttga cccgctgctg caaaaacatt ccctccaatg ccacctccgt gactctgggc 180
tgcctggcca cgggctactt cccggagccg gtgatggtga cctgggacac aggctccctc 240
aacgggacaa ctatgacctt accagccacc accctcacgc tctctggtca ctatgccacc 300
atcagcttgc tgaccgtctc gggtgcgtgg gccaagcaga tgttcacctg ccgtgtggca 360
cacactccat cgtccacaga ctgggtcgac aacaaaacct tcagcggtaa gagagggcca 420
agctcagaga ccacagttcc caggagtgcc aggctgaggg ctggcagagt gggcaggggt 480
tgagggggtg ggtgggctca aacgtgggaa cacccagcat gcctggggac ccgggccagg 540
acgtgggggc aagaggaggg cacacagagc tcagagaggc caacaaccct catgaccacc 600
agctctcccc cagtctgctc cagggacttc accccgccca ccgtgaagat cttacagtcg 660
tcctgcgacg gcggcgggca cttccccccg accatccagc tcctgtgcct cgtctctggg 720
tacaccccag ggactatcaa catcacctgg ctggaggacg ggcaggtcat ggacgtggac 780
ttgtccaccg cctctaccac gcaggagggt gagctggcct ccacacaaag cgagctcacc 840
ctcagccaga agcactggct gtcagaccgc acctacacct gccaggtcac ctatcaaggt 900
cacacctttg aggacagcac caagaagtgt gcaggtacgt tcccacctgc cctggtggcc 960
gccacggagg ccagagaaga ggggcgggtg ggcctcacac agccctccgg tgtaccacag 1020
attccaaccc gagaggggtg agcgcctacc taagccggcc cagcccgttc gacctgttca 1080
tccgcaagtc gcccacgatc acctgtctgg tggtggacct ggcacccagc aaggggaccg 1140
tgaacctgac ctggtcccgg gccagtggga agcctgtgaa ccactccacc agaaaggagg 1200

agaagcagcg caatggcacg ttaaccgtca cgtccaccct gccggtgggc acccgagact 1260
ggatcgaggg ggagacctac cagtgcaggg tgacccaccc ccacctgccc agggccctca 1320
tgcggtccac gaccaagacc agcggtgagc catgggcagg ccggggtcgt gggggaaggg 1380
agggagcgag tgagcggggc ccgggctgac cccacgtctg gccacaggcc cgcgtgctgc 1440
cccggaagtc tatgcgtttg cgacgccgga gtggccgggg agccgggaca agcgcaccct 1500
cgcctgcctg atccagaact tcatgcctga ggacatctcg gtgcagtggc tgcacaacga 1560
ggtgcagctc ccggacgccc ggcacagcac gacgcagccc cgcaagacca agggctccgg 1620
cttcttcgtc ttcagccgcc tggaggtgac cagggccgaa tgggagcaga aagatgagtt 1680
catctgccgt gcagtccatg aggcagcgag cccctcacag accgtccagc gagcggtgtc 1740
tgtaaatccc ggtaaatgac gtactcctgc ctccctccct cccagggctc catccagctg 1800
tgcagtgggg aggactggcc agaccttctg tccactgttg caatgacccc aggaagctac 1860
ccccaataaa ctgtgcctgc tcagagcccc agtacaccca ttcttgggag cgggcagggc 1920

<210> 45
<211> 574
<212> PRT
<213> Homo sapiens

<400> 45


<210> 46
<211> 2213
<212> DNA
<213> Homo sapiens

<400> 46
gctctagaac tagtggatcc cccgggctgc aggaattctc taaagaagcc cctgggagca 60
cagctcatca ccatggactg gacctggagg ttcctctttg tggtggcagc agctacaggt 120
gtccagtccc aggtgcagct ggtgcagtct ggggctgagg tgaagaagcc tgggtcctcg 180
gtgaaggtct cctgcaaggc ttctggaggc accttcagca gctatgctat cagctgggtg 240
cgacaggccc ctggacaagg gcttgagtgg atgggaggga tcatccctat ctttggtaca 300
gcaaactacg cacagaagtt ccagggcaga gtcacgatta ccgcggacga atccacgagc 360
acagcctaca tggagctgag cagcctgaga tctgaggaca cggccgtgta ttactgtgcg 420
aaaaccggga tcctggggcc gtatagcagt ggctggtacc cgaactcgga ctactactac 480
tacggtatgg acgtctgggg ccaagggacc acggtcaccg tctcctcagg gagtgcatcc 540
gccccaaccc ttttccccct cgtctcctgt gagaattccc cgtcggatac gagcagcgtg 600
gccgttggct gcctcgcaca ggacttcctt cccgactcca tcactttctc ctggaaatac 660
aagaacaact ctgacatcag cagcacccgg ggcttcccat cagtcctgag agggggcaag 720
tacgcagcca cctcacaggt gctgctgcct tccaaggacg tcatgcaggg cacagacgaa 780
cacgtggtgt gcaaagtcca gcaccccaac ggcaacaaag aaaagaacgt gcctcttcca 840
gtgattgctg agctgcctcc caaagtgagc gtcttcgtcc caccccgcga cggcttcttc 900
ggcaaccccc gcagcaagtc caagctcatc tgccaggcca cgggtttcag tccccggcag 960
attcaggtgt cctggctgcg cgaggggaag caggtggggt ctggcgtcac cacggaccag 1020
gtgcaggctg aggccaaaga gtctgggccc acgacctaca aggtgaccag cacactgacc 1080
atcaaagaga gcgactggct cagccagagc atgttcacct gccgcgtgga tcacaggggc 1140
ctgaccttcc agcagaatgc gtcctccatg tgtgtccccg atcaagacac agccatccgg 1200
gtcttcgcca tccccccatc ctttgccagc atcttcctca ccaagtccac caagttgacc 1260
tgcctggtca cagacctgac cacctatgac agcgtgacca tctcctggac ccgccagaat 1320
ggcgaagctg tgaaaaccca caccaacatc tccgagagcc accccaatgc cactttcagc 1380
gccgtgggtg aggccagcat ctgcgaggat gactggaatt ccggggagag gttcacgtgc 1440
accgtgaccc acacagacct gccctcgcca ctgaagcaga ccatctcccg gcccaagggg 1500
gtggccctgc acaggcccga tgtctacttg ctgccaccag cccgggagca gctgaacctg 1560
cgggagtcgg ccaccatcac gtgcctggtg acgggcttct ctcccgcgga cgtcttcgtg 1620
cagtggatgc agagggggca gcccttgtcc ccggagaagt atgtgaccag cgccccaatg 1680
cctgagcccc aggccccagg ccggtacttc gcccacagca tcctgaccgt gtccgaagag 1740
gaatggaaca cgggggagac ctacacctgc gtggtggccc atgaggccct gcccaacagg 1800
gtcaccgaga ggaccgtgga caagtccacc gagggggagg tgagcgccga cgaggagggc 1860
tttgagaacc tgtgggccac cgcctccacc ttcatcgtcc tcttcctcct gagcctcttc 1920
tacagtacca ccgtcacctt gttcaaggtg aaatgatccc aacagaagaa catcggagac 1980
cagagagagg aactcaaagg ggcgctgcct ccgggtctgg ggtcctggcc tgcgtggcct 2040
gttggcacgt gtttctcttc ccgcccggcc tccagttgtg tgctctcaca caggcttcct 2100
tctcgaccgg caggggctgg ctggcttgca ggccacgagg tgggctctac cccacactgc 2160
tttgctgtgt atacgcttgt tgccctgaaa taaatatgca cattttatcc atg       2213

<210> 47
<211> 627
<212> PRT
<213> Homo sapiens

<400> 47


<210> 48
<211> 822
<212> PRT
<213> Artificial Sequence

<220>
<223> Description of Artificial Sequence: Protein
      encoded by plasmid pSSPICAMHuA2

<400> 48


<210> 49

<400> 49
000


<210> 50
<211> 159
<212> PRT
<213> Homo sapiens

<400> 50
Met Glu Asn His Leu Leu Phe Trp Gly Val Leu Ala Val Phe Ile Lys
  1               5                  10                  15

Ala Val His Val Lys Ala Gln Glu Asp Glu Arg Ile Val Leu Val Asp
             20                  25                  30

Asn Lys Cys Lys Cys Ala Arg Ile Thr Ser Arg Ile Ile Arg Ser Ser
         35                  40                  45

Glu Asp Pro Asn Glu Asp Ile Val Glu Arg Asn Ile Arg Ile Ile Val
     50                  55                  60

Pro Leu Asn Asn Arg Glu Asn Ile Ser Asp Pro Thr Ser Pro Leu Arg
 65                  70                  75                  80

Thr Arg Phe Val Tyr His Leu Ser Asp Leu Cys Lys Lys Cys Asp Pro
                 85                  90                  95

Thr Glu Val Glu Leu Asp Asn Gln Ile Val Thr Ala Thr Gln Ser Asn
            100                 105                 110

Ile Cys Asp Glu Asp Ser Ala Thr Glu Thr Cys Tyr Thr Tyr Asp Arg
        115                 120                 125

Asn Lys Cys Tyr Thr Ala Val Val Pro Leu Val Tyr Gly Gly Glu Thr
    130                 135                 140

Lys Met Val Glu Thr Ala Leu Thr Pro Asp Ala Cys Tyr Pro Asp
145                 150                 155


<210> 51
<211> 602
<212> PRT
<213> Homo sapiens

<400> 51
Met Val Leu Phe Val Leu Thr Cys Leu Leu Ala Val Phe Pro Ala Ile
1               5                   10                  15

Ser Thr Lys Ser Pro Ile Phe Gly Pro Glu Glu Val Asn Ser Val Glu
            20                  25                  30

Gly Asn Ser Val Ser Ile Thr Cys Tyr Tyr Pro Pro Thr Ser Val Asn
        35                  40                  45

Arg Thr Arg Lys Tyr Trp Cys Arg Gln Gly Ala Arg Gly Gly Cys Ile
    50                  55                  60

Thr Leu Ile Ser Ser Glu Gly Tyr Val Ser Ser Lys Tyr Ala Gly Arg
65                  70                  75                  80

Ala Asn Leu Thr Asn Phe Pro Glu Asn Gly Thr Phe Val Val Asn Ile
                85                  90                  95

Ala Gln Leu Ser Gln Asp Asp Ser Gly Arg Tyr Lys Cys Gly Leu Gly
            100                 105                 110

Ile Asn Ser Arg Gly Leu Ser Phe Asp Val Ser Leu Glu Val Ser Gln
        115                 120                 125

Gly Pro Gly Leu Leu Asn Asp Thr Lys Val Tyr Thr Val Asp Leu Gly
    130                 135                 140

Arg Thr Val Thr Ile Asn Cys Pro Phe Lys Thr Glu Asn Ala Gln Lys
145                 150                 155                 160

Arg Lys Ser Leu Tyr Lys Gln Ile Gly Leu Tyr Pro Val Leu Val Ile
                165                 170                 175

Asp Ser Ser Gly Tyr Val Asn Pro Asn Tyr Thr Gly Arg Ile Arg Leu
            180                 185                 190

Asp Ile Gln Gly Thr Gly Gln Leu Leu Phe Ser Val Val Ile Asn Gln
        195                 200                 205

Leu Arg Leu Ser Asp Ala Gly Gln Tyr Leu Cys Gln Ala Gly Asp Asp
    210                 215                 220

Ser Asn Ser Asn Lys Lys Asn Ala Asp Leu Gln Val Leu Lys Pro Glu
225                 230                 235                 240

Pro Glu Leu Val Tyr Glu Asp Leu Arg Gly Ser Val Thr Phe Cys Ala
                245                 250                 255

Leu Gly Pro Glu Val Ala Asn Val Ala Lys Phe Leu Cys Arg Gln Ser
            260                 265                 270

Ser Gly Glu Asn Cys Asp Val Val Val Asn Thr Leu Gly Lys Arg Ala
        275                 280                 285

Pro Ala Phe Glu Gly Arg Ile Leu Leu Asn Pro Gln Asp Lys Asp Gly
    290                 295                 300

Ser Phe Ser Val Val Ile Thr Gly Leu Arg Lys Glu Asp Ala Gly Arg
305                 310                 315                 320

Tyr Leu Cys Gly Ala Ser Asp Gly Gln Leu Gln Glu Gly Ser Pro Ile
                325                 330                 335

Gln Ala Trp Gln Leu Phe Val Asn Glu Glu Ser Thr Ile Pro Arg Ser
            340                 345                 350

Pro Thr Val Val Lys Gly Val Ala Gly Ser Ser Val Ala Val Leu Cys
        355                 360                 365

Pro Tyr Asn Arg Lys Glu Ser Lys Ser Ile Lys Tyr Trp Cys Leu Trp
    370                 375                 380

Glu Gly Ala Gln Asn Gly Arg Cys Pro Leu Leu Val Asp Ser Glu Gly
385                 390                 395                 400

Trp Val Lys Ala Gln Tyr Glu Gly Arg Leu Ser Leu Leu Glu Glu Pro
                405                 410                 415

Gly Asn Gly Thr Phe Thr Val Ile Leu Asn Gln Leu Thr Ser Arg Asp
            420                 425                 430

Ala Gly Phe Tyr Trp Cys Leu Thr Asn Gly Asp Thr Leu Trp Arg Thr
        435                 440                 445

Thr Val Glu Ile Lys Ile Ile Glu Gly Glu Pro Asn Leu Lys Val Pro

450                 455                 460

Gly Asn Val Thr Ala Val Leu Gly Glu Thr Leu Lys Val Pro Cys Phe
465                 470                 475                 480

Pro Cys Lys Phe Ser Ser Tyr Glu Lys Tyr Trp Cys Lys Trp Asn Asn
                485                 490                 495

Thr Gly Cys Gln Ala Leu Pro Ser Gln Asp Glu Gly Pro Ser Lys Ala
            500                 505                 510

Phe Val Asn Cys Asp Glu Asn Ser Arg Leu Val Ser Leu Thr Leu Asn
        515                 520                 525

Leu Val Thr Arg Ala Asp Glu Gly Trp Tyr Trp Cys Gly Val Lys Gln
    530                 535                 540

Gly Phe Tyr Gly Glu Thr Ala Ala Val Tyr Val Ala Val Glu Glu Arg
545                 550                 555                 560

Lys Ala Ala Gly Ser Arg Asp Val Ser Leu Ala Lys Ala Asp Ala Ala
                565                 570                 575

Pro Asp Glu Lys Val Leu Asp Ser Gly Phe Arg Glu Ile Glu Asn Lys
            580                 585                 590

Ala Ile Gln Asp Pro Arg Leu Phe Ala Glu
        595                 600

<210> 52
<211> 2533
<212> DNA
<213> Homo sapiens

<400> 52
ggtccaactg caggcctgtg gtgcaggagc tgtgtgacca tggggctgtc accaggcctc 60
tctgtgctgg gttcctccag tatagaggag aggcagtata gaggagaggg ccgcgtcctc 120
acagtgcatt ctgtgttcca gcatccccga ccagccccaa ggtcttcccg ctgagcctct 180
gcagcaccca gccagatggg aacgtggtca tcgcctgcct ggtccagggc ttcttccccc 240
aggagccact cagtgtgacc tggagcgaaa gcggacaggg cgtgaccgcc agaaacttcc 300
cacccagcca ggatgcctcc ggggacctgt acaccacgag cagccagctg accctgccgg 360
ccacacagtg cctagccggc aagtccgtga catgccacgt gaagcactac acgaatccca 420
gccaggatgt gactgtgccc tgcccaggtc agagggcagg ctggggagtg gggcggggcc 480
accccgtcgt gccctgacac tgcgcctgca cccgtgttcc ccacagggag ccgccccttc 540
actcacacca gagtggaccc cgggccgagc cccaggaggt ggtggtggac aggccaggag 600
gggcgaggcg ggggcatggg gaagtatgtg ctgaccagct caggccatct ctccactcca 660
gttccctcaa ctccacctac cccatctccc tcaactccac ctaccccatc tccctcatgc 720
tgccaccccc gactgtcact gcaccgaccg gccctcgagg acctgctctt aggttcagaa 780
gcgaacctca cgtgcacact gaccggcctg agagatgcct caggtgtcac cttcacctgg 840
acgccctcaa gtgggaagag cgctgttcaa ggaccacctg agcgtgacct ctgtggctgc 900
tacagcgtgt ccagtgtcct gccgggctgt gccgagccat ggaaccatgg gaagaccttc 960
acttgcactg ctgcctaccc cgagtccaag accccgctaa ccgccaccct ctcaaaatcc 1020
ggtgggtcca gaccctgctc ggggccctgc tcagtgctct ggtttgcaaa gcatattcct 1080
ggcctgcctc ctccctccca atcctgggct ccagtgctca tgccaagtac acagggaaac 1140
tgaggcaggc tgaggggcca ggacacagcc cggggtgccc accagagcag aggggctctc 1200
tcatcccctg cccagccccc tgacctggct ctctaccctc caggaaacac attccggccc 1260
gaggtccacc tgctgccgcc gccgtcggag gagctggccc tgaacgagct ggtgacgctg 1320
acgtgcctgg cacgcggctt cagccccaag gacgtgctgg ttcgctggct gcaggggtca 1380
caggagctgc cccgcgagaa gtacctgact tgggcatccc ggcaggagcc cagccagggc 1440
accaccacct tcgctgtgac cagcatactg cgcgtggcag ccgaggactg gaagaagggg 1500
gacaccttct cctgcatggt gggccacgag gccctgccgc tggccttcac acagaagacc 1560
atcgaccgct tggcgggtaa acccacccat gtcaatgtgt ctgttgtcat ggcggaggtg 1620
gacggcacct gctactgagc cgcccgcctg tccccacccc tgaataaact ccatgctccc 1680
ccaagcagcc ccacgcttcc atccggcgcc tgtctgtcca tcctcagggt ctcagcactt 1740
gggaaagggc cagggcatgg acagggaaga ataccccctg ccctgagcct cggggggccc 1800
ctggcacccc catgagactt tccaccctgg tgtgagtgtg agttgtgagt gtgagagtgt 1860
gtggtgcagg aggcctcgct ggtgtgagat cttaggtctg ccaaggcagg cacagcccag 1920
gatgggttct gagagacgca catgccccgg acagttctga gtgagcagtg gcatggccgt 1980
ttgtccctga gagagccgcc tctggctgta gctgggaggg aatagggagg gtaaaaggag 2040
caggctagcc aagaaaggcg caggtagtgg caggagcggc gagggagtga ggggctggac 2100
tccagggccc cactgggagg acaagctcca ggagggcccc accaccctag tgggtgggcc 2160
tcaggacgtc ccactgacgc atgcaggaag gggcacctcc ccttaaccac actgctctgt 2220
acggggcacg tgggcacagg tgcacactca cactcacata tatgcctgag ccctgcagga 2280
gcggaacgtt cacagcccag acccagttcc agaaaagcca ggggagtccc ctcccaagcc 2340
cccaagctca gcctgctccc ctaggcccct ctggcttccc tgtgtttcca ctgtgcacag 2400
atcaggcacc aactccacag acccctccca ggcagcccct gctccctgcc tggccaagtc 2460
tcccatccct tcctaagccc aactaggacc caaagcatag acagggaggg gccacgtggg 2520
gtggcatcag aag                                                    2533

<210> 53
<211> 2516
<212> DNA
<213> Homo sapiens

<400> 53
ggtccaaccg caggcccatg gtgcaggagc tgtgtaacct atggggctgt caccaggcct 60
ctctgtgctg ggttcctcca gtgtagagga gaggcaggta cagcctgtcc tcctggggac 120
atggcatgag ggccgcgtcc tcacagcgca ttctgtgttc cagcatcccc gaccagcccc 180
aaggtcttcc cgctgagcct cgacagcacc ccccaagatg ggaacgtggt cgtcgcatgc 240
ctggtccagg gcttcttccc ccaggagcca ctcagtgtga cctggagcga aagcggacag 300
aacgtgaccg ccagaaactt cccacctagc caggatgcct ccggggacct gtacaccacg 360
agcagccagc tgaccctgcc ggccacacag tgcccagacg gcaagtccgt gacatgccac 420
gtgaagcact acacgaatcc cagccaggat gtgactgtgc cctgcccagg tcagagggca 480
ggctggggag tggggcgggg ccaccccgtc ctgccctgac actgcgcctg cacccgtgtt 540
ccccacaggg agccgcccct tcactcacac cagagtggac cccgggccga gccccaggag 600
gtggtggtgg acaggccagg aggggcgagg cgggggcacg gggaagggcg ttctgaccag 660
ctcaggccat ctctccactc cagttccccc acctccccca tgctgccacc cccgactgtc 720
gctgcaccga ccggccctcg aggacctgct cttaggttca gaagcgaacc tcacgtgcac 780
actgaccggc ctgagagatg cctctggtgc caccttcacc tggacgccct caagtgggaa 840
gagcgctgtt caaggaccac ctgagcgtga cctctgtggc tgctacagcg tgtccagtgt 900
cctgcctggc tgtgcccagc catggaacca tggggagacc ttcacctgca ctgctgccca 960
ccccgagttg aagaccccac taaccgccaa catcacaaaa tccggtgggt ccagaccctg 1020
ctcggggccc tgctcagtgc tctggtttgc aaagcatatt cccggcctgc ctcctccctc 1080
ccaatcctgg gctccagtgc tcatgccaag tacacaggga aactgaggca ggctgagggg 1140
ccaggacaca gcccagggtg cccaccagag cagaggggct ctctcatccc ctgcccagcc 1200
ccctgacctg gctctctacc ctccaggaaa cacattccgg cccgaggtcc acctgctgcc 1260
gccgccgtcg gaggagctgg ccctgaacga gctggtgacg ctgacgtgcc tggcacgtgg 1320
cttcagcccc aaggatgtgc tggttcgctg gctgcagggg tcacaggagc tgccccgcga 1380
gaagtacctg acttgggcat cccggcagga gcccagccag ggcaccacca ccttcgctgt 1440
gaccagcata ctgcgcgtgg cagccgagga ctggaagaag ggggacacct tctcctgcat 1500
ggtgggccac gaggccctgc cgctggcctt cacacagaag accatcgacc gcttggcggg 1560
taaacccacc catgtcaatg tgtctgttgt catggcggag gtggacggca cctgctactg 1620
agccgcccgc ctgtccccac ccctgaataa actccatgct cccccaagca gccccacgct 1680
tccatccggc gcctgtctgt ccatcctcag ggtctcagca cttgggaaag ggccagggca 1740
tggacaggga agaatacccc ctgccctgag cctcgggggg cccctggcac ccccatgaga 1800
ctttccaccc tggtgtgagt gtgagttgtg agtgtgagag tgtgtggtgc aggaggcctc 1860
gctggtgtga gatcttaggt ctgccaaggc aggcacagcc caggatgggt tctgagagac 1920
gcacatgccc cggacagttc tgagtgagca gtggcatggc cgtttgtccc tgagagagcc 1980
gcctctggct gtagctggga gggaataggg agggtaaaag gagcaggcta gccaagaaag 2040
gcgcaggtag tggcaggagc ggcgagggag tgaggggctg gactccaggg ccccactggg 2100
aggacaagct ccaggagggc cccaccaccc tagtgggtgg gcctcaggac gtcccactga 2160
cgcatgcagg aaggggcacc tccccttaac cacactgctc tgtacggggc acgtgggcac 2220
acatgcacac tcacactcac atatacgcct gagccctgca ggagtggaac gttcacagcc 2280
cagacccagt tccagaaaag ccaggggagt cccctcccaa gcccccaagc tcagcctgct 2340
cccccaggcc cctctggctt ccctgtgttt ccactgtgca cagatcaggc accaactcca 2400
cagacccctc ccaggcagcc cctgctccct gcctggccaa gtctcccatc ccttcctaag 2460
cccaactagg acccaaagca tagacaggga ggggccgcgt ggggtggcat cagaag 2516

<210> 54
<211> 2009
<212> DNA
<213> Homo sapiens

<400> 54
agctttctgg ggcaggccag gcctgacctt ggctttgggg cagggagggg gctaaggtga 60
ggcaggtggc gccagcaggt gcacacccaa tgcccatgag cccagacact ggacgctgaa 120
cctcgcggac agttaagaac ccaggggcct ctgcgcctgg gcccagctct gtcccacacc 180
gcggtcacat ggcaccacct ctcttgcagc ctccaccaag ggcccatcgg tcttccccct 240
ggcaccctcc tccaagagca cctctggggg cacagcggcc ctgggctgcc tggtcaagga 300
ctacttcccc gaaccggtga cggtgtcgtg gaactcaggc gccctgacca gcggcgtgca 360
caccttcccg gctgtcctac agtcctcagg actctactcc ctcagcagcg tggtgaccgt 420
gccctccagc agcttgggca cccagaccta catctgcaac gtgaatcaca agcccagcaa 480
caccaaggtg gacaagaaag ttggtgagag gccagcacag ggagggaggg tgtctgctgg 540
aagcaggctc agcgctcctg cctggacgca tcccggctat gcagccccag tccagggcag 600
caaggcaggc cccgtctgcc tcttcacccg gagcctctgc ccgccccact catgctcagg 660
gagagggtct tctggctttt tcccaggctc tgggcaggca caggctaggt gcccctaacc 720
caggccctgc acacaaaggg gcaggtgctg ggctcagacc tgccaagagc catatccggg 780
aggaccctgc ccctgaccta agcccacccc aaaggccaaa ctctccactc cctcagctcg 840
gacaccttct ctcctcccag attccagtaa ctcccaatct tctctctgca gagcccaaat 900
cttgtgacaa aactcacaca tgcccaccgt gcccaggtaa gccagcccag gcctcgccct 960
ccagctcaag gcgggacagg tgccctagag tagcctgcat ccagggacag gccccagccg 1020
ggtgctgaca cgtccacctc catctcttcc tcagcacctg aactcctggg gggaccgtca 1080
gtcttcctct tccccccaaa acccaaggac accctcatga tctcccggac ccctgaggtc 1140
acatgcgtgg tggtggacgt gagccacgaa gaccctgagg tcaagttcaa ctggtacgtg 1200
gacggcgtgg aggtgcataa tgccaagaca aagccgcggg aggagcagta caacagcacg 1260
taccgggtgg tcagcgtcct caccgtcctg caccaggact ggctgaatgg caaggagtac 1320
aagtgcaagg tctccaacaa agccctccca gcccccatcg agaaaaccat ctccaaagcc 1380
aaaggtggga cccgtggggt gcgagggcca catggacaga ggccggctcg gcccaccctc 1440
tgccctgaga gtgaccgctg taccaacctc tgtcctacag ggcagccccg agaaccacag 1500
gtgtacaccc tgcccccatc ccgggatgag ctgaccaaga accaggtcag cctgacctgc 1560
ctggtcaaag gcttctatcc cagcgacatc gccgtggagt gggagagcaa tgggcagccg 1620
gagaacaact acaagaccac gcctcccgtg ctggactccg acggctcctt cttcctctac 1680
agcaagctca ccgtggacaa gagcaggtgg cagcagggga acgtcttctc atgctccgtg 1740
atgcatgagg ctctgcacaa ccactacacg cagaagagcc tctccctgtc tccgggtaaa 1800
tgagtgcgac ggccggcaag ccccgctccc cgggctctcg cggtcgcacg aggatgcttg 1860
gcacgtaccc cctgtacata cttcccgggc gcccagcatg gaaataaagc acccagcgct 1920
gccctgggcc cctgcgagac tgtgatggtt ctttccacgg gtcaggccga gtctgaggcc 1980
tgagtggcat gagggaggca gagcgggtc 2009

<210> 55
<211> 2009
<212> DNA
<213> Homo sapiens

<400> 55
agctttctgg ggcgagccgg gcctgacttt ggctttgggg cagggagtgg gctaaggtga 60
ggcaggtggc gccagccagg tgcacaccca atgcccgtga gcccagacac tggaccctgc 120
ctggaccctc gtggatagac aagaaccgag gggcctctgc gcctgggccc agctctgtcc 180
cacaccgcgg tcacatggca ccacctctct tgcagcctcc accaagggcc catcggtctt 240
ccccctggcg ccctgctcca ggagcacctc cgagagcaca gccgccctgg gctgcctggt 300
caaggactac ttccccgaac cggtgacggt gtcgtggaac tcaggcgctc tgaccagcgg 360
cgtgcacacc ttcccagctg tcctacagtc ctcaggactc tactccctca gcagcgtggt 420
gaccgtgccc tccagcaact tcggcaccca gacctacacc tgcaacgtag atcacaagcc 480
cagcaacacc aaggtggaca agacagttgg tgagaggcca gctcagggag ggagggtgtc 540
tgctggaagc caggctcagc cctcctgcct ggacgcaccc cggctgtgca gccccagccc 600
agggcagcaa ggcaggcccc atctgtctcc tcacccggag gcctctgccc gccccactca 660
tgctcaggga gagggtcttc tggctttttc caccaggctc caggcaggca caggctgggt 720
gcccctaccc caggcccttc acacacaggg gcaggtgctt ggctcagacc tgccaaaagc 780
catatccggg aggaccctgc ccctgaccta agccgacccc aaaggccaaa ctgtccactc 840
cctcagctcg gacaccttct ctcctcccag atccgagtaa ctcccaatct tctctctgca 900
gagcgcaaat gttgtgtcga gtgcccaccg tgcccaggta agccagccca ggcctcgccc 960
tccagctcaa ggcgggacag gtgccctaga gtagcctgca tccagggaca ggccccagct 1020
gggtgctgac acgtccacct ccatctcttc ctcagcacca cctgtggcag gaccgtcagt 1080
cttcctcttc cccccaaaac ccaaggacac cctcatgatc tcccggaccc ctgaggtcac 1140
gtgcgtggtg gtggacgtga gccacgaaga ccccgaggtc cagttcaact ggtacgtgga 1200
cggcgtggag gtgcataatg ccaagacaaa gccacgggag gagcagttca acagcacgtt 1260
ccgtgtggtc agcgtcctca ccgttgtgca ccaggactgg ctgaacggca aggagtacaa 1320
gtgcaaggtc tccaacaaag gcctcccagc ccccatcgag aaaaccatct ccaaaaccaa 1380
aggtgggacc cgcggggtat gagggccaca tggacagagg ccggctcggc ccaccctctg 1440
ccctgggagt gaccgctgtg ccaacctctg tccctacagg gcagccccga gaaccacagg 1500
tgtacaccct gcccccatcc cgggaggaga tgaccaagaa ccaggtcagc ctgacctgcc 1560
tggtcaaagg cttctacccc agcgacatcg ccgtggagtg ggagagcaat gggcagccgg 1620
agaacaacta caagaccaca cctcccatgc tggactccga cggctccttc ttcctctaca 1680
gcaagctcac cgtggacaag agcaggtggc agcaggggaa cgtcttctca tgctccgtga 1740
tgcatgaggc tctgcacaac cactacacgc agaagagcct ctccctgtct ccgggtaaat 1800
gagtgccacg gccggcaagc ccccgctccc caggctctcg gggtcgcgtg aggatgcttg 1860
gcacgtaccc cgtgtacata cttcccaggc acccagcatg gaaataaagc acccagcgct 1920
gccctgggcc cctgcgagac tgtgatggtt ctttccgtgg gtcaggccga gtctgaggcc 1980
tgagtggcat gagggaggca gagtgggtc 2009

<210> 56
<211> 2590
<212> DNA
<213> Homo sapiens

<400> 56
agctttctgg ggcaggccag gcctgacttt ggctgggggc agggaggggg ctaaggtgac 60
gcaggtggcg ccagccaggc gcacacccaa tgcccgtgag cccagacact ggaccctgcc 120
tggaccctcg tggatagaca agaaccgagg ggcctctgcg ccctgggccc agctctgtcc 180
cacaccgcag tcacatggcg ccatctctct tgcagcttcc accaagggcc catcggtctt 240
ccccctggcg ccctgctcca ggagcacctc tgggggcaca gcggccctgg gctgcctggt 300
caaggactac ttccccgaac cggtgacggt gtcgtggaac tcaggcgccc tgaccagcgg 360
cgtgcacacc ttcccggctg tcctacagtc ctcaggactc tactccctca gcagcgtggt 420
gaccgtgccc tccagcagct tgggcaccca gacctacacc tgcaacgtga atcacaagcc 480
cagcaacacc aaggtggaca agagagttgg tgagaggcca gcgcagggag ggagggtgtc 540
tgctggaagc caggctcagc cctcctgcct ggacgcatcc cggctgtgca gtcccagccc 600
agggcagcaa ggcaggcccc gtctgactcc tcacccggag cctctgcccg ccccactcat 660
gctcagggag agggtcttct ggctttttcc accaggctcc gggcaggcac aggctggatg 720
cccctacccc aggcccttca cacacagggg caggtgctgc gctcagagct gccaaaagcc 780
atatccagga ggaccctgcc cctgacctaa gcccacccca aaggccaaac tctctactca 840
ctcagctcag acaccttctc tcttcccaga tctgagtaac tcccaatctt ctctctgcag 900
agctcaaaac cccacttggt gacacaactc acacatgccc acggtgccca ggtaagccag 960
cccaggactc gccctccagc tcaaggcggg acaagagccc tagagtggcc tgagtccagg 1020
gacaggcccc agcagggtgc tgacgcatcc acctccatcc cagatccccg taactcccaa 1080
tcttctctct gcagagccca aatcttgtga cacacctccc ccgtgcccac ggtgcccagg 1140
taagccagcc caggcctcac cctccagctc aaggcaggac aagagcccta gagtggcctg 1200
agtccaggga caggccccag cagggtgctg acgcgtccac ctccatccca gatccccgta 1260
actcccaatc ttctctctgc agagcccaaa tcttgtgaca cacctccccc atgcccacgg 1320
tgcccaggta agccagccca ggcctcgccc tccagctcaa ggcgggacaa gagccctaga 1380
gtggcctgag tccagggaca ggccccagca gggtgctgac gcatccacct ccatcccaga 1440
tccccgtaac tcccaatctt ctctctgcag agcccaaatc ttgtgacaca cctcccccgt 1500
gcccaaggtg cccaggtaag ccagcccagg cctcgccctc cagctcaagg caggacaggt 1560
gccctagagt ggcctgcatc cagggacagg tcccagtcgg gtgctgacac atctgcctcc 1620
atctcttcct cagcacctga actcctggga ggaccgtcag tcttcctctt ccccccaaaa 1680
cccaaggata cccttatgat ttcccggacc cctgaggtca cgtgcgtggt ggtggacgtg 1740
agccacgaag accccgaggt ccagttcaag tggtacgtgg acggcgtgga ggtgcataat 1800
gccaagacaa agccgcggga ggagcagtac aacagcacgt tccgtgtggt cagcgtcctc 1860
accgtcctgc accaggactg gctgaacggc aaggagtaca agtgcaaggt ctccaacaaa 1920
gccctcccag cccccatcga gaaaaccatc tccaaaacca aaggtgggac ccgcggggta 1980
tgagggccac atggacagag gccagcttga cccaccctct gccctgggag tgaccgctgt 2040
gccaacctct gtccctacag gacagccccg agaaccacag gtgtacaccc tgcccccatc 2100
ccgggaggag atgaccaaga accaggtcag cctgacctgc ctggtcaaag gcttctaccc 2160
cagcgacatc gccgtggagt gggagagcag cgggcagccg gagaacaact acaacaccac 2220

gcctcccatg ctggactccg acggctcctt cttcctctac agcaagctca ccgtggacaa 2280
gagcaggtgg cagcagggga acatcttctc atgctccgtg atgcatgagg ctctgcacaa 2340
ccgcttcacg cagaagagcc tctccctgtc tccgggtaaa tgagtgcgac agccggcaag 2400
cccccgctcc ccgggctctc ggggtcgcgc gaggatgctt ggcacgtacc ccgtgtacat 2460
acttcccggg cacccagcat ggaaataaag cacccagcgc tgccctgggc ccctgtgaga 2520
ctgtgatggt tctttccacg ggtcaggccg agtctgaggc ctgagtgaca tgagggaggc 2580
agagcgggtc 2590

<210> 57
<211> 2028
<212> DNA
<213> Homo sapiens

<400> 57
agctttctgg ggcaggccgg gcctgacttt ggctgggggc agggaggggg ctaaggtgac 60
gcaggtggcg ccagccaggt gcacacccaa tgcccatgag cccagacact ggaccctgca 120
tggaccatcg cggatagaca agaaccgagg ggcctctgcg ccctgggccc agctctgtcc 180
cacaccgcgg tcacatggca ccacctctct tgcagcttcc accaagggcc catccgtctt 240
ccccctggcg ccctgctcca ggagcacctc cgagagcaca gccgccctgg gctgcctggt 300
caaggactac ttccccgaac cggtgacggt gtcgtggaac tcaggcgccc tgaccagcgg 360
cgtgcacacc ttcccggctg tcctacagtc ctcaggactc tactccctca gcagcgtggt 420
gaccgtgccc tccagcagct tgggcacgaa gacctacacc tgcaacgtag atcacaagcc 480
cagcaacacc aaggtggaca agagagttgg tgagaggcca gcacagggag ggagggtgtc 540
tgctggaagc caggctcagc cctcctgcct ggacgcaccc cggctgtgca gccccagccc 600
agggcagcaa ggcatgcccc atctgtctcc tcacccggag gcctctgacc accccactca 660
tgctcaggga gagggtcttc tggatttttc caccaggctc ccggcaccac aggctggatg 720
cccctacccc aggccctgcg catacagggc aggtgctgcg ctcagacctg ccaagagcca 780
tatccgggag gaccctgccc ctgacctaag cccaccccaa aggccaaact ctccactccc 840
tcagctcaga caccttctct cctcccagat ctgagtaact cccaatcttc tctctgcaga 900
gtccaaatat ggtcccccat gcccatcatg cccaggtaag ccaacccagg cctcgccctc 960
cagctcaagg cgggacaggt gccctagagt agcctgcatc cagggacagg ccccagccgg 1020
gtgctgacgc atccacctcc atctcttcct cagcacctga gttcctgggg ggaccatcag 1080
tcttcctgtt ccccccaaaa cccaaggaca ctctcatgat ctcccggacc cctgaggtca 1140
cgtgcgtggt ggtggacgtg agccaggaag accccgaggt ccagttcaac tggtacgtgg 1200
atggcgtgga ggtgcataat gccaagacaa agccgcggga ggagcagttc aacagcacgt 1260
accgtgtggt cagcgtcctc accgtcctgc accaggactg gctgaacggc aaggagtaca 1320
agtgcaaggt ctccaacaaa ggcctcccgt cctccatcga gaaaaccatc tccaaagcca 1380
aaggtgggac ccacggggtg cgagggccac acggacagag gccagctcgg cccaccctct 1440
gccctgggag tgaccgctgt gccaacctct gtccctacag ggcagccccg agagccacag 1500
gtgtacaccc tgcccccatc ccaggaggag atgaccaaga accaggtcag cctgacctgc 1560
ctggtcaaag gcttctaccc cagcgacatc gccgtggagt gggagagcaa tgggcagccg 1620
gagaacaact acaagaccac gcctcccgtg ctggactccg acggctcctt cttcctctac 1680
agcaggctaa ccgtggacaa gagcaggtgg caggagggga atgtcttctc atgctccgtg 1740
atgcatgagg ctctgcacaa ccactacaca cagaagagcc tctccctgtc tctgggtaaa 1800
tgagtgccag ggccggcaag cccccgctcc ccgggctctc ggggtcgcgc gaggatgctt 1860
ggcacgtacc ccgtctacat acttcccagg cacccagcat ggaaataaag cacccaccac 1920
tgccctgggc ccctgtgaga ctgtgatggt tctttccacg ggtcaggccg agtctgaggc 1980
ctgagtgaca tgagggaggc agagcgggtc ccactgtccc cacactgg 2028

<210> 58
<211> 106
<212> DNA
<213> Homo sapiens

<400> 58
tgccacccca ggactctgtc ttccagcacc caccaaggct ccggatgtgt tccccatcat 60
atcagggtgc agacacccaa aggataacag ccctgtggtc ctggca 106

<210> 59
<211> 1725
<212> DNA
<213> Homo sapiens

<400> 59
atggactgga cctggatcct cttcttggtg gcagcagcca cgcgagtcca ctcccagacg 60
cagttggtgc agtctggggc tgaggtgagg aagcctgggg catcagtgag ggtctcctgc 120
aaggcttctg gatacacctt catcgactcc tatatccact ggatacgaca ggcccctggg 180
cacgggcttg agtgggtggg atggatcaac cctaacagtg gtggcacaaa ctatgctccg 240
agatttcagg gcagggtcac catgaccaga gacgcgtcct tcagtacagc ctacatggac 300
ctgagaagtc tgagatctga cgactcggcc gtgttttact gtgcgaaaag tgaccctttt 360
tggagtgatt attataactt tgactactcg tacactttgg acgtctgggg ccaagggacc 420
acggtcaccg tctcctcagc ctccacacag agcccatccg tcttccccct gacccgctgc 480
tgcaaaaaca ttccctccaa tgccacctcc gtgactctgg gctgcctggc cacgggctac 540
ttcccggagc cggtgatggt gacctgggac acaggctccc tcaacgggac aactatgacc 600
ttaccagcca ccaccctcac gctctctggt cactatgcca ccatcagctt gctgaccgtc 660
tcgggtgcgt gggccaagca gatgttcacc tgccgtgtgg cacacactcc atcgtccaca 720
gactgggtcg acaacaaaac cttcagcgtc tgctccaggg acttcacccc gcccaccgtg 780
aagatcttac agtcgtcctg cgacggcggc gggcacttcc ccccgaccat ccagctcctg 840
tgcctcgtct ctgggtacac cccagggact atcaacatca cctggctgga ggacgggcag 900
gtcatggacg tggacttgtc caccgcctct accacgcagg agggtgagct ggcctccaca 960
caaagcgagc tcaccctcag ccagaagcac tggctgtcag accgcaccta cacctgccag 1020
gtcacctatc aaggtcacac ctttgaggac agcaccaaga agtgtgcaga ttccaacccg 1080
agaggggtga gcgcctacct aagccggccc agcccgttcg acctgttcat ccgcaagtcg 1140

cccacgatca cctgtctggt ggtggacctg gcacccagca aggggaccgt gaacctgacc 1200
tggtcccggg ccagtgggaa gcctgtgaac cactccacca gaaaggagga gaagcagcgc 1260
aatggcacgt taaccgtcac gtccaccctg ccggtgggca cccgagactg gatcgagggg 1320
gagacctacc agtgcagggt gacccacccc cacctgccca gggccctcat gcggtccacg 1380
accaagacca gcggcccgcg tgctgccccg gaagtctatg cgtttgcgac gccggagtgg 1440
ccggggagcc gggacaagcg caccctcgcc tgcctgatcc agaacttcat gcctgaggac 1500
atctcggtgc agtggctgca caacgaggtg cagctcccgg acgcccggca cagcacgacg 1560
cagccccgca agaccaaggg ctccggcttc ttcgtcttca gccgcctgga ggtgaccagg 1620
gccgaatggg agcagaaaga tgagttcatc tgccgtgcag tccatgaggc agcgagcccc 1680
tcacagaccg tccagcgagc ggtgtctgta aatcccggta aatga 1725

<210> 60
<211> 428
<212> PRT
<213> Homo sapiens

<400> 60
Ala Ser Thr Gln Ser Pro Ser Val Phe Pro Leu Thr Arg Cys Cys Lys
1               5                   10                  15

Asn Ile Pro Ser Asn Ala Thr Ser Val Thr Leu Gly Cys Leu Ala Thr
            20                  25                  30

Gly Tyr Phe Pro Glu Pro Val Met Val Thr Trp Asp Thr Gly Ser Leu
        35                  40                  45

Asn Gly Thr Thr Met Thr Leu Pro Ala Thr Thr Leu Thr Leu Ser Gly
    50                  55                  60

His Tyr Ala Thr Ile Ser Leu Leu Thr Val Ser Gly Ala Trp Ala Lys
65                  70                  75                  80

Gln Met Phe Thr Cys Arg Val Ala His Thr Pro Ser Ser Thr Asp Trp
                85                  90                  95

Val Asp Asn Lys Thr Phe Ser Val Cys Ser Arg Asp Phe Thr Pro Pro
            100                 105                 110

Thr Val Lys Ile Leu Gln Ser Ser Cys Asp Gly Gly Gly His Phe Pro
        115                 120                 125

Pro Thr Ile Gln Leu Leu Cys Leu Val Ser Gly Tyr Thr Pro Gly Thr
    130                 135                 140

Ile Asn Ile Thr Trp Leu Glu Asp Gly Gln Val Met Asp Val Asp Leu
145                 150                 155                 160

Ser Thr Ala Ser Thr Thr Gln Glu Gly Glu Leu Ala Ser Thr Gln Ser
                165                 170                 175

Glu Leu Thr Leu Ser Gln Lys His Trp Leu Ser Asp Arg Thr Tyr Thr
            180                 185                 190

Cys Gln Val Thr Tyr Gln Gly His Thr Phe Glu Asp Ser Thr Lys Lys
        195                 200                 205

Cys Ala Asp Ser Asn Pro Arg Gly Val Ser Ala Tyr Leu Ser Arg Pro
    210                 215                 220

Ser Pro Phe Asp Leu Phe Ile Arg Lys Ser Pro Thr Ile Thr Cys Leu
225                 230                 235                 240

Val Val Asp Leu Ala Pro Ser Lys Gly Thr Val Asn Leu Thr Trp Ser
                245                 250                 255

Arg Ala Ser Gly Lys Pro Val Asn His Ser Thr Arg Lys Glu Glu Lys
            260                 265                 270

Gln Arg Asn Gly Thr Leu Thr Val Thr Ser Thr Leu Pro Val Gly Thr
        275                 280                 285

Arg Asp Trp Ile Glu Gly Glu Thr Tyr Gln Cys Arg Val Thr His Pro
    290                 295                 300

His Leu Pro Arg Ala Leu Met Arg Ser Thr Thr Lys Thr Ser Gly Pro
305                 310                 315                 320

Arg Ala Ala Pro Glu Val Tyr Ala Phe Ala Thr Pro Glu Trp Pro Gly
                325                 330                 335

Ser Arg Asp Lys Arg Thr Leu Ala Cys Leu Ile Gln Asn Phe Met Pro
            340                 345                 350

Glu Asp Ile Ser Val Gln Trp Leu His Asn Glu Val Gln Leu Pro Asp
        355                 360                 365

Ala Arg His Ser Thr Thr Gln Pro Arg Lys Thr Lys Gly Ser Gly Phe
    370                 375                 380

Phe Val Phe Ser Arg Leu Glu Val Thr Arg Ala Glu Trp Glu Gln Lys
385                 390                 395                 400

Asp Glu Phe Ile Cys Arg Ala Val His Glu Ala Ala Ser Pro Ser Gln
                405                 410                 415

Thr Val Gln Arg Ala Val Ser Val Asn Pro Gly Lys
            420                 425


<210> 61
<211> 1884
<212> DNA
<213> Homo sapiens

<400> 61
atggactgga cctggaggtt cctctttgtg gtggcagcag ctacaggtgt ccagtcccag 60
gtgcagctgg tgcagtctgg ggctgaggtg aagaagcctg ggtcctcggt gaaggtctcc 120
tgcaaggctt ctggaggcac cttcagcagc tatgctatca gctgggtgcg acaggcccct 180
ggacaagggc ttgagtggat gggagggatc atccctatct ttggtacagc aaactacgca 240
cagaagttcc agggcagagt cacgattacc gcggacgaat ccacgagcac agcctacatg 300
gagctgagca gcctgagatc tgaggacacg gccgtgtatt actgtgcgaa aaccgggatc 360
ctgggggcgt atagcagtgg ctggtacccg aactcggact actactacta cggtatggac 420
gtctggggcc aagggaccac ggtcaccgtc tcctcaggga gtgcatccgc cccaaccctt 480
ttccccctcg tctcctgtga gaattccccg tcggatacga gcagcgtggc cgttggctgc 540
ctcgcacagg acttccttcc cgactccatc actttctcct ggaaatacaa gaacaactct 600
gacatcagca gcacccgggg cttcccatca gtcctgagag ggggcaagta cgcagccacc 660
tcacaggtgc tgctgccttc caaggacgtc atgcagggca cagacgaaca cgtggtgtgc 720
aaagtccagc accccaacgg caacaaagaa aagaacgtgc ctcttccagt gattgctgag 780
ctgcctccca aagtgagcgt cttcgtccca ccccgcgacg gcttcttcgg caacccccgc 840
agcaagtcca agctcatctg ccaggccacg ggtttcagtc cccggcagat tcaggtgtcc 900
tggctgcgcg aggggaagca ggtggggtct ggcgtcacca cggaccaggt gcaggctgag 960
gccaaagagt ctgggcccac gacctacaag gtgaccagca cactgaccat caaagagagc 1020
gactggctca gccagagcat gttcacctgc cgcgtggatc acaggggcct gaccttccag 1080
cagaatgcgt cctccatgtg tgtccccgat caagacacag ccatccgggt cttcgccatc 1140
cccccatcct ttgccagcat cttcctcacc aagtccacca agttgacctg cctggtcaca 1200
gacctgacca cctatgacag cgtgaccatc tcctggaccc gccagaatgg cgaagctgtg 1260
aaaacccaca ccaacatctc cgagagccac cccaatgcca ctttcagcgc cgtgggtgag 1320
gccagcatct gcgaggatga ctggaattcc ggggagaggt tcacgtgcac cgtgacccac 1380
acagacctgc cctcgccact gaagcagacc atctcccggc ccaagggggt ggccctgcac 1440
aggcccgatg tctacttgct gccaccagcc cgggagcagc tgaacctgcg ggagtcggcc 1500
accatcacgt gcctggtgac gggcttctct cccgcggacg tcttcgtgca gtggatgcag 1560
agggggcagc ccttgtcccc ggagaagtat gtgaccagcg ccccaatgcc tgagccccag 1620
gccccaggcc ggtacttcgc ccacagcatc ctgaccgtgt ccgaagagga atggaacacg 1680
ggggagacct acacctgcgt ggtggcccat gaggccctgc ccaacagggt caccgagagg 1740
accgtggaca agtccaccga gggggaggtg agcgccgacg aggagggctt tgagaacctg 1800
tgggccaccg cctccacctt catcgtcctc ttcctcctga gcctcttcta cagtaccacc 1860
gtcaccttgt tcaaggtgaa atga                                        1884


<210> 62
<211> 454
<212> PRT
<213> Homo sapiens

<400> 62
Gly Ser Ala Ser Ala Pro Thr Leu Phe Pro Leu Val Ser Cys Glu Asn
1               5                   10                  15

Ser Pro Ser Asp Thr Ser Ser Val Ala Val Gly Cys Leu Ala Gln Asp
            20                  25                  30

Phe Leu Pro Asp Ser Ile Thr Phe Ser Trp Lys Tyr Lys Asn Asn Ser
        35                  40                  45

Asp Ile Ser Ser Thr Arg Gly Phe Pro Ser Val Leu Arg Gly Gly Lys
    50                  55                  60

Tyr Ala Ala Thr Ser Gln Val Leu Leu Pro Ser Lys Asp Val Met Gln
65                  70                  75                  80

Gly Thr Asp Glu His Val Val Cys Lys Val Gln His Pro Asn Gly Asn
                85                  90                  95

Lys Glu Lys Asn Val Pro Leu Pro Val Ile Ala Glu Leu Pro Pro Lys
            100                 105                 110

Val Ser Val Phe Val Pro Pro Arg Asp Gly Phe Phe Gly Asn Pro Arg
        115                 120                 125

Ser Lys Ser Lys Leu Ile Cys Gln Ala Thr Gly Phe Ser Pro Arg Gln
    130                 135                 140

Ile Gln Val Ser Trp Leu Arg Glu Gly Lys Gln Val Gly Ser Gly Val
145                 150                 155                 160

Thr Thr Asp Gln Val Gln Ala Glu Ala Lys Glu Ser Gly Pro Thr Thr
                165                 170                 175

Tyr Lys Val Thr Ser Thr Leu Thr Ile Lys Glu Ser Asp Trp Leu Ser
            180                 185                 190

Gln Ser Met Phe Thr Cys Arg Val Asp His Arg Gly Leu Thr Phe Gln
        195                 200                 205

Gln Asn Ala Ser Ser Met Cys Val Pro Asp Gln Asp Thr Ala Ile Arg
    210                 215                 220

Val Phe Ala Ile Pro Pro Ser Phe Ala Ser Ile Phe Leu Thr Lys Ser
225                 230                 235                 240

Thr Lys Leu Thr Cys Leu Val Thr Asp Leu Thr Thr Tyr Asp Ser Val
                245                 250                 255

Thr Ile Ser Trp Thr Arg Gln Asn Gly Glu Ala Val Lys Thr His Thr
            260                 265                 270

Asn Ile Ser Glu Ser His Pro Asn Ala Thr Phe Ser Ala Val Gly Glu
        275                 280                 285

Ala Ser Ile Cys Glu Asp Asp Trp Asn Ser Gly Glu Arg Phe Thr Cys
    290                 295                 300

Thr Val Thr His Thr Asp Leu Pro Ser Pro Leu Lys Gln Thr Ile Ser
305                 310                 315                 320

Arg Pro Lys Gly Val Ala Leu His Arg Pro Asp Val Tyr Leu Leu Pro
                325                 330                 335

Pro Ala Arg Glu Gln Leu Asn Leu Arg Glu Ser Ala Thr Ile Thr Cys
            340                 345                 350

Leu Val Thr Gly Phe Ser Pro Ala Asp Val Phe Val Gln Trp Met Gln
        355                 360                 365

Arg Gly Gln Pro Leu Ser Pro Glu Lys Tyr Val Thr Ser Ala Pro Met
    370                 375                 380

Pro Glu Pro Gln Ala Pro Gly Arg Tyr Phe Ala His Ser Ile Leu Thr
385                 390                 395                 400

Val Ser Glu Glu Glu Trp Asn Thr Gly Glu Thr Tyr Thr Cys Val Val
                405                 410                 415

Ala His Glu Ala Leu Pro Asn Arg Val Thr Glu Arg Thr Val Asp Lys
            420                 425                 430

Ser Thr Gly Lys Pro Thr Leu Tyr Asn Val Ser Leu Val Met Ser Asp
        435                 440                 445

Thr Ala Gly Thr Cys Tyr
    450


<210> 63
<211> 532
<212> PRT
<213> Homo sapiens

<400> 63
Met Ala Pro Ser Ser Pro Arg Pro Ala Leu Pro Ala Leu Leu Val Leu
1               5                   10                  15

Leu Gly Ala Leu Phe Pro Gly Pro Gly Asn Ala Gln Thr Ser Val Ser
            20                  25                  30

Pro Ser Lys Val Ile Leu Pro Arg Gly Gly Ser Val Leu Val Thr Cys
        35                  40                  45

Ser Thr Ser Cys Asp Gln Pro Lys Leu Leu Gly Ile Glu Thr Pro Leu
    50                  55                  60

Pro Lys Lys Glu Leu Leu Leu Pro Gly Asn Asn Arg Lys Val Tyr Glu
65                  70                  75                  80

Leu Ser Asn Val Gln Glu Asp Ser Gln Pro Met Cys Tyr Ser Asn Cys
                85                  90                  95

Pro Asp Gly Gln Ser Thr Ala Lys Thr Phe Leu Thr Val Tyr Trp Thr
            100                 105                 110

Pro Glu Arg Val Glu Leu Ala Pro Leu Pro Ser Trp Gln Pro Val Gly
        115                 120                 125

Lys Asn Leu Thr Leu Arg Cys Gln Val Glu Gly Gly Ala Pro Arg Ala
    130                 135                 140

Asn Leu Thr Val Val Leu Leu Arg Gly Glu Lys Glu Leu Lys Arg Glu
145                 150                 155                 160

Pro Ala Val Gly Glu Pro Ala Glu Val Thr Thr Thr Val Leu Val Arg
                165                 170                 175

Arg Asp His His Gly Ala Asn Phe Ser Cys Arg Thr Glu Leu Asp Leu
            180                 185                 190

Arg Pro Gln Gly Leu Glu Leu Phe Glu Asn Thr Ser Ala Pro Tyr Gln
        195                 200                 205

Leu Gln Thr Phe Val Leu Pro Ala Thr Pro Pro Gln Leu Val Ser Pro
    210                 215                 220

Arg Val Leu Glu Val Asp Thr Gln Gly Thr Val Val Cys Ser Leu Asp
225                 230                 235                 240

Gly Leu Phe Pro Val Ser Glu Ala Gln Val His Leu Ala Leu Gly Asp
                245                 250                 255

Gln Arg Leu Asn Pro Thr Val Thr Tyr Gly Asn Asp Ser Phe Ser Ala
            260                 265                 270

Lys Ala Ser Val Ser Val Thr Ala Glu Asp Glu Gly Thr Gln Arg Leu
        275                 280                 285

Thr Cys Ala Val Ile Leu Gly Asn Gln Ser Gln Glu Thr Leu Gln Thr
    290                 295                 300

Val Thr Ile Tyr Ser Phe Pro Ala Pro Asn Val Ile Leu Thr Lys Pro
305                 310                 315                 320

Glu Val Ser Glu Gly Thr Glu Val Thr Val Lys Cys Glu Ala His Pro
                325                 330                 335

Arg Ala Lys Val Thr Leu Asn Gly Val Pro Ala Gln Pro Leu Gly Pro
            340                 345                 350

Arg Ala Gln Leu Leu Leu Lys Ala Thr Pro Glu Asp Asn Gly Arg Ser
        355                 360                 365

Phe Ser Cys Ser Ala Thr Leu Glu Val Ala Gly Gln Leu Ile His Lys
    370                 375                 380

Asn Gln Thr Arg Glu Leu Arg Val Leu Tyr Gly Pro Arg Leu Asp Glu
385                 390                 395                 400

Arg Asp Cys Pro Gly Asn Trp Thr Trp Pro Glu Asn Ser Gln Gln Thr
                405                 410                 415

Pro Met Cys Gln Ala Trp Gly Asn Pro Leu Pro Glu Leu Lys Cys Leu
            420                 425                 430

Lys Asp Gly Thr Phe Pro Leu Pro Ile Gly Glu Ser Val Thr Val Thr
        435                 440                 445

Arg Asp Leu Glu Gly Thr Tyr Leu Cys Arg Ala Arg Ser Thr Gln Gly
    450                 455                 460

Glu Val Thr Arg Glu Val Thr Val Asn Val Leu Ser Pro Arg Tyr Glu
465                 470                 475                 480

Ile Val Ile Ile Thr Val Val Ala Ala Ala Val Ile Met Gly Thr Ala
                485                 490                 495

Gly Leu Ser Thr Tyr Leu Tyr Asn Arg Gln Arg Lys Ile Lys Lys Tyr
            500                 505                 510

Arg Leu Gln Gln Ala Gln Lys Gly Thr Pro Met Lys Pro Asn Thr Gln
        515                 520                 525

Ala Thr Pro Pro
    530

<210> 64

<211> 275
<212> PRT
<213> Homo sapiens

<400> 64
Met Ser Ser Phe Gly Tyr Arg Thr Leu Thr Val Ala Leu Phe Thr Leu
1               5                   10                  15

Ile Cys Cys Pro Gly Ser Asp Glu Lys Val Phe Glu Val His Val Arg
            20                  25                  30

Pro Lys Lys Leu Ala Val Glu Pro Lys Gly Ser Leu Glu Val Asn Cys
        35                  40                  45

Ser Thr Thr Cys Asn Gln Pro Glu Val Gly Gly Leu Glu Thr Ser Leu
    50                  55                  60

Asp Lys Ile Leu Leu Asp Glu Gln Ala Gln Trp Lys His Tyr Leu Val
65                  70                  75                  80

Ser Asn Ile Ser His Asp Thr Val Leu Gln Cys His Phe Thr Cys Ser
                85                  90                  95

Gly Lys Gln Glu Ser Met Asn Ser Asn Val Ser Val Tyr Gln Pro Pro
            100                 105                 110

Arg Gln Val Ile Leu Thr Leu Gln Pro Thr Leu Val Ala Val Gly Lys
        115                 120                 125

Ser Phe Thr Ile Glu Cys Arg Val Pro Thr Val Glu Pro Leu Asp Ser
    130                 135                 140

Leu Thr Leu Phe Leu Phe Arg Gly Asn Glu Thr Leu His Tyr Glu Thr
145                 150                 155                 160

Phe Gly Lys Ala Ala Pro Ala Pro Gln Glu Ala Thr Ala Thr Phe Asn
                165                 170                 175

Ser Thr Ala Asp Arg Glu Asp Gly His Arg Asn Phe Ser Cys Leu Ala
            180                 185                 190

Val Leu Asp Leu Met Ser Arg Gly Gly Asn Ile Phe His Lys His Ser
        195                 200                 205

Ala Pro Lys Met Leu Glu Ile Tyr Glu Pro Val Ser Asp Ser Gln Met
    210                 215                 220

Val Ile Ile Val Thr Val Val Ser Val Leu Leu Ser Leu Phe Val Thr
225                 230                 235                 240

Ser Val Leu Leu Cys Phe Ile Phe Gly Gln His Leu Arg Gln Gln Arg
                245                 250                 255

Met Gly Thr Tyr Gly Val Arg Ala Ala Trp Arg Arg Leu Pro Gln Ala
            260                 265                 270

Phe Arg Pro
        275


<210> 65
<211> 547
<212> PRT
<213> Homo sapiens

<400> 65
Met Ala Thr Met Val Pro Ser Val Leu Trp Pro Arg Ala Cys Trp Thr
1               5                   10                  15

Leu Leu Val Cys Cys Leu Leu Thr Pro Gly Val Gln Gly Gln Glu Phe
            20                  25                  30

Leu Leu Arg Val Glu Pro Gln Asn Pro Val Leu Ser Ala Gly Gly Ser
        35                  40                  45

Leu Phe Val Asn Cys Ser Thr Asp Cys Pro Ser Ser Glu Lys Ile Ala
    50                  55                  60

Leu Glu Thr Ser Leu Ser Lys Glu Leu Val Ala Ser Gly Met Gly Trp
65                  70                  75                  80

Ala Ala Phe Asn Leu Ser Asn Val Thr Gly Asn Ser Arg Ile Leu Cys
                85                  90                  95

Ser Val Tyr Cys Asn Gly Ser Gln Ile Thr Gly Ser Ser Asn Ile Thr
            100                 105                 110

Val Tyr Gly Leu Pro Glu Arg Val Glu Leu Ala Pro Leu Pro Pro Trp
        115                 120                 125

Gln Pro Val Gly Gln Asn Phe Thr Leu Arg Cys Gln Val Glu Gly Gly
    130                 135                 140

Ser Pro Arg Thr Ser Leu Thr Val Val Leu Leu Arg Trp Glu Glu Glu
145                 150                 155                 160

Leu Ser Arg Gln Pro Ala Val Glu Glu Pro Ala Glu Val Thr Ala Thr
                165                 170                 175

Val Leu Ala Ser Arg Asp Asp His Gly Ala Pro Phe Ser Cys Arg Thr
            180                 185                 190

Glu Leu Asp Met Gln Pro Gln Gly Leu Gly Leu Phe Val Asn Thr Ser
        195                 200                 205

Ala Pro Arg Gln Leu Arg Thr Phe Val Leu Pro Val Thr Pro Pro Arg
    210                 215                 220

Leu Val Ala Pro Arg Phe Leu Glu Val Glu Thr Ser Trp Pro Val Asp
225                 230                 235                 240

Cys Thr Leu Asp Gly Leu Phe Pro Ala Ser Glu Ala Gln Val Tyr Leu
                245                 250                 255

Ala Leu Gly Asp Gln Met Leu Asn Ala Thr Val Met Asn His Gly Asp
            260                 265                 270

Thr Leu Thr Ala Thr Ala Thr Ala Thr Ala Arg Ala Asp Gln Glu Gly
        275                 280                 285

Ala Arg Glu Ile Val Cys Asn Val Thr Leu Gly Gly Glu Arg Arg Glu
    290                 295                 300

Ala Arg Glu Asn Leu Thr Val Phe Ser Phe Leu Gly Pro Ile Val Asn
305                 310                 315                 320

Leu Ser Glu Pro Thr Ala His Glu Gly Ser Thr Val Thr Val Ser Cys
                325                 330                 335

Met Ala Gly Ala Arg Val Gln Val Thr Leu Asp Gly Val Pro Ala Ala
            340                 345                 350

Ala Pro Gly Gln Pro Ala Gln Leu Gln Leu Asn Ala Thr Glu Ser Asp
        355                 360                 365

Asp Gly Arg Ser Phe Phe Cys Ser Ala Thr Leu Glu Val Asp Gly Glu
    370                 375                 380

Phe Leu His Arg Asn Ser Ser Val Gln Leu Arg Val Leu Tyr Gly Pro
385                 390                 395                 400

Lys Ile Asp Arg Ala Thr Cys Pro Gln His Leu Lys Trp Lys Asp Lys
                405                 410                 415

Thr Arg His Val Leu Gln Cys Gln Ala Arg Gly Asn Pro Tyr Pro Glu
            420                 425                 430

Leu Arg Cys Leu Lys Glu Gly Ser Ser Arg Glu Val Pro Val Gly Ile
        435                 440                 445

Pro Phe Phe Val Asn Val Thr His Asn Gly Thr Tyr Gln Cys Gln Ala
    450                 455                 460

Ser Ser Ser Arg Gly Lys Tyr Thr Leu Val Val Val Met Asp Ile Glu
465                 470                 475                 480

Ala Gly Ser Ser His Phe Val Pro Val Phe Val Ala Val Leu Leu Thr
                485                 490                 495

Leu Gly Val Val Thr Ile Val Leu Ala Leu Met Tyr Val Phe Arg Glu
            500                 505                 510

His Gln Arg Ser Gly Ser Tyr His Val Arg Glu Glu Ser Thr Tyr Leu
        515                 520                 525

Pro Leu Thr Ser Met Gln Pro Thr Glu Ala Met Gly Glu Glu Pro Ser
    530                 535                 540

Arg Ala Glu
545

<210> 66
<211> 577
<212> PRT
<213> Homo sapiens

<400> 66
Gly Val Pro Glu Glu Leu Phe Glu Val Ser Ile Trp Pro Ser Gln Ala
1               5                   10                  15

Leu Val Glu Phe Gly Gln Ser Leu Val Val Asn Cys Ser Thr Thr Cys
            20                  25                  30

Pro Asp Pro Gly Pro Ser Gly Ile Glu Thr Phe Leu Lys Lys Thr Gln
        35                  40                  45

Val Gly Lys Gly Pro Gln Trp Lys Glu Phe Leu Leu Glu Asp Val Thr
    50                  55                  60

Glu Asn Ser Ile Leu Gln Cys Phe Phe Ser Cys Ala Gly Ile Gln Lys
65                  70                  75                  80

Asp Thr Ser Leu Gly Ile Thr Val Tyr Gln Pro Pro Glu Gln Val Ile
                85                  90                  95

Leu Glu Leu Gln Pro Ala Trp Val Ala Val Asp Glu Ala Phe Thr Val
            100                 105                 110

Lys Cys His Val Pro Ser Val Ala Pro Leu Glu Ser Leu Thr Leu Ala
        115                 120                 125

Leu Leu Gln Gly Asn Gln Glu Leu His Arg Lys Asn Phe Thr Ser Leu
    130                 135                 140

Ala Val Ala Ser Gln Arg Ala Glu Val Ile Ile Ser Val Arg Ala Gln
145                 150                 155                 160

Lys Glu Asn Asp Arg Cys Asn Ser Ser Cys His Ala Glu Leu Asp Leu
                165                 170                 175

Ser Leu Gln Gly Gly Arg Leu Phe Gln Gly Ser Ser Pro Ile Arg Ile
            180                 185                 190

Val Arg Ile Phe Glu Phe Ser Gln Ser Pro His Ile Trp Val Ser Ser
        195                 200                 205

Leu Leu Glu Ala Gly Met Ala Glu Thr Val Ser Cys Glu Val Ala Arg
    210                 215                 220

Val Phe Pro Ala Lys Glu Val Met Phe His Met Phe Leu Glu Asp Gln
225                 230                 235                 240

Glu Leu Ser Ser Phe Leu Ser Trp Glu Gly Asp Thr Ala Trp Ala Asn
                245                 250                 255

Ala Thr Ile Arg Thr Met Glu Ala Gly Asp Gln Glu Leu Ser Cys Phe
            260                 265                 270

Ala Ser Leu Gly Ala Met Glu Gln Lys Thr Arg Lys Leu Val His Ser
        275                 280                 285

Tyr Asn Lys Trp Pro Gly Ser Ser Phe Phe Ile Arg Val Leu Cys Cys
    290                 295                 300

Lys His Arg Val Thr Gly Trp Phe Gly Cys Arg His Pro Cys Cys Pro
305                 310                 315                 320

Leu Leu Gly Met Leu Ser Ser Glu His Glu Ser Ser Ser Phe Ser Gly
                325                 330                 335

Phe Pro Pro Pro Ile Leu Glu Leu Lys Glu Ser Tyr Pro Leu Ala Gly
            340                 345                 350

Thr Asp Ile Asn Val Thr Cys Ser Gly His Val Leu Thr Ser Pro Ser
        355                 360                 365

Pro Thr Leu Arg Leu Gln Gly Ala Pro Asp Leu Pro Ala Gly Glu Pro
    370                 375                 380

Ala Trp Leu Leu Leu Thr Ala Arg Glu Glu Asp Asp Gly Asn Phe Ser
385                 390                 395                 400

Cys Glu Ala Ser Leu Val Val Gln Gly Gln Arg Leu Met Lys Thr Thr
                405                 410                 415

Val Ile Gln Leu His Ile Leu Cys Lys Pro Gln Leu Glu Glu Ser Ser
            420                 425                 430

Cys Pro Gly Lys Gln Thr Trp Leu Glu Gly Met Glu His Thr Leu Ala
        435                 440                 445

Cys Val Pro Lys Gly Asn Pro Ala Pro Ala Leu Val Cys Thr Trp Asn
    450                 455                 460

Gly Val Val Phe Asp Leu Glu Val Pro Gln Lys Ala Thr Asn His Thr
465                 470                 475                 480

Gly Thr Tyr Arg Tyr Thr Ala Thr Asn Gln Leu Gly Ser Val Ser Lys
                485                 490                 495

Asp Ile Ala Val Ile Val Gln Gly Leu Asp Glu Gly Ile Ser Ser Thr
            500                 505                 510

Leu Phe Val Ile Ile Thr Val Ala Leu Gly Val Gly Val Ile Thr Ile
        515                 520                 525

Ala Leu Tyr Leu Ser Tyr Arg Pro Cys Lys Val Asp Arg Arg Lys Leu
    530                 535                 540

Leu Tyr Arg Gln Lys Glu Glu Asp Lys Glu Glu Glu Ser Gln Phe Ala
545                 550                 555                 560

Val Gln Glu Glu Lys Ser Thr Thr His Ile Ile Asp Ser Tyr Leu Ile
                565                 570                 575

Glu


<210> 67
<211> 924
<212> PRT
<213> Homo sapiens

<400> 67
Met Pro Gly Pro Ser Pro Gly Leu Arg Arg Ala Leu Leu Gly Leu Trp
1               5                   10                  15

Ala Ala Leu Gly Leu Gly Leu Phe Gly Leu Ser Ala Val Ser Gln Glu
            20                  25                  30

Pro Phe Trp Ala Asp Leu Gln Pro Arg Val Ala Phe Val Glu Arg Gly
        35                  40                  45

Gly Ser Leu Trp Leu Asn Cys Ser Thr Asn Cys Pro Arg Pro Glu Arg
    50                  55                  60

Gly Gly Leu Glu Thr Ser Leu Arg Arg Asn Gly Thr Gln Arg Gly Leu
65                  70                  75                  80

Arg Trp Leu Ala Arg Gln Leu Val Asp Ile Arg Glu Pro Glu Thr Gln
                85                  90                  95

Pro Val Cys Phe Phe Arg Cys Ala Arg Arg Thr Leu Gln Ala Arg Gly
            100                 105                 110

Leu Ile Arg Thr Phe Gln Arg Pro Asp Arg Val Glu Leu Met Pro Leu
        115                 120                 125

Pro Pro Trp Gln Pro Val Gly Glu Asn Phe Thr Leu Ser Cys Arg Val
    130                 135                 140

Pro Gly Ala Gly Pro Arg Ala Ser Leu Thr Leu Thr Leu Leu Arg Gly
145                 150                 155                 160

Ala Gln Glu Leu Ile Arg Arg Ser Phe Ala Gly Glu Pro Pro Arg Ala
                165                 170                 175

Arg Gly Ala Val Leu Thr Ala Thr Val Leu Ala Arg Arg Glu Asp His
            180                 185                 190

Gly Ala Asn Phe Ser Cys Arg Ala Glu Leu Asp Leu Arg Pro His Gly
        195                 200                 205

Leu Gly Leu Phe Glu Asn Ser Ser Ala Pro Arg Glu Leu Arg Thr Phe
    210                 215                 220

Ser Leu Ser Pro Asp Ala Pro Arg Leu Ala Ala Pro Arg Leu Leu Glu
225                 230                 235                 240

Val Gly Ser Glu Arg Pro Val Ser Cys Thr Leu Asp Gly Leu Phe Pro
                245                 250                 255

Ala Ser Glu Ala Arg Val Tyr Leu Ala Leu Gly Asp Gln Asn Leu Ser

260                 265                 270

Pro Asp Val Thr Leu Glu Gly Asp Ala Phe Val Ala Thr Ala Thr Ala
        275                 280                 285

Thr Ala Ser Ala Glu Gln Glu Gly Ala Arg Gln Leu Ile Cys Asn Val
    290                 295                 300

Thr Leu Gly Gly Glu Asn Arg Glu Thr Arg Glu Asn Val Thr Ile Tyr
305                 310                 315                 320

Ser Phe Pro Ala Pro Leu Leu Thr Leu Ser Glu Pro Ser Val Ser Glu
                325                 330                 335

Gly Gln Met Val Thr Val Thr Cys Ala Ala Gly Thr Gln Ala Leu Val
            340                 345                 350

Thr Leu Glu Gly Val Pro Ala Ala Val Pro Gly Gln Pro Ala Gln Leu
        355                 360                 365

Gln Leu Asn Ala Thr Glu Asn Asp Asp Arg Arg Ser Phe Phe Cys Asp
    370                 375                 380

Ala Thr Leu Asp Val Asp Gly Glu Thr Leu Ile Lys Asn Arg Ser Ala
385                 390                 395                 400

Glu Leu Arg Val Leu Tyr Ala Pro Arg Leu Asp Asp Ser Asp Cys Pro
                405                 410                 415

Arg Ser Trp Thr Trp Pro Glu Gly Pro Glu Gln Thr Leu Arg Cys Glu
            420                 425                 430

Ala Arg Gly Asn Pro Glu Pro Ser Val His Cys Ala Arg Ser Asp Gly
        435                 440                 445

Gly Ala Val Leu Ala Leu Gly Leu Leu Gly Pro Val Thr Arg Ala Leu
    450                 455                 460

Ser Gly Thr Tyr Arg Cys Lys Ala Ala Asn Asp Gln Gly Glu Ala Val
465                 470                 475                 480

Lys Asp Val Thr Leu Thr Val Glu Tyr Ala Pro Ala Leu Asp Ser Val
                485                 490                 495

Gly Cys Pro Glu Arg Ile Thr Trp Leu Glu Gly Thr Glu Ala Ser Leu
            500                 505                 510

Ser Cys Val Ala His Gly Val Pro Pro Pro Asp Val Ile Cys Val Arg
        515                 520                 525

Ser Gly Glu Leu Gly Ala Val Ile Glu Gly Leu Leu Arg Val Ala Arg
    530                 535                 540

Glu His Ala Gly Thr Tyr Arg Cys Glu Ala Thr Asn Pro Arg Gly Ser
545                 550                 555                 560

Ala Ala Lys Asn Val Ala Val Thr Val Glu Tyr Gly Pro Arg Phe Glu
                565                 570                 575

Glu Pro Ser Cys Pro Ser Asn Trp Thr Trp Val Glu Gly Ser Gly Arg
            580                 585                 590

Leu Phe Ser Cys Glu Val Asp Gly Lys Pro Gln Pro Ser Val Lys Cys
        595                 600                 605

Val Gly Ser Gly Gly Ala Thr Glu Gly Val Leu Leu Pro Leu Ala Pro
    610                 615                 620

Pro Asp Pro Ser Pro Arg Ala Pro Arg Ile Pro Arg Val Leu Ala Pro
625                 630                 635                 640

Gly Ile Tyr Val Cys Asn Ala Thr Asn Arg His Gly Ser Val Ala Lys
                645                 650                 655

Thr Val Val Val Ser Ala Glu Ser Pro Pro Glu Met Asp Glu Ser Thr
            660                 665                 670

Cys Pro Ser His Gln Thr Trp Leu Glu Gly Ala Glu Ala Ser Ala Leu
        675                 680                 685

Ala Cys Ala Ala Arg Gly Arg Pro Ser Pro Gly Val Arg Cys Ser Arg
    690                 695                 700

Glu Gly Ile Pro Trp Pro Glu Gln Gln Arg Val Ser Arg Glu Asp Ala
705                 710                 715                 720

Gly Thr Tyr His Cys Val Ala Thr Asn Ala His Gly Thr Asp Ser Arg
                725                 730                 735

Thr Val Thr Val Gly Val Glu Tyr Arg Pro Val Val Ala Glu Leu Ala
            740                 745                 750

Ala Ser Pro Pro Gly Gly Val Arg Pro Gly Gly Asn Phe Thr Leu Thr
        755                 760                 765

Cys Arg Ala Glu Ala Trp Pro Pro Ala Gln Ile Ser Trp Arg Ala Pro
    770                 775                 780

Pro Gly Ala Leu Asn Ile Gly Leu Ser Ser Asn Asn Ser Thr Leu Ser
785                 790                 795                 800

Val Ala Gly Ala Met Gly Ser His Gly Gly Glu Tyr Glu Cys Ala Arg
                805                 810                 815

Thr Asn Ala His Gly Arg His Ala Arg Arg Ile Thr Val Arg Val Ala
            820                 825                 830

Gly Pro Trp Leu Trp Val Ala Val Gly Gly Ala Ala Gly Gly Ala Ala
        835                 840                 845

Leu Leu Ala Ala Gly Ala Gly Leu Ala Phe Tyr Val Gln Ser Thr Ala
    850                 855                 860

Cys Lys Lys Gly Glu Tyr Asn Val Gln Glu Ala Glu Ser Ser Gly Glu
865                 870                 875                 880

Ala Val Cys Leu Asn Gly Ala Gly Gly Gly Ala Gly Gly Ala Ala Gly
                885                 890                 895

Ala Glu Gly Gly Pro Glu Ala Ala Gly Gly Ala Ala Glu Ser Pro Ala
            900                 905                 910

Glu Gly Glu Val Phe Ala Ile Gln Leu Thr Ser Ala
        915                 920


<210> 68
<211> 406
<212> PRT
<213> Homo sapiens

<400> 68
Met Asp Phe Gly Leu Ala Leu Leu Leu Ala Gly Leu Leu Gly Leu Leu
1               5                   10                  15

Leu Gly Gln Ser Leu Gln Val Lys Pro Leu Gln Val Glu Pro Pro Glu
            20                  25                  30

Pro Val Val Ala Val Ala Leu Gly Ala Ser Arg Gln Leu Thr Cys Arg
        35                  40                  45

Leu Ala Cys Ala Asp Arg Gly Ala Ser Val Gln Trp Arg Gly Leu Asp
    50                  55                  60

Thr Ser Leu Gly Ala Val Gln Ser Asp Thr Gly Arg Ser Val Leu Thr
65                  70                  75                  80

Val Arg Asn Ala Ser Leu Ser Ala Ala Gly Thr Arg Val Cys Val Gly
                85                  90                  95

Ser Cys Gly Gly Arg Thr Phe Gln His Thr Val Gln Leu Leu Val Tyr
            100                 105                 110

Ala Phe Pro Asp Gln Leu Thr Val Ser Pro Ala Ala Leu Val Pro Gly
        115                 120                 125

Asp Pro Glu Val Ala Cys Thr Ala His Lys Val Thr Pro Val Asp Pro
    130                 135                 140

Asn Ala Leu Ser Phe Ser Leu Leu Val Gly Gly Gln Glu Leu Glu Gly
145                 150                 155                 160

Ala Gln Ala Leu Gly Pro Glu Val Gln Glu Glu Glu Glu Glu Pro Gln
                165                 170                 175

Gly Asp Glu Asp Val Leu Phe Arg Val Thr Glu Arg Trp Arg Leu Pro
            180                 185                 190

Pro Leu Gly Thr Pro Val Pro Pro Ala Leu Tyr Cys Gln Ala Thr Met
        195                 200                 205

Arg Leu Pro Gly Leu Glu Leu Ser His Arg Gln Ala Ile Pro Val Leu

210                 215                 220

His Ser Pro Thr Ser Pro Glu Pro Pro Asp Thr Thr Ser Pro Glu Pro
225                 230                 235                 240

Pro Asn Thr Thr Ser Pro Glu Ser Pro Asp Thr Thr Ser Pro Glu Ser
                245                 250                 255

Pro Asp Thr Thr Ser Gln Glu Pro Pro Asp Thr Thr Ser Gln Glu Pro
            260                 265                 270

Pro Asp Thr Thr Ser Gln Glu Pro Pro Asp Thr Thr Ser Pro Glu Pro
        275                 280                 285

Pro Asp Lys Thr Ser Pro Glu Pro Ala Pro Gln Gln Gly Ser Thr His
    290                 295                 300

Thr Pro Arg Ser Pro Gly Ser Thr Arg Thr Arg Arg Pro Glu Ile Ser
305                 310                 315                 320

Gln Ala Gly Pro Thr Gln Gly Glu Val Ile Pro Thr Gly Ser Ser Lys
                325                 330                 335

Pro Ala Gly Asp Gln Leu Pro Ala Ala Leu Trp Thr Ser Ser Ala Val
            340                 345                 350

Leu Gly Leu Leu Leu Leu Ala Leu Pro Thr Tyr His Leu Trp Lys Arg
        355                 360                 365

Cys Arg His Leu Ala Glu Asp Asp Thr His Pro Pro Ala Ser Leu Arg
    370                 375                 380

Leu Leu Pro Gln Val Ser Ala Trp Ala Gly Leu Arg Gly Thr Gly Gln
385                 390                 395                 400

Val Gly Ile Ser Pro Ser
                405


<210> 69
<211> 739
<212> PRT
<213> Homo sapiens

<400> 69
Met Pro Gly Lys Met Val Val Ile Leu Gly Ala Ser Asn Ile Leu Trp
1               5                   10                  15

Ile Met Phe Ala Ala Ser Gln Ala Phe Lys Ile Glu Thr Thr Pro Glu
            20                  25                  30

Ser Arg Tyr Leu Ala Gln Ile Gly Asp Ser Val Ser Leu Thr Cys Ser
        35                  40                  45

Thr Thr Gly Cys Glu Ser Pro Phe Phe Ser Trp Arg Thr Gln Ile Asp
    50                  55                  60

Ser Pro Leu Asn Gly Lys Val Thr Asn Glu Gly Thr Thr Ser Thr Leu
65                  70                  75                  80

Thr Met Asn Pro Val Ser Phe Gly Asn Glu His Ser Tyr Leu Cys Thr
                85                  90                  95

Ala Thr Cys Glu Ser Arg Lys Leu Glu Lys Gly Ile Gln Val Glu Ile
            100                 105                 110

Tyr Ser Phe Pro Lys Asp Pro Glu Ile His Leu Ser Gly Pro Leu Glu
        115                 120                 125

Ala Gly Lys Pro Ile Thr Val Lys Cys Ser Val Ala Asp Val Tyr Pro
    130                 135                 140

Phe Asp Arg Leu Glu Ile Asp Leu Leu Lys Gly Asp His Leu Met Lys
145                 150                 155                 160

Ser Gln Glu Phe Leu Glu Asp Ala Asp Arg Lys Ser Leu Glu Thr Lys
                165                 170                 175

Ser Leu Glu Val Thr Phe Thr Pro Val Ile Glu Asp Ile Gly Lys Val
            180                 185                 190

Leu Val Cys Arg Ala Lys Leu His Ile Asp Glu Met Asp Ser Val Pro
        195                 200                 205

Thr Val Arg Gln Ala Val Lys Glu Leu Gln Val Tyr Ile Ser Pro Lys
    210                 215                 220

Asn Thr Val Ile Ser Val Asn Pro Ser Thr Lys Leu Gln Glu Gly Gly
225                 230                 235                 240

Ser Val Thr Met Thr Cys Ser Ser Glu Gly Leu Pro Ala Pro Glu Ile
                245                 250                 255

Phe Trp Ser Lys Lys Leu Asp Asn Gly Asn Leu Gln His Leu Ser Gly
            260                 265                 270

Asn Ala Thr Leu Thr Leu Ile Ala Met Arg Met Glu Asp Ser Gly Ile
        275                 280                 285

Tyr Val Cys Glu Gly Val Asn Leu Ile Gly Lys Asn Arg Lys Glu Val
    290                 295                 300

Glu Leu Ile Val Gln Glu Lys Pro Phe Thr Val Glu Ile Ser Pro Gly
305                 310                 315                 320

Pro Arg Ile Ala Ala Gln Ile Gly Asp Ser Val Met Leu Thr Cys Ser
                325                 330                 335

Val Met Gly Cys Glu Ser Pro Ser Phe Ser Trp Arg Thr Gln Ile Asp
            340                 345                 350

Ser Pro Leu Ser Gly Lys Val Arg Ser Glu Gly Thr Asn Ser Thr Leu
        355                 360                 365

Thr Leu Ser Pro Val Ser Phe Glu Asn Glu His Ser Tyr Leu Cys Thr
    370                 375                 380

Val Thr Cys Gly His Lys Lys Leu Glu Lys Gly Ile Gln Val Glu Leu
385                 390                 395                 400

Tyr Ser Phe Pro Arg Asp Pro Glu Ile Glu Met Ser Gly Gly Leu Val
                405                 410                 415

Asn Gly Ser Ser Val Thr Val Ser Cys Lys Val Pro Ser Val Tyr Pro
            420                 425                 430

Leu Asp Arg Leu Glu Ile Glu Leu Leu Lys Gly Glu Thr Ile Leu Glu
        435                 440                 445

Asn Ile Glu Phe Leu Glu Asp Thr Asp Met Lys Ser Leu Glu Asn Lys
    450                 455                 460

Ser Leu Glu Met Thr Phe Ile Pro Thr Ile Glu Asp Thr Gly Lys Ala
465                 470                 475                 480

Leu Val Cys Gln Ala Lys Leu His Ile Asp Asp Met Glu Phe Glu Pro
                485                 490                 495

Lys Gln Arg Gln Ser Thr Gln Thr Leu Tyr Val Asn Val Ala Pro Arg
            500                 505                 510

Asp Thr Thr Val Leu Val Ser Pro Ser Ser Ile Leu Glu Glu Gly Ser
        515                 520                 525

Ser Val Asn Met Thr Cys Leu Ser Gln Gly Phe Pro Ala Pro Lys Ile
    530                 535                 540

Leu Trp Ser Arg Gln Leu Pro Asn Gly Glu Leu Gln Pro Leu Ser Glu
545                 550                 555                 560

Asn Ala Thr Leu Thr Leu Ile Ser Thr Lys Met Glu Asp Ser Gly Val
                565                 570                 575

Tyr Leu Cys Glu Gly Ile Asn Gln Ala Gly Arg Ser Arg Lys Glu Val
            580                 585                 590

Glu Leu Ile Ile Gln Val Thr Pro Lys Asp Ile Lys Leu Thr Ala Phe
        595                 600                 605

Pro Ser Glu Ser Val Lys Glu Gly Asp Thr Val Ile Ile Ser Cys Thr
    610                 615                 620

Cys Gly Asn Val Pro Glu Thr Trp Ile Ile Leu Lys Lys Lys Ala Glu
625                 630                 635                 640

Thr Gly Asp Thr Val Leu Lys Ser Ile Asp Gly Ala Tyr Thr Ile Arg
                645                 650                 655

Lys Ala Gln Leu Lys Asp Ala Gly Val Tyr Glu Cys Glu Ser Lys Asn
            660                 665                 670

Lys Val Gly Ser Gln Leu Arg Ser Leu Thr Leu Asp Val Gln Gly Arg

675                     680                     685

Glu Asn Asn Lys Asp Tyr Phe Ser Pro Glu Leu Leu Val Leu Tyr Phe
    690                     695                     700

Ala Ser Ser Leu Ile Ile Pro Ala Ile Gly Met Ile Ile Tyr Phe Ala
705                     710                     715                     720

Arg Lys Ala Asn Met Lys Gly Ser Tyr Ser Leu Val Glu Ala Gln Lys
                725                     730                     735

Ser Lys Val

<210> 70
<211> 537
<212> PRT
<213> Mus musculus

<400> 70
Met Ala Ser Thr Arg Ala Lys Pro Thr Leu Pro Leu Leu Leu Ala Leu
1               5                   10                  15

Val Thr Val Val Ile Pro Gly Pro Gly Asp Ala Gln Val Ser Ile His
            20                  25                  30

Pro Arg Glu Ala Phe Leu Pro Gln Gly Gly Ser Val Gln Val Asn Cys
        35                  40                  45

Ser Ser Ser Cys Lys Glu Asp Leu Ser Leu Gly Leu Glu Thr Gln Trp
    50                  55                  60

Leu Lys Asp Glu Leu Glu Ser Gly Pro Asn Trp Lys Leu Phe Glu Leu
65                  70                  75                  80

Ser Glu Ile Gly Glu Asp Ser Ser Pro Leu Cys Phe Glu Asn Cys Gly
                85                  90                  95

Thr Val Gln Ser Ser Ala Ser Ala Thr Ile Thr Val Tyr Ser Phe Pro
            100                 105                 110

Glu Ser Val Glu Leu Arg Pro Leu Pro Ala Trp Gln Gln Val Gly Lys
        115                 120                 125

Asp Leu Thr Leu Arg Cys His Val Asp Gly Gly Ala Pro Arg Thr Gln
    130                 135                 140

Leu Ser Ala Val Leu Leu Arg Gly Glu Glu Ile Leu Ser Arg Gln Pro
145                 150                 155                 160

Val Gly Gly His Pro Lys Asp Pro Lys Glu Ile Thr Phe Thr Val Leu
                165                 170                 175

Ala Ser Arg Gly Asp His Gly Ala Asn Phe Ser Cys Arg Thr Glu Leu
            180                 185                 190

Asp Leu Arg Pro Gln Gly Leu Ala Leu Phe Ser Asn Val Ser Glu Ala
        195                 200                 205

Arg Ser Leu Arg Thr Phe Asp Leu Pro Ala Thr Ile Pro Lys Leu Asp
    210                 215                 220

Thr Pro Asp Leu Leu Glu Val Gly Thr Gln Gln Lys Leu Phe Cys Ser
225                 230                 235                 240

Leu Glu Gly Leu Phe Pro Ala Ser Glu Ala Arg Ile Tyr Leu Glu Leu
                245                 250                 255

Gly Gly Gln Met Pro Thr Gln Glu Ser Thr Asn Ser Ser Asp Ser Val
            260                 265                 270

Ser Ala Thr Ala Leu Val Glu Val Thr Glu Glu Phe Asp Arg Thr Leu
        275                 280                 285

Pro Leu Arg Cys Val Leu Glu Leu Ala Asp Gln Ile Leu Glu Thr Gln
    290                 295                 300

Arg Thr Leu Thr Val Tyr Asn Phe Ser Ala Pro Val Leu Thr Leu Ser
305                 310                 315                 320

Gln Leu Glu Val Ser Glu Gly Ser Gln Val Thr Val Lys Cys Glu Ala
                325                 330                 335

His Ser Gly Ser Lys Val Val Leu Leu Ser Gly Val Glu Pro Arg Pro
            340                 345                 350

Pro Thr Pro Gln Val Gln Phe Thr Leu Asn Ala Ser Ser Glu Asp His
        355                 360                 365

Lys Arg Ser Phe Phe Cys Ser Ala Ala Leu Glu Val Ala Gly Lys Phe
    370                 375                 380

Leu Phe Lys Asn Gln Thr Leu Glu Leu His Val Leu Tyr Gly Pro Arg
385                 390                 395                 400

Leu Asp Glu Thr Asp Cys Leu Gly Asn Trp Thr Trp Gln Glu Gly Ser
                405                 410                 415

Gln Gln Thr Leu Lys Cys Gln Ala Trp Gly Asn Pro Ser Pro Lys Met
            420                 425                 430

Thr Cys Arg Arg Lys Ala Asp Gly Ala Leu Leu Pro Ile Gly Val Val
        435                 440                 445

Lys Ser Val Lys Gln Glu Met Asn Gly Thr Tyr Val Cys His Ala Phe
    450                 455                 460

Ser Ser His Gly Asn Val Thr Arg Asn Val Tyr Leu Thr Val Leu Tyr
465                 470                 475                 480

His Ser Gln Asn Asn Trp Thr Ile Ile Ile Leu Val Pro Val Leu Leu
                485                 490                 495

Val Ile Val Gly Leu Val Met Ala Ala Ser Tyr Val Tyr Asn Arg Gln
            500                 505                 510

Arg Lys Ile Arg Ile Tyr Lys Leu Gln Lys Ala Gln Glu Glu Ala Ile
        515                 520                 525

Lys Leu Lys Gly Gln Ala Pro Pro Pro
    530                 535

<210> 71
<211> 537
<212> PRT
<213> Mus musculus

<400> 71
Met Ala Ser Thr Arg Ala Lys Pro Thr Leu Pro Leu Leu Leu Ala Leu
1               5                   10                  15

Val Thr Val Val Ile Pro Gly Pro Gly Asp Ala Gln Val Ser Ile His
            20                  25                  30

Pro Arg Glu Ala Phe Leu Pro Gln Gly Gly Ser Val Gln Val Asn Cys
        35                  40                  45

Ser Ser Ser Cys Lys Glu Asp Leu Ser Leu Gly Leu Glu Thr Gln Trp
    50                  55                  60

Leu Lys Asp Glu Leu Glu Ser Gly Pro Asn Trp Lys Leu Phe Glu Leu
65                  70                  75                  80

Ser Glu Ile Gly Glu Asp Ser Ser Pro Leu Cys Phe Glu Asn Cys Gly
                85                  90                  95

Thr Val Gln Ser Ser Ala Ser Ala Thr Ile Thr Val Tyr Ser Phe Pro
            100                 105                 110

Glu Ser Val Glu Leu Arg Pro Leu Pro Ala Trp Gln Gln Val Gly Lys
        115                 120                 125

Asp Leu Thr Leu Arg Cys His Val Asp Gly Gly Ala Pro Arg Thr Gln
    130                 135                 140

Leu Ser Ala Val Leu Leu Arg Gly Glu Glu Ile Leu Ser Arg Gln Pro
145                 150                 155                 160

Val Gly Gly His Pro Lys Asp Pro Lys Glu Ile Thr Phe Thr Val Leu
                165                 170                 175

Ala Ser Arg Gly Asp His Gly Ala Asn Phe Ser Cys Arg Thr Glu Leu
            180                 185                 190

Asp Leu Arg Pro Gln Gly Leu Ala Leu Phe Ser Asn Val Ser Glu Ala
        195                 200                 205

Arg Ser Leu Arg Thr Phe Asp Leu Pro Ala Thr Ile Pro Lys Leu Asp
    210                 215                 220

Thr Pro Asp Leu Leu Glu Val Gly Thr Gln Gln Lys Leu Phe Cys Ser

225                 230                 235                 240

Leu Glu Ala Leu Phe Pro Ala Ser Glu Ala Arg Ile Tyr Leu Glu Leu
                245                 250                 255

Gly Gly Gln Met Pro Thr Gln Glu Ser Thr Asn Ser Ser Asp Ser Val
            260                 265                 270

Ser Ala Thr Ala Leu Val Glu Val Thr Glu Glu Phe Asp Arg Thr Leu
        275                 280                 285

Pro Leu Arg Cys Val Leu Glu Leu Ala Asp Gln Ile Leu Glu Thr Gln
    290                 295                 300

Arg Thr Leu Thr Val Tyr Asn Phe Ser Ala Pro Val Leu Thr Leu Ser
305                 310                 315                 320

Gln Leu Glu Val Ser Glu Gly Ser Gln Val Thr Val Lys Cys Glu Ala
                325                 330                 335

His Ser Gly Ser Lys Val Val Leu Leu Ser Gly Val Glu Pro Arg Pro
            340                 345                 350

Pro Thr Pro Gln Val Gln Phe Thr Leu Asn Ala Ser Ser Glu Asp His
        355                 360                 365

Lys Arg Ser Phe Phe Cys Ser Ala Ala Leu Glu Val Ala Gly Lys Phe
    370                 375                 380

Leu Phe Lys Asn Gln Thr Leu Glu Leu His Val Leu Tyr Gly Pro Arg
385                 390                 395                 400

Leu Asp Glu Thr Asp Cys Leu Gly Asn Trp Thr Trp Gln Glu Gly Ser
                405                 410                 415

Gln Gln Thr Leu Lys Cys Gln Ala Trp Gly Asn Pro Ser Pro Lys Met
            420                 425                 430

Thr Cys Arg Arg Lys Ala Asp Gly Ala Leu Leu Pro Ile Gly Val Val
        435                 440                 445

Lys Ser Val Lys Gln Glu Met Asn Gly Thr Tyr Val Cys His Ala Phe
    450                 455                 460

Ser Ser His Gly Asn Val Thr Arg Asn Val Tyr Leu Thr Val Leu Tyr
465                 470                 475                 480

His Ser Gln Asn Asn Trp Thr Ile Ile Ile Leu Val Pro Val Leu Leu
                485                 490                 495

Val Ile Val Gly Leu Val Met Ala Ala Ser Tyr Val Tyr Asn Arg Gln
            500                 505                 510

Arg Lys Ile Arg Ile Tyr Lys Leu Gln Lys Ala Gln Glu Glu Ala Ile
        515                 520                 525

Lys Leu Lys Gly Gln Ala Pro Pro Pro
    530                 535


<210> 72
<211> 527
<212> PRT
<213> Cricetulus griseus

<400> 72
Met Ala Pro Thr Arg Ala Arg Pro Thr Pro Pro Leu Leu Leu Ala Leu
1               5                   10                  15

Val Ala Val Val Ile Pro Gly Pro Gly Ser Ala Gln Val Ser Ile His
            20                  25                  30

Pro Lys Glu Ala Phe Leu Pro Arg Gly Ala Ser Met Gln Val Asn Cys
        35                  40                  45

Ser Ser Ser Cys Ser Glu Asn Leu Ser Leu Gly Leu Glu Thr Gln Trp
    50                  55                  60

Pro Lys Val Glu Leu Asp His Gly His Asn Trp Lys Leu Phe Glu Leu
65                  70                  75                  80

Ser Asp Ile Gly Asp Asp Ser Lys Pro Leu Cys Phe Glu Asn Cys Gly
                85                  90                  95

Pro Ile Gln Ser Ser Ala Ser Ala Thr Ile Val Leu Tyr Ser Phe Pro
            100                 105                 110

Glu Arg Val Glu Leu Asp Arg Leu Pro Thr Trp Gln Pro Val Gly Lys
        115                 120                 125

Asn Leu Thr Leu Arg Cys Leu Val Asp Gly Gly Thr Pro Arg Ser Gln
    130                 135                 140

Leu Ser Val Lys Leu Leu Arg Gly Gly Glu Val Leu His Gln Glu Pro
145                 150                 155                 160

Val Gly Val Asp Ser Arg Asn Pro Lys Glu Val Thr Val Thr Val Leu
                165                 170                 175

Ala Ser Arg Asp Asp His Gly Ala Asn Phe Ser Cys Arg Thr Glu Leu
            180                 185                 190

Asp Leu Arg Pro Gln Gly Leu Ala Leu Phe Pro Asn Val Ser Val Ile
        195                 200                 205

Arg Gln Leu Trp Thr Phe Asp Leu Pro Val Thr Glu Pro Lys Leu Asp
    210                 215                 220

Thr Pro Asp Leu Leu Glu Val Gly Thr Val Gln Lys Val Met Cys Ser
225                 230                 235                 240

Leu Gly Gly Leu Phe Pro Ala Ala Glu Ala Arg Ile Thr Leu Glu Leu
                245                 250                 255

Gly Gly His Thr Leu Thr Ser Lys Ser Thr Asn His Arg Asp Leu Val
            260                 265                 270

Ser Ala Thr Ala Leu Val Thr Ala Glu Met Glu Gly Thr Gln Gln Leu
        275                 280                 285

Arg Cys Val Leu Glu Leu Ala Asp Gln Ile Leu Lys Ala Glu Arg Thr
    290                 295                 300

Leu Ser Ile Tyr Asn Phe Ser Ala Pro Val Leu Thr Leu Ser Gln Gln
305                 310                 315                 320

Glu Val Ser Glu Gly Ser Gln Val Thr Val Lys Cys Glu Ala Gln Gly
                325                 330                 335

Gly Ala Gln Val Arg Leu Ser Gly Ala Pro Pro Gly Gln Val Gln Phe
            340                 345                 350

Thr Leu Asn Ala Ser Ser Glu Asp His Glu Arg Ile Phe Thr Cys Ser
        355                 360                 365

Ala Ala Leu Arg Val Ala Gly Gln Glu Leu Leu Lys Asn Gln Thr Leu
    370                 375                 380

Lys Leu His Val Leu Tyr Gly Pro Arg Leu Asp Glu Asn Asp Cys Pro
385                 390                 395                 400

Gly Asn Trp Thr Trp Pro Glu Gly Ser Gln Gln Asn Leu Ser Cys Gln
                405                 410                 415

Ala Phe Gly Asn Pro Pro Pro Lys Leu Thr Cys Ser Arg Lys Thr Asp
            420                 425                 430

Gly Ala Leu Leu Pro Ile Gly Glu Val Lys Thr Val Thr Trp Ala Met
        435                 440                 445

Asn Gly Thr Tyr Val Cys His Ala Val Ser Ser His Gly Asn Ile Thr
    450                 455                 460

Arg Glu Val Phe Leu Lys Val Leu Pro Lys Ser Pro Ile Trp Pro Ile
465                 470                 475                 480

Ile Ile Ile Val Val Ile Leu Ala Thr Val Val Phe Val Gly Val Leu
                485                 490                 495

Thr Ile Tyr Ile Tyr Asn Arg Gln Arg Lys Ile Arg Ile Tyr Lys Leu
            500                 505                 510

Gln Arg Ala Gln Glu Glu Ala Met Lys Leu Lys Val Pro Pro His
        515                 520                 525

<210> 73
<211> 544
<212> PRT
<213> Bos taurus

<400> 73

Met Ile Ala Ser Gly Pro Pro Pro Arg Val Tyr Trp Thr Ser Leu Ile
1               5                   10                  15

Phe Leu Leu Leu Ala Cys Cys Leu Leu Pro Thr Gly Ala Gln Gly Gln
            20                  25                  30

Thr Tyr Gln Val Arg Val Glu Pro Lys Asp Pro Val Val Pro Phe Gly
        35                  40                  45

Glu Pro Leu Val Val Asn Cys Thr Leu Asp Cys Pro Gly Pro Gly Leu
    50                  55                  60

Ile Ser Leu Glu Thr Ala Leu Ser Lys Glu Pro His Ser Arg Gly Leu
65                  70                  75                  80

Gly Trp Ala Ala Phe Arg Leu Thr Asn Val Thr Gly Asp Met Glu Ile
                85                  90                  95

Leu Cys Ser Gly Ile Cys Asn Lys Ser Gln Val Val Gly Phe Ser Asn
            100                 105                 110

Ile Thr Val Phe Gly Phe Pro Lys Arg Val Glu Leu Ala Pro Leu Pro
        115                 120                 125

Leu Trp Gln Pro Val Gly Glu Glu Leu Asn Leu Ser Cys Leu Val Ser
    130                 135                 140

Gly Gly Ala Pro Arg Ala His Leu Ser Val Val Leu Leu Arg Gly Glu
145                 150                 155                 160

Glu Glu Leu Gly Arg Gln Pro Leu Gly Lys Glu Glu Pro Ala Lys Val
                165                 170                 175

Thr Phe Met Val Gln Pro Arg Arg Glu Asp His Gly Thr Asn Phe Ser
            180                 185                 190

Cys Arg Ser Glu Leu Asp Leu Arg Ser Gln Gly Leu Glu Leu Phe Gln
        195                 200                 205

Asn Thr Ser Ala Pro Arg Lys Leu Gln Thr Tyr Ala Met Pro Lys Thr
    210                 215                 220

Ala Pro Arg Leu Val Phe Pro Arg Phe Trp Glu Met Glu Thr Ser Trp
225                 230                 235                 240

Pro Val Asn Cys Ser Leu Asn Gly Leu Phe Pro Ala Ser Glu Ala His
                245                 250                 255

Ile Gln Leu Ala Leu Gly Asn Gln Met Leu Asn Ala Thr Val Val Ser
            260                 265                 270

His Ala Asp Thr Leu Thr Ala Thr Ala Thr Ala Lys Thr Glu Gln Glu
        275                 280                 285

Gly Thr Gln Glu Ile Val Cys Asn Val Thr Leu Gly Val Glu Asn Arg
    290                 295                 300

Glu Thr Arg Glu Ser Leu Val Ala Tyr Arg Phe Gln Gly Pro Asn Leu
305                 310                 315                 320

Asn Leu Ser Glu Ser Asn Ala Thr Glu Gly Thr Pro Val Thr Val Thr
                325                 330                 335

Cys Ala Ala Gly Pro Gln Val Gln Val Met Leu Asp Gly Val Pro Ala
            340                 345                 350

Ala Val Pro Gly Gln Pro Ala Gln Leu Gln Leu Lys Ala Thr Glu Met
        355                 360                 365

Asp Asp Arg Arg Thr Phe Phe Cys Asn Ala Thr Leu Lys Val His Gly
    370                 375                 380

Val Thr Leu His Arg Asn Arg Ser Ile Gln Leu Arg Val Leu Tyr Gly
385                 390                 395                 400

Pro Thr Ile Asp Arg Ala Lys Cys Pro Gln Arg Leu Met Trp Lys Glu
                405                 410                 415

Lys Thr Met His Ile Leu Gln Cys Gln Ala Arg Gly Asn Pro Asn Pro
            420                 425                 430

Gln Leu Gln Cys Leu Arg Glu Gly Ser Lys Phe Lys Val Pro Val Gly
        435                 440                 445

Ile Pro Phe Leu Val Leu Leu Asn Tyr Ser Gly Thr Tyr Ser Cys Gln
    450                 455                 460

Ala Ala Ser Ser Arg Gly Thr Asp Lys Met Leu Val Met Met Asp Val
465                 470                 475                 480

Gln Gly Arg Asn Pro Val Thr Ile Asn Ile Val Leu Gly Val Leu Ala
                485                 490                 495

Ile Leu Gly Leu Val Thr Leu Ala Ala Ala Ser Val Tyr Val Phe Trp
            500                 505                 510

Val Gln Arg Gln His Asp Ile Tyr His Leu Thr Pro Arg Ser Thr Arg
        515                 520                 525

Trp Arg Leu Thr Ser Thr Gln Pro Val Thr Val Ala Glu Glu Leu Ser
    530                 535                 540


<210> 74
<211> 537
<212> PRT
<213> Sus scrofa

<400> 74
Met Ala Pro Gly Ala Thr His Pro Gly Gln Leu Ala Leu Leu Ala Leu
1               5                   10                  15

Leu Leu Pro Leu Leu Gly Ala Leu Leu Pro Gly Leu Gly Gly Ala Glu
            20                  25                  30

Ile Ser Met Trp Pro Leu Asn Thr Ile Ile Pro Lys Gly Gly Ser Met
        35                  40                  45

Lys Val Asn Cys Ser Val Ala Cys Asp Gly Asn Ile Thr Ser Phe Gly
    50                  55                  60

Leu Glu Thr His Trp His Lys Thr Glu Val Asp His Arg Asp Lys Trp
65                  70                  75                  80

Lys Ile Phe Glu Leu Ser Asn Val Glu Asn Asp Gly Thr Leu Leu Cys
                85                  90                  95

His Ala Val Cys Gln Gly Asn Gln Thr Gln Val Gln Gly Asn Leu Thr
            100                 105                 110

Val Tyr Trp Phe Pro Glu Tyr Val Lys Leu Ala Asn Leu Ser Trp Gln
        115                 120                 125

Arg Glu Gly Gln His Phe Asn Leu Ser Cys Gln Val Ser Gly Gly Ala
    130                 135                 140

Pro Arg Thr Asn Leu Ser Ala Val Leu Phe Arg Gly Glu Glu Glu Leu
145                 150                 155                 160

Phe Arg Gln Ser Val Gly Met Glu Glu Pro Ala Asn Val Thr Phe Arg
                165                 170                 175

Met Leu Ala Ser Arg Lys Asp His Gly Ala Asn Phe Ser Cys Arg Thr
            180                 185                 190

Glu Leu Asn Leu Gln Pro Gln Gly Leu Glu Leu Phe Trp Asn Ser Ser
        195                 200                 205

Ala Pro Leu Lys Leu Gln Thr Tyr Val Leu Pro Ala Thr His Pro His
    210                 215                 220

Leu Ala Thr Pro Glu Leu Val Glu Val Gly Thr Pro Val Ser Val Asn
225                 230                 235                 240

Cys Ser Leu Asp Gly Leu Phe Pro Ala Ser Glu Ala Thr Val His Leu
                245                 250                 255

Ala Arg Gly Asp His Arg Pro Pro Leu Thr Ile Thr His Asn Gly Asp
            260                 265                 270

Ser Leu Leu Ala Lys Thr Trp Ile Asn Gly Thr Glu Lys Glu Gln Gly
        275                 280                 285

Thr Gln Tyr Leu Val Cys Glu Ile Met Leu Ala Asp Glu Lys Val Val
    290                 295                 300

Thr Lys Lys Asn Val Thr Phe Tyr Ser Phe Pro Pro Pro Asn Leu Thr
305                 310                 315                 320

Leu Ser Glu Pro Glu Val Ser Glu Gly Thr Thr Val Ser Ile Glu Cys
                325                 330                 335

Gln Ala His Gly Glu Ala Val Val Thr Leu Asn Glu Val Pro Ala Glu
            340                 345                 350

Pro Pro Ser Gln Arg Ala Gln Leu Lys Leu Asn Val Ser Ala Glu Asp
        355                 360                 365

His Gly Arg Ser Phe Ser Cys Ser Ala Ala Leu Thr Val Ala Gly His
    370                 375                 380

Val Leu Tyr Lys Asn Gln Thr Gln Val Leu Ser Val Leu Tyr Gly Pro
385                 390                 395                 400

Arg Leu Asp Glu Arg Asp Cys Pro Gly Asn Trp Thr Trp Pro Glu Gly
                405                 410                 415

Ser His Gln Thr Leu Thr Cys Gln Ala Arg Gly Asn Pro Thr Pro Lys
            420                 425                 430

Leu Ile Cys Arg Arg Glu Gly Asp Gly Ala Leu Leu Pro Thr Gly Asp
        435                 440                 445

Leu Gly Pro Val Lys Arg Glu Ile Thr Gly Thr Tyr Gln Cys Gln Ala
    450                 455                 460

Thr Ser Ser Arg Gly Val Ala Thr Arg Val Val Val Val Asn Val Ile
465                 470                 475                 480

His Asn Gln Asn Asn Met Val Ile Ile Ile Pro Val Ala Ala Val Ala
                485                 490                 495

Ile Leu Gly Ser Val Gly Val Ala Ala Tyr Ile Tyr Asn Tyr Gln Arg
            500                 505                 510

Lys Ile Gln Lys Tyr Glu Leu Gln Lys Ala Gln Glu Asn Ala Ala Met
        515                 520                 525

Lys Leu Ser Thr Pro Ala Ser Pro Pro
    530                 535


<210> 75
<211> 912
<212> PRT
<213> Oryctolagus cuniculus

<400> 75
Met Pro Gly Pro Ser Pro Gly Leu Arg Ala Leu Leu Gly Phe Trp Val
1               5                   10                  15

Ala Leu Gly Leu Gly Ile Leu Arg Leu Ser Ala Val Ala Gln Glu Pro
            20                  25                  30

Phe Trp Ala Asp Leu Gln Pro Arg Val Ala Leu Val Glu Arg Gly Gly
        35                  40                  45

Ser Leu Trp Leu Asn Cys Ser Thr Asn Cys Pro Arg Pro Glu Arg Gly
    50                  55                  60

Gly Leu Glu Thr Ser Leu Arg Arg Asn Gly Pro Glu Gly Leu Arg Trp
65                  70                  75                  80

Arg Ala Arg Gln Leu Val Asp Ile Arg Glu Pro Glu Thr Gln Pro Val
                85                  90                  95

Cys Phe Phe Arg Cys Ala Ala Thr Leu Gln Ala Arg Gly Leu Ile Arg
            100                 105                 110

Thr Phe Gln Arg Pro Asp Arg Val Glu Leu Val Pro Leu Pro Pro Trp
        115                 120                 125

Gln Pro Val Gly Glu Asn Phe Thr Leu Ser Cys Arg Val Pro Gly Ala
    130                 135                 140

Gly Pro Arg Gly Ser Leu Thr Leu Thr Leu Leu Arg Gly Ala Gln Glu
145                 150                 155                 160

Leu Ile Arg Arg Ser Phe Ala Gly Glu Pro Ala Arg Ala Arg Gly Ala
                165                 170                 175

Val Leu Thr Ala Thr Val Leu Ala Arg Arg Glu Asp His Gly Ala Asn
            180                 185                 190

Phe Ser Cys Arg Ala Glu Leu Asp Leu Arg Pro Gln Gly Leu Ala Leu
        195                 200                 205

Phe Glu Asn Ser Ser Ala Pro Arg Gln Leu Trp Thr Tyr Ala Leu Pro

210                 215                 220

Leu Asp Ser Pro Arg Leu Leu Ala Pro Arg Val Leu Glu Val Asp Ser
225                 230                 235                 240

Gln Ser Leu Val Ser Cys Thr Leu Asp Gly Leu Phe Pro Ala Ser Glu
                245                 250                 255

Ala Gly Val His Leu Ala Leu Gly Asp Lys Arg Leu Asn Pro Glu Val
            260                 265                 270

Thr Leu Glu Gly Asp Ala Ile Val Ala Thr Ala Thr Ala Thr Ala Glu
        275                 280                 285

Glu Glu Gly Ile Lys Gln Leu Val Cys Ala Val Thr Leu Gly Gly Glu
    290                 295                 300

Arg Arg Glu Ser Arg Glu Asn Val Thr Val Tyr Ser Phe Pro Ala Pro
305                 310                 315                 320

Leu Leu Thr Leu Ser Glu Pro Ser Ala Pro Glu Gly Lys Leu Val Thr
                325                 330                 335

Val Thr Cys Thr Ala Gly Ala Arg Ala Leu Val Thr Leu Glu Gly Val
            340                 345                 350

Pro Ala Ala Ala Pro Gly Gln Pro Ala Gln Leu Gln Phe Asn Ala Ser
        355                 360                 365

Glu Ser Asp Asp Gly Arg Ser Phe Phe Cys Asp Ala Thr Leu Glu Leu
    370                 375                 380

Asp Gly Glu Thr Leu Ser Lys Asn Gly Ser Ala Glu Leu Arg Val Leu
385                 390                 395                 400

Tyr Ala Pro Arg Leu Asp Asp Ala Asp Cys Pro Arg Ser Trp Thr Trp
                405                 410                 415

Pro Glu Gly Pro Glu Gln Thr Leu Arg Cys Glu Ala Arg Gly Asn Pro
            420                 425                 430

Thr Pro Ala Val His Cys Ala Arg Ser Asp Gly Gly Ala Val Leu Ala
        435                 440                 445

Leu Gly Leu Leu Gly Pro Val Thr Arg Ala Leu Ala Gly Thr Tyr Arg
    450                 455                 460

Cys Thr Ala Ala Asn Val Gln Gly Glu Ala Val Lys Asp Val Thr Leu
465                 470                 475                 480

Thr Val Glu Tyr Ala Pro Ala Leu Asp Ser Val Gly Cys Pro Glu Arg
                485                 490                 495

Val Thr Trp Leu Glu Gly Thr Glu Ala Ser Leu Ser Cys Val Ala His
            500                 505                 510

Gly Val Pro Pro Pro Ser Val Ser Cys Val Arg Phe Arg Gln Ala Asp
        515                 520                 525

Val Ile Glu Gly Leu Leu Leu Val Ala Arg Glu His Ala Gly Thr Tyr
    530                 535                 540

Arg Cys Glu Ala Ile Asn Ala Arg Ala Leu Ala Lys Asn Val Ala Val
545                 550                 555                 560

Thr Val Glu Tyr Gly Pro Ser Phe Glu Glu Arg Ser Cys Pro Ser Asn
                565                 570                 575

Trp Thr Trp Val Glu Gly Ser Glu Gln Leu Phe Ser Cys Glu Val Glu
            580                 585                 590

Gly Lys Pro Gln Pro Ser Val Gln Cys Val Gly Ser Glu Gly Ala Ser
        595                 600                 605

Glu Gly Leu Leu Leu Pro Leu Ala Pro Leu Asn Pro Ser Pro Ser Asp
    610                 615                 620

Pro Ser Val Pro Arg Asp Leu Ala Pro Gly Ile Tyr Val Cys Asn Ala
625                 630                 635                 640

Thr Asn Pro Leu Gly Ser Ala Val Lys Thr Val Val Val Ser Ala Glu
                645                 650                 655

Ser Pro Pro Gln Met Asp Asp Ser Thr Cys Pro Ser Asp Gln Thr Trp
            660                 665                 670

Leu Glu Gly Ala Glu Ala Ala Gly Pro Ala Cys Ala Arg Gly Arg Pro
        675                 680                 685

Ser Pro Arg Val Arg Cys Ser Arg Glu Gly Ala Pro Arg Pro Ala Arg
    690                 695                 700

Pro Arg Val Ser Arg Glu Asp Ala Gly Thr Tyr Leu Cys Val Ala Thr
705                 710                 715                 720

Asn Ala His Gly Ser Asp Ser Arg Thr Val Thr Val Gly Val Glu Tyr
                725                 730                 735

Arg Pro Val Val Ala Glu Leu Ala Ala Ser Pro Ser Gly Gly Val Arg
            740                 745                 750

Pro Gly Gly Asn Phe Thr Leu Thr Cys Arg Ala Glu Ala Trp Pro Pro
        755                 760                 765

Ala Gln Ile Ser Trp Arg Ala Pro Pro Gly Ala Pro Asn Ile Gly Leu
    770                 775                 780

Ser Ser Asn Asn Ser Thr Leu Ser Val Pro Gly Ala Met Gly Ser His
785                 790                 795                 800

Gly Gly Glu Tyr Glu Cys Glu Ala Thr Asn Ala His Gly His Ala Arg
                805                 810                 815

Arg Ile Thr Val Arg Val Ala Gly Pro Trp Leu Trp Ile Ala Val Gly
            820                 825                 830

Gly Ala Val Gly Gly Ala Val Leu Leu Ala Ala Gly Ala Gly Leu Ala
        835                 840                 845

Phe Tyr Val Gln Ser Thr Ala Cys Lys Lys Gly Glu Tyr Asn Val Gln
    850                 855                 860

Glu Ala Glu Ser Ser Gly Glu Ala Val Cys Leu Asn Gly Ala Gly Gly
865                 870                 875                 880

Gly Ala Gly Ser Gly Ala Glu Gly Gly Pro Glu Ala Glu Asp Ser Ala
                885                 890                 895

Glu Ser Pro Ala Gly Gly Glu Val Phe Ala Ile Gln Leu Thr Ser Ala
            900                 905                 910


<210> 76
<211> 917
<212> PRT
<213> Mus musculus

<400> 76
Met Pro Gly Pro Ser Pro Gly Leu Arg Arg Ala Leu Leu Gly Leu Trp
1               5                   10                  15

Ala Ala Leu Gly Leu Gly Ile Leu Gly Ile Ser Ala Val Ala Leu Glu
            20                  25                  30

Pro Phe Trp Ala Asp Leu Gln Pro Arg Val Ala Leu Val Glu Pro Gly
        35                  40                  45

Gly Ser Leu Trp Leu Asn Cys Ser Thr Asn Cys Pro Arg Pro Glu Arg
    50                  55                  60

Gly Gly Leu Glu Thr Ser Leu Arg Arg Asn Gly Thr Gln Arg Gly Leu
65                  70                  75                  80

Arg Trp Leu Ala Arg Gln Leu Val Asp Ile Arg Glu Pro Glu Thr Gln
                85                  90                  95

Pro Val Cys Phe Phe Arg Cys Ala Arg Arg Thr Leu Gln Ala Arg Gly
            100                 105                 110

Leu Ile Arg Thr Phe Gln Arg Pro Asp Arg Val Glu Leu Val Pro Leu
        115                 120                 125

Pro Ser Trp Gln Pro Val Gly Glu Asn Phe Thr Leu Ser Cys Arg Val
    130                 135                 140

Pro Gly Ala Gly Pro Arg Ala Ser Leu Thr Leu Thr Leu Leu Arg Gly
145                 150                 155                 160

Gly Gln Glu Leu Ile Arg Arg Ser Phe Val Gly Glu Pro Pro Arg Ala
                165                 170                 175

Arg Gly Ala Met Leu Thr Ala Arg Val Leu Ala Arg Arg Glu Asp His

180                 185                 190

Arg Val Asn Phe Ser Cys Leu Ala Glu Leu Asp Leu Arg Pro His Gly
        195                 200                 205

Leu Gly Leu Phe Ala Asn Ser Ser Ala Pro Arg Gln Leu Arg Thr Phe
    210                 215                 220

Ala Met Pro Pro His Ser Pro Ser Leu Ile Ala Pro Arg Val Leu Glu
225                 230                 235                 240

Val Asp Ser Glu Arg Pro Val Thr Cys Thr Leu Asp Gly Leu Phe Pro
                245                 250                 255

Ala Pro Glu Ala Gly Val Tyr Leu Ser Leu Gly Asp Gln Arg Leu Asn
            260                 265                 270

Pro Asn Val Thr Leu Asp Gly Asp Ser Leu Val Ala Thr Ala Thr Ala
        275                 280                 285

Thr Ala Ser Ala Glu Gln Glu Gly Thr Lys Gln Leu Met Cys Val Val
    290                 295                 300

Thr Leu Gly Gly Glu Thr Arg Glu Thr Gln Glu Asn Leu Thr Val Tyr
305                 310                 315                 320

Ser Phe Pro Thr Pro Leu Leu Thr Leu Ser Glu Pro Glu Ala Pro Glu
                325                 330                 335

Gly Lys Met Val Thr Ile Ser Cys Trp Ala Gly Ala Arg Ala Leu Val
            340                 345                 350

Thr Leu Glu Gly Ile Pro Ala Ala Val Pro Gly Gln Pro Ala Glu Leu
        355                 360                 365

Gln Leu Asn Val Thr Lys Asn Asp Asp Lys Arg Gly Phe Phe Cys Asp
    370                 375                 380

Ala Ala Leu Asp Val Asp Gly Glu Thr Leu Arg Lys Asn Gln Ser Ser
385                 390                 395                 400

Glu Leu Arg Val Leu Tyr Ala Pro Arg Leu Asp Asp Leu Asp Cys Pro
                405                 410                 415

Arg Ser Trp Thr Trp Pro Glu Gly Pro Glu Gln Thr Leu His Cys Glu
            420                 425                 430

Ala Arg Gly Asn Pro Glu Pro Ser Val His Cys Ala Arg Pro Glu Gly
        435                 440                 445

Gly Ala Val Leu Ala Leu Gly Leu Leu Gly Pro Val Thr Arg Ala Leu
    450                 455                 460

Ala Gly Thr Tyr Arg Cys Thr Ala Val Asn Gly Gln Gly Gln Ala Val
465                 470                 475                 480

Lys Asp Val Thr Leu Thr Val Glu Tyr Ala Pro Ala Leu Asp Ser Val
                485                 490                 495

Gly Cys Pro Glu His Ile Thr Trp Leu Glu Gly Thr Glu Ala Ser Leu
            500                 505                 510

Ser Cys Val Ala Pro Gly Val Pro Pro Pro Ser Val Ser Cys Val Arg
        515                 520                 525

Ser Gly Lys Glu Glu Val Met Glu Gly Pro Leu Arg Val Ala Arg Glu
    530                 535                 540

His Ala Gly Thr Tyr Arg Cys Glu Ala Ile Asn Ala Arg Gly Ser Ala
545                 550                 555                 560

Ala Lys Asn Val Ala Val Thr Val Glu Tyr Gly Pro Ser Phe Glu Glu
                565                 570                 575

Leu Gly Cys Pro Ser Asn Trp Thr Trp Val Glu Gly Ser Gly Lys Leu
            580                 585                 590

Phe Ser Cys Glu Val Asp Gly Lys Pro Glu Pro Arg Val Glu Cys Val
        595                 600                 605

Gly Ser Glu Gly Ala Ser Glu Gly Ile Val Leu Pro Leu Val Ser Ser
    610                 615                 620

Asn Ser Gly Pro Arg Asn Ser Met Thr Pro Gly Asn Leu Ser Pro Gly
625                 630                 635                 640

Ile Tyr Leu Cys Asn Ala Thr Asn Arg His Gly Ser Thr Val Lys Thr
                645                 650                 655

Val Val Val Ser Ala Glu Ser Pro Pro Gln Met Asp Glu Ser Ser Cys
            660                 665                 670

Pro Ser His Gln Thr Trp Leu Glu Gly Ala Glu Ala Thr Ala Leu Ala
        675                 680                 685

Cys Ser Ala Arg Gly Arg Pro Ser Pro Arg Val His Cys Ser Arg Glu
    690                 695                 700

Gly Ala Ala Arg Leu Glu Arg Leu Gln Val Ser Arg Glu Asp Ala Gly
705                 710                 715                 720

Thr Tyr Arg Cys Val Ala Thr Asn Ala His Gly Thr Asp Ser Arg Thr
                725                 730                 735

Val Thr Val Gly Val Glu Tyr Arg Pro Val Val Ala Glu Leu Ala Ala
            740                 745                 750

Ser Pro Pro Ser Val Arg Pro Gly Gly Asn Phe Thr Leu Thr Cys Arg
        755                 760                 765

Ala Glu Ala Trp Pro Pro Ala Gln Ile Ser Trp Arg Ala Pro Pro Gly
    770                 775                 780

Ala Leu Asn Leu Gly Leu Ser Ser Asn Asn Ser Thr Leu Ser Val Ala
785                 790                 795                 800

Gly Ala Met Gly Ser His Gly Gly Glu Tyr Glu Cys Ala Ala Thr Asn
                805                 810                 815

Ala His Gly Arg His Ala Arg Arg Ile Thr Val Arg Val Ala Gly Pro
            820                 825                 830

Trp Leu Trp Val Ala Val Gly Gly Ala Ala Gly Gly Ala Ala Leu Leu
        835                 840                 845

Ala Ala Gly Ala Gly Leu Ala Phe Tyr Val Gln Ser Thr Ala Cys Lys
    850                 855                 860

Lys Gly Glu Tyr Asn Val Gln Glu Ala Glu Ser Ser Gly Glu Ala Val
865                 870                 875                 880

Cys Leu Asn Gly Ala Gly Gly Thr Pro Gly Ala Glu Gly Gly Ala Glu
                885                 890                 895

Thr Pro Gly Thr Ala Glu Ser Pro Ala Asp Gly Glu Val Phe Ala Ile
            900                 905                 910

Gln Leu Thr Ser Ser
        915


<210> 77
<211> 548
<212> PRT
<213> Mus musculus

<400> 77
Met Lys Met Leu Leu Leu Gly Val Trp Thr Leu Leu Ala Leu Ile Pro
1               5                   10                  15

Cys Pro Gly Ala Ala Glu Glu Leu Phe Gln Val Ser Val His Pro Asn
            20                  25                  30

Glu Ala Leu Val Glu Phe Gly His Ser Leu Thr Val Asn Cys Ser Thr
        35                  40                  45

Thr Cys Pro Asp Pro Gly Pro Ser Gly Ile Glu Thr Phe Leu Lys Lys
    50                  55                  60

Thr Gln Leu Ser Lys Gly Ser Gln Trp Lys Glu Phe Leu Leu Glu Asp
65                  70                  75                  80

Ile Thr Glu Asp Leu Val Leu Gln Cys Phe Phe Ser Cys Ala Gly Glu
                85                  90                  95

Gln Lys Asp Thr Val Leu Ala Ile Thr Met Tyr Gln Pro Pro Glu Gln
            100                 105                 110

Val Ile Leu Asp Leu Gln Pro Glu Trp Val Ala Val Asp Glu Ala Phe
        115                 120                 125

Thr Val Thr Cys His Val Pro Ser Val Ala Pro Leu Gln Ser Leu Thr

130                 135                 140

Leu Thr Leu Leu Gln Gly Asp Gln Glu Leu His Arg Lys Asp Phe Leu
145                 150                 155                 160

Ser Leu Ser Leu Val Ser Gln Arg Ala Glu Val Thr Ala Thr Val Arg
                165                 170                 175

Ala His Arg Asp Asn Asp Arg Arg Asn Phe Ser Cys Arg Ala Glu Leu
            180                 185                 190

Asp Leu Ser Pro His Gly Gly Gly Leu Phe His Gly Ser Ser Ala Thr
        195                 200                 205

Lys Gln Leu Arg Ile Phe Glu Phe Ser Gln Asn Pro Gln Ile Trp Val
    210                 215                 220

Pro Ser Leu Leu Glu Val Gly Lys Ala Glu Ile Val Ser Cys Glu Val
225                 230                 235                 240

Thr Arg Val Phe Pro Ala Gln Glu Ala Val Phe Arg Met Phe Leu Glu
                245                 250                 255

Asp Gln Glu Leu Ser Pro Phe Ser Ser Trp Arg Glu Asp Ala Ala Trp
            260                 265                 270

Ala Ser Ala Thr Ile Gln Ala Met Glu Thr Gly Asp Gln Glu Leu Thr
        275                 280                 285

Cys Leu Val Ser Leu Gly Pro Val Glu Gln Lys Thr Arg Lys Pro Val
    290                 295                 300

Tyr Val Tyr Ser Phe Pro Pro Pro Ile Leu Glu Ile Glu Asp Ala Tyr
305                 310                 315                 320

Pro Leu Ala Gly Thr Asp Val Asn Val Thr Cys Ser Gly His Val Leu
                325                 330                 335

Thr Ser Pro Ser Pro Thr Leu Arg Leu Gln Gly Ser Leu Asn His Ser
            340                 345                 350

Ala Pro Gly Lys Pro Ala Trp Leu Leu Phe Thr Ala Arg Glu Glu Asp
        355                 360                 365

Asp Gly Arg Thr Leu Ser Cys Glu Ala Ser Leu Glu Val Gln Gly Gln
    370                 375                 380

Arg Leu Val Arg Thr Thr Glu Ser Gln Leu His Val Leu Tyr Lys Pro
385                 390                 395                 400

Arg Phe Gln Glu Ser Arg Cys Pro Gly Asn Gln Ile Trp Val Glu Gly
                405                 410                 415

Met His Gln Met Leu Ala Cys Ile Pro Glu Gly Asn Pro Thr Pro Val
            420                 425                 430

Leu Val Cys Val Trp Asn Gly Met Ile Phe Asp Leu Asp Val Pro Gln
        435                 440                 445

Lys Ala Thr Gln Asn His Thr Gly Thr Tyr Cys Cys Thr Ala Thr Asn
    450                 455                 460

Pro Leu Gly Ser Val Ser Lys Asp Ile Thr Ile Ile Val Gln Gly Leu
465                 470                 475                 480

Pro Glu Gly Ile Ser Ser Ser Thr Ile Phe Ile Ile Ile Ile Phe Thr
                485                 490                 495

Leu Gly Met Ala Val Ile Thr Val Ala Leu Tyr Leu Asn Tyr Gln Pro
            500                 505                 510

Cys Lys Gly Asn Ser Arg Lys Arg Met His Arg Pro Arg Glu Gln Ser
        515                 520                 525

Lys Gly Glu Glu Ser Gln Phe Ser Asp Ile Arg Ala Glu Glu Cys His
    530                 535                 540

Ala His Leu Cys
545


<210> 78
<211> 548
<212> PRT
<213> Rattus norvegicus

<400> 78
Met Lys Met Leu Leu Leu Gly Ile Trp Thr Leu Leu Ala Leu Ile Pro
1               5                   10                  15

Cys Pro Gly Thr Thr Glu Val Leu Phe Gln Val Ser Val His Pro Asn
            20                  25                  30

Gln Ala Leu Val Glu Phe Gly His Ser Leu Thr Ile Asn Cys Ser Thr
        35                  40                  45

Thr Cys Pro Asp Pro Gly Pro Ser Gly Ile Glu Thr Phe Leu Lys Lys
    50                  55                  60

Thr Gln Leu Ser Lys Gly Ser Gln Trp Lys Glu Phe Leu Leu Glu Gly
65                  70                  75                  80

Ile Thr Glu Asn Ser Val Leu Gln Cys Phe Phe Ser Cys Ala Gly Val
                85                  90                  95

Gln Lys Asp Thr Ala Leu Asp Ile Thr Met Tyr Gln Pro Pro Glu Gln
            100                 105                 110

Val Ile Leu Asp Leu Gln Pro Glu Trp Val Ala Ile Asp Glu Ala Phe
        115                 120                 125

Thr Val Lys Cys His Val Pro Ser Val Ala Pro Leu Gln Ser Leu Thr
    130                 135                 140

Leu Thr Leu Leu Gln Gly Asp Gln Glu Leu His Arg Lys Asp Phe Leu
145                 150                 155                 160

Ser Leu Ser Leu Val Ser Gln Arg Ala Glu Val Thr Val Asn Val Arg
                165                 170                 175

Ala Gln Arg Glu Asn Asp Arg His Asn Phe Ser Cys Arg Ala Glu Leu
            180                 185                 190

Asp Leu Ser Pro His Gly Gly Gly Leu Phe His Gly Ser Ser Ala Thr
        195                 200                 205

Lys Gln Leu Arg Ile Phe Glu Phe Ser Gln Asn Pro Gln Ile Leu Val
    210                 215                 220

Pro Ser Leu Leu Glu Val Gly Met Ala Glu Thr Met Ser Cys Glu Val
225                 230                 235                 240

Val Arg Val Phe Pro Ala Gln Glu Ala Val Phe Arg Met Phe Leu Glu
                245                 250                 255

Asp Gln Glu Leu Ser Pro Phe Ser Ser Trp Lys Gly Asp Ala Ala Trp
            260                 265                 270

Ala Ser Ala Thr Ile Gln Ala Met Glu Thr Gly Asp Gln Glu Leu Thr
        275                 280                 285

Cys Leu Val Ser Val Gly Pro Val Glu Gln Lys Ala Arg Lys Pro Val
    290                 295                 300

His Val Tyr Ser Phe Pro Pro Pro Val Leu Glu Ile Glu Asp Ala Tyr
305                 310                 315                 320

Pro Gln Ala Gly Thr Asp Val Asn Val Thr Cys Ser Gly His Val Leu
                325                 330                 335

Thr Ser Pro Ser Pro Thr Leu Arg Leu Gln Gly Ser Leu Asn Leu Ser
            340                 345                 350

Ala Pro Gly Glu Pro Ala Trp Leu Arg Phe Thr Ala Arg Glu Glu Asp
        355                 360                 365

Asp Gly Arg Thr Leu Ser Cys Glu Ala Ser Leu Val Val Gln Gly Gln
    370                 375                 380

Arg Leu Val Lys Thr Thr Lys Ile Gln Leu His Val Leu Tyr Lys Pro
385                 390                 395                 400

Arg Phe Gln Glu Ser Asp Cys Pro Gly Asn Gln Ile Trp Val Glu Gly
                405                 410                 415

Met Asp Gln Met Leu Ala Cys Ile Pro Glu Gly Asn Pro Ile Pro Ala
            420                 425                 430

Leu Val Cys Ile Trp Asn Gly Met Thr Phe Asp Leu Glu Val Pro Gln
        435                 440                 445

Lys Ala Thr Gln Asn His Thr Gly Thr Tyr Ser Cys Thr Ala Thr Asn

450                 455                 460

Ser Leu Gly Ser Val Ser Lys Asp Ile Ala Val Leu Val Gln Gly Leu
465                 470                 475                 480

His Glu Gly Ile Ser Ser Ser Thr Ile Phe Ile Ile Ile Ile Phe Thr
                485                 490                 495

Leu Gly Met Ala Val Ile Thr Ile Ala Leu Tyr Leu Asn Tyr Gln Pro
            500                 505                 510

Cys Lys Arg Asn Gly Arg Lys Arg Thr His Arg Gln Lys Glu Gln Asn
        515                 520                 525

Lys Gly Gly Glu Arg Gln Phe Ser Asp Ile Gln Ala Glu Glu Cys His
    530                 535                 540

Ala His Leu Cys
545


<210> 79
<211> 396
<212> PRT
<213> Mus musculus

<400> 79
Met Gly Ala Pro Ser Ala Leu Pro Leu Leu Leu Leu Leu Ala Cys Ser
1               5                   10                  15

Trp Ala Pro Gly Gly Ala Asn Leu Ser Gln Asp Asp Ser Gln Pro Trp
            20                  25                  30

Thr Ser Asp Glu Thr Val Val Ala Gly Gly Thr Val Val Leu Lys Cys
        35                  40                  45

Gln Val Lys Asp His Glu Asp Ser Ser Leu Gln Trp Ser Asn Pro Ala
    50                  55                  60

Gln Gln Thr Leu Tyr Phe Gly Glu Lys Arg Ala Leu Arg Asp Asn Arg
65                  70                  75                  80

Ile Gln Leu Val Ser Ser Thr Pro His Glu Leu Ser Ile Ser Ile Ser
                85                  90                  95

Asn Val Ala Leu Ala Asp Glu Gly Glu Tyr Thr Cys Ser Ile Phe Thr
            100                 105                 110

Met Pro Val Arg Thr Ala Lys Ser Leu Val Thr Val Leu Gly Ile Pro
        115                 120                 125

Gln Lys Pro Ile Ile Thr Gly Tyr Lys Ser Ser Leu Arg Glu Lys Glu
    130                 135                 140

Thr Ala Thr Leu Asn Cys Gln Ser Ser Gly Ser Lys Pro Ala Ala Gln
145                 150                 155                 160

Leu Thr Trp Arg Lys Gly Asp Gln Glu Leu His Gly Asp Gln Thr Arg
                165                 170                 175

Ile Gln Glu Asp Pro Asn Gly Lys Thr Phe Thr Val Ser Ser Ser Val
            180                 185                 190

Ser Phe Gln Val Thr Arg Glu Asp Asp Gly Ala Asn Ile Val Cys Ser
        195                 200                 205

Val Asn His Glu Ser Leu Lys Gly Ala Asp Arg Ser Thr Ser Gln Arg
    210                 215                 220

Ile Glu Val Leu Tyr Thr Pro Thr Ala Met Ile Arg Pro Glu Pro Ala
225                 230                 235                 240

His Pro Arg Glu Gly Gln Lys Leu Leu Leu His Cys Glu Gly Arg Gly
                245                 250                 255

Asn Pro Val Pro Gln Gln Tyr Val Trp Val Lys Glu Gly Ser Glu Pro
            260                 265                 270

Pro Leu Lys Met Thr Gln Glu Ser Ala Leu Ile Phe Pro Phe Leu Asn
        275                 280                 285

Lys Ser Asp Ser Gly Thr Tyr Gly Cys Thr Ala Thr Ser Asn Met Gly
    290                 295                 300

Ser Tyr Thr Ala Tyr Phe Thr Leu Asn Val Asn Asp Pro Ser Pro Val
305                 310                 315                 320

Pro Ser Ser Ser Ser Thr Tyr His Ala Ile Ile Gly Gly Ile Val Ala
                325                 330                 335

Phe Ile Val Phe Leu Leu Leu Ile Leu Leu Ile Phe Leu Gly His Tyr
            340                 345                 350

Leu Ile Arg His Lys Gly Thr Tyr Leu Thr His Glu Ala Lys Gly Ser
        355                 360                 365

Asp Asp Ala Pro Asp Ala Asp Thr Ala Ile Ile Asn Ala Glu Gly Gly
    370                 375                 380

Gln Ser Gly Gly Asp Asp Lys Lys Glu Tyr Phe Ile
385                 390                 395

<210> 80
<211> 662
<212> PRT
<213> Homo sapiens

<400> 80


<210> 81
<211> 505
<212> PRT
<213> Pan troglodytes

<400> 81
Gln Thr Ser Val Ser Pro Pro Lys Val Ile Leu Pro Arg Gly Gly Ser
1               5                   10                  15

Val Gln Val Thr Cys Ser Thr Ser Cys Asp Gln Pro Asp Leu Leu Gly
            20                  25                  30

Ile Glu Thr Pro Leu Pro Lys Lys Glu Leu Leu Leu Gly Gly Asn Asn
        35                  40                  45

Trp Lys Val Tyr Glu Leu Ser Asn Val Gln Glu Asp Ser Gln Pro Met
    50                  55                  60

Cys Tyr Ser Asn Cys Pro Asp Gly Gln Ser Thr Ala Lys Thr Phe Leu
65                  70                  75                  80

Thr Val Tyr Trp Thr Pro Glu Arg Val Glu Leu Ala Pro Leu Pro Ser

85                  90                  95

Trp Gln Pro Val Gly Lys Asp Leu Thr Leu Arg Cys Gln Val Glu Gly
            100                 105                 110

Gly Ala Pro Arg Ala Asn Leu Thr Val Val Leu Leu Arg Gly Glu Lys
        115                 120                 125

Glu Leu Lys Arg Glu Pro Ala Val Gly Glu Pro Ala Glu Val Thr Thr
    130                 135                 140

Thr Val Leu Val Glu Arg Asp His His Gly Ala Asn Phe Ser Cys Arg
145                 150                 155                 160

Thr Glu Leu Asp Leu Arg Pro Gln Gly Leu Gln Leu Phe Glu Asn Thr
                165                 170                 175

Ser Ala Pro His Gln Leu Gln Thr Phe Val Leu Pro Ala Thr Pro Pro
            180                 185                 190

Gln Leu Val Ser Pro Arg Val Leu Glu Val Asp Thr Gln Gly Thr Val
        195                 200                 205

Val Cys Ser Leu Asp Gly Leu Phe Pro Val Leu Glu Ala Gln Val His
    210                 215                 220

Leu Ala Leu Gly Asp Gln Arg Leu Asn Pro Thr Val Thr Tyr Gly Asn
225                 230                 235                 240

Asp Ser Phe Ser Ala Lys Ala Ser Val Ser Val Thr Ala Glu Asp Glu
                245                 250                 255

Gly Thr Gln Arg Leu Thr Cys Ala Val Ile Leu Gly Asn Gln Ser Arg
            260                 265                 270

Glu Thr Leu Gln Thr Val Thr Ile Tyr Ser Phe Pro Ala Pro Asn Val
        275                 280                 285

Ile Leu Thr Lys Pro Glu Val Ser Glu Gly Thr Glu Val Thr Val Lys
    290                 295                 300

Cys Glu Ala His Pro Arg Ala Lys Val Thr Leu Asn Gly Val Pro Ala
305                 310                 315                 320

Gln Pro Val Gly Pro Arg Val Gln Leu Leu Leu Lys Ala Thr Pro Glu
                325                 330                 335

Asp Asn Gly Arg Ser Phe Ser Cys Ser Ala Thr Leu Glu Val Ala Gly
            340                 345                 350

Gln Leu Ile His Lys Asn Gln Thr Arg Glu Leu Arg Val Leu Tyr Gly
        355                 360                 365

Pro Arg Leu Asp Glu Arg Asp Cys Pro Gly Asn Trp Thr Trp Pro Glu
    370                 375                 380

Asn Ser Gln Gln Thr Pro Met Cys Gln Ala Ser Gly Asn Pro Leu Pro
385                 390                 395                 400

Glu Leu Lys Cys Leu Lys Asp Gly Thr Phe Pro Leu Pro Val Gly Glu
                405                 410                 415

Ser Val Thr Val Thr Arg Asp Leu Glu Gly Thr Tyr Leu Cys Arg Ala
            420                 425                 430

Arg Ser Thr Gln Gly Glu Val Thr Arg Lys Val Thr Val Asn Val Leu
        435                 440                 445

Ser Pro Arg Tyr Glu Ile Val Ile Ile Thr Val Val Ala Ala Ala Val
    450                 455                 460

Ile Met Gly Thr Ala Gly Leu Ser Thr Tyr Leu Tyr Asn Arg Gln Arg
465                 470                 475                 480

Lys Ile Arg Lys Tyr Arg Leu Gln Gln Ala Gln Lys Gly Thr Pro Met
                485                 490                 495

Lys Pro Asn Thr Gln Ala Thr Pro Pro
            500                 505

<210> 82
<211> 447
<212> PRT
<213> Mus musculus

<220>
<221> MOD_RES
<222> (12)
<223> Any amino acid

<220>
<221> MOD_RES
<222> (77)..(80)
<223> Any amino acid

<220>
<221> MOD_RES
<222> (145)..(147)
<223> Any amino acid

<220>
<221> MOD_RES
<222> (155)..(163)
<223> Any amino acid

<220>
<221> MOD_RES
<222> (268)..(269)
<223> Any amino acid

<220>
<221> MOD_RES
<222> (279)
<223> Any amino acid

<220>
<221> MOD_RES
<222> (282)
<223> Any amino acid

<400> 82
Glu Asp Ser Gln Pro Met Cys Tyr Ser Asn Cys Xaa Asp Gly Gln Ser
1               5                   10                  15

Thr Ala Lys Thr Phe Leu Thr Val Tyr Trp Thr Pro Glu Arg Val Glu
            20                  25                  30

Leu Ala Pro Leu Pro Ser Trp Gln Pro Val Gly Lys Asn Leu Thr Leu
        35                  40                  45

Arg Cys Gln Val Glu Gly Gly Ala Pro Arg Ala Asn Leu Thr Val Val
    50                  55                  60

Leu Leu Arg Gly Glu Lys Glu Leu Lys Arg Glu Pro Xaa Xaa Xaa Xaa
65                  70                  75                  80

Pro Ala Glu Val Thr Thr Thr Val Leu Val Arg Arg Asp His His Gly
                85                  90                  95

Ala Asn Phe Ser Cys Arg Thr Glu Leu Asp Leu Arg Pro Gln Gly Leu
            100                 105                 110

Glu Leu Phe Glu Asn Thr Ser Ala Pro Tyr Gln Leu Gln Thr Phe Val
        115                 120                 125

Leu Pro Ala Thr Pro Pro Gln Leu Val Ser Pro Arg Val Leu Glu Val
    130                 135                 140

Xaa Xaa Xaa Gly Thr Val Val Cys Ser Leu Xaa Xaa Xaa Xaa Xaa Xaa
145                 150                 155                 160

Xaa Xaa Xaa Gln Val His Leu Ala Leu Gly Asp Gln Arg Leu Asn Pro
                165                 170                 175

Thr Val Thr Tyr Gly Asn Asp Ser Phe Ser Ala Lys Ala Ser Val Ser
            180                 185                 190

Val Thr Ala Glu Asp Glu Gly Thr Gln Arg Leu Thr Cys Ala Val Ile
        195                 200                 205

Leu Gly Asn Gln Ser Gln Glu Thr Leu Gln Thr Val Thr Ile Tyr Ser
    210                 215                 220

Phe Pro Ala Pro Asn Val Ile Leu Thr Lys Pro Glu Val Ser Glu Gly
225                 230                 235                 240

Thr Glu Val Thr Val Lys Cys Glu Ala His Pro Arg Ala Lys Val Thr
                245                 250                 255

Leu Asn Gly Val Pro Ala Gln Pro Leu Gly Pro Xaa Xaa Gln Leu Leu
            260                 265                 270

Leu Lys Ala Thr Pro Glu Xaa Asn Gly Xaa Ser Phe Ser Cys Ser Ala
        275                 280                 285

Thr Leu Glu Val Ala Gly Gln Leu Ile His Lys Asn Gln Thr Arg Glu
    290                 295                 300

Leu Arg Val Leu Tyr Gly Pro Arg Leu Asp Glu Arg Asp Cys Pro Gly
305                 310                 315                 320

Asn Trp Thr Trp Pro Glu Asn Ser Gln Gln Thr Pro Met Cys Gln Ala
                325                 330                 335

Trp Gly Asn Pro Leu Pro Glu Leu Lys Cys Leu Lys Asp Gly Thr Phe
            340                 345                 350

Pro Leu Pro Ile Gly Glu Ser Val Thr Val Thr Arg Asp Leu Glu Gly
        355                 360                 365

Thr Tyr Leu Cys Arg Ala Arg Ser Thr Gln Gly Glu Val Thr Arg Glu
    370                 375                 380

Val Thr Val Asn Val Leu Ser Pro Arg Tyr Glu Ile Val Ile Ile Thr
385                 390                 395                 400

Val Val Ala Ala Ala Val Ile Met Gly Thr Ala Gly Leu Ser Thr Tyr
                405                 410                 415

Leu Tyr Asn Arg Gln Arg Lys Ile Lys Lys Tyr Arg Leu Gln Gln Ala
            420                 425                 430

Gln Lys Gly Thr Pro Met Lys Pro Asn Thr Gln Ala Thr Pro Pro
        435                 440                 445


<210> 83
<211> 528
<212> PRT
<213> Canis familiaris

<400> 83
Ala Pro Ala Leu Pro Arg Leu Pro Ala Leu Leu Ala Leu Leu Gly Ala
1               5                   10                  15

Leu Leu Pro Gly Leu Gly Gly Ala Gln Thr Ser Val Asp Pro Ala Glu
            20                  25                  30

Ala Ile Ile Leu Arg Gly Gly Ser Val Gln Val Asn Cys Ser Thr Ser
        35                  40                  45

Cys Asn Gln Thr Ser Ile Phe Gly Leu Glu Thr Leu Leu Thr Lys Thr
    50                  55                  60

Glu Val Thr Ser Gly Asp Asn Trp Val Leu Phe Glu Leu Thr Asp Val
65                  70                  75                  80

Gln Glu Asp Ser Lys Leu Ile Cys Phe Ser Asn Cys His Asp Glu Thr
                85                  90                  95

Met Ala Pro Ile Asp Leu Thr Val Tyr Trp Phe Pro Glu Arg Val Glu
            100                 105                 110

Leu Ala Pro Leu Pro Arg Trp Gln Pro Val Gly Glu Asn Leu Thr Met
        115                 120                 125

Thr Cys Gln Val Ala Gly Gly Ala Pro Arg Thr Asn Leu Thr Val Val
    130                 135                 140

Leu Leu Arg Gly Glu Glu Glu Leu Ser Arg Gln Pro Ala Val Gly Glu
145                 150                 155                 160

Pro Ala Glu Val Thr Phe Thr Val Ala Val Gly Arg Glu Asp His Leu
                165                 170                 175

Ala Asn Phe Ser Cys Arg Thr Asp Leu Asp Leu Arg His Arg Gly Leu
            180                 185                 190

Gly Leu Phe Gln Asn Ser Ser Ala Pro Arg Gln Leu Gln Thr Phe Val
        195                 200                 205

Leu Pro Glu Thr Pro Pro Arg Leu Ala Thr Pro Pro Ile Val Glu Val
    210                 215                 220

Gly Thr Gln Trp Ser Val Asp Cys Thr Met Asp Gly Val Phe Pro Ala
225                 230                 235                 240

Ser Glu Ala Gln Val His Leu Ala Leu Ala Glu Glu Arg Leu His Ser
                245                 250                 255

Thr Val Leu Tyr Lys Lys Asp Ser Leu Leu Ala Thr Ala Asn Val Lys
            260                 265                 270

Ala Asn Pro Glu Asp Glu Gly Thr Gln Gln Leu Trp Cys Glu Val Thr
        275                 280                 285

Leu Gly Asp Glu Asn Arg Arg Trp Gln Glu Asn Val Thr Phe Tyr Ser
    290                 295                 300

Phe Pro Ala Pro Asn Leu Thr Leu Ser Glu Pro Glu Val Ser Glu Trp
305                 310                 315                 320

Thr Thr Val Thr Val Glu Cys Glu Ala Pro Ala Gly Val Val Val Ser
                325                 330                 335

Leu Ser Gly Leu Pro Ser Gly Leu Ala Val Pro Arg Ala Gln Phe Gln
            340                 345                 350

Leu Asn Ala Ser Ala Ala Asp Asn Arg Arg Ser Phe Ser Cys Ser Ala
        355                 360                 365

Ala Leu Glu Val Ala Gly His Met Leu Gln Lys Asn Gln Thr Arg Glu
    370                 375                 380

Leu His Val Leu Tyr Gly Pro Arg Leu Asp Gln Arg Asp Cys Pro Gly
385                 390                 395                 400

Asn Trp Thr Trp Glu Glu Gly Phe His Gln Thr Leu Lys Cys Gln Ala
                405                 410                 415

Trp Gly Asn Pro Val Pro Glu Leu Lys Cys His Arg Lys Gly Asp Asp
            420                 425                 430

Ala Leu Leu Pro Ile Gly Asp Leu Arg Pro Val Lys Arg Glu Val Ala
        435                 440                 445

Gly Thr Tyr Leu Cys Gln Ala Arg Ser Pro Arg Gly Glu Ile Thr Arg
    450                 455                 460

Glu Val Val Ile Asn Val Ile Tyr His Gln Asn Asn Ile Leu Ile Ile
465                 470                 475                 480

Ile Leu Val Thr Thr Ile Val Ile Leu Gly Thr Val Ser Val Ala Ala
                485                 490                 495

Tyr Leu Tyr Asn Arg Gln Arg Lys Ile Gln Lys Tyr Lys Leu Gln Lys
            500                 505                 510

Ala Gln Glu Ala Ala Ala Met Lys Leu Asn Thr Pro Ala Thr Pro Pro
        515                 520                 525


<210> 84
<211> 535
<212> PRT
<213> Bos taurus

<400> 84
Met Ala Leu Gly Ala Ala Pro Ala Ala Gln Leu Ala Leu Leu Ala Leu
  1               5                  10                  15

Leu Gly Thr Leu Leu Pro Gly Pro Gly Gly Ala Gly Ile Ser Ile His
             20                  25                  30

Pro Ser Lys Ala Ile Ile Pro Arg Gly Asp Ser Leu Thr Val Asn Cys
         35                  40                  45

Ser Asn Ser Cys Asp Gln Lys Ser Thr Phe Gly Leu Glu Thr Val Leu
     50                  55                  60

Ile Lys Glu Glu Val Gly Arg Gly Asp Asn Trp Lys Val Phe Gln Leu
 65                  70                  75                  80

Arg Asp Val Gln Glu Asp Ile Glu Leu Phe Cys Tyr Ser Asn Cys His
                 85                  90                  95

Lys Glu Gln Thr Ile Ala Ser Met Asn Leu Thr Val Tyr Trp Phe Pro
            100                 105                 110

Glu His Val Glu Leu Ala Pro Leu Pro Leu Trp Gln Pro Val Gly Glu
        115                 120                 125

Glu Leu Asn Leu Ser Cys Leu Val Ser Gly Gly Ala Pro Arg Ala His

130                 135                 140

Leu Ser Val Val Leu Leu Arg Gly Glu Glu Glu Leu Gly Arg Gln Pro
145                 150                 155                 160

Val Gly Lys Gly Glu Pro Ala Lys Val Met Phe Thr Val Gln Ser Arg
                165                 170                 175

Arg Glu Asp His Gly Thr Asn Phe Ser Cys Arg Trp Glu Leu Asp Leu
            180                 185                 190

Arg Ser Gln Gly Leu Glu Leu Phe Gln Asn Thr Ser Ala Pro Arg Lys
        195                 200                 205

Leu Gln Thr Tyr Val Leu Pro Ser Ile Asp Pro His Leu Glu Val Pro
    210                 215                 220

Pro Ile Val Glu Val Gly Ser Arg Trp Pro Val Asn Cys Thr Leu Asp
225                 230                 235                 240

Gly Leu Phe Pro Ala Ser Asp Ala Lys Val Tyr Leu Val Leu Gly Asp
                245                 250                 255

Gln Lys Leu Glu Ser Asn Ile Thr Tyr Asp Gly Asp Ser Val Leu Ala
            260                 265                 270

Lys Ala Trp Met Glu Glu Asn Glu Glu Gly Thr His Ser Leu Lys Cys
        275                 280                 285

Ser Val Thr Leu Gly Glu Val Ser Arg Arg Thr Gln Glu Asn Val Thr
    290                 295                 300

Val Tyr Ser Phe Pro Leu Pro Thr Leu Thr Leu Ser Pro Pro Glu Val
305                 310                 315                 320

Ser Glu Trp Thr Thr Val Thr Val Glu Cys Val Thr Arg Asp Gly Ala
                325                 330                 335

Val Val Lys Leu Asn Gly Thr Ser Ala Val Pro Pro Gly Pro Arg Ala
            340                 345                 350

Gln Leu Lys Leu Asn Ala Ser Ala Ser Asp His Arg Ser Asn Phe Ser
        355                 360                 365

Cys Ser Ala Ala Leu Glu Ile Ala Gly Gln Val Val His Lys His Gln
    370                 375                 380

Thr Leu Glu Leu His Val Leu Tyr Gly Pro Arg Leu Asp Gln Arg Asp
385                 390                 395                 400

Cys Pro Gly Asn Trp Thr Trp Gln Glu Gly Ser Glu Gln Thr Leu Lys
                405                 410                 415

Cys Glu Ala Gln Gly Asn Pro Ile Pro Lys Leu Asn Cys Ser Arg Lys
            420                 425                 430

Gly Asp Gly Ala Ser Leu Pro Ile Gly Asp Leu Arg Pro Val Arg Arg
        435                 440                 445

Glu Val Ala Gly Thr Tyr Leu Cys Arg Ala Thr Ser Ala Arg Gly Arg
    450                 455                 460

Val Thr Arg Glu Val Val Leu Asn Val Leu His Gly Gln Asn Ile Leu
465                 470                 475                 480

Asp Ile Val Ile Pro Val Val Ala Val Thr Leu Ile Leu Gly Ala Leu
                485                 490                 495

Gly Thr Ala Gly Tyr Val Tyr Asn Tyr Gln Arg Lys Ile Gln Lys Tyr
            500                 505                 510

Glu Leu Gln Lys Ala Arg Lys Ala Gln Glu Glu Ala Ala Leu Lys Leu
        515                 520                 525

Asn Ala Gln Ser Thr Pro Pro
    530                 535


<210> 85
<211> 530
<212> PRT
<213> Ovis aries

<400> 85
Met Ala Pro Gly Ala Ala Pro Ala Ala Leu Leu Ala Leu Leu Val Leu
1               5                   10                  15

Leu Gly Thr Leu Leu Pro Gly Ser Gly Gly Ala Glu Ile Ser Ile His
            20                  25                  30

Pro Pro Lys Ala Ile Ile Pro Arg Gly Gly Ser Leu Arg Val Asn Cys
        35                  40                  45

Ser Ile Ser Cys Asp Arg Lys Thr Thr Phe Gly Leu Glu Thr Val Leu
    50                  55                  60

Asn Lys Glu Glu Val Ser Arg Gly Pro Asn Trp Lys Val Phe Glu Leu
65                  70                  75                  80

Ser Asp Val Gln Glu Glu Ile Asn Pro Leu Cys Tyr Ser Asn Cys His
                85                  90                  95

Gly Glu Gln Ile Val Ala Ser Met Asn Leu Thr Ile Tyr Trp Phe Pro
            100                 105                 110

Glu Arg Val Glu Leu Ala Pro Leu Pro Leu Trp Gln Pro Val Gly Glu
        115                 120                 125

Glu Leu Asn Leu Ser Cys Gln Val Ser Gly Gly Gly Pro Arg His His
    130                 135                 140

Leu Ser Met Val Leu Leu Arg Gly Glu Glu Glu Leu Asp Arg Gln Pro
145                 150                 155                 160

Val Gly Lys Glu Glu Pro Ala Glu Val Thr Phe Met Val Gln Pro Arg
                165                 170                 175

Arg Glu Asp His Gly Thr Ser Phe Ser Cys Arg Trp Glu Leu Asp Leu
            180                 185                 190

Arg Ser Gln Gly Leu Glu Leu Phe Gln Asn Thr Ser Ala Pro Arg Lys
        195                 200                 205

Leu Gln Thr Tyr Val Leu Pro Ser Thr Asp Pro His Leu Glu Ala Pro
    210                 215                 220

Pro Val Val Glu Val Gly Ser Arg Trp Pro Val Lys Cys Thr Leu Asp
225                 230                 235                 240

Gly Leu Phe Pro Ala Ser Asp Ala Glu Val Tyr Val Gln Leu Gly Asp
                245                 250                 255

Gln Lys Leu Glu Ser Asn Ile Thr Tyr Asn Gly Asp Ser Val Leu Ala
            260                 265                 270

Glu Ala Trp Thr Glu Glu Asn Glu Glu Gly Thr His Ser Leu Arg Cys
        275                 280                 285

Ser Val Ser Leu Gly Glu Lys Ile Arg Arg Thr Arg Gly Ser Val Thr
    290                 295                 300

Met Tyr Ser Phe Pro Leu Pro Thr Leu Thr Leu Ser Pro Pro Glu Val
305                 310                 315                 320

Ser Glu Trp Thr Thr Val Thr Val Glu Cys Val Thr Arg Asp Gly Ala
                325                 330                 335

Val Val Arg Leu Asn Gly Val Ser Ala Glu Pro Pro Gly Pro Arg Ala
            340                 345                 350

Gln Leu Lys Leu Asn Val Ser Ala Asp Asp His Gly Ser Asn Phe Ser
        355                 360                 365

Cys Ser Ala Ala Leu Lys Ile Ala Gly Gln Glu Val His Lys Ile Gln
    370                 375                 380

Thr Arg Glu Leu His Val Leu Tyr Gly Pro Arg Leu Asp Gln Arg Asp
385                 390                 395                 400

Cys Leu Gly Asn Trp Thr Trp Gln Glu Gly Ser Glu Gln Thr Leu Lys
                405                 410                 415

Cys Ala Ala Arg Gly Asn Pro Ile Pro Lys Leu Asn Cys Ser Arg Lys
            420                 425                 430

Gly Asp Gly Ala Ser Leu Pro Ile Gly Asp Leu Arg Pro Val Thr Arg
        435                 440                 445

Glu Val Ala Gly Thr Tyr Leu Cys Trp Ala Thr Ser Ala Arg Gly Gly
    450                 455                 460

Val Thr Arg Glu Val Val Leu Asn Val Leu Tyr Gly Gln Asn Ile Leu

465                 470                 475                 480

Asp Ile Val Ile Pro Val Val Ala Val Thr Leu Ile Leu Gly Thr Leu
                485                 490                 495

Gly Thr Ala Gly Tyr Ile Tyr Asn Tyr Gln Arg Lys Ile Gln Lys Tyr
            500                 505                 510

Glu Leu Gln Lys Ala Gln Lys Glu Ala Ala Leu Lys Leu Lys Ser Thr
        515                 520                 525

Pro Pro
    530


<210> 86
<211> 545
<212> PRT
<213> Rattus norvegicus

<400> 86
Met Ala Ser Thr Arg Ala Arg Pro Met Leu Pro Leu Leu Leu Val Leu
1               5                   10                  15

Val Ala Val Val Ile Pro Gly Pro Val Gly Ala Gln Val Ser Ile His
            20                  25                  30

Pro Thr Glu Ala Phe Leu Pro Arg Gly Gly Ser Val Gln Val Asn Cys
        35                  40                  45

Ser Ser Ser Cys Glu Asp Glu Asn Leu Gly Leu Gly Leu Glu Thr Asn
    50                  55                  60

Trp Met Lys Asp Glu Leu Ser Ser Gly His Asn Trp Lys Leu Phe Lys
65                  70                  75                  80

Leu Ser Asp Ile Gly Glu Asp Ser Arg Pro Leu Cys Phe Glu Asn Cys
                85                  90                  95

Gly Thr Thr Gln Ser Ser Ala Ser Ala Thr Ile Thr Val Tyr Ser Phe
            100                 105                 110

Pro Glu Arg Val Glu Leu Asp Pro Leu Pro Ala Trp Gln Gln Val Gly
        115                 120                 125

Lys Asn Leu Ile Leu Arg Cys Leu Val Glu Gly Gly Ala Pro Arg Thr
    130                 135                 140

Gln Leu Ser Val Val Leu Leu Arg Gly Asn Glu Thr Leu Ser Arg Gln
145                 150                 155                 160

Ala Val Asp Gly Asp Pro Lys Glu Ile Thr Phe Thr Val Leu Ala Ser
                165                 170                 175

Arg Gly Asp His Gly Ala Asn Phe Ser Cys Phe Thr Glu Leu Asp Leu
            180                 185                 190

Arg Pro Gln Gly Leu Ser Leu Phe Lys Asn Val Ser Glu Val Arg Gln
        195                 200                 205

Leu Arg Thr Phe Asp Leu Pro Thr Arg Val Leu Lys Leu Asp Thr Pro
    210                 215                 220

Asp Leu Leu Glu Val Gly Thr Gln Gln Lys Phe Leu Cys Ser Leu Glu
225                 230                 235                 240

Gly Leu Phe Pro Ala Ser Glu Ala Gln Ile Tyr Leu Glu Met Gly Gly
                245                 250                 255

Gln Met Leu Thr Leu Glu Ser Thr Asn Ser Arg Asp Phe Val Ser Ala
            260                 265                 270

Thr Ala Ser Val Glu Val Thr Glu Lys Leu Asp Arg Thr Leu Gln Leu
        275                 280                 285

Arg Cys Val Leu Glu Leu Ala Asp Gln Thr Leu Glu Met Glu Lys Thr
    290                 295                 300

Leu Arg Ile Tyr Asn Phe Ser Ala Pro Ile Leu Thr Leu Ser Gln Pro
305                 310                 315                 320

Glu Val Ser Glu Gly Asp Gln Val Thr Val Lys Cys Glu Ala His Gly
                325                 330                 335

Gly Ala Gln Val Val Leu Leu Asn Ser Thr Ser Pro Arg Pro Pro Thr
            340                 345                 350

Ser Gln Gly Thr Ser Pro Arg Pro Pro Thr Ser Gln Ile Gln Phe Thr
        355                 360                 365

Leu Asn Ala Ser Pro Glu Asp His Lys Arg Arg Phe Phe Cys Ser Ala
    370                 375                 380

Ala Leu Glu Val Asp Gly Lys Ser Leu Phe Lys Asn Gln Thr Leu Glu
385                 390                 395                 400

Leu His Val Leu Tyr Gly Pro His Leu Asp Lys Lys Asp Cys Leu Gly
                405                 410                 415

Asn Trp Thr Trp Gln Glu Gly Ser Gln Gln Thr Leu Thr Cys Gln Pro
            420                 425                 430

Gln Gly Asn Pro Ala Pro Asn Leu Thr Cys Ser Arg Lys Ala Asp Gly
        435                 440                 445

Val Pro Leu Pro Ile Gly Met Val Lys Ser Val Lys Arg Glu Met Asn
    450                 455                 460

Gly Thr Tyr Lys Cys Arg Ala Phe Ser Ser Arg Gly Ser Ile Thr Arg
465                 470                 475                 480

Asp Val His Leu Thr Val Leu Tyr His Asp Gln Asn Thr Trp Val Ile
                485                 490                 495

Ile Val Gly Val Leu Val Leu Ile Ile Ala Gly Phe Val Ile Val Ala
            500                 505                 510

Ser Ile Tyr Thr Tyr Tyr Arg Gln Arg Lys Ile Arg Ile Tyr Lys Leu
        515                 520                 525

Gln Lys Ala Gln Glu Glu Ala Leu Lys Leu Lys Val Gln Ala Pro Pro
    530                 535                 540

Pro
545


<210> 87
<211> 917
<212> PRT
<213> Rattus norvegicus

<400> 87
Met Pro Gly Pro Ser Pro Gly Leu Arg Arg Thr Leu Leu Gly Leu Trp
1               5                   10                  15

Ala Ala Leu Gly Leu Gly Ile Leu Gly Ile Ser Ala Val Ala Leu Glu
            20                  25                  30

Pro Phe Trp Ala Asp Leu Gln Pro Arg Val Ala Leu Val Glu Arg Gly
        35                  40                  45

Gly Ser Leu Trp Leu Asn Cys Ser Thr Asn Cys Pro Arg Pro Glu Arg
    50                  55                  60

Gly Gly Leu Glu Thr Ser Leu Arg Arg Asn Gly Thr Gln Arg Gly Leu
65                  70                  75                  80

Arg Trp Leu Ala Arg Gln Leu Val Asp Ile Arg Glu Pro Glu Thr Gln
                85                  90                  95

Pro Val Cys Phe Phe Arg Cys Ala Arg Arg Thr Leu Gln Ala Arg Gly
            100                 105                 110

Leu Ile Arg Thr Phe Gln Arg Pro Asp Arg Val Glu Leu Val Pro Leu
        115                 120                 125

Pro Pro Trp Gln Pro Val Gly Glu Asn Phe Thr Leu Ser Cys Arg Val
    130                 135                 140

Pro Gly Ala Gly Pro Arg Ala Ser Leu Thr Leu Thr Leu Leu Arg Gly
145                 150                 155                 160

Gly Gln Glu Leu Ile Arg Arg Ser Phe Val Gly Glu Pro Pro Arg Ala
                165                 170                 175

Arg Gly Ala Met Leu Thr Ala Thr Val Leu Ala Arg Arg Glu Asp His
            180                 185                 190

Arg Ala Asn Phe Ser Cys Leu Ala Glu Leu Asp Leu Arg Pro His Gly
        195                 200                 205

Leu Gly Leu Phe Ala Asn Ser Ser Ala Pro Arg Gln Leu Arg Thr Phe
    210                 215                 220

Ala Met Pro Pro Leu Ser Pro Ser Leu Ile Ala Pro Arg Phe Leu Glu
225                 230                 235                 240

Val Gly Ser Glu Arg Pro Val Thr Cys Thr Leu Asp Gly Leu Phe Pro
                245                 250                 255

Ala Pro Glu Ala Gly Val Tyr Leu Ser Leu Gly Asp Gln Arg Leu His
            260                 265                 270

Pro Asn Val Thr Leu Asp Gly Glu Ser Leu Val Ala Thr Ala Thr Ala
        275                 280                 285

Thr Ala Ser Glu Glu Gln Glu Gly Thr Lys Gln Leu Met Cys Ile Val
    290                 295                 300

Thr Leu Gly Gly Glu Ser Arg Glu Thr Gln Glu Asn Leu Thr Val Tyr
305                 310                 315                 320

Ser Phe Pro Ala Pro Leu Leu Thr Leu Ser Glu Pro Glu Ala Pro Glu
                325                 330                 335

Gly Lys Met Val Thr Val Ser Cys Trp Ala Gly Ala Arg Ala Leu Val
            340                 345                 350

Thr Leu Glu Gly Ile Pro Ala Ala Val Pro Gly Gln Pro Ala Glu Leu
        355                 360                 365

Gln Leu Asn Val Thr Lys Asn Asp Asp Lys Arg Gly Phe Phe Cys Asp
    370                 375                 380

Ala Ala Leu Asp Val Asp Gly Glu Thr Leu Arg Lys Asn Gln Ser Ser
385                 390                 395                 400

Glu Leu Arg Val Leu Tyr Ala Pro Arg Leu Asp Asp Leu Asp Cys Pro
                405                 410                 415

Arg Ser Trp Thr Trp Pro Glu Gly Pro Glu Gln Thr Leu His Cys Glu
            420                 425                 430

Ala Arg Gly Asn Pro Glu Pro Ser Val His Cys Ala Arg Pro Asp Gly
        435                 440                 445

Gly Ala Val Leu Ala Leu Gly Leu Leu Gly Pro Val Thr Arg Ala Leu
    450                 455                 460

Ala Gly Thr Tyr Arg Cys Thr Ala Ile Asn Gly Gln Gly Gln Ala Val
465                 470                 475                 480

Lys Asp Val Thr Leu Thr Val Glu Tyr Ala Pro Ala Leu Asp Ser Val
                485                 490                 495

Gly Cys Pro Glu Arg Ile Thr Trp Leu Glu Gly Thr Glu Ala Ser Leu
            500                 505                 510

Ser Cys Val Ala His Gly Val Pro Pro Pro Ser Val Ser Cys Val Arg
        515                 520                 525

Ser Gly Lys Glu Glu Val Met Glu Gly Pro Leu Arg Val Ala Arg Glu
    530                 535                 540

His Ala Gly Thr Tyr Arg Cys Glu Ala Ile Asn Ala Arg Gly Ser Ala
545                 550                 555                 560

Ala Lys Asn Val Ala Val Thr Val Glu Tyr Gly Pro Ser Phe Glu Glu
                565                 570                 575

Leu Gly Cys Pro Ser Asn Trp Thr Trp Val Glu Gly Ser Gly Lys Leu
            580                 585                 590

Phe Ser Cys Glu Val Asp Gly Lys Pro Glu Pro Arg Val Glu Cys Val
        595                 600                 605

Gly Ser Glu Gly Ala Ser Glu Gly Val Val Leu Pro Leu Val Ser Ser
    610                 615                 620

Asn Ser Gly Ser Arg Asn Ser Met Thr Pro Gly Asn Leu Ser Pro Gly
625                 630                 635                 640

Ile Tyr Leu Cys Asn Ala Thr Asn Arg His Gly Ser Thr Val Lys Thr
                645                 650                 655

Val Val Val Ser Ala Glu Ser Pro Pro Gln Met Asp Glu Ser Ser Cys
            660                 665                 670

Pro Ser His Gln Thr Trp Leu Glu Gly Ala Glu Ala Thr Ala Leu Ala
        675                 680                 685

Cys Ser Ala Arg Gly Arg Pro Ser Pro Arg Val Arg Cys Ser Arg Glu
    690                 695                 700

Gly Ala Ala Arg Leu Glu Arg Leu Gln Val Ser Arg Glu Asp Ala Gly
705                 710                 715                 720

Thr Tyr Leu Cys Val Ala Thr Asn Ala His Gly Thr Asp Ser Arg Thr
                725                 730                 735

Val Thr Val Gly Val Glu Tyr Arg Pro Val Val Ala Glu Leu Ala Ala
            740                 745                 750

Ser Pro Pro Ser Val Arg Pro Gly Gly Asn Phe Thr Leu Thr Cys Arg
        755                 760                 765

Ala Glu Ala Trp Pro Pro Ala Gln Ile Ser Trp Arg Ala Pro Pro Gly
    770                 775                 780

Ala Leu Asn Leu Gly Leu Ser Ser Asn Asn Ser Thr Leu Ser Val Ala
785                 790                 795                 800

Gly Ala Met Gly Ser His Gly Gly Glu Tyr Glu Cys Ala Ala Thr Asn
                805                 810                 815

Ala His Gly Arg His Ala Arg Arg Ile Thr Val Arg Val Ala Gly Pro
            820                 825                 830

Trp Leu Trp Val Ala Val Gly Gly Ala Ala Gly Gly Ala Ala Leu Leu
        835                 840                 845

Ala Ala Gly Ala Gly Leu Ala Phe Tyr Val Gln Ser Thr Ala Cys Lys
    850                 855                 860

Lys Gly Glu Tyr Asn Val Gln Glu Ala Glu Ser Ser Gly Glu Ala Val
865                 870                 875                 880

Cys Leu Asn Gly Ala Gly Gly Thr Pro Gly Ala Glu Gly Gly Ala Glu
                885                 890                 895

Thr Pro Gly Thr Ala Glu Ser Pro Ala Asp Gly Glu Val Phe Ala Ile
            900                 905                 910

Gln Leu Thr Ser Ser
        915


<210> 88
<211> 151
<212> PRT
<213> Homo sapiens

<220>
<221> MOD_RES
<222> (12)
<223> Any amino acid

<220>
<221> MOD_RES
<222> (77)..(81)
<223> Any amino acid

<220>
<221> MOD_RES
<222> (132)
<223> Any amino acid

<220>
<221> MOD_RES
<222> (145)..(147)
<223> Any amino acid

<400> 88
Glu Asp Ser Gln Pro Met Cys Tyr Ser Asn Cys Xaa Asp Gly Gln Ser
1               5                   10                  15

Thr Ala Lys Thr Phe Leu Thr Val Tyr Trp Thr Pro Glu Arg Val Glu
            20                  25                  30

Leu Ala Pro Leu Pro Ser Trp Gln Pro Val Gly Lys Asn Leu Thr Leu
        35                  40                  45

Arg Cys Gln Val Glu Gly Gly Ala Pro Arg Ala Asn Leu Thr Val Val

50                  55                  60

Leu Leu Arg Gly Glu Lys Glu Leu Lys Arg Glu Pro Xaa Xaa Xaa Xaa
65                  70                  75                  80

Xaa Ala Glu Val Thr Thr Thr Val Leu Val Arg Arg Asp His His Gly
                85                  90                  95

Ala Asn Phe Ser Cys Arg Thr Glu Leu Asp Leu Arg Pro Gln Gly Leu
            100                 105                 110

Glu Leu Phe Glu Asn Thr Ser Ala Pro Tyr Gln Leu Gln Thr Phe Val
        115                 120                 125

Leu Pro Ala Xaa Pro Pro Gln Leu Val Ser Pro Arg Val Leu Glu Val
    130                 135                 140

Xaa Xaa Xaa Gly Thr Val Val
145                 150


<210> 89
<211> 1252
<212> PRT
<213> Rattus norvegicus

<400> 89
   Met Gly Ala Lys Arg Val Thr Val Arg Gly Ala Arg Thr Ser Pro Ile
   1               5                   10                  15
   His Arg Met Ser Ser Leu Thr Pro Leu Leu Leu Met Gly Met Leu Thr
               20                  25                  30
   Ser Gly Leu Ala Glu Ser Pro Val Pro Thr Ser Ala Pro Arg Gly Phe
           35                  40                  45
   Trp Ala Leu Ser Glu Asn Leu Thr Ala Val Glu Gly Thr Thr Val Lys
       50                  55                  60
   Leu Trp Cys Gly Val Arg Ala Pro Gly Ser Val Val Gln Trp Ala Lys
   65                  70                  75                  80
   Asp Gly Leu Leu Leu Gly Pro Asn Pro Lys Met Pro Gly Phe Pro Arg
                   85                  90                  95
   Tyr Ser Leu Glu Gly Asp Arg Ala Lys Gly Glu Phe His Leu Leu Ile
               100                 105                 110
   Glu Ala Cys Asp Leu Ser Asp Asp Ala Glu Tyr Glu Cys Gln Val Gly
           115                 120                 125
   Arg Ser Glu Leu Gly Pro Glu Leu Val Ser Pro Lys Val Ile Leu Ser
       130                 135                 140
   Ile Leu Val Ser Pro Lys Val Leu Leu Leu Thr Pro Glu Ala Gly Ser
   145                 150                 155                 160
   Thr Val Thr Trp Val Ala Gly Gln Glu Tyr Val Val Thr Cys Val Ser
                   165                 170                 175
   Gly Asp Ala Lys Pro Ala Pro Asp Ile Thr Phe Ile Gln Ser Gly Arg
               180                 185                 190
   Thr Ile Leu Asp Val Ser Ser Asn Val Asn Glu Gly Ser Glu Glu Lys
           195                 200                 205
   Leu Cys Ile Thr Glu Ala Glu Ala Arg Val Ile Pro Gln Ser Ser Asp
       210                 215                 220
   Asn Gly Gln Leu Leu Val Cys Glu Gly Ser Asn Pro Ala Leu Asp Thr
   225                 230                 235                 240
   Pro Ile Lys Ala Ser Phe Thr Met Asn Ile Leu Phe Pro Pro Gly Pro
                   245                 250                 255
   Pro Val Ile Asp Trp Pro Gly Leu Asn Glu Gly His Val Arg Ala Gly
               260                 265                 270
   Glu Asn Leu Glu Leu Pro Cys Thr Ala Arg Gly Gly Asn Pro Pro Ala
           275                 280                 285
   Thr Leu Gln Trp Leu Lys Asn Gly Lys Pro Val Ser Thr Ala Trp Gly
       290                 295                 300
   Thr Glu His Ala Gln Ala Val Ala His Ser Val Leu Val Met Thr Val
   305                 310                 315                 320
   Arg Pro Glu Asp His Gly Ala Arg Leu Ser Cys Gln Ser Tyr Asn Ser
                   325                 330                 335
   Val Ser Ala Gly Thr Gln Glu Arg Ser Ile Thr Leu Gln Val Thr Phe
               340                 345                 350
   Pro Pro Ser Ala Ile Thr Ile Leu Gly Ser Val Ser Gln Ser Glu Asn
           355                 360                 365
   Lys Asn Val Thr Leu Cys Cys Leu Thr Lys Ser Ser Arg Pro Arg Val
       370                 375                 380
   Leu Leu Arg Trp Trp Leu Gly Gly Arg Gln Leu Leu Pro Thr Asp Glu
   385                 390                 395                 400
   Thr Val Met Asp Gly Leu His Gly Gly His Ile Ser Met Ser Asn Leu
                   405                 410                 415
   Thr Phe Leu Val Arg Arg Glu Asp Asn Gly Leu Pro Leu Thr Cys Glu
               420                 425                 430
   Ala Phe Ser Asp Ala Phe Ser Lys Glu Thr Phe Lys Lys Ser Leu Thr
           435                 440                 445
   Leu Asn Val Lys Tyr Pro Ala Gln Lys Leu Trp Ile Glu Gly Pro Pro
       450                 455                 460
   Glu Gly Gln Tyr Ile Arg Thr Gly Thr Arg Val Arg Leu Val Cys Leu
   465                 470                 475                 480
   Ala Ile Gly Gly Asn Pro Asp Pro Ser Leu Ile Trp Phe Lys Asp Ser
                   485                 490                 495
   Arg Pro Val Ser Glu Pro Arg Gln Pro Gln Glu Pro Arg Arg Val Gln
               500                 505                 510
   Leu Gly Ser Val Glu Lys Ser Gly Ser Thr Phe Ser Arg Glu Leu Val
           515                 520                 525
   Leu Ile Ile Gly Pro Pro Asp Asn Arg Ala Lys Phe Ser Cys Lys Ala
       530                 535                 540
   Gly Gln Leu Ser Ala Ser Thr Gln Leu Val Val Gln Phe Pro Pro Thr
   545                 550                 555                 560
   Asn Leu Thr Ile Leu Ala Asn Ser Ser Ala Leu Arg Pro Gly Asp Ala
                   565                 570                 575
   Leu Asn Leu Thr Cys Val Ser Ile Ser Ser Asn Pro Pro Val Asn Leu
               580                 585                 590
   Ser Trp Asp Lys Glu Gly Glu Arg Leu Glu Asp Val Ala Ala Lys Pro
           595                 600                 605
   Gln Ser Ala Pro Phe Lys Gly Ser Ala Ala Ser Arg Ser Val Phe Leu
       610                 615                 620
   Arg Val Ser Ser Arg Asp His Gly Gln Arg Val Thr Cys Arg Ala His
   625                 630                 635                 640
   Ser Glu Ala Leu Arg Glu Thr Val Ser Ser Phe Tyr Arg Phe Asn Val
                   645                 650                 655
   Leu Tyr Pro Pro Glu Phe Leu Gly Glu Gln Val Arg Ala Val Thr Val
               660                 665                 670
   Val Glu Gln Gly Gln Val Leu Leu Pro Val Ser Val Ser Ala Asn Pro
           675                 680                 685
   Ala Pro Glu Ala Phe Asn Trp Thr Phe Arg Gly Tyr Arg Leu Ser Pro
       690                 695                 700
   Ala Gly Gly Pro Arg His Arg Ile Leu Ser Gly Gly Ala Leu Gln Leu
   705                 710                 715                 720
   Trp Asn Val Thr Arg Ala Asp Asp Gly Phe Tyr Gln Leu His Cys Gln
                   725                 730                 735
   Asn Ser Glu Gly Thr Ala Glu Ala Leu Leu Lys Leu Asp Val His Tyr
               740                 745                 750
   Ala Pro Thr Ile Arg Ala Leu Arg Asp Pro Thr Glu Val Asn Val Gly
           755                 760                 765
   Gly Ser Val Asp Ile Val Cys Thr Val Asp Ala Asn Pro Ile Leu Pro
           770                 775                 780
   Glu Met Phe Ser Trp Glu Arg Leu Gly Glu Glu Glu Glu Asp Leu Asn
   785                 790                 795                 800
   Leu Asp Asp Met Glu Lys Val Ser Lys Gly Ser Thr Gly Arg Leu Arg
                   805                 810                 815
   Ile Arg Gln Ala Lys Leu Ser Gln Ala Gly Ala Tyr Gln Cys Ile Val
               820                 825                 830
   Asp Asn Gly Val Ala Pro Ala Ala Arg Gly Leu Val Arg Leu Val Val
           835                 840                 845
   Arg Phe Ala Pro Gln Val Asp Gln Pro Thr Pro Leu Thr Lys Val Ala
       850                 855                 860
   Ala Ala Gly Asp Ser Thr Ser Ser Ala Thr Leu His Cys Arg Ala Arg
   865                 870                 875                 880
   Gly Val Pro Asn Ile Asp Phe Thr Trp Thr Lys Asn Gly Val Pro Leu
                   885                 890                 895
   Asp Leu Gln Asp Pro Arg Tyr Thr Glu His Arg Tyr His Gln Gly Val
               900                 905                 910
   Val His Ser Ser Leu Leu Thr Ile Ala Asn Val Ser Ala Ala Gln Asp
           915                 920                 925
   Tyr Ala Leu Phe Lys Cys Thr Ala Thr Asn Ala Leu Gly Ser Asp His
       930                 935                 940
   Thr Asn Ile Gln Leu Val Ser Ile Ser Arg Pro Asp Pro Pro Leu Gly
   945                 950                 955                 960
   Leu Lys Val Val Ser Ile Ser Pro His Ser Val Gly Leu Glu Trp Lys
                   965                 970                 975
   Pro Gly Phe Asp Gly Gly Leu Pro Gln Arg Phe Gln Ile Arg Tyr Glu
               980                 985                 990
   Ala Leu Glu Thr Pro Gly Phe Leu His Val Asp Val Leu Pro Thr Gln
           995                 1000                1005
   Ala Thr Thr Phe Thr Leu Thr Gly Leu Lys Pro Ser Thr Arg Tyr Arg
       1010                1015                1020
   Ile Trp Leu Leu Ala Ser Asn Ala Leu Gly Asp Ser Gly Leu Thr Asp
   1025                1030                1035                1040
   Lys Gly Ile Gln Val Ser Val Thr Thr Pro Gly Pro Asp Gln Ala Pro
                   1045                1050                1055
   Glu Asp Thr Asp His Gln Leu Pro Thr Glu Leu Pro Pro Gly Pro Pro
               1060                1065                1070
   Arg Leu Pro Leu Leu Pro Val Leu Phe Ala Val Gly Gly Leu Leu Leu
           1075                1080                1085
   Leu Ser Asn Ala Ser Cys Val Gly Gly Leu Leu Trp Arg Arg Arg Leu
       1090                1095                1100
   Arg Arg Leu Ala Glu Glu Ile Ser Glu Lys Thr Glu Ala Gly Ser Glu
   1105                1110                1115                1120
   Asp Arg Ile Arg Asn Glu Tyr Glu Glu Ser Gln Trp Thr Gly Asp Arg
                   1125                1130                1135
   Asp Thr Arg Ser Ser Thr Val Ser Thr Ala Glu Val Asp Pro Asn Tyr
               1140                1145                1150
   Tyr Ser Met Arg Asp Phe Ser Pro Gln Leu Pro Pro Thr Leu Glu Glu
           1155                1160                1165
   Val Leu Tyr His Gln Gly Ala Glu Gly Glu Asp Met Ala Phe Pro Gly
       1170                1175                1180
   His Leu His Asp Glu Val Glu Arg Ala Tyr Gly Pro Pro Gly Ala Trp
   1185                1190                1195                1200
   Gly Pro Leu Tyr Asp Glu Val Arg Met Asp Pro Tyr Asp Leu Arg Trp
                   1205                1210                1215
   Pro Glu Val Gln Cys Glu Asp Pro Arg Gly Ile Tyr Asp Gln Val Ala
               1220                1225                1230
   Ala Asp Met Asp Ala Val Glu Ala Ser Ser Leu Pro Phe Glu Leu Arg
           1235                1240                1245
   Gly His Leu Val
       1250


<210> 90
<211> 1256
<212> PRT
<213> Mus musculus

<400> 90
Met Gly Ala Lys Glu Val Thr Val Arg Gly Pro Gly Ala Ser Pro Val
1               5                   10                  15

His Arg Thr Cys Arg Leu Ile Pro Leu Leu Leu Ala Gly Met Leu Thr
            20                  25                  30

Thr Gly Leu Ala Gln Ser Pro Val Pro Thr Ser Ala Pro Arg Gly Phe
        35                  40                  45

Trp Ala Leu Ser Glu Asn Leu Thr Val Val Glu Gly Ser Thr Ile Lys
    50                  55                  60

Leu Trp Cys Gly Val Arg Ala Pro Gly Ser Val Val Gln Trp Ala Lys
65                  70                  75                  80

Asp Gly Leu Leu Leu Gly Pro Asn Pro Lys Ile Pro Gly Phe Pro Arg
                85                  90                  95

Tyr Ser Leu Glu Gly Asp Ser Ala Lys Gly Glu Phe His Leu Leu Ile
            100                 105                 110

Glu Ala Cys Asp Leu Ser Asp Asp Ala Glu Tyr Glu Cys Gln Val Gly
        115                 120                 125

Arg Ser Glu Leu Gly Pro Glu Leu Val Ser Pro Arg Val Ile Leu Ser
    130                 135                 140

Val Leu Val Pro Pro Lys Val Leu Gln Leu Thr Pro Glu Ala Gly Ser
145                 150                 155                 160

Thr Val Thr Trp Val Ala Gly Gln Glu Tyr Val Val Thr Cys Val Ser
                165                 170                 175

Gly Gly Ala Lys Pro Ala Pro Asp Ile Ile Phe Ile Gln Gly Gly Arg
            180                 185                 190

Thr Val Glu Asp Val Ser Ser Ser Val Asn Glu Gly Ser Glu Glu Lys
        195                 200                 205

Leu Phe Phe Thr Glu Ala Glu Ala Arg Val Thr Pro Gln Ser Ser Asp
    210                 215                 220

Asn Gly Gln Leu Leu Val Cys Glu Gly Ser Asn Pro Ala Leu Ala Thr
225                 230                 235                 240

Pro Ile Lys Ala Ser Phe Thr Met Asn Ile Leu Phe Pro Pro Gly Pro
                245                 250                 255

Pro Val Ile Asp Trp Pro Gly Leu Asn Glu Gly His Val Arg Ala Gly
            260                 265                 270

Glu Asn Leu Glu Leu Pro Cys Ile Ala Arg Gly Gly Asn Pro Pro Ala
        275                 280                 285

Thr Leu Gln Trp Leu Lys Asn Gly Lys Pro Val Ser Ile Ala Trp Gly
    290                 295                 300

Thr Glu His Ala Gln Ala Val Ala His Ser Val Leu Val Met Thr Val
305                 310                 315                 320

Arg Pro Glu Asp His Gly Ala Arg Leu Ser Cys Gln Ser Tyr Asn Ser
                325                 330                 335

Val Ser Ala Glu Thr Gln Glu Arg Ser Ile Thr Leu Gln Val Thr Phe
            340                 345                 350

Pro Pro Ser Ala Val Thr Ile Leu Gly Ser Thr Ser Gln Ser Glu Asn
        355                 360                 365

Lys Asn Val Thr Leu Cys Cys Leu Thr Lys Ser Ser Arg Pro Arg Val
    370                 375                 380

Leu Leu Arg Trp Trp Leu Gly Gly Arg Gln Leu Leu Pro Thr Asp Glu

385                 390                 395                 400

Thr Val Met Asp Gly Leu His Gly Gly His Ile Ser Met Ser Asn Leu
                405                 410                 415

Thr Leu Leu Val Lys Arg Glu Asp Asn Gly Leu Ser Leu Thr Cys Glu
            420                 425                 430

Ala Phe Ser Asp Ala Phe Ser Lys Glu Thr Phe Lys Lys Ser Leu Thr
        435                 440                 445

Leu Asn Val Lys Tyr Pro Ala Gln Lys Leu Trp Ile Glu Gly Pro Pro
    450                 455                 460

Glu Gly Gln Ser Ile Arg Thr Gly Thr Arg Val Arg Leu Val Cys Leu
465                 470                 475                 480

Ala Ile Gly Gly Asn Pro Glu Pro Ser Leu Thr Trp Leu Lys Asp Ser
                485                 490                 495

Arg Pro Val Asn Asp Pro Arg Gln Ser Gln Glu Pro Arg Arg Val Gln
            500                 505                 510

Leu Gly Ser Val Glu Lys Ser Gly Ser Thr Phe Ser Arg Glu Leu Val
        515                 520                 525

Leu Ile Ile Gly Pro Pro Asp Asn Leu Ala Lys Phe Ser Cys Lys Ala
    530                 535                 540

Gly Gln Leu Ser Ala Ser Thr Gln Leu Val Val Gln Phe Pro Pro Thr
545                 550                 555                 560

Asn Leu Thr Ile Leu Ala Asn Ser Ser Ala Leu Arg Pro Gly Asp Ala
                565                 570                 575

Leu Asn Leu Thr Cys Val Ser Ile Ser Ser Asn Pro Pro Val Asn Leu
            580                 585                 590

Ser Leu Asp Lys Glu Gly Glu Arg Leu Asp Asp Val Ala Ala Lys Pro
        595                 600                 605

Gln Ser Ala Pro Phe Lys Gly Ser Ala Ala Ser Arg Ser Val Phe Leu
    610                 615                 620

Arg Val Ser Ser Arg Asp His Gly His Arg Val Thr Cys Arg Ala His
625                 630                 635                 640

Ser Glu Ala Leu Arg Glu Thr Val Ser Ser Phe Tyr Arg Leu Asn Val
                645                 650                 655

Leu Tyr Pro Pro Glu Phe Leu Gly Glu Gln Val Arg Ala Val Thr Val
            660                 665                 670

Val Glu Gln Gly Gln Ala Leu Leu Pro Val Ser Val Ser Ala Asn Pro
        675                 680                 685

Ala Pro Glu Ala Phe Asn Trp Thr Phe Arg Gly Tyr Arg Leu Ser Pro
    690                 695                 700

Ala Gly Gly Pro Arg His Arg Ile Leu Ser Gly Gly Ala Leu Gln Leu
705                 710                 715                 720

Trp Asn Val Thr Arg Ala Asp Asp Gly Phe Tyr Gln Leu His Cys Gln
                725                 730                 735

Asn Ser Glu Gly Thr Ala Glu Ala Leu Leu Lys Leu Asp Val His Tyr
            740                 745                 750

Ala Pro Thr Ile Arg Ala Leu Lys Asp Pro Thr Glu Val Asn Val Gly
        755                 760                 765

Gly Ser Val Asp Ile Val Cys Thr Val Asp Ala Asn Pro Ile Leu Pro
    770                 775                 780

Glu Met Phe Ser Trp Glu Arg Leu Gly Glu Asp Glu Glu Glu Leu Asn
785                 790                 795                 800

Leu Asp Asp Met Glu Lys Met Ser Lys Gly Ser Thr Gly Arg Leu Arg
                805                 810                 815

Ile Arg Gln Ala Lys Leu Ser Gln Ala Gly Ala Tyr Gln Cys Ile Val
            820                 825                 830

Asp Asn Gly Val Ala Pro Ala Ala Arg Gly Leu Val Arg Leu Val Val
        835                 840                 845

Arg Phe Ala Pro Gln Val Asp His Pro Thr Pro Leu Thr Lys Val Ala
    850                 855                 860

Ala Ala Gly Asp Ser Thr Ser Ser Ala Thr Leu His Cys Arg Ala Arg
865                 870                 875                 880

Gly Val Pro Asn Ile Asp Phe Thr Trp Thr Lys Asn Gly Val Pro Leu
                885                 890                 895

Asp Leu Gln Asp Pro Arg Tyr Thr Glu His Lys Tyr His Gln Gly Val
            900                 905                 910

Val His Ser Ser Leu Leu Thr Ile Ala Asn Val Ser Ala Ala Gln Asp
        915                 920                 925

Tyr Ala Leu Phe Lys Cys Thr Ala Thr Asn Ala Leu Gly Ser Asp His
    930                 935                 940

Thr Asn Ile Gln Leu Val Ser Ile Ser Arg Pro Asp Pro Pro Leu Gly
945                 950                 955                 960

Leu Lys Val Val Ser Val Ser Pro His Ser Val Gly Leu Glu Trp Lys
                965                 970                 975

Pro Gly Phe Asp Gly Gly Leu Pro Gln Arg Phe Gln Ile Arg Tyr Glu
            980                 985                 990

Ala Leu Glu Thr Pro Gly Phe Leu Tyr Met Asp Val Leu Pro Ala Gln
        995                 1000                1005

Ala Thr Thr Phe Thr Leu Thr Gly Leu Lys Pro Ser Thr Arg Tyr Arg
    1010                1015                1020

Ile Trp Leu Leu Ala Ser Asn Ala Leu Gly Asp Ser Gly Leu Thr Asp
1025                1030                1035                1040

Lys Gly Ile Gln Val Ser Ile Thr Thr Pro Gly Leu Asp Gln Ala Pro
                1045                1050                1055

Glu Asp Thr Asp Gln Pro Leu Pro Thr Glu Gln Pro Pro Gly Pro Pro
            1060                1065                1070

Arg Leu Pro Leu Leu Pro Val Leu Phe Ala Val Gly Gly Leu Leu Leu
        1075                1080                1085

Leu Ser Asn Ala Ser Cys Val Gly Gly Leu Leu Trp Arg Arg Arg Leu
    1090                1095                1100

Arg Arg Leu Ala Glu Glu Ile Ser Glu Lys Thr Glu Ala Gly Ser Glu
1105                1110                1115                1120

Glu Asp Arg Ile Arg Asn Glu Tyr Glu Glu Ser Gln Trp Thr Gly Asp
                1125                1130                1135

Arg Asp Thr Arg Ser Ser Thr Val Ser Thr Ala Glu Val Asp Pro His
            1140                1145                1150

Tyr Tyr Ser Met Arg Asp Phe Ser Pro Gln Leu Pro Pro Thr Leu Glu
        1155                1160                1165

Glu Val Ser Tyr Arg Gln Ala Phe Thr Gly Ile Glu Asp Glu Asp Met
    1170                1175                1180

Ala Phe Pro Gly His Leu Tyr Asp Glu Val Glu Arg Val Tyr Gly Pro
1185                1190                1195                1200

Pro Gly Val Trp Gly Pro Leu Tyr Asp Glu Val Gln Met Asp Pro Tyr
                1205                1210                1215

Asp Leu Arg Trp Pro Glu Val Lys Tyr Glu Asp Pro Arg Gly Ile Tyr
            1220                1225                1230

Asp Gln Val Ala Ala Asp Met Asp Ala Gly Glu Pro Gly Ser Leu Pro
        1235                1240                1245

Phe Glu Leu Arg Gly His Leu Val
    1250                1255


<210> 91
<211> 29
<212> DNA
<213> Artificial Sequence

<220>
<223> Description of Artificial Sequence: Primer

<400> 91
gacctgtact tcattttgga caaatcagg                                          29

<210> 92
<211> 34
<212> DNA
<213> Artificial Sequence

<220>
<223> Description of Artificial Sequence: Primer

<400> 92
gagctcaaaa ttgagtggat gatgccttgc agag                                    34

<210> 93
<211> 27
<212> DNA
<213> Artificial Sequence

<220>
<223> Description of Artificial Sequence: Primer

<400> 93
ggtaccactt ctctcaatcc aactttc                                            27

<210> 94
<211> 49
<212> DNA
<213> Artificial Sequence

<220>
<223> Description of Artificial Sequence: Primer

<400> 94
gtccaaaatg aagtacaggt cagccaaact agtagaggtg aacaaaagc                    49

<210> 95
<211> 28
<212> DNA
<213> Artificial Sequence

<220>
<223> Description of Artificial Sequence: Primer

<400> 95
gggggacgca gggaggatgg gggtccag                                           28

<210> 96
<211> 30
<212> DNA
<213> Artificial Sequence

<220>
<223> Description of Artificial Sequence: Primer

<400> 96
gagctcccgt cagaacagtg tgtggtggtg                                         30

<210> 97
<211> 46
<212> DNA
<213> Artificial Sequence

<220>
<223> Description of Artificial Sequence: Primer

<400> 97
atcctccctg cgtcccccag ccaaactagt agaggtgaac aaaagc                       46

<210> 98
<211> 1650
<212> DNA
<213> Unknown Organism

<220>
<221> CDS
<222> (34)..(1647)

<220>
<223> Description of Unknown Organism: ATR-IgA2 fusion
      nucleotide

<400> 98
ggtaccactt ctctcaatcc aactttctaa aca atg gct tct aaa cct ttc ttg   54
                                     Met Ala Ser Lys Pro Phe Leu
                                     1               5

tct ctt ctt tct ttg tct ttg ctt ttg ttc acc tct act agt ttg gct   102
Ser Leu Leu Ser Leu Ser Leu Leu Leu Phe Thr Ser Thr Ser Leu Ala
        10                  15                  20

gac ctg tac ttc att ttg gac aaa tca gga agt gtg ctg cac cac tgg   150
Asp Leu Tyr Phe Ile Leu Asp Lys Ser Gly Ser Val Leu His His Trp
    25                  30                  35

aat gaa atc tat tac ttt gtg gaa cag ttg gct cac aaa ttc atc agc   198
Asn Glu Ile Tyr Tyr Phe Val Glu Gln Leu Ala His Lys Phe Ile Ser
40                  45                  50                  55

cca cag ttg aga atg tcc ttt att gtt ttc tcc acc cga gga aca acc   246
Pro Gln Leu Arg Met Ser Phe Ile Val Phe Ser Thr Arg Gly Thr Thr
                60                  65                  70

tta atg aaa ctg aca gaa gac aga gaa caa atc cgt caa ggc cta gaa   294
Leu Met Lys Leu Thr Glu Asp Arg Glu Gln Ile Arg Gln Gly Leu Glu
            75                  80                  85

gaa ctc cag aaa gtt ctg cca gga gga gac act tac atg cat gaa gga   342
Glu Leu Gln Lys Val Leu Pro Gly Gly Asp Thr Tyr Met His Glu Gly
        90                  95                  100

ttt gaa agg gcc agt gag cag att tat tat gaa aac aga caa ggg tac   390
Phe Glu Arg Ala Ser Glu Gln Ile Tyr Tyr Glu Asn Arg Gln Gly Tyr
    105                 110                 115

agg aca gcc agc gtc atc att gct ttg act gat gga gaa ctc cat gaa   438
Arg Thr Ala Ser Val Ile Ile Ala Leu Thr Asp Gly Glu Leu His Glu
120                 125                 130                 135

gat ctc ttt ttc tat tca gag agg gag gct aat agg tct cga gat ctt   486
Asp Leu Phe Phe Tyr Ser Glu Arg Glu Ala Asn Arg Ser Arg Asp Leu
                140                 145                 150

ggt gca att gtt tac tgt gtt ggt gtg aaa gat ttc aat gag aca cag   534
Gly Ala Ile Val Tyr Cys Val Gly Val Lys Asp Phe Asn Glu Thr Gln
            155                 160                 165

ctg gcc cgg att gcg gac agt aag gat cat gtg ttt ccc gtg aat gac   582
Leu Ala Arg Ile Ala Asp Ser Lys Asp His Val Phe Pro Val Asn Asp
        170                 175                 180

ggc ttt cag gct ctg caa ggc atc atc cac tca att ttg agc tct gct   630
Gly Phe Gln Ala Leu Gln Gly Ile Ile His Ser Ile Leu Ser Ser Ala
    185                 190                 195

tcc cca acc agc cct aag gtc ttc cct ctc agc ctt gac agc acc cct   678
Ser Pro Thr Ser Pro Lys Val Phe Pro Leu Ser Leu Asp Ser Thr Pro
200                 205                 210                 215

caa gat ggt aat gtt gtc gtt gct tgc ctt gtc cag ggt ttc ttc cct   726
Gln Asp Gly Asn Val Val Val Ala Cys Leu Val Gln Gly Phe Phe Pro
                220                 225                 230

cag gag cca ctc tct gtt acc tgg tct gaa tct gga cag aat gtt acc   774
Gln Glu Pro Leu Ser Val Thr Trp Ser Glu Ser Gly Gln Asn Val Thr
            235                 240                 245

gcc aga aac ttc cca cct agc cag gat gcc tcc ggt gac ctc tac acc   822
Ala Arg Asn Phe Pro Pro Ser Gln Asp Ala Ser Gly Asp Leu Tyr Thr
        250                 255                 260

acc agc tct cag ctc acc ctt cca gcc acc cag tgc cca gat ggt aag   870
Thr Ser Ser Gln Leu Thr Leu Pro Ala Thr Gln Cys Pro Asp Gly Lys
    265                 270                 275

tcc gtt acc tgc cat gtt aag cac tac acc aac tcc agc cag gat gtt   918
Ser Val Thr Cys His Val Lys His Tyr Thr Asn Ser Ser Gln Asp Val
280                 285                 290                 295

act gtt cca tgc cgt gtt cca cca cct cca cca tgc tgc cac cca cgt   966
Thr Val Pro Cys Arg Val Pro Pro Pro Pro Pro Cys Cys His Pro Arg
                300                 305                 310

ctc tct ctt cac cgt cct gcc ctt gag gac ttg ctc ttg ggt tct gaa   1014

Leu Ser Leu His Arg Pro Ala Leu Glu Asp Leu Leu Leu Gly Ser Glu
            315                 320                 325

gct aac ctc acc tgc acc ctc acc ggt ctc aga gat gcc tct ggt gcc 1062
Ala Asn Leu Thr Cys Thr Leu Thr Gly Leu Arg Asp Ala Ser Gly Ala
        330                 335                 340

acc ttc acc tgg acc cca agc tct ggt aag agc gct gtt caa gga cca 1110
Thr Phe Thr Trp Thr Pro Ser Ser Gly Lys Ser Ala Val Gln Gly Pro
    345                 350                 355

cct gag cgt gac ctc tgt gga tgc tac tct gtt agc tct gtt ctt cct 1158
Pro Glu Arg Asp Leu Cys Gly Cys Tyr Ser Val Ser Ser Val Leu Pro
360                 365                 370                 375

ggt tgt gcc cag cct tgg aac cac ggt gag acc ttc acc tgc act gct 1206
Gly Cys Ala Gln Pro Trp Asn His Gly Glu Thr Phe Thr Cys Thr Ala
                380                 385                 390

gcc cac cca gag ttg aag acc cca ctt acc gcc aac atc acc aag tcc 1254
Ala His Pro Glu Leu Lys Thr Pro Leu Thr Ala Asn Ile Thr Lys Ser
            395                 400                 405

gga aac acc ttc cgt ccc gag gtc cac ctc ttg cca cca cca tct gag 1302
Gly Asn Thr Phe Arg Pro Glu Val His Leu Leu Pro Pro Pro Ser Glu
        410                 415                 420

gag ctt gcc ctc aat gag ctt gtt acc ctc acc tgc ctt gct cgt gga 1350
Glu Leu Ala Leu Asn Glu Leu Val Thr Leu Thr Cys Leu Ala Arg Gly
    425                 430                 435

ttc agc cca aag gat gtt ctt gtt agg tgg ctt cag gga tct cag gag 1398
Phe Ser Pro Lys Asp Val Leu Val Arg Trp Leu Gln Gly Ser Gln Glu
440                 445                 450                 455

ctt cca cgt gag aag tac ctc act tgg gct tcc cgt cag gag cca agc 1446
Leu Pro Arg Glu Lys Tyr Leu Thr Trp Ala Ser Arg Gln Glu Pro Ser
                460                 465                 470

cag gga act acc acc tac gct gtt acc agc atc ctt cgt gtt gct gct 1494
Gln Gly Thr Thr Thr Tyr Ala Val Thr Ser Ile Leu Arg Val Ala Ala
            475                 480                 485

gag gac tgg aag aag ggt gag acc ttc tcc tgc atg gtt ggt cac gag 1542
Glu Asp Trp Lys Lys Gly Glu Thr Phe Ser Cys Met Val Gly His Glu
        490                 495                 500

gcc ctt cca ctt gcc ttc acc cag aag acc att gat cgt ttg gct gga 1590
Ala Leu Pro Leu Ala Phe Thr Gln Lys Thr Ile Asp Arg Leu Ala Gly
    505                 510                 515

aag cca acc cac atc aat gtt tct gtt gtc atg gct gag gct gat gga 1638
Lys Pro Thr His Ile Asn Val Ser Val Val Met Ala Glu Ala Asp Gly
520                 525                 530                 535

acc tgc tac taa 1650
Thr Cys Tyr

<210> 99
<211> 538
<212> PRT
<213> Unknown Organism

<220>
<223> Description of Unknown Organism: ATR-IgA2 fusion
amino acid

<400> 99
Met Ala Ser Lys Pro Phe Leu Ser Leu Leu Ser Leu Ser Leu Leu Leu
1               5                   10                  15

Phe Thr Ser Thr Ser Leu Ala Asp Leu Tyr Phe Ile Leu Asp Lys Ser
            20                  25                  30

Gly Ser Val Leu His His Trp Asn Glu Ile Tyr Tyr Phe Val Glu Gln
        35                  40                  45

Leu Ala His Lys Phe Ile Ser Pro Gln Leu Arg Met Ser Phe Ile Val
    50                  55                  60

Phe Ser Thr Arg Gly Thr Thr Leu Met Lys Leu Thr Glu Asp Arg Glu
65                  70                  75                  80

Gln Ile Arg Gln Gly Leu Glu Glu Leu Gln Lys Val Leu Pro Gly Gly
                85                  90                  95

Asp Thr Tyr Met His Glu Gly Phe Glu Arg Ala Ser Glu Gln Ile Tyr
            100                 105                 110

Tyr Glu Asn Arg Gln Gly Tyr Arg Thr Ala Ser Val Ile Ile Ala Leu
        115                 120                 125

Thr Asp Gly Glu Leu His Glu Asp Leu Phe Phe Tyr Ser Glu Arg Glu
    130                 135                 140

Ala Asn Arg Ser Arg Asp Leu Gly Ala Ile Val Tyr Cys Val Gly Val
145                 150                 155                 160

Lys Asp Phe Asn Glu Thr Gln Leu Ala Arg Ile Ala Asp Ser Lys Asp
                165                 170                 175

His Val Phe Pro Val Asn Asp Gly Phe Gln Ala Leu Gln Gly Ile Ile
            180                 185                 190

His Ser Ile Leu Ser Ser Ala Ser Pro Thr Ser Pro Lys Val Phe Pro
        195                 200                 205

Leu Ser Leu Asp Ser Thr Pro Gln Asp Gly Asn Val Val Val Ala Cys
    210                 215                 220

Leu Val Gln Gly Phe Phe Pro Gln Glu Pro Leu Ser Val Thr Trp Ser
225                 230                 235                 240

Glu Ser Gly Gln Asn Val Thr Ala Arg Asn Phe Pro Pro Ser Gln Asp
                245                 250                 255

Ala Ser Gly Asp Leu Tyr Thr Thr Ser Ser Gln Leu Thr Leu Pro Ala
            260                 265                 270

Thr Gln Cys Pro Asp Gly Lys Ser Val Thr Cys His Val Lys His Tyr
        275                 280                 285

Thr Asn Ser Ser Gln Asp Val Thr Val Pro Cys Arg Val Pro Pro Pro
    290                 295                 300

Pro Pro Cys Cys His Pro Arg Leu Ser Leu His Arg Pro Ala Leu Glu
305                 310                 315                 320

Asp Leu Leu Leu Gly Ser Glu Ala Asn Leu Thr Cys Thr Leu Thr Gly
                325                 330                 335

Leu Arg Asp Ala Ser Gly Ala Thr Phe Thr Trp Thr Pro Ser Ser Gly
            340                 345                 350

Lys Ser Ala Val Gln Gly Pro Pro Glu Arg Asp Leu Cys Gly Cys Tyr
        355                 360                 365

Ser Val Ser Ser Val Leu Pro Gly Cys Ala Gln Pro Trp Asn His Gly
    370                 375                 380

Glu Thr Phe Thr Cys Thr Ala Ala His Pro Glu Leu Lys Thr Pro Leu
385                 390                 395                 400

Thr Ala Asn Ile Thr Lys Ser Gly Asn Thr Phe Arg Pro Glu Val His
                405                 410                 415

Leu Leu Pro Pro Pro Ser Glu Glu Leu Ala Leu Asn Glu Leu Val Thr
            420                 425                 430

Leu Thr Cys Leu Ala Arg Gly Phe Ser Pro Lys Asp Val Leu Val Arg
        435                 440                 445

Trp Leu Gln Gly Ser Gln Glu Leu Pro Arg Glu Lys Tyr Leu Thr Trp
    450                 455                 460

Ala Ser Arg Gln Glu Pro Ser Gln Gly Thr Thr Thr Tyr Ala Val Thr
465                 470                 475                 480

Ser Ile Leu Arg Val Ala Ala Glu Asp Trp Lys Lys Gly Glu Thr Phe
                485                 490                 495

Ser Cys Met Val Gly His Glu Ala Leu Pro Leu Ala Phe Thr Gln Lys
            500                 505                 510

Thr Ile Asp Arg Leu Ala Gly Lys Pro Thr His Ile Asn Val Ser Val
        515                 520                 525

Val Met Ala Glu Ala Asp Gly Thr Cys Tyr
    530                 535

<210> 100
<211> 6602
<212> DNA
<213> Unknown Organism

<220>
<223> Description of Unknown Organism:
      pGPTV-kan-ocs-ATR-IgA2

<400> 100
ctggccggcg ccagatctgg ggaacctgtg gttggcatgc acatacaaat ggacgaacgg 60
ataaaccttt tcacgccctt ttaaatatcc gattattcta ataaacgctc ttttctctta 120
ggtttacccg ccaatatatc ctgtcaaaca ctgatagttt aaactgaagg cgggaaacga 180
caatctgatc atgagcggag aattaaggga gtcacgttat gacccccgcc gatgacgcgg 240
acaagccgtt ttacgtttgg aactgacaga accgcaacgt tgaaggagcc actcagccga 300
tctgaattca ctgctttaat gagatatgcg agacgcctat gatcgcatga tatttgcttt 360
caattctgtt gtgcacgttg taaaaaacct gagcatgtgt agctcagatc cttaccgccg 420
gtttcggttc attctaatga atatatcacc cgttactatc gtatttttat gaataatatt 480
ctccgttcaa tttactgatt gtaccctact acttatatgt acaatattaa aatgaaaaca 540
atatattgtg ctgaataggt ttatagcgac atctatgata gagcgccaca ataacaaaca 600
attgcgtttt attattacaa atccaatttt gagctcggcg cgccagctgg acatcatgtt 660
ggatatgaaa caactattat ttatctacat gttttagatg ttatctgatt atttttatac 720
gtagtcttct attgatgagg agtctaaggc tatagaatta tatatctaaa tgattaatat 780
atatattatt aataattaac aataattaat atattataat ttatatatat atattttata 840
ttattataat aatattctta caaatataat tattatattc gacggtatcg gggcaattgt 900
attcgacggt atcgcgataa gctcgcggat ccctgaaagc gacgttggat gttaacatct 960
acaaattgcc ttttcttatc gaccatgtac gtaagcgctt acgtttttgg tggacccttg 1020
aggaaactgg tagctgttgt gggcctgtgg tctcaagatg gatcattaat ttccaccttc 1080
acctacgatg gggggcatcg caccggtgag taatattgta cggctaagag cgaatttggc 1140
ctgtaggatc cctgaaagcg acgttggatg ttaacatcta caaattgcct tttcttatcg 1200
accatgtacg taagcgctta cgtttttggt ggacccttga ggaaactggt agctgttgtg 1260
ggcctgtggt ctcaagatgg atcattaatt tccaccttca cctacgatgg ggggcatcgc 1320
accggtgagt aatattgtac ggctaagagc gaatttggcc tgtaggatcc ctgaaagcga 1380
cgttggatgt taacatctac aaattgcctt ttcttatcga ccatgtacgt aagcgcttac 1440
gtttttggtg gacccttgag gaaactggta gctgttgtgg gcctgtggtc tcaagatgga 1500
tcattaattt ccaccttcac ctacgatggg gggcatcgca ccggtgagta atattgtacg 1560
gctaagagcg aatttggcct gtaggatccg cgagctggtc aatcccattg cttttgaagc 1620
agctcaacat tgatctcttt ctcgatcgag ggagattttt caaatcagtg cgcaagacgt 1680
gacgtaagta tccgagtcag tttttatttt tctactaatt tggtcgttta tttcggcgtg 1740
taggacatgg caaccgggcc tgaatttcgc gggtattctg tttctattcc aactttttct 1800
tgatccgcag ccattaacga cttttgaata gatacgctga cacgccaagc ctcgctagtc 1860
aaaagtgtac caaacaacgc tttacagcaa gaacggaatg cgcgtgacgc tcgcggtgac 1920
gccatttcgc cttttcagaa atggataaat agccttgctt cctattatat cttcccttaa 1980
ttaaggtacc acttctctca atccaacttt ctaaacaatg gcttctaaac ctttcttgtc 2040
tcttctttct ttgtctttgc ttttgttcac ctctactagt ttggctgacc tgtacttcat 2100
tttggacaaa tcaggaagtg tgctgcacca ctggaatgaa atctattact ttgtggaaca 2160
gttggctcac aaattcatca gcccacagtt gagaatgtcc tttattgttt tctccacccg 2220
aggaacaacc ttaatgaaac tgacagaaga cagagaacaa atccgtcaag gcctagaaga 2280
actccagaaa gttctgccag gaggagacac ttacatgcat gaaggatttg aaagggccag 2340
tgagcagatt tattatgaaa acagacaagg gtacaggaca gccagcgtca tcattgcttt 2400
gactgatgga gaactccatg aagatctctt tttctattca gagagggagg ctaataggtc 2460
tcgagatctt ggtgcaattg tttactgtgt tggtgtgaaa gatttcaatg agacacagct 2520
ggcccggatt gcggacagta aggatcatgt gtttcccgtg aatgacggct ttcaggctct 2580
gcaaggcatc atccactcaa ttttgagctc tgcttcccca accagcccta aggtcttccc 2640
tctcagcctt gacagcaccc ctcaagatgg taatgttgtc gttgcttgcc ttgtccaggg 2700
tttcttccct caggagccac tctctgttac ctggtctgaa tctggacaga atgttaccgc 2760
cagaaacttc ccacctagcc aggatgcctc cggtgacctc tacaccacca gctctcagct 2820
cacccttcca gccacccagt gcccagatgg taagtccgtt acctgccatg ttaagcacta 2880
caccaactcc agccaggatg ttactgttcc atgccgtgtt ccaccacctc caccatgctg 2940
ccacccacgt ctctctcttc accgtcctgc ccttgaggac ttgctcttgg gttctgaagc 3000
taacctcacc tgcaccctca ccggtctcag agatgcctct ggtgccacct tcacctggac 3060
cccaagctct ggtaagagcg ctgttcaagg accacctgag cgtgacctct gtggatgcta 3120
ctctgttagc tctgttcttc ctggttgtgc ccagccttgg aaccacggtg agaccttcac 3180
ctgcactgct gcccacccag agttgaagac cccacttacc gccaacatca ccaagtccgg 3240
aaacaccttc cgtcccgagg tccacctctt gccaccacca tctgaggagc ttgccctcaa 3300
tgagcttgtt accctcacct gccttgctcg tggattcagc ccaaaggatg ttcttgttag 3360
gtggcttcag ggatctcagg agcttccacg tgagaagtac ctcacttggg cttcccgtca 3420
ggagccaagc cagggaacta ccacctacgc tgttaccagc atccttcgtg ttgctgctga 3480
ggactggaag aagggtgaga ccttctcctg catggttggt cacgaggccc ttccacttgc 3540
cttcacccag aagaccattg atcgtttggc tggaaagcca acccacatca atgtttctgt 3600
tgtcatggct gaggctgatg gaacctgcta ctaagatctg tgaattcctg cagcccgggg 3660
gatccactag ttctagctag agcggccgcc accgcggtgg cgaattaaca gaggtggatg 3720
gacagacccg ttcttacacc ggactgggcg cgggatagga tattcagatt gggatgggat 3780
tgagcttaaa gccggcgctg agaccatgct caaggtaggc aatgtcctca gcgtcgagcc 3840
cggcatctat gtcgagggca ttggtggagc gcgcttcggg gataccgtgc ttgtaactga 3900
gaccggatat gaggccctca ctccgcttga tcttggcaaa gatatttgac gcatttatta 3960
gtatgtgtta attttcattt gcagtgcagt attttctatt cgatctttat gtaattcgtt 4020
acaattaata aatattcaaa tcagattatt gactgtcatt tgtatcaaat cgtgtttaat 4080
ggatattttt attataatat tgatgataat tcactggccg tcgttttaca acgtcgtgac 4140
tgggaaaacc ctggcgttac ccaacttaat cgccttgcag cacatccccc tttcgccagc 4200
tggcgcgcca agcttcacgc tgccgcaagc actcagggcg caagggctgc taaaggaagc 4260
ggaacacgta gaaagccagt ccgcagaaac ggtgctgacc ccggatgaat gtcagctact 4320
ggctatctgg acaagggaaa acgcaagcgc aaagagaaag caggtagctt gcagtgggct 4380
tacatggcga tagctagact gggcggtttt atggacagca agcgaaccgg aattgccagc 4440
tggggcgccc tctggtaagg ttgggaagcc ctgcaaagta aactggatgg ctttcttgcc 4500

gccaaggatc tgatggcgca ggggatcaag atcatgagcg gagaattaag ggagtcacgt 4560
tatgaccccc gccgatgacg cgggacaagc cgttttacgt ttggaactga cagaaccgca 4620
acgttgaagg agccactcag ccgcgggttt ctggagttta atgagctaag cacatacgtc 4680
agaaaccatt attgcgcgtt caaaagtcgc ctaaggtcac tatcagctag caaatatttc 4740
ttgtcaaaaa tgctccactg acgttccata aattcccctc ggtatccaat tagagtctca 4800
tattcactct caatccagat ctggatcgtt tcgcatgatt gaacaagatg gattgcacgc 4860
aggttctccg gccgcttggg tggagaggct attcggctat gactgggcac aacagacaat 4920
cggctgctct gatgccgccg tgttccggct gtcagcgcag gggcgcccgg ttctttttgt 4980
caagaccgac ctgtccggtg ccctgaatga actgcaggac gaggcagcgc ggctatcgtg 5040
gctggccacg acgggcgttc cttgcgcagc tgtgctcgac gttgtcactg aagcgggaag 5100
ggactggctg ctattgggcg aagtgccggg gcaggatctc ctgtcatctc accttgctcc 5160
tgccgagaaa gtatccatca tggctgatgc aatgcggcgg ctgcatacgc ttgatccggc 5220
tacctgccca ttcgaccacc aagcgaaaca tcgcatcgag cgagcacgta ctcggatgga 5280
agccggtctt gtcgatcagg atgatctgga cgaagagcat caggggctcg cgccagccga 5340
actgttcgcc aggctcaagg cgcgcatgcc cgacggcgat gatctcgtcg tgacccatgg 5400
cgatgcctgc ttgccgaata tcatggtgga aaatggccgc ttttctggat tcatcgactg 5460
tggccggctg ggtgtggcgg accgctatca ggacatagcg ttggctaccc gtgatattgc 5520
tgaagagctt ggcggcgaat gggctgaccg cttcctcgtg ctttacggta tcgccgctcc 5580
cgattcgcag cgcatcgcct tctatcgcct tcttgacgag ttcttctgag cgggactctg 5640
aggatccccc gatgagctaa gctagctata tcatcaattt atgtattaca cataatatcg 5700
cactcagtct ttcatctacg gcaatgtacc agctgatata atcagttatt gaaatatttc 5760
tgaatttaaa cttgcatcaa taaatttatg tttttgcttg gactataata cctgacttgt 5820
tattttatca ataaatattt aaactatatt tctttcaaga tgggaattaa ttcactggcc 5880
gtcgttttac aacgtcgtga ctgggaaaac cctggcgtta cccaacttaa tcgccttgca 5940
gcacatcccc ctttcgccag ctggcgtaat agcgaagagg cccgcaccga tcgcccttcc 6000
caacagttgc gcagcctgaa tggcgcccgc tcctttcgct ttcttccctt cctttctcgc 6060
cacgttcgcc ggctttcccc gtcaagctct aaatcggggg ctccctttag ggttccgatt 6120
tagtgcttta cggcacctcg accccaaaaa acttgatttg ggtgatggtt cacgtagtgg 6180
gccatcgccc tgatagacgg tttttcgccc tttgacgttg gagtccacgt tctttaatag 6240
tggactcttg ttccaaactg gaacaacact caaccctatc tcgggctatt cttttgattt 6300
ataagggatt ttgccgattt cggaaccacc atcaaacagg attttcgcct gctggggcaa 6360
accagcgtgg accgcttgct gcaactctct cagggccagg cggtgaaggg caatcagctg 6420
ttgcccgtct cactggtgaa aagaaaaacc accccagtac attaaaaacg tccgcaatgt 6480
gttattaagt tgtctaagcg tcaatttgtt tacaccacaa tatatcctgc caccagccag 6540
ccaacagctc cccgaccggc agctcggcac aaaatcacca ctcgatacag gcagcccatc 6600
ag 6602

<210> 101
<211> 7129
<212> DNA
<213> Unknown Organism

<220>
<223> Description of Unknown Organism:
      pGPTV-hpt-ocs-35SJ/SC

<400> 101
ctgatgggct gcctgtatcg agtggtgatt ttgtgccgag ctgccggtcg gggagctgtt 60
ggctggctgg tggcaggata tattgtggtg taaacaaatt gacgcttaga caacttaata 120
acacattgcg gacgttttta atgtactggg gtggtttttc ttttcaccag tgagacgggc 180
aacagctgat tgcccttcac cgcctggccc tgagagagtt gcagcaagcg gtccacgctg 240
gtttgcccca gcaggcgaaa atcctgtttg atggtggttc cgaaatcggc aaaatccctt 300
ataaatcaaa agaatagccc gagatagggt tgagtgttgt tccagtttgg aacaagagtc 360
cactattaaa gaacgtggac tccaacgtca aagggcgaaa aaccgtctat cagggcgatg 420
gcccactacg tgaaccatca cccaaatcaa gttttttggg gtcgaggtgc cgtaaagcac 480
taaatcggaa ccctaaaggg agcccccgat ttagagcttg acggggaaag ccggcgaacg 540
tggcgagaaa ggaagggaag aaagcgaaag gagcgggcgc cattcaggct gcgcaactgt 600
tgggaagggc gatcggtgcg ggcctcttcg ctattacgcc agctggcgaa agggggatgt 660
gctgcaaggc gattaagttg ggtaacgcca gggttttccc agtcacgacg ttgtaaaacg 720
acggccagtg aattaattcc catcttgaaa gaaatatagt ttaaatattt attgataaaa 780
taacaagtca ggtattatag tccaagcaaa aacataaatt tattgatgca agtttaaatt 840
cagaaatatt tcaataactg attatatcag ctggtacatt gccgtagatg aaagactgag 900
tgcgatatta tgtgtaatac ataaattgat gatatagcta gcttagctca tcgggggatc 960
ccggtcggca tctactctat tcctttgccc tcggacgagt gctggggcgt cggtttccac 1020
tatcggcgag tacttctaca cagccatcgg tccagacggc cgcgcttctg cgggcgattt 1080
gtgtacgccc gacagtcccg gctccggatc ggacgattgc gtcgcatcga ccctgcgccc 1140
aagctgcatc atcgaaattg ccgtcaacca agctctgata gagttggtca agaccaatgc 1200
ggagcatata cgcccggagc cgcggcgatc ctgcaagctc cggatgcctc cgctcgaagt 1260
agcgcgtctg ctgctccata caagccaacc acggcctcca gaagaagatg ttggcgacct 1320
cgtattggga atccccgaac atcgcctcgc tccagtcaat gaccgctgtt atgcggccat 1380
tgtccgtcag gacattgttg gagccgaaat ccgcgtgcac gaggtgccgg acttcggggc 1440
agtcctcggc ccaaagcatc agctcatcga gagcctgcgc gacggacgca ctgacggtgt 1500
cgtccatcac agtttgccag tgatacacat ggggatcagc aatcgcgcat atgaaatcac 1560
gccatgtagt gtattgaccg attccttgcg gtccgaatgg gccgaacccg ctcgtctggc 1620
taagatcggc cgcagcgatc gcatccatgg cctccgcgac cggctgcaga acagcgggca 1680
gttcggtttc aggcaggtct tgcaacgtga caccctgtgc acggcgggag atgcaatagg 1740
tcaggctctc gctgaatgcc ccaatgtcaa gcacttccgg aatcgggagc gcggccgatg 1800
caaagtgccg ataaacataa cgatctttgt agaaaccatc ggcgcagcta tttacccgca 1860
ggacatatcc acgccctcct acatcgaagc tgaaagcacg agattcttcg ccctccgaga 1920
gctgcatcag gtcggagacg ctgtcgaact tttcgatcag aaacttctcg acagacgtcg 1980
cggtgagttc aggctttttc atatcttatt gcccccctag agtcgagatc tggattgaga 2040
gtgaatatga gactctaatt ggataccgag gggaatttat ggaacgtcag tggagcattt 2100
ttgacaagaa atatttgcta gctgatagtg accttaggcg acttttgaac gcgcaataat 2160
ggtttctgac gtatgtgctt agctcattaa actccagaaa cccgcggctg agtggctcct 2220
tcaacgttgc ggttctgtca gttccaaacg taaaacggct tgtcccgcgt catcggcggg 2280

ggtcataacg tgactccctt aattctccgc tcatgatctt gatcccctgc gccatcagat 2340
ccttggcggc aagaaagcca tccagtttac tttgcagggc ttcccaacct taccagaggg 2400
cgccccagct ggcaattccg gttcgcttgc tgtccataaa accgcccagt ctagctatcg 2460
ccatgtaagc ccactgcaag ctacctgctt tctctttgcg cttgcgtttt cccttgtcca 2520
gatagccagt agctgacatt catccggggt cagcaccgtt tctgcggact ggctttctac 2580
gtgttccgct tcctttagca gcccttgcgc cctgagtgct tgcggcagcg tgaagcttgg 2640
cgcgccagct ggacatcatg ttggatatga aacaactatt atttatctac atgttttaga 2700
tgttatctga ttatttttat acgtagtctt ctattgatga ggagtctaag gctatagaat 2760
tatatatcta aatgattaat atatatatta ttaataatta acaataatta atatattata 2820
atttatatat atatatttta tattattata ataatattct tacaaatata attattatat 2880
tcgacggtat cggggcaatt gattcccgat cctatctgtc acttcatcaa aaggacagta 2940
gaaaaggaag gtggcaccta caaatgccat cattgcgata aaggaaaggc tatcattcaa 3000
gatgcctctg ccgacagtgg tcccaaagat ggacccccac ccacgaggag catcgtggaa 3060
aaagaagacg ttccaaccac gtcttcaaag caagtggatt gatgtgatat ctccactgac 3120
gtaagggatg acgcacaatc ccactatcct tcgcaagacc cttcctctat ataaggaagt 3180
tcatttcatt tggagaggac acgctgaaat caccagtctc tctctacaag gtaccatggt 3240
gctcttcgtg ctcacctgcc tgctggcggt cttcccagcc atctccacga agagtcccat 3300
atttggtccc gaggaggtga atagtgtgga aggtaactca gtgtccatca cgtgctacta 3360
cccacccacc tctgtcaacc ggcacacccg gaagtactgg tgccggcagg gagctagagg 3420
tggctgcata accctcatct cctcggaggg ctacgtctcc agcaaatatg caggcagggc 3480
taacctcacc aacttcccgg agaacggcac atttgtggtg aacattgccc agctgagcca 3540
ggatgactcc gggcgctaca agtgtggcct gggcatcaat agccgaggcc tgtcctttga 3600
tgtcagcctg gaggtcagcc agggtcctgg gctcctaaat gacactaaag tctacacagt 3660
ggacctgggc agaacggtga ccatcaactg ccctttcaag actgagaatg ctcaaaagag 3720
gaagtccttg tacaagcaga taggcctgta ccctgtgctg gtcatcgact ccagtggtta 3780
tgtgaatccc aactatacag gaagaatacg ccttgatatt cagggtactg gccagttact 3840
gttcagcgtt gtcatcaacc aactcaggct cagcgatgct gggcagtatc tctgccaggc 3900
tggggatgat tccaatagta ataagaagaa tgctgacctc caagtgctaa agcccgagcc 3960
cgagctggtt tatgaagacc tgaggggctc agtgaccttc cactgtgccc tgggccctga 4020
ggtggcaaac gtggccaaat ttctgtgccg acagagcagt ggggaaaact gtgacgtggt 4080
cgtcaacacc ctggggaaga gggccccagc ctttgagggc aggatcctgc tcaaccccca 4140
ggacaaggat ggctcattca gtgtggtgat cacaggcctg aggaaggagg atgcagggcg 4200
ctacctgtgt ggagcccatt cggatggtca gctgcaggaa ggctcgccta tccaggcctg 4260
gcaactcttc gtcaatgagg agtccacgat tccccgcagc cccactgtgg tgaagggggt 4320
ggcaggaagc tctgtggccg tgctctgccc ctacaaccgt aaggaaagca aaagcatcaa 4380
gtactggtgt ctctgggaag gggcccagaa tggccgctgc cccctgctgg tggacagcga 4440
ggggtgggtt aaggcccagt acgagggccg cctctccctg ctggaggagc caggcaacgg 4500
caccttcact gtcatcctca accagctcac cagccgggac gccggcttct actggtgtct 4560
gaccaacggc gatactctct ggaggaccac cgtggagatc aagattatcg aaggagaacc 4620
aaacctcaag gttcccggga atgtcacggc tgtgctggga gagactctca aggtcccctg 4680
tcactttcca tgcaaattct cctcgtacga gaaatactgg tgcaagtgga ataacacggg 4740
ctgccaggcc ctgcccagcc aagacgaagg ccccagcaag gccttcgtga actgtgacga 4800
gaacagccgg cttgtctccc tgaccctgaa cctggtgacc agggctgatg agggctggta 4860
ctggtgtgga gtgaagcagg gccacttcta tggagagact gcagccgtct atgtggcagt 4920
tgaagagagg aaggcagcgg ggtcccgcga tgtcagccta gcgaaggcag acgctgctcc 4980
tgatgagaag gtgctagact ctggttttcg ggagattgag aacaaagcca ttcaggatcc 5040
caggcttttt gcagagtgaa ttcgttcgta tcatcggttt cgacaacgtt cgtcaagttc 5100
aatgcatcag tttcattgcg cacacaccag aatcctactg agttcgagta ttatggcatt 5160
gggaaaactg tttttcttgt accatttgtt gtgcttgtaa tttactgtgt tttttattcg 5220
gttttcgcta tcgaactgtg aaatggaaat ggatggagaa gagttaatga atgatatggt 5280
ccttttgttc attctcaaat taatattatt tgttttttct cttatttgtt gtgtgttgaa 5340
tttgaaatta taagagatat gcaaacattt tgttttgagt aaaaatgtgt caaatcgtgg 5400
cctctaatga ccgaagttaa tatgaggagt aaaacacttg tagttgtcga cggtatcgat 5460
attaattccc gatcctatct gtcacttcat caaaaggaca gtagaaaagg aaggtggcac 5520
ctacaaatgc catcattgcg ataaaggaaa ggctatcatt caagatgcct ctgccgacag 5580
tggtcccaaa gatggacccc cacccacgag gagcatcgtg gaaaaagaag acgttccaac 5640
cacgtcttca aagcaagtgg attgatgtga tatctccact gacgtaaggg atgacgcaca 5700
atcccactat ccttcgcaag acccttcctc tatataagga agttcatttc atttggagag 5760
gacacgctga aatcaccagt ctctctctag agtaccatgg agaaccattt gcttttctgg 5820
ggagtcctgg cggtttttat taaggctgtt catgtgaaag cccaagaaga tgaaaggatt 5880
gttcttgttg acaacaaatg taagtgtgcc cggattactt ccaggatcat ccgttcttcc 5940
gaagatccta atgaggacat tgtggagaga aacatccgaa ttattgttcc tctgaacaac 6000
agggagaata tctctgatcc cacctcacca ttgagaacca gatttgtgta ccatttgtct 6060
gacctctgta aaaaatgtga tcctacagaa gtggagctgg ataatcagat agttactgct 6120
acccagagca atatctgtga tgaagacagt gctacagaga cctgctacac ttatgacaga 6180
aacaagtgct acacagctgt ggtcccactc gtatatggtg gtgagaccaa aatggtggaa 6240
acagccttaa ccccagatgc ctgctatcct gactgaatcc gcggcgatga gctaagctag 6300
ctatatcatc aatttatgta ttacacataa tatcgcactc agtctttcat ctacggcaat 6360
gtaccagctg atataatcag ttattgaaat atttctgaat ttaaacttgc atcaataaat 6420
ttatgttttt gcttggacta taatacctga cttgttattt tatcaataaa tatttaaact 6480
atatttcttt caagagctca aaattggatt tgtaataata aaacgcaatt gtttgttatt 6540
gtggcgctct atcatagatg tcgctataaa cctattcagc acaatatatt gttttcattt 6600
taatattgta catataagta gtagggtaca atcagtaaat tgaacggaga atattattca 6660
taaaaatacg atagtaacgg gtgatatatt cattagaatg aaccgaaacc ggcggtaagg 6720
atctgagcta cacatgctca ggttttttac aacgtgcaca acagaattga aagcaaatat 6780
catgcgatca taggcgtctc gcatatctca ttaaagcagt gaattcagat cggctgagtg 6840
gctccttcaa cgttgcggtt ctgtcagttc caaacgtaaa acggcttgtc ccgcgtcatc 6900
ggcggggtca taacgtgact cccttaattc tccgctcatg atcagattgt cgtttcccgc 6960
cttcagttta aactatcagt gtttgacagg atatattggc gggtaaacct aagagaaaag 7020
agcgtttatt agaataatcg gatatttaaa agggcgtgaa aaggtttatc cgttcgtcca 7080
tttgtatgtg catgccaacc acaggttccc cagatctggc gccggccag 7129
30905.004.WO

## Description

### RELATED APPLICATIONS

This application claims priority as a continuation-in-part application of Larrick andWycoff, International Patent Application Ser. No. PCT/US01/13932, filed April 28, 2001, and entitled NOVEL IMMUNOADHESIN FOR THE PREVENTION OF RHINOVIRUS INFECTION, which in turn claims priority to United States Provisional Application Ser. No. 60/200,298, filed April 28, 2000, and entitled the same. Each of these applications is herein incorporated by reference in its entirety, including all figures, drawings, and sequence listings.

### STATEMENT REGARDING FEDERALLY SPONSORED RESEARCH OR DEVELOPMENT

Federal research support was provided in the form of an SBIR Phase I grant (R43 AI43122) and SBIR Phase II grant (2R44AI43122-02).

### FIELD OF THE INVENTION

The present invention relates to immunoadhesins, their production from plants, and their use in the treatment and prevention of toxicity and pathogen-mediated ailments such as anthrax and the common cold.

### BACKGROUND OF THE INVENTION

The common cold is generally a relatively mild disease. However, significant complications resulting from colds, such as otitis media, sinusitis and asthma exacerbations are common. Human rhinoviruses (HRV) cause up to 50% of all adult colds and 25% of colds in children (Bella and Rossmann, J Struct Biol. 128:69-74, 1999, and Sperber and Hayden, Antimicrob Agents Chemother. 32:409-19, 1988). The cost to society runs into billions of dollar per year. These small, nonenveloped RNA viruses represent a subgroup of picornavirus (Rueckert, Virology, pp. 507-548, eds. Fields, et al., Raven Press, Ltd. New York, 1990) X-ray crystallography of rhinovirus identified a capsid 300 Å in diameter (1 Å = 0.1 nm) with icosahedral symmetry, constructed from sixty copies each of the viral coat proteins VP1, VP2, and VP3 (Rossmann, Nature 317:145-153, 1985). A surface depression or "canyon" on HRV was suggested as the receptor binding site (Colonno, et al., Proc Natl Acad Sci U S A. 85:5449-5453, 1985; Rossmann, et al. Nature 317:145-153, 1985). Of the 102 characterized HRV serotypes, 91 (known as the major group) share as their receptor a cell surface glycoprotein known as intercellular adhesion molecule-1 (ICAM-1) (Greve, et al., Cell 56:839-847, 1989; Staunton, et al., Cell 56:849-853, 1989); the binding site is located within N-terminal domain 1 (Greve, et al., J Virol. 65:6015-6023, 1991; Staunton, et al., Cell 61:243-254, 1990).

ICAM-1 is a membrane protein with five extracellular domains, a hydrophobic transmembrane domain, and a short cytoplasmic domain. ICAM-1 is expressed on many cells important in immune and inflammatory responses, and is inducible on others (Casasnovas, et al., Proc Natl Acad Sci USA. 95:4134-9, 1998). ICAM-1 functions as a ligand for the leukocyte integrins LFA-1 and Mac-1 (Springer, Cell. 76:301-14, 1994; Staunton et al., Cell 61:243-254, 1990). On the cell surface, ICAM-1 is primarily a dimer due to association of the transmembrane domains (Miller, et al., JExp Med. 182:1231-41, 1995; Reilly, et al J Immunol. 155:529-32, 1995).

Recombinant, soluble forms of ICAM-1 (sICAM-1) consisting of the five extracellular domains were shown to be effective in blocking rhinovirus infection of human cells *in vitro* (Greve, et al., J Virol. 65:6015-6023, 1991; Marlin, et al., Nature. 344:70-2, 1990). Evaluation of sICAM-1 activity against a spectrum of laboratory strains and field isolates showed that all major strains of HRV are sensitive to sICAM-1. Minor strains, which do not use ICAM as a receptor, were unaffected by sICAM-1 *(*Crump et al., Antiviral Chem Chemother. 4:323-327, 1993; Ohlin, et al., Antimicrob Agents Chemother. 38:1413-5, 1994).

The anti-viral activity of soluble ICAM-1 *in vitro* appears to be mediated by more than one mechanism. These mechanisms include competition with cell-surface ICAM-1 for binding sites, interference with virus entry or uncoating, and direct inactivation by premature release of viral RNA and formation of empty capsids (Arruda, et al., Antimicrob Agents Chemother. 36:1186-1191, 1992; Greve, et al., J Virol. 65:6015-6023, 1991; Marlin, et al., Nature 344:70-2, 1990; Martin et al., J Virol. 67:3561-8, 1993).

The host range of HRV is restricted to primates. A recent study showed that soluble ICAM-1 was effective in preventing rhinovirus infection in chimpanzees (Huguenel, et al., Am JRespir Crit Care Med. 155:1206-10, 1997). Although chimpanzees do not show clinical symptoms, infection was demonstrated by measuring seroconversion and virus shedding. A single dose of 10 mg of soluble ICAM-1 as an intranasal spray was effective at preventing infection by HRV-16 when co-administered with HRV, or when the virus was administered ten minutes later.

A human clinical trial with soluble ICAM-1 showed that it reduced the severity of experimental HRV colds (Turner, et al., JAMA 281:1797-804, 1999). In this trial a total of 196 subjects received either soluble ICAM-1 or placebo in various formulations. Some subjects were given soluble ICAM-1 or placebo starting seven hours before inoculation with HRV 39 and others were started twelve hours after virus inoculation. Medications were administered as either an intranasal solution or powder, given in six daily doses for seven days (a total of 4.4 mg per day). In this study, soluble ICAM-1 did not prevent infection, as measured by either virus isolation or seroconversion (infection rate of 92% for placebo-treated vs. 85% of soluble ICAM-1 treated). However, soluble ICAM-1 did have an impact on all measures of illness. The total symptom score was reduced by 45%, the proportion of subjects with clinical colds was reduced 23% and nasal mucus weight was reduced by 56%. There was not a significant difference between the use of powder or solution formulations, or between pre- and post-inoculation groups. Treatment with soluble ICAM-1 did not result in any adverse effects or evidence of absorption through the nasal mucosa. Also, there was no inhibition of the development of anti-HRV type-specific antibodies.

As discussed, ICAM-1 is dimeric on the cell surface. Martin et al., in J Virol. 67:3561-8, (1993) first proposed that multivalent binding to HRV by a multimeric soluble ICAM might result in a higher effective affinity, termed avidity, and thus facilitate uncoating of the virus. They constructed multivalent, ICAM-1/immunoglobulin molecules, postulating that these would be more effective than monovalent soluble ICAM-1 in neutralizing HRV and thus would have increased therapeutic utility. These ICAM-1/immunoglobulin molecules included ICAM-1 amino-terminal domains 1 and 2 fused to the hinge and constant domains of the heavy chains of IgA1 (IC1-2D/IgA), IgM (IC1-2D/IgM) and IgG1(IC1-2D/IgG). In addition, five extracellular domains were fused to IgA1 (IC1-5D/IgA). These ICAM-1/immunoglobulin molecules were compared with soluble forms of ICAM-1 having two (sIC1-2D) and five (sIC1-5D) domains in assays of HRV binding, infectivity and conformation. The ICAM-1/IgA immunoglobulin (IC1-SD/IgA) was 200 times, and the ICAM-1/IgM immunoglobulin (IC1-2D/IgM) and ICAM-1/IgG immunoglobulin molecules (IC1-2D/IgG) were 25 and 10 times, more effective than soluble ICAM-1. These molecules were highly effective in inhibiting rhinovirus binding to cells and disrupting the conformation of the virus capsid. The ICAM-1/IgA immunoglobulin molecules were effective in the nanomolar concentration range. Comparison of IC1-2D/IgA and IC1-2D/IgG showed that the class of Ig constant region used had a large impact on efficacy.

A subsequent study compared the inhibitory activities of soluble ICAM-1 and IC1-SD/IgA against nine major HRV serotypes and a variant of HRV-39 selected for moderate resistance to soluble ICAM-1 (Crump, et al., Antimicrob Agents Chemother. 38:1425-7, 1993). IC1-SD/IgA was more potent than monomeric soluble ICAM-1 by 50 to 143 times on a weight basis and by 60 to 170 times on a molar basis against the standard serotypes. The HRV-39 variant was 38-fold more resistant to soluble ICAM-1 than the wild-type, and it was only 5-fold more resistant to IC1-5D/IgA. This is consistent with the hypothesis that virus escape from inhibition by multivalent molecules would be expected to occur at lower frequency than virus escape from inhibition by monomeric soluble receptor (Martin, et al., J Virol. 67:3561-8, 1993). An assay designed to measure viral inactivation showed that HRV-39 and HRV-13 were not directly inactivated to a significant extent by soluble ICAM-1 (<0.5 log₁₀ reduction in infectivity). However, incubation with IC1-5D/IgA resulted in a reduction of infectivity of these same viruses by about 1.0 log₁₀ (Crump, et al., Antimicrob Agents Chemother. 38:1425-7, 1994). Results by Martin et al. (J Virol. 67:3561-8, 1993) suggest that the greater the valence, the greater the effectiveness of the molecules. Dimeric and decameric forms of IC1-2/IgM were separable by sucrose gradient sedimentation. The decameric form was five times more effective than the dimeric form at blocking binding of HRV to HeLa cells.

The ICAM-1/immunoglobulin molecules that have been described suffer from several drawbacks, including the laborious production techniques and high costs associated with those production methods. In addition, the previously described ICAM-1/immunoglobulin molecules have limited stability as multimers in the harsh environment in which the molecule must inactivate rhinoviruses.

Applicants' previous, commonly owned application, International Application Ser. No. PCT/US01/13932, described the construction, purification, and use of chimeric immunoadhesin molecules, with examples and claims directed to treating or preventing viral infections and diseases, e.g., the common cold. There is a need for similar agents for the treatment and prevention of toxicity and other pathogen-mediated diseases and ailments, e.g., bacterial infections and diseases, such as anthrax. The bioterrorism scare following September 11, 2001 underscores this need.

The tripartite toxin secreted by *Bacillus anthracis,* the causative agent of anthrax, helps the bacterium evade the immune system and can kill the host during a systemic infection. Two components of the toxin enzymatically modify substrates within the cytosol of mammalian cells: oedema factor (OF) is an adenylate cyclase that impairs host defences through a variety of mechanisms including inhibiting phagocytosis; lethal factor (LF) is a zinc-dependent protease that cleaves mitogen-activated protein kinase kinase and causes lysis of macrophages. Protective antigen (PA), the third component, binds to a cellular receptor and mediates delivery of the enzymatic components to the cytosol. After binding to the cell-surface receptor, PA is cleaved into two fragments by a furin-like protease. The amino-terminal fragment, PA₂₀, dissociates into the medium, and this allows the carboxy-terminal fragment, PA₆₃ to heptamerize and bind LF and OF. The resulting complexes of [PA₆₃]₇ with OF and/or LF are taken up into cells by receptor-mediated endocytosis and moved to a low-pH endosomal compartment. There, the acidic environment induces a conformational change in [PA₆₃]₇ that allows it to insert into the membrane and form a pore. This conversion promotes the translocation of bound OF and LF across the endosomal membrane to the cytosol.

The immunoadhesins of the present invention may be tailored to combat any pathogenic agent or poison and has significant advantages over what has been described in the art. The immunoadhesins of the present invention that are expressed in plants would be tetrameric, rather than only dimeric. Immunoadhesins having multiple binding sites have a higher effective affinity for the pathogen/toxicant, thereby increasing the effectiveness of the immunoadhesin. In addition, the association of secretory component and immunoglobulin J chain with the immunoadhesin of the present invention increases the stability of the immunoadhesin in the mucosal environment (Corthesy, Biochem Soc Trans. 25:471-475, 1997). Secretory IgA, which is associated with secretory component, is the antibody isotype normally found in mucosal secretions, including milk and colostrum. Unlike other antibody isotypes, SIgA can pass through the gut with very little proteolytic degradation. It also is very stable in crude plant preparations at room temperature. A function of the secretory component appears to be to protect the antibody from the harsh environment of the mucosa (Paul, Fundamental Immunology, Raven Press, NY, Third Edition, pp. 303-304, 1993). Furthermore, the immunoadhesins of the present invention are significantly less expensive to produce in plants than in animal cell culture, and production in plants would make it safer for human use, since plants are not known to harbor any animal viruses.

The preceding discussed documents as well as those which follow may be useful in understanding the invention but are not admitted to be prior art to the invention:
Bäumlein H, Wobus U, Pustell J, Kafatos FC (1986) The legumin gene family: structure of a B type gene of Vicia faba and a possible legumin gene specific regulatory element. Nucl. Acids Res. 14: 2707-2713
Becker D, Kemper E, Schell J, Masterson R (1992) New plant binary vectors with selectable markers located proximal to the left T-DNA border. Plant Mol. Biol. 20: 1195-1197
Bradley KA, Mogridge J, Mourez M, Collier RJ, Young JAT (2001) Identification of the cellular receptor for anthrax toxin. Nature 414: pre-publication
Chintalacharuvu KR, Raines M, Morrison SL (1994) Divergence of human alpha-chain constant region gene sequences. A novel recombinant alpha 2 gene. Journal of Immunology 152: 5299-5304
Crump et al. (1994) Comparative Antirhinoviral Activities of Soluble Intercellular Adhesion Molecule-1 (sICAM-1) and Chimeric ICAM-1Immunoglobulin A Molecule. Antimicrobial Agents and Chemotherapy, 38:6, p. 1425-1427
Depicker A, Stachel S, Dhaese P, Zambryski P, Goodman HM (1982) Nopaline synthase: transcript mapping and DNA sequence. J. Mol. Appl. Genet. 1: 561-573
Gielen J, De Beuckeleer M, Seurinck J, Deboeck F, De Greve H, Lemmers M, Van Montagu M, Schell J (1984) The complete nucleotide sequence of the TL-DNA of the Agrobacterium tumefaciens plasmid pTiAch5. Embo J 3: 835-46
Greve et al.(1991) EP 0468257, Multimeric form of human rhinovirus receptor protein.
Horsch RB, Fry JE, Hoffmann NL, Eichholtz D, Rogers SG, Fraley RT (1985) A simple and general method for transferring genes into plants. Science 227: 1229-1231
Ingelbrecht I, Breyne P, Vancompernolle K, Jacobs A, Van Montagu M, Depicker A (1991) Transcriptional interference in transgenic plants. Gene 109: 239-242
MacDonald MH, Mogen BD, Hunt AG (1991) Characterization of the polyadenylation signal from the T-DNA-encoded octopine synthase gene. Nucleic Acids Res 19: 5575-81
Martin et al. (1993), Efficient Neutralization and Disruption of Rhinovirus by Chimeric ICAM-1/Immunoglobulin Molecules. J. of Virology, 67:6, p. 3561-3568.
Mogen BD, MacDonald MH, Leggewie G, Hunt AG (1992) Several distinct types of sequence elements are required for efficient mRNA 3' end formation in a pea rbcS gene. Mol Cell Biol 12: 5406-14
Ni M, Cui D, Einstein J, Narasimhulu S, Vergara CE, Gelvin SB (1995) Strength and tissue specificity of chimeric promoters derived from the octopine and mannopine synthase genes. Plant Journal 7: 661-676
Sawant SV, Singh PK, Gupta SK, Madnala R, Tuli R (1999) Conserved nucleotide sequences in highly expressed genes in plants. Journal of Genetics 78: 123-131
St Croix B, Rago C, Velculescu V, Traverso G, Romans KE, Montgomery E, Lal A, Riggins GJ, Lengauer C, Vogelstein B, Kinzler KW (2000) Genes expressed in human tumor endothelium. Science 289: 1197-202.
Yamamoto YY, Tsuji H, Obokata J (1995) 5'-leader of a photosystem I gene in Nicotiana sylvestris, psaDb, contains a translational enhancer. J Biol Chem 270: 12466-70**.**

### SUMMARY OF THE INVENTION

The present invention contemplates an immunoadhesin comprising a chimeric molecule having a toxin receptor protein linked to at least a portion of an immunoglobulin heavy chain, wherein J chain and secretory component are associated with the chimeric molecule. A toxin receptor as used here in is a receptor molecule or a part of a receptor molecule, at least a portion of which is a protein or peptide found on the surface of or in the cells of a host organism, to which toxicants, e.g., poisons or pathogenic organisms such as viruses, bacteria, fungi, parasites (or a molecule produced by such pathogenic organism) etc. attach as part of the disease generating process. In a preferred embodiment the toxin receptor will be the extra-cellular domain of a receptor molecule. The toxin receptor may be glycosylated or non-glycosylated. Alterations and modifications to the receptor protein or portion thereof are also contemplated, provided such modifications do not destroy the ability of the receptor to bind the toxin, toxicant, pathogen, or pathogen component.

In some embodiments in which the receptor protein is a viral receptor protein, the immunoadhesin of the present invention is comprised of a rhinovirus receptor protein made of any combination of extracellular domains 1, 2, 3, 4 and 5 of the rhinovirus receptor protein, ICAM-1, linked to an immunoglobulin heavy chain. Also contemplated by the present invention are immunoadhesins of the present invention in which the immunoglobulin is IgA, IgA1, IgA2, IgG1, IgG2, IgG3, IgG4, IgM, IgD, IgE or a chimeric immunoglobulin heavy chain made up of domains or segments from different immunoglobulin isotypes.

In other preferred embodiments of the present invention, the immunoadhesin comprises multiple chimeric ICAM-1 molecules associated with J chain and secretory component. The increase in valency results in a higher effective affinity for the rhinovirus, thereby increasing the effectiveness of the immunoadhesin.

In a preferred embodiment of the present invention, all proteins used to make the immunoadhesin of the present invention are human proteins. In addition to production in plants or plant cells, the present invention contemplates an immunoadhesin expressed in mammalian cells, hairy root cultures, plant cells in tissue culture, and heterologous cells derived from plants, vertebrates or invertebrates.

In preferred embodiments of the present invention, the immunoadhesins are expressed, in plants, including monocotyledonous plants and dicotyledonous plants as a part of the plants genome. Expression in plants, as opposed to expression in cultured cells, allows for a significant reduction in the cost of producing the immunoadhesin.

The present invention contemplates an immunoadhesin having plant-specific glycosylation. A gene coding for a polypeptide having within its amino acid sequence, the glycosylation signal asparagine-X-serine/threonine, where X can be any amino acid residue, is glycosylated via oligosaccharides linked to the asparagine residue of the sequence when expressed in a plant cell. See Marshall, Ann. Rev. Biochem., 41:673 (1972) and Marshall, Biochem. Soc. Symp., 40:17 (1974) for a general review of the polypeptide sequences that function as glycosylation signals. These signals are recognized in both mammalian and in plant cells. At the end of their maturation, proteins expressed in plants or plant cells have a different pattern of glycosylation than do proteins expressed in other types of cells, including mammalian cells and insect cells. Detailed studies characterizing plant-specific glycosylation and comparing it with glycosylation in other cell types have been performed, for example, in studies described by Cabanes-Macheteau et al., Glycobiology 9(4):365-372 (1999), and Altmann, Glycoconjugate J. 14:643-646 (1997). These groups and others have shown that plant-specific glycosylation generates glycans that have xylose linked β(1,2) to mannose, but xylose is not linked β(1,2) to mannose as a result of glycosylation in mammalian and insect cells. Plant-specific glycosylation results in a fucose linked α(1,3) to the proximal GlcNAc, while glycosylation in mammalian cells results in a fucose linked α(1,6) to the proximal GlcNAc. Furthermore, plant-specific glycosylation does not result in the addition of a sialic acid to the terminus of the protein glycan, whereas in glycosylation in mammalian cells, sialic acid is added.

In other embodiments, the immunoadhesin of the present invention is part of a composition comprising plant material and the immunoadhesin, associated with J chain and secretory component. The plant material present may be plant cell walls, plant organelles, plant cytoplasms, intact plant cells, viable plants, and the like. The particular plant materials or plant macromolecules that may be present include ribulose bisphosphate carboxylase, light harvesting complex, pigments, secondary metabolites or chlorophyll. Compositions of the present invention may have an immunoadhesin concentration of between 0.001 % and 99.9% mass excluding water. In other embodiments, the immunoadhesin is present in a concentration of 0.01 % to 99% mass excluding water. In other embodiments, the compositions of the present invention have plant material or plant macromolecules present at a concentration of 0.01 % to 99% mass excluding water.

The present invention also contemplates methods for the treatment or prevention of human rhinovirus infection in a subject, including reducing the infection by human rhinovirus of host cells susceptible to infection by the virus, or reducing the initiation or spread of the common cold due to human rhinovirus, by a method comprising contacting the virus with an immunoadhesin of the present invention, wherein the immunoadhesin binds to the human rhinovirus and reduces infectivity. The immunoadhesin could mediate infection by competition with cell-surface ICAM-1 for binding sites, interference with virus entry or uncoating, and/or direct inactivation by premature release of viral RNA and formation of empty capsids (Arruda, et al., Antimicrob. Agents Chemother. 36:1186-1191, 1992; Greve, et al., J. Virol. 65:6015-6023, 1991; Martin, et al., Nature 344:70-2, 1990; Martin, et al., J. Virol. 67:3561-8, 1993). In another embodiment, human rhinovirus infection in a subject is treated by a method comprising intranasally administering to the subj ect an effective amount of an immunoadhesin of the present invention, wherein the immunoadhesin reduces human rhinovirus infectivity.

Other aspects of the invention contemplate immunoadhesins, compositions, and methods of use thereof in which the immunoadhesins are active against a bacterium or bacteria. In such aspects, the immunoadhesins contain a receptor protein that binds a bacterium of interest, e.g., *Bacillus anthracis,* or a pathogenic component thereof, e.g., protective antigen (PA). In some preferred embodiments for the treatment or prevention of anthrax, that receptor protein is the Anthrax toxin receptor protein or a portion thereof. The portion can be an extracellular domain or a portion of that domain. Additional or alternative embodiments can track those already described for ICAM immunoadhesins, discussed above. For example, at least a portion of an immunoglobulin heavy chain, a J chain, and a secretory component as described above are also present in some preferred embodiments.

In another distinct aspect the invention features a method for reducing or preventing the binding of toxin or pathogen (e.g., the protective antigen (PA) of *Bacillus anthracis*) to host cells susceptible to damage by said toxin or pathogen by contacting the toxin or pathogen with immunoadhesins that bind to it, thereby decoying the toxin or pathogen, and masking and/or ameliorating its pathological effect. Other aspects feature methods of reducing mortality and morbity based on this concept.

In poison contexts, the invention also features poison antidotes that comprise a poison receptor or portion thereof linked to an immunoahesin.

While anthrax and the common cold are two enumerated targets in various aspects of the invention, the invention as concerns immunoadhesins generally can make use of any known receptor protein or portion thereof that can bindto a component of any pathogen or toxicant, which component is required by that pathogen to exert its pathogenic or toxic effect. In addition to natural pathogens, toxicants include but are not limited to venom, carcinogens, mutagens, or other metabolic inhibitors or accelerators that can have a negative effect on cells, tissues, organs, or organisms. The principle described herein can thus be used to prevent, treat, ameliorate, or modulate any type of toxicity or pathogen-mediated disease or symptom caused by such..

### BRIEF DESCRIPTION OF THE DRAWINGS

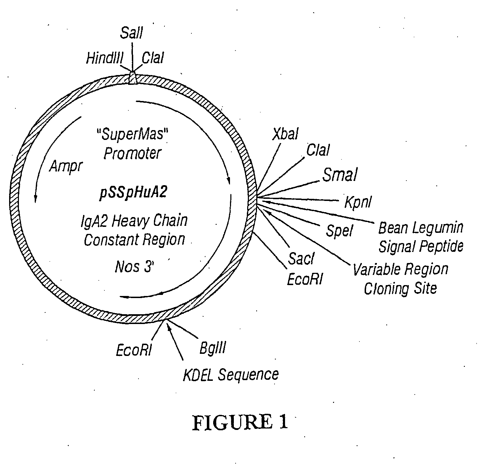

FIG. 1 illustrates pSSPHuA2, vector in which DNAs encoding a chimeric ICAM-1 molecule containing the first five domains of human ICAM-1 and the Fc region of human IgA2m(2) were fused [SEQ ID NO:9, 48]. This vector contains the SuperMas promoter for driving the expression of a signal peptide and the constant regions of the human IgA2m(2) heavy-chain. Sequences encoding ICAM domains 1-5 were amplified, by PCR, to contain convenient restriction sites (5' SpeI and 3' Spe I) for insertion between the signal peptide and the Cα1 domain. DNA encoding an ER retention signal (RSEKDEL) [SEQ ID NO:5] was appended to the 3' end of the heavy-chain in order to boost the expression level of the construct.
FIG. 2 illustrates a chimeric ICAM molecule. 2A shows the DNA expression cassette from which the chimeric ICAM-1 molecule was produced. 2B shows the amino acid sequence, after signal peptide cleavage, of the mature form of the fusion protein [SEQ ID NO:8]. Amino acids introduced by the cloning procedure are underlined and mark the junction between the five extracellular domains of ICAM-1 and the Cα1-Cα3 domains of the IgA2m(2) heavy chain. The bolded N's indicate the fifteen potential glycosylation sites.

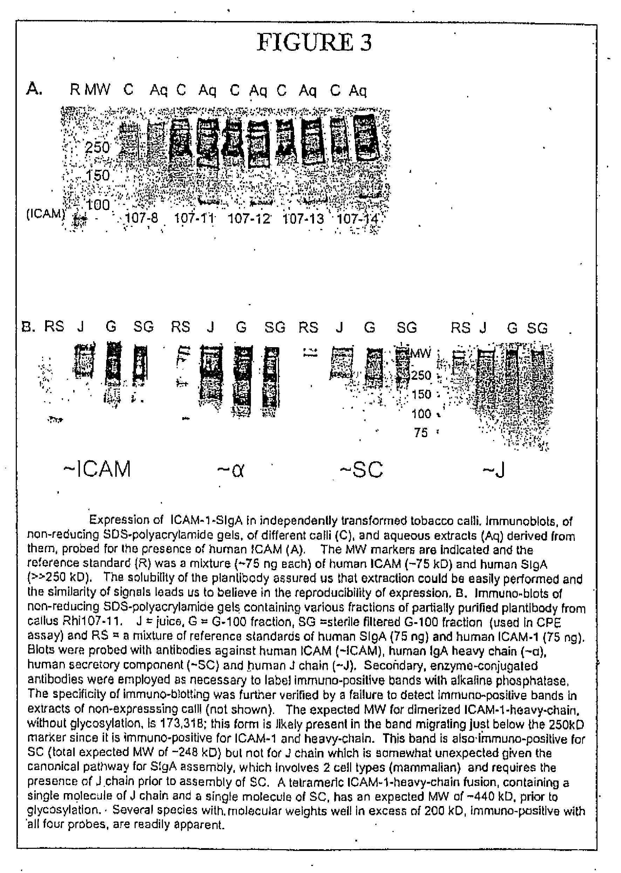

FIG. 3 illustrates the expression of the immunoadhesin in independently transformed tobacco calli. 3A shows immunoblots of non-reducing SDS-polyacrylamide gels on which samples containing different transformed tobacco calli (C) and aqueous extracts (Aq) were run and probed for the presence of human ICAM. The molecular weight markers are indicated, and the reference standard (R) was a mixture (~75 ng each) of human ICAM (~75 kD) and human SigA (>>250 kD). 3B shows immunoblots of nonreducing SDS-polyacrylamide gels containing various fractions of partially purified immunoadhesin from callus Rhi107-11. The purification fractions analyzed were juice (J), G-100 fraction (G), sterile filtered G-100 fraction (SG), and a mixture of reference standards of human SigA (75 ng) and human ICAM-1 (75ng) (RS).
   Blots were probed with antibodies against human ICAM (~ICAM), human IgA heavy chain (~α), human secretory component (~SC) and human J chain (~J). Secondary, enzyme-conjugated antibodies were employed as necessary to label immune-positive bands with alkaline phosphatase.
FIG. 4 illustrates the results of an enzyme-linked immunosorbent assay (ELISA) showing competition between plant extract and soluble ICAM-1 for binding to an anti-ICAM mAb. For the assay, 96-well plates were coated with 0.25 µg soluble ICAM-1/ml. The squares represent the increasing concentrations of sICAM and the circles represent the increasing amounts of callus extract (sterile filtered fraction from G-100) used to compete with the adhered ICAM for a constant amount of a mouse (anti-human ICAM) antibody.

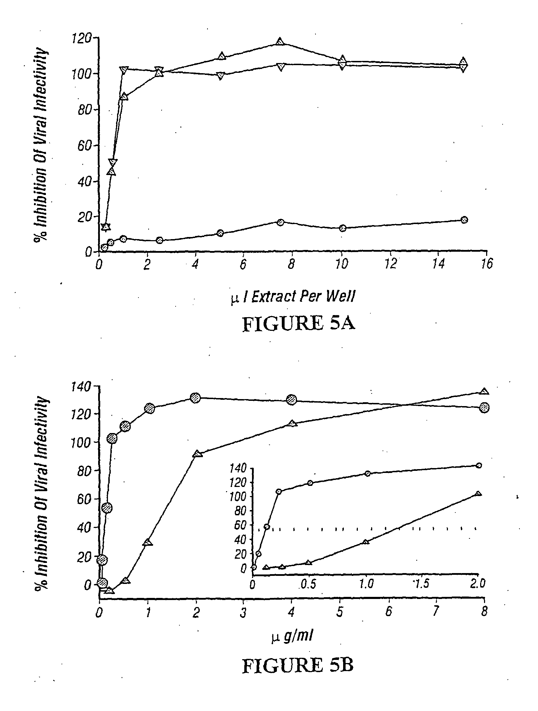

FIG. 5 illustrates the results of an assay showing the ability of an immunoadhesin to inhibit human rhinovirus killing of HeLa cells (cytopathic effect, or CPE, assay). 5A shows the results of an assay comparing the CPE of human rhinovirus on HeLa cells in the presence of partially purified extracts containing either the immunoadhesin in the ICAM-Fc fusion (IC1-SD/IgA) or containing an antibody against doxorubicin. (The right side-up and upside-down triangles represent two extracts derived from Rhi107-11, containing the immunoadhesin.) 5B shows the results of an assay comparing the CPE of human rhinovirus on HeLa cells in the presence of soluble human ICAM-1 or an extract from the immunoadhesin in the ICAM-Fc fusion (IC1-5D/IgA). The Inset shows scale expansion in the range of the IC50 for soluble ICAM (1.35 µg/ml) and for IC1-5D/IgA (0.12 µg/ml; 11.3 fold-less).

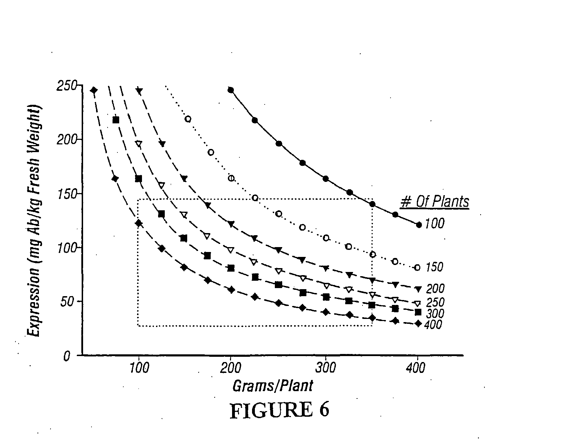

FIG. 6 shows an evaluation of the production necessities for making 1 gram of finished immunoadhesin. In this diagram, the number of plants needed for 1 g of immunoadhesin, at 20% yield, at expected levels of expression and plant weight is illustrated. At different levels of immunoadhesin expression (mg/kg fresh weight) and overall recovery (set at 20%), the weight of each plant, and so the total number of plants, may be determined for a specified production target (1 g/harvest) within a window (dotted square) of reasonable possibilities. The number of required plants decreases, inversely, with the number of specified growth and re-growth periods. The expected biomass production, a function of time and growth conditions, influences the time to harvest and the time between harvests. These growth periods can be adjusted to the realities of the purification schedule by staggering planting and harvesting dates.
FIG. 7 shows the coding and amino acid sequences of each of the immunoglobulin genes and proteins listed in Table 2 [SEQ ID NO:15 through 47 and SEQ ID NO:52 through 62].
FIG. 8 shows the sequences of plasmids used to transform plants, as described in Example 2, for use in studies of the expression of immunoadhesins of the present invention.
FIG. 8A shows the nucleotide [SEQ ID NO:9] and protein [SEQ ID NO:48] sequences for plasmids PSSpICAMHuA2
FIG 8B shows the nucleotide and protein [SEQ ID NO:10] sequence for the bean legumin signal peptide.
FIG 8C shows the nucleotide [SEQ ID NO:11] and amino acid [SEQ ID NO:50] sequence of the protein coding region of pSHuJ.
FIG 8D shows the nucleotide [SEQ ID NO:12] and amino acid [SEQ ID NO:51] sequence of protein coding region of pSHuSC.
FIG 8E shows the nucleotide sequence [SEQ ID NO:13] of plasmid pBMSP-1.
FIG 8F shows the nucleotide sequence [SEQ ID NO:14] of plasmid pBMSP-1spJSC.

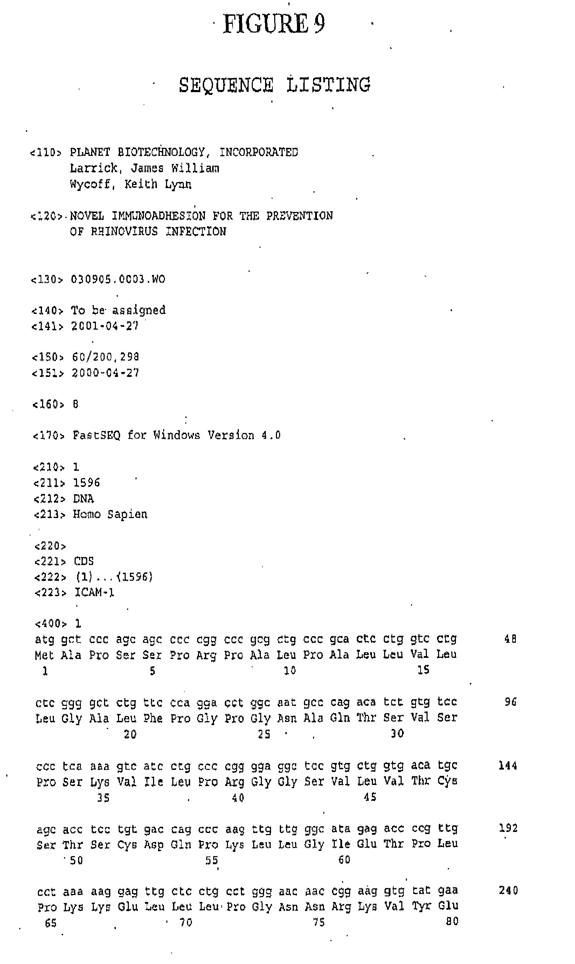

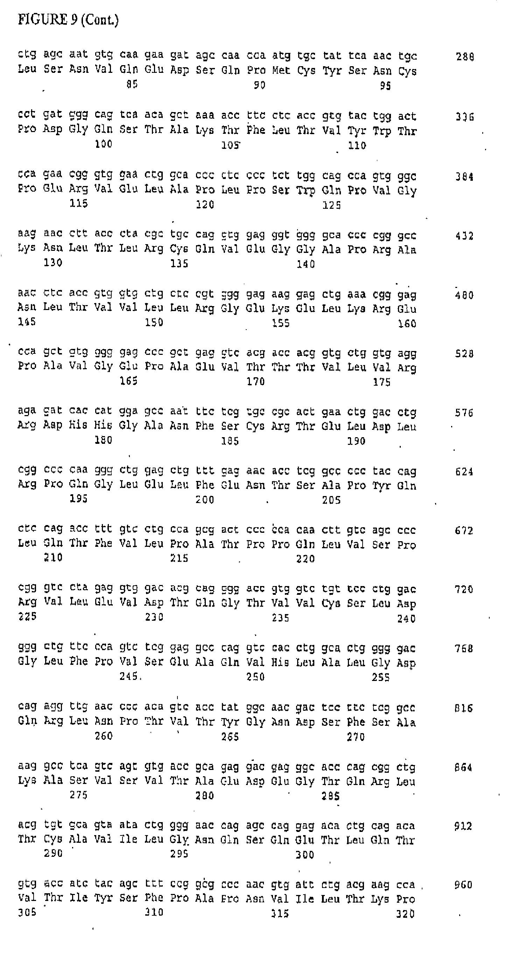

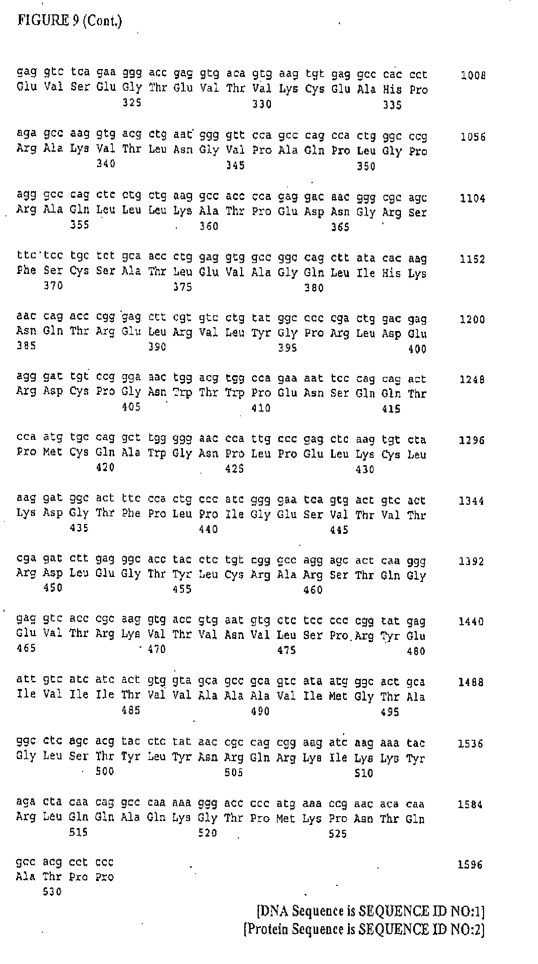

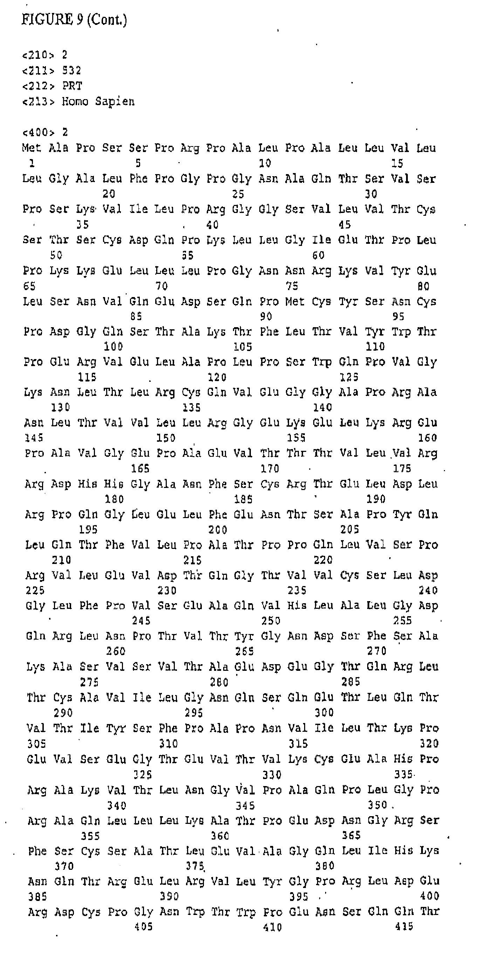

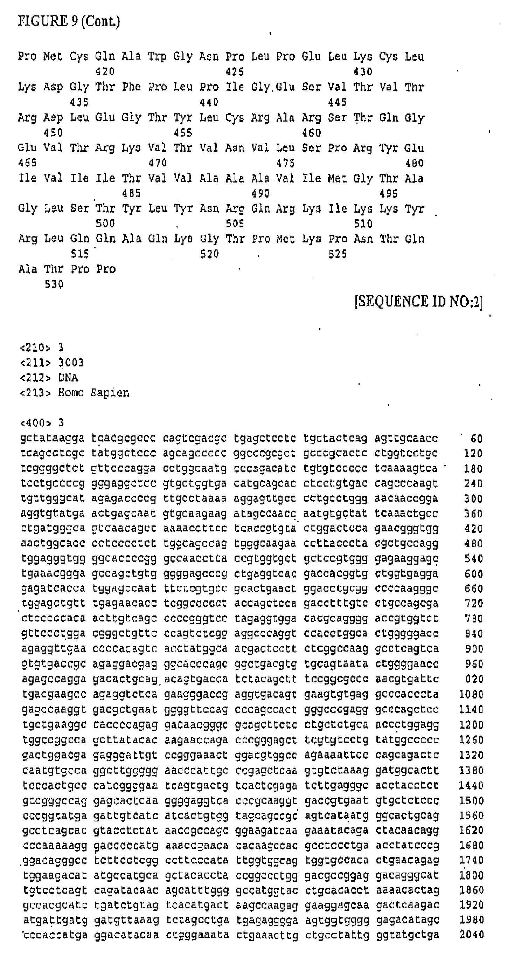

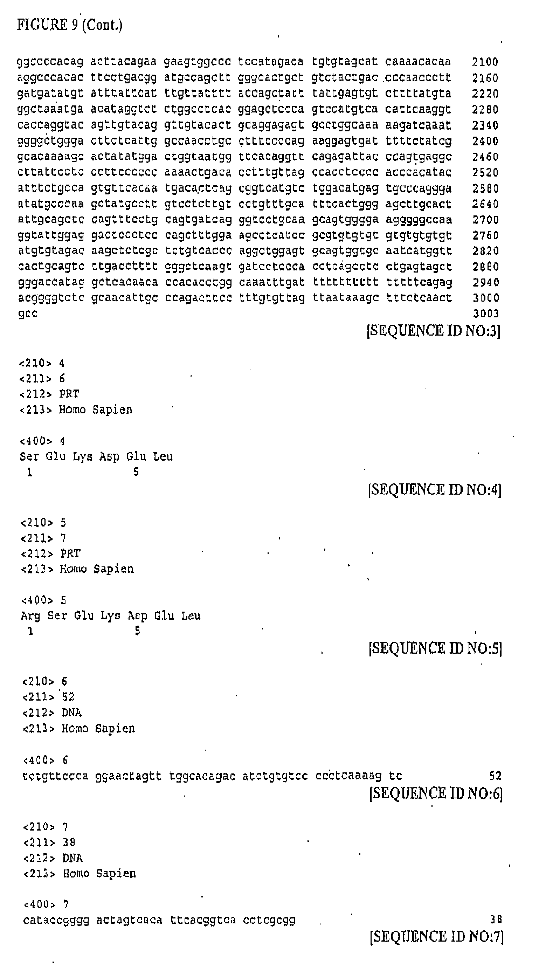

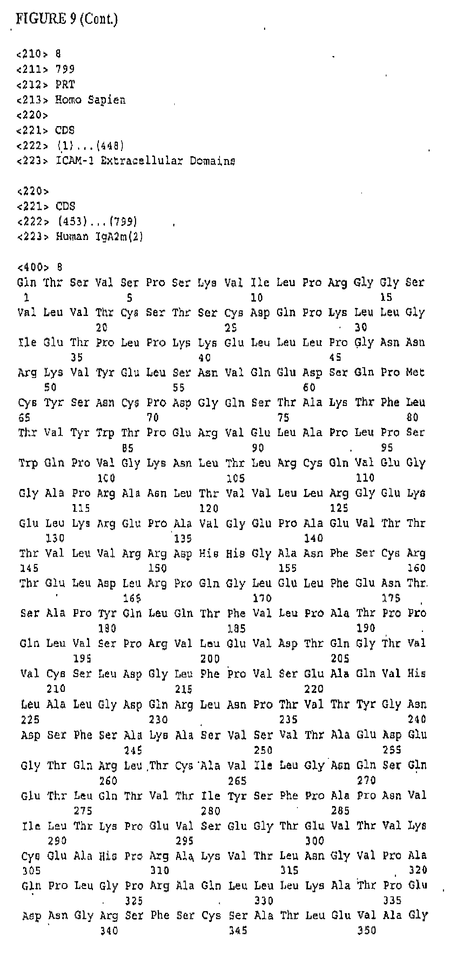

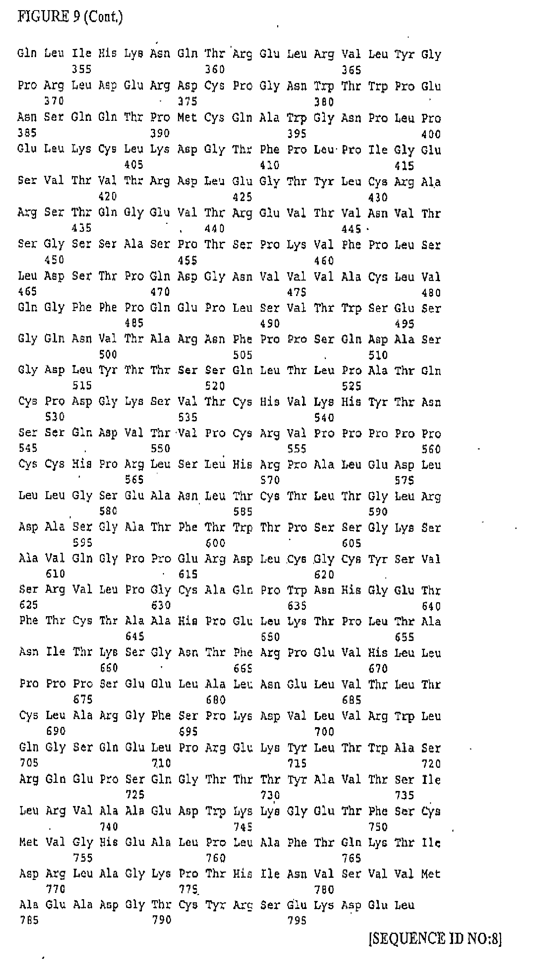

FIG. 9 contains nucleotide and protein sequences SEQ ID NO:1; SEQ ID NO:2; SEQ ID NO:3; SEQ ID NO:4; SEQ ID NO:5; SEQ ID NO:6; SEQ ID NO:7; SEQ ID NO:8, for ICAM-1, and human IgA2 and other nucleotide sequences.
FIG. 10 shows the full nucleotide (SEQ ID NO:98) and amino acid sequence (SEQ ID NO:99) of the ATR-IgA2 fusion (an immunoadhesin).
FIG. 11 shows the sequence (SEQ ID NO:100) between the T-DNA borders of the plasmid pGPTV-kan-ocs-ATR-IgA2.
FIG. 12 shows the sequence (SEQ ID NO:101) between the T-DNA borders of the plasmid pGPTV-hpt-ocs-35SJ/SC.

### DETAILED DESCRIPTION OF THE INVENTION

### A. Definitions

As used herein, the following abbreviations and terms include, but are not necessarily limited to, the following definitions.

The practice of the present invention will employ, unless otherwise indicated, conventional techniques of immunology, molecular biology, microbiology, cell biology and recombinant DNA, which are within the skill of the art. See, e.g., Sambrook, et al., Molecular Cloning: A Laboratory Manual, 2nd edition (1989); Current Protocols In Molecular Biology (F.M. Ausubel, et al. eds., (1987)); the series Methods In Enzymology (Academic Press, Inc.); M.J. MacPherson, et al., eds. Pcr 2: A Practical Approach (1995); Harlow and Lane, eds, Antibodies: A Laboratory Manual (1988), and H. Jones, Methods In Molecular Biology vol. 49, "Plant Gene Transfer And Expression Protocols" (1995).

Immunoglobulin molecule or Antibody. A polypeptide or multimeric protein containing the immunologically active portions of an immunoglobulin heavy chain and immunoglobulin light chain covalently coupled together and capable of specifically combining with antigen. The immunoglobulins or antibody molecules are a large family of molecules that include several types of molecules such as IgD, IgG, IgA, secretory IgA (SIgA), IgM, and IgE.

Construct or Vector. An artificially assembled DNA segment to be transferred into a target plant tissue or cell. Typically, the construct will include the gene or genes of a particular interest, a marker gene and appropriate control sequences. The term "plasmid" refers to an autonomous, self-replicating extrachromosomal DNA molecule. In a preferred embodiment, the plasmid constructs of the present invention contain sequences coding for heavy and light chains of an antibody. Plasmid constructs containing suitable regulatory elements are also referred to as "expression cassettes." In a preferred embodiment, a plasmid construct can also contain a screening or selectable marker, for example an antibiotic resistance gene.

Selectable marker. A gene that encodes a product that allows the growth of transgenic tissue on a selective medium. Non-limiting examples of selectable markers include genes encoding for antibiotic resistance, e.g., ampicillin, kanamycin, or the like. Other selectable markers will be known to those of skill in the art.

Transgenic plant. Genetically engineered plant or progeny of genetically engineered plants. The transgenic plant usually contains material from at least one unrelated organism, such as a virus, another plant or animal.

Chimeric ICAM-1 molecule: The fusion of any combination of the extracellular domains 1, 2, 3, 4 and 5 of ICAM-1 with at least a part of an immunoglobulin heavy chain protein, made by linking ICAM-1 sequence upstream of an immunoglobulin heavy chain gene sequence and expressing the encoded protein from the construct. In antibacterial embodiments, one or more receptor proteins effective to bind a bacterium or bacteria of interest or subcomponent thereof, such as a protein produced by the bacteria and required for the bacteria to exert its pathogenic effect, are used instead of, or in addition to, ICAM-1. Many such receptor proteins are known and can be implemented for use in appropriate aspects of the invention by those of ordinary skill in the art without exercising undue experimentation. An example is provided below utilizing one such protein that binds to a protein involved in the pathogenic mechanism caused by the bacterium that causes human anthrax. The same concept can be used to target other viruses besides rhinoviruses, e.g., by making use of appropriate host receptor proteins in immunoadhesin form.

Chimeric immunoglobulin heavy chain: An immunoglobulin derived heavy chain having at least a portion of its amino acid sequence derived from an immunoglobulin heavy chain of a different isotype or subtype or some other peptide, polypeptide or protein. Typically, a chimeric immunoglobulin heavy chain has its amino acid residue sequence derived from at least two different isotypes or subtypes of immunoglobulin heavy chain.

Dicotyledonous plants (dicots): Flowering plants whose embryos have two seed halves or cotyledons. Examples of dicots are: tobacco; tomato; the legumes including alfalfa; oaks; maples; roses; mints; squashes; daisies; walnuts; cacti; violets and buttercups.

Effective amount: An effective amount of an immunoadhesin of the present invention is sufficient to detectably inhibit viral or bacterial infection (as the case may be), cytotoxicity or replication; or to reduce the severity or length of infection.

Human rhinovirus (HRV): A nonenveloped RNA virus representing a subgroup of picornavirus, that is a major cause of the common cold in humans. Rhinoviruses are described in Rhinoviruses, Reoviruses, and Parvoviruses, pp. 1057-1059, Zinsser Microbiology, Joklik et al., eds. Appleton and Lange (1992).

Immunoadhesin : A complex containing a chimeric receptor protein molecule, and optionally containing secretory component, and J chain.

Immunoglobulin heavy chain: A polypeptide that contains at least a portion of the antigen binding domain of an immunoglobulin and at least a portion of a variable region of an immunoglobulin heavy chain or at least a portion of a constant region of an immunoglobulin heavy chain. Thus, the immunoglobulin derived heavy chain has significant regions of amino acid sequence homology with a member of the immunoglobulin gene superfamily. For example, the heavy chain in an Fab fragment is an immunoglobulin-derived heavy chain.

Immunoglobulin light chain: A polypeptide that contains at least a portion of the antigen binding domain of an immunoglobulin and at least a portion of the variable region or at least a portion of a constant region of an immunoglobulin light chain. Thus, the immunoglobulin-derived light chain has significant regions of amino acid homology with a member of the immunoglobulin gene superfamily.

Immunoglobulin molecule: A protein containing the immunologically-active portions of an immunoglobulin heavy chain and immunoglobulin light chain covalently coupled together and capable of specifically combining with antigen.

ICAM-1: Intercellular adhesion molecule-1. In humans, ICAM-1 functions as the receptor for human rhinovirus.

J chain: A polypeptide that is involved in the polymerization of immunoglobulins and transport of polymerized immunoglobulins through epithelial cells. See, The Immunoglobulin Helper: The J Chain in Immunoglobulin Genes, at pg. 345, Academic Press (1989). J chain is found in pentameric IgM and dimeric IgA and typically attached via disulphide bonds. J chain has been studied in both mouse and human.

Monocotyledonous plants (monocots): Flowering plants whose embryos have one cotyledon or seed leaf. Examples of monocots are: lilies; grasses; corn; grains, including oats, wheat and barley; orchids; irises; onions and palms.

Glycosylation: The modification of a protein by oligosaccharides. See, Marshall, Ann. Rev. Biochem., 41:673 (1972) and Marshall, Biochem. Soc. Symp., 40:17 (1974) for a general review of the polypeptide sequences that function as glycosylation signals. These signals are recognized in both mammalian and in plant cells.

Plant-specific glycosylation: The glycosylation pattern found on plant-expressed proteins, which is different from that found in proteins made in mammalian or insect cells. Proteins expressed in plants or plant cells have a different pattern of glycosylation than do proteins expressed in other types of cells, including mammalian cells and insect cells. Detailed studies characterizing plant-specific glycosylation and comparing it with glycosylation in other cell types have been performed by Cabanes-Macheteau et al., Glycobiology 9(4):365-372 (1999), Lerouge et al., Plant Molecular Biology 38:31-48 (1998) and Altmann, Glycoconjugate J. 14:643-646 (1997). Plant-specific glycosylation generates glycans that have xylose linked β(1,2) to mannose. Neither mammalian nor insect glycosylation generate xylose linked β(1,2) to mannose. Plants do not have a sialic acid linked to the terminus of the glycan, whereas mammalian cells do. In addition, plant-specific glycosylation results in a fucose linked α(1,3) to the proximal GlcNAc, while glycosylation in mammalian cells results in a fucose linked α(1,6) to the proximal GlcNAc.

Secretory component (SC): A component of secretory immunoglobulins that helps to protect the immunoglobulin against inactivating agents thereby increasing the biological effectiveness of secretory immunoglobulin. The secretory component may be from any mammal or rodent including mouse or human.

sICAM: A naturally-occurring soluble truncated form of ICAM-1 lacking both the hydrophobic transmembrane domain and the carboxy-terminal cytoplasmic domain of ICAM.

The articles, patents and patent applications cited in this document are incorporated into this document as if set forth in full.

Although much of the discussion which follows is directed to ICAM-1 immunoadhesin aspects and embodiments, it will be clear to one of ordinary skill that other antiviral or antibacterial immunoadhesins can be similarly produced by incorporating receptor protein molecules other than ICAM-1. Indeed, examples 10-12 are directed to antibacterial embodiments in which the anthrax toxin receptor (ATR) is used instead of ICAM-1.

### B. Immunoadhesins Containing Chimeric ICAM Molecules

The present invention provides novel methods for producing immunoadhesin molecules containing chimeric receptor proteins, e.g., ICAM-1 receptor proteins. ICAM-1 immunoadhesins, for example, contain chimeric ICAM-1 molecules made up of a rhinovirus receptor protein linked to a portion of an immunoglobulin heavy chain molecule in association with J chain and secretory component. The chimeric ICAM-1 molecules of such aspects of the present invention contain two molecules derived from different sources: a rhinovirus receptor protein portion and an immunoglobulin chain portion. The rhinovirus receptor protein is derived from the intercellular adhesion molecule 1 (ICAM-1). The nucleotide sequence for the human rhinovirus receptor ICAM-1 has been determined and characterized by Staunton, et al., Cell 52:925-933 (1988); Greve, et al. Cell 56:839-847 (1989); Greve, et al. J. Virology 65:6015-6023 (1991); Staunton, et al., Cell, 61:243-254 (1990) and described in Sequence ID No. 3 and GenBank accession no. M24283.

The ICAM-1 molecule is a membrane protein (SEQ ID NOS: 1 and 2) that has 5 extracellular domains, a hydrophobic transmembrane domain and a short cytoplasmic domain. These features have been described by Casasnovas, et al., Proc. Natl. Acad. Sci. U.S.A., 95:4134-4139 (1998) and Staunton, et al, Cell 52:925-933 (1988). Of particular use in appropriate aspects of the present invention are the domains of the ICAM-1 molecule that are responsible for the binding of human rhinoviruses which have been localized to the N-terminal domains 1 and 2 (Greve, et al., J. Virol., 65:6015-6023 1991, and Staunton, et al., Cell, 61:243-245 1990. Such aspects also contemplate(s) rhinovirus receptor protein portions which include any combination of extracellular domains 1, 2, 3, 4, and 5 of the ICAM-1 molecule. In particular preferred embodiments, the rhinovirus receptor protein portion includes domains 1 and 2 of the ICAM-1 molecule and in other preferred embodiments domains 1, 2, 3, 4 and 5 of the ICAM-1 molecule are present.

The boundaries of the 5 extracellular domains of ICAM-1 are well known in the art and described in Staunton, et al., Cell 52:925-933 (1988). The approximated domain boundaries are shown in Table 1 below [SEQ ID NO:2].

**Table 1**

| ICAM-1 Domains | Amino Acids |
|---|---|
| 1 | 1-88 |
| 2 | 89-105 |
| 3 | 106-284 |
| 4 | 285-385 |
| 5 | 386-453 |

As used in some aspects and embodiments of the present invention, the ICAM-1 domain 1 is from about residue 1 to about residue 88; domain 2 is from about residue 89 to about residue 105; domain 3 is from about residue 106 to about residue 284; domain 4 is from about residue 285 to about 385; and domain 5 is from about residue 386 to 453. One of skill in the art will understand that the exact boundaries of these domains may vary.

The chimeric ICAM-1 molecules preferably contain at least a portion of an IgM or IgA heavy chain which allows that immunoglobulin heavy chain to bind to immunoglobulin J chain and thereby binds to the secretory component. It is contemplated that the portion of the chimeric ICAM-1 molecule derived from the immunoglobulin heavy chain may be comprised of individual domains selected from the IgA heavy chain or the IgM heavy chain or from some other isotype of heavy chain. It is also contemplated that an immunoglobulin domain derived from an immunoglobulin heavy chain other than IgA or IgM or from an immunoglobulin light chain may be molecularly engineered to bind immunoglobulin J chain and thus may be used to produce immunoglobulins and immunoadhesins of the present invention.

One skilled in the art will understand that immunoglobulins consist of domains which are approximately 100-110 amino acid residues. These various domains are well known in the art and have known boundaries. The removal of a single domain and its replacement with a domain of another antibody molecule is easily achieved with modem molecular biology. The domains are globular structures which are stabilized by intrachain disulfide bonds. This confers a discrete shape and makes the domains a self-contained unit that can be replaced or interchanged with other similarly shaped domains. The heavy chain constant region domains of the immunoglobulins confer various properties known as antibody effector functions on a particular molecule containing that domain. Example effector functions include complement fixation, placental transfer, binding to staphyloccal protein, binding to streptococcal protein G, binding to mononuclear cells, neutrophils or mast cells and basophils. The association of particular domains and particular immunoglobulin isotypes with these effector functions is well known and for example, described in Immunology, Roitt et al., Mosby St. Louis, Mo. (1993 3rd Ed.)

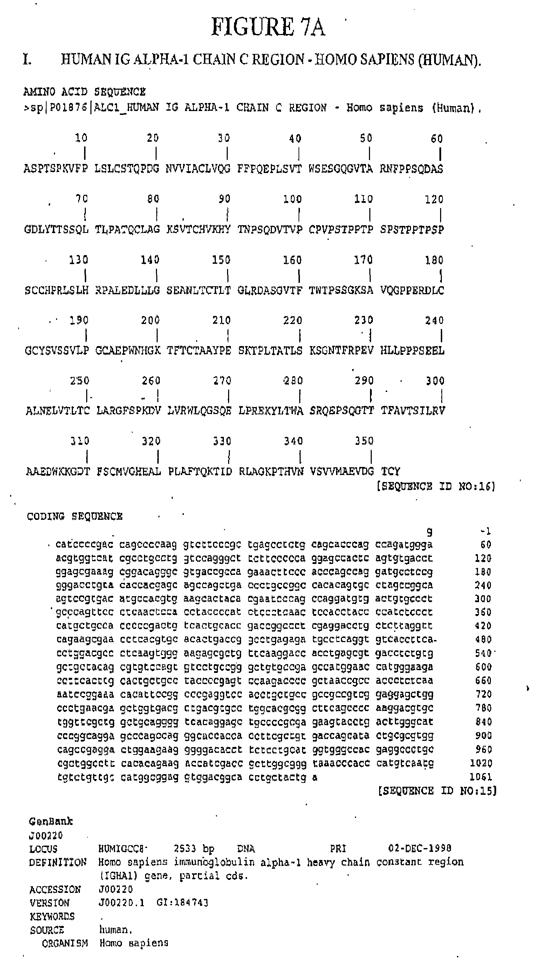

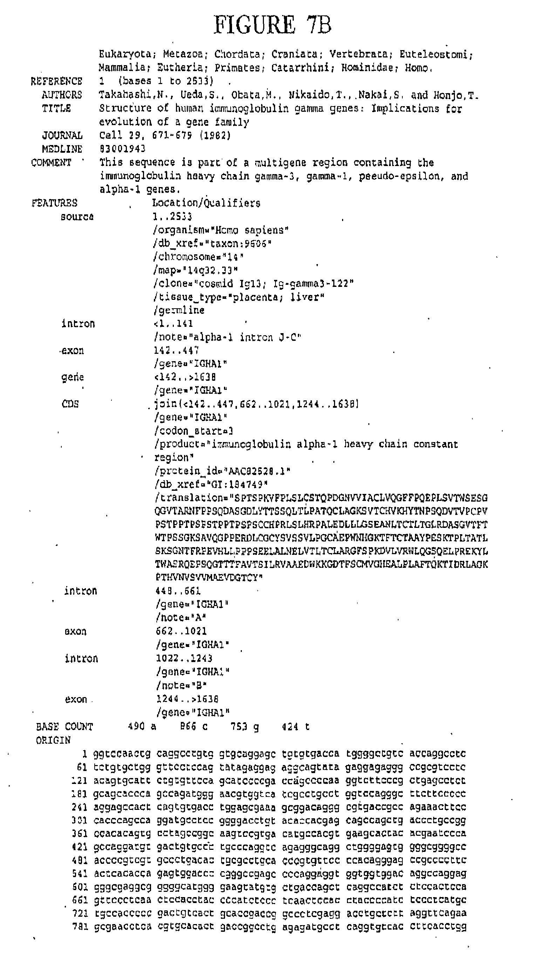

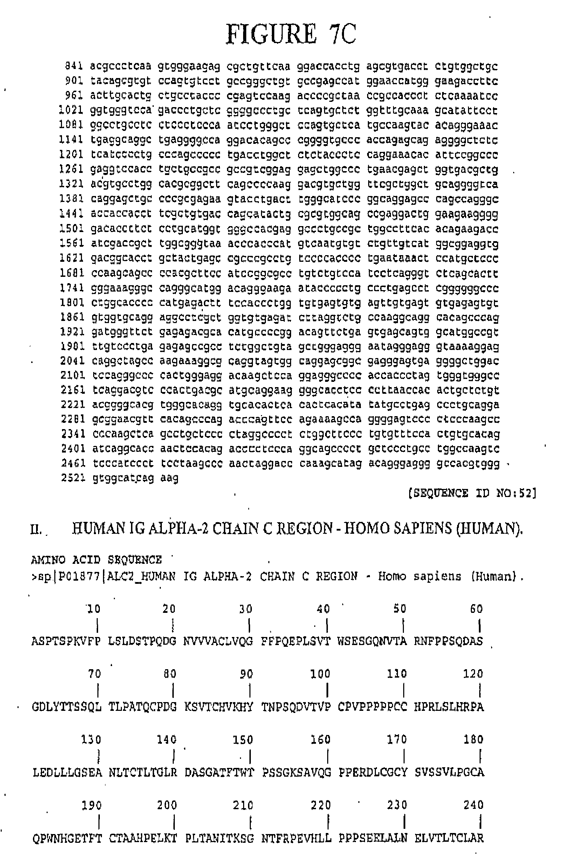

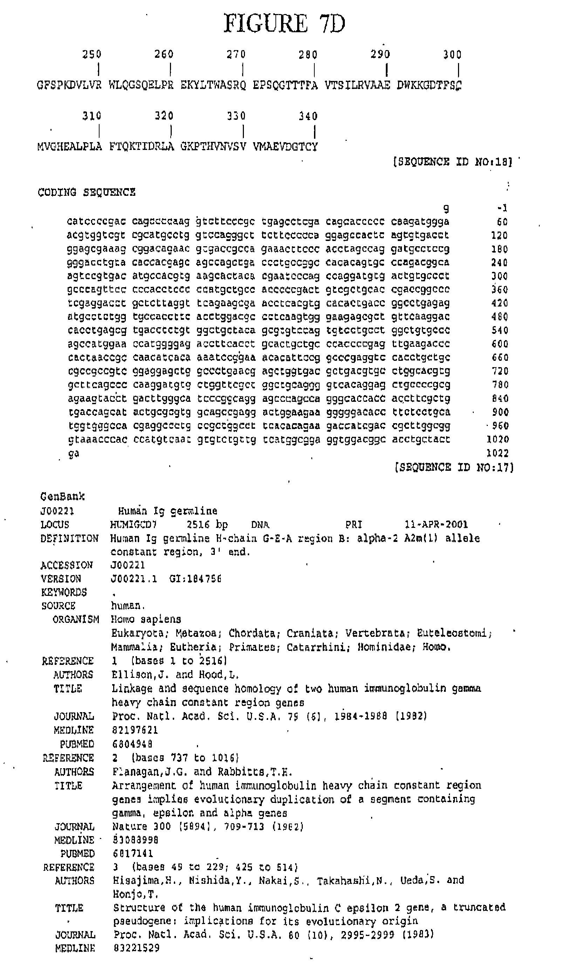

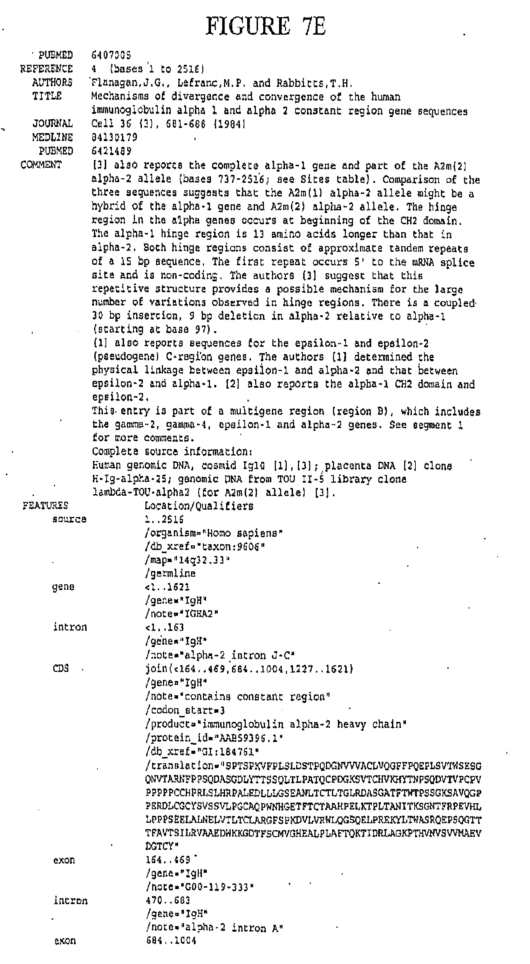

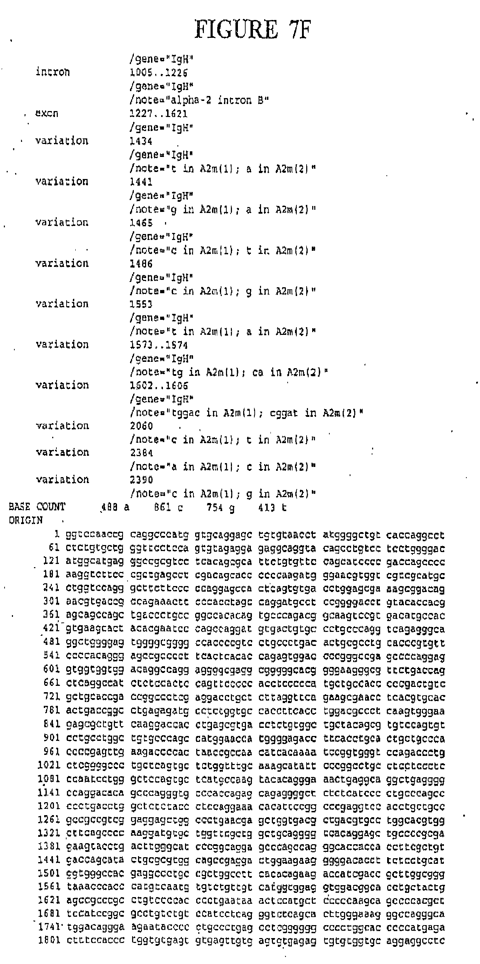

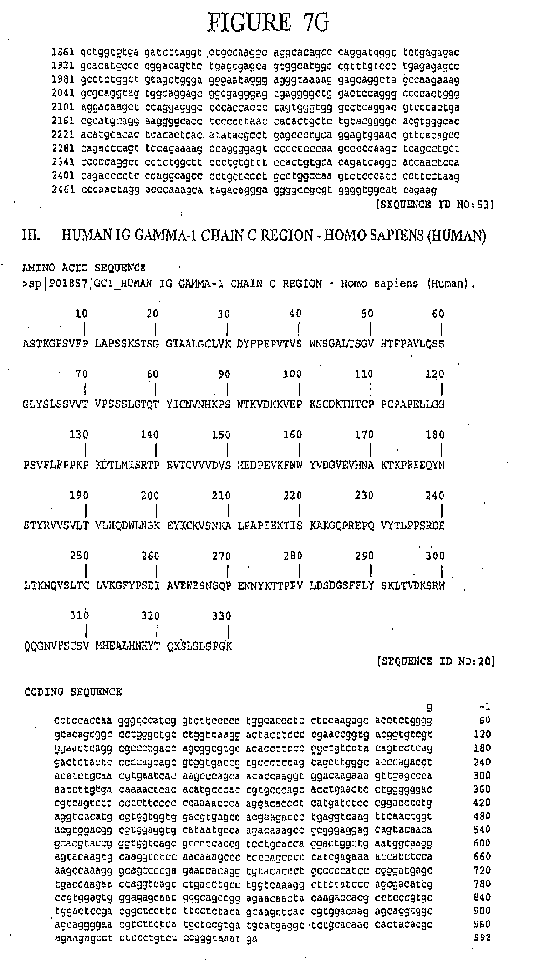

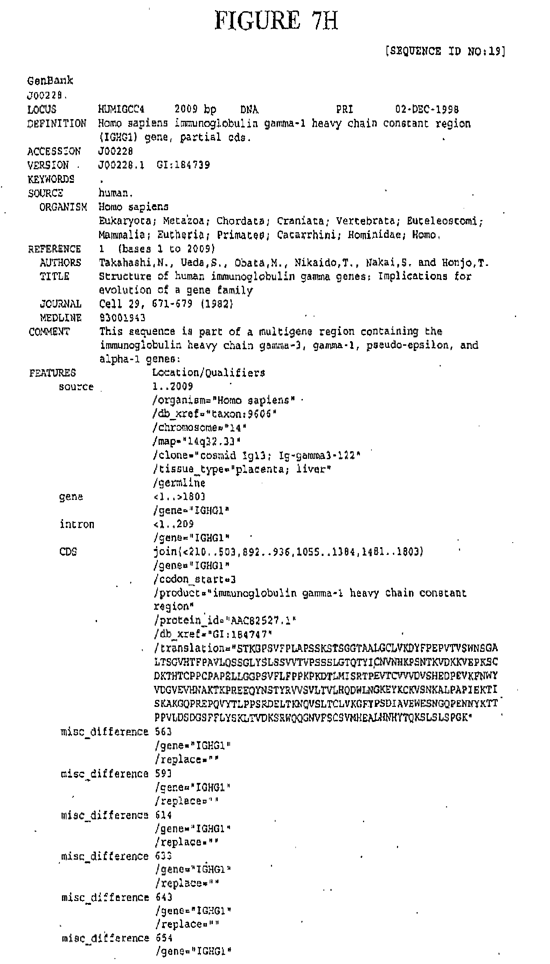

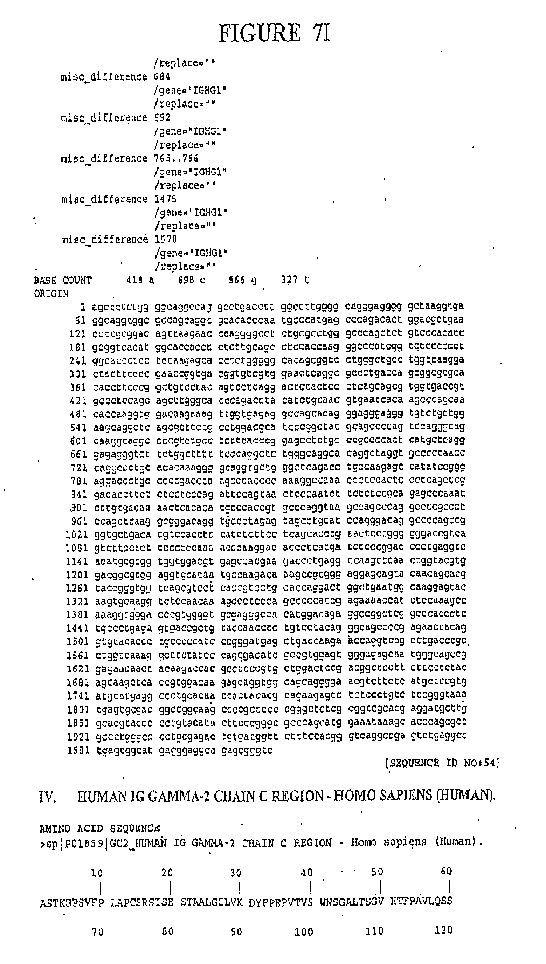

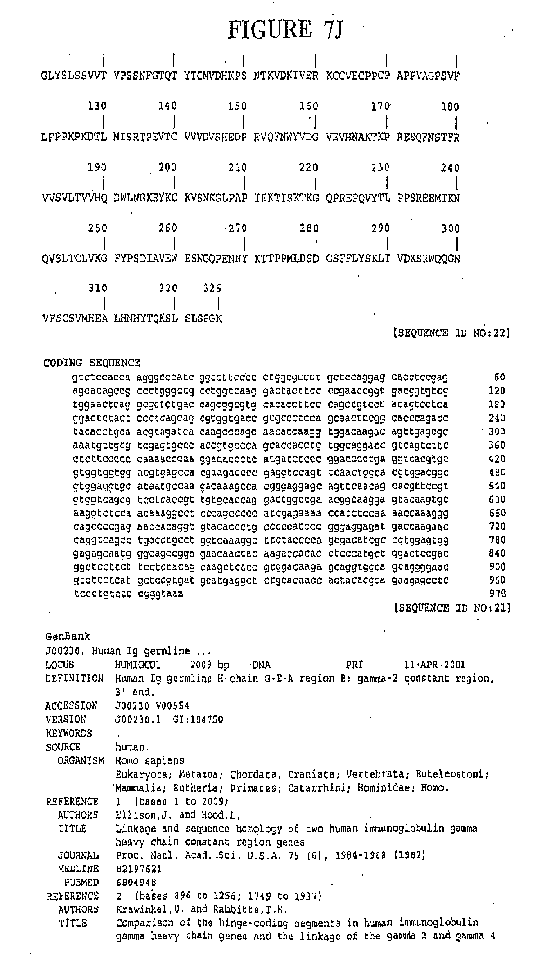

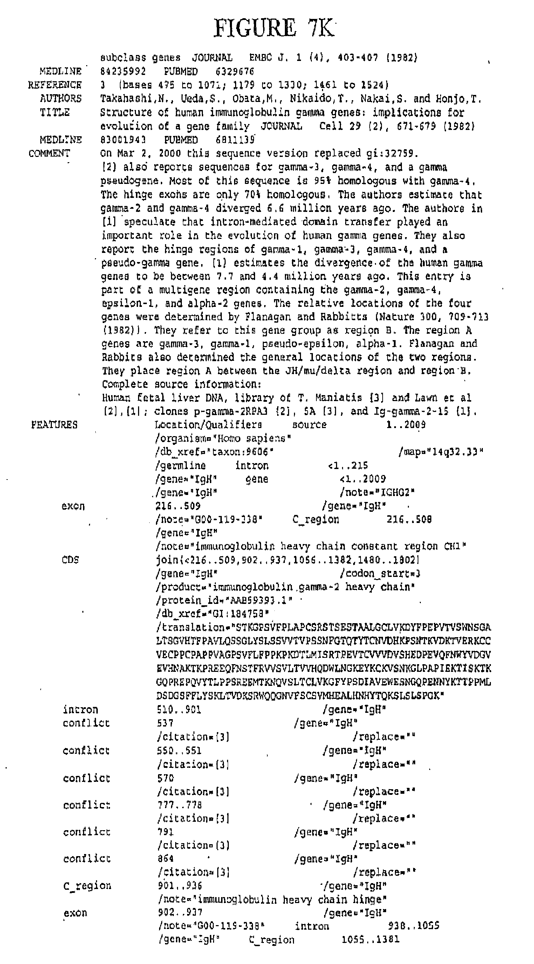

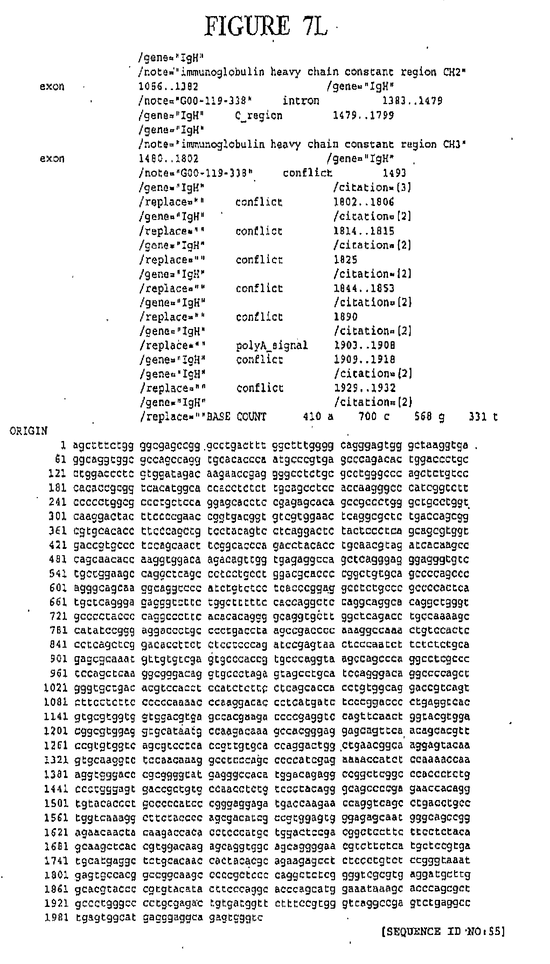

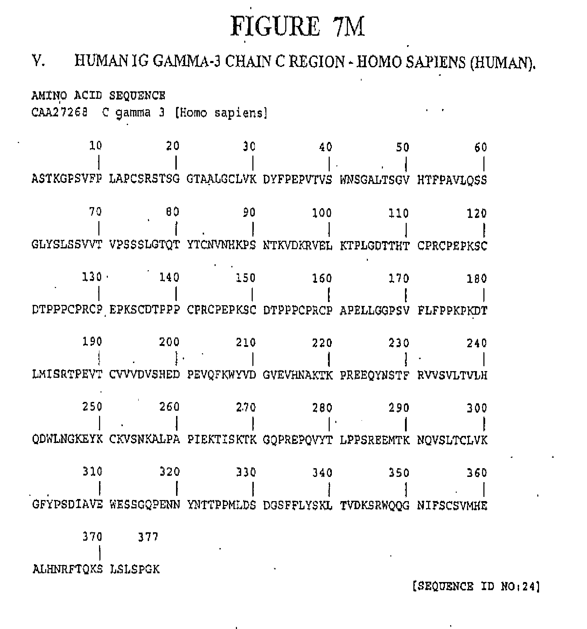

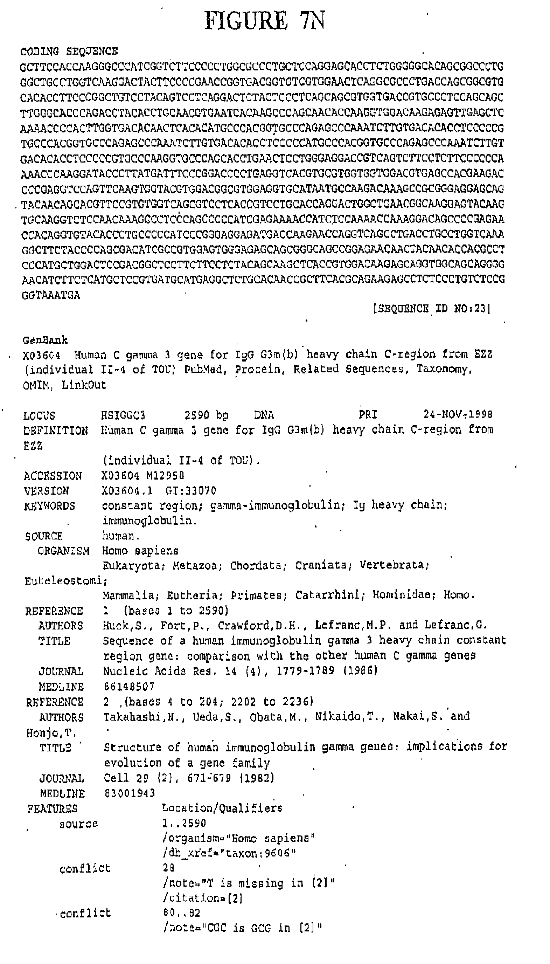

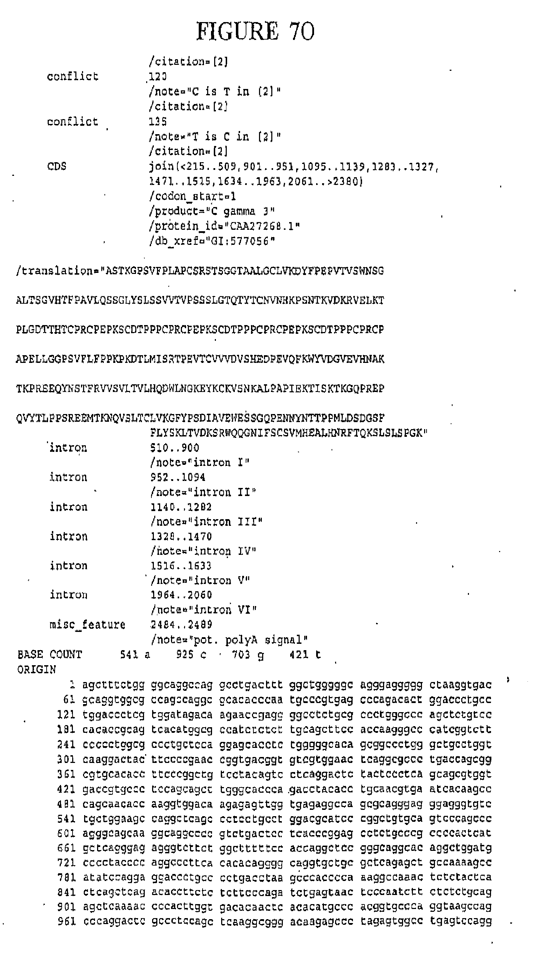

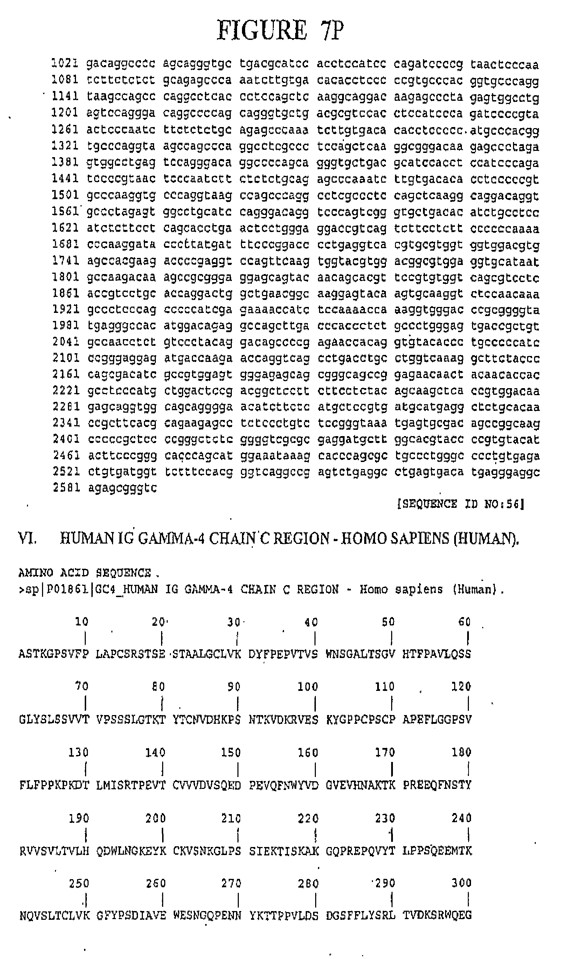

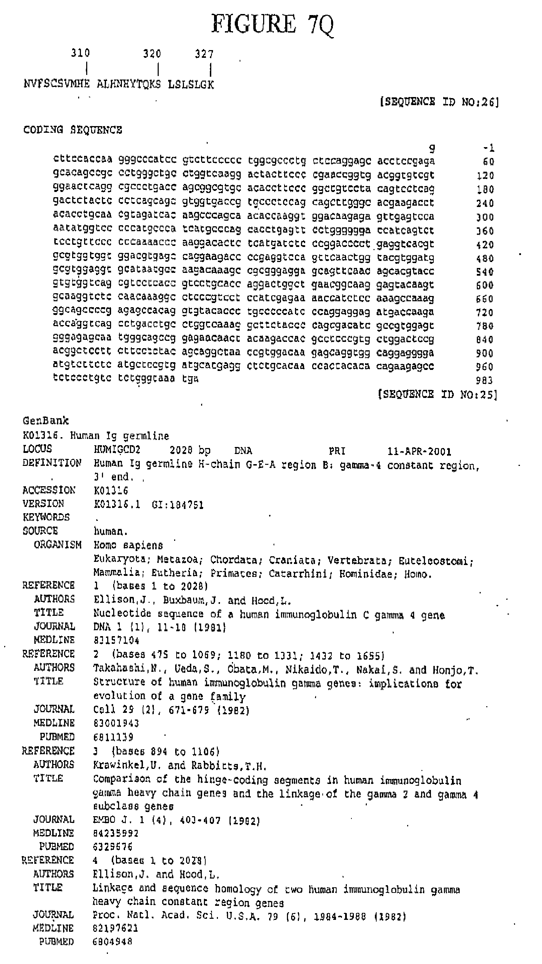

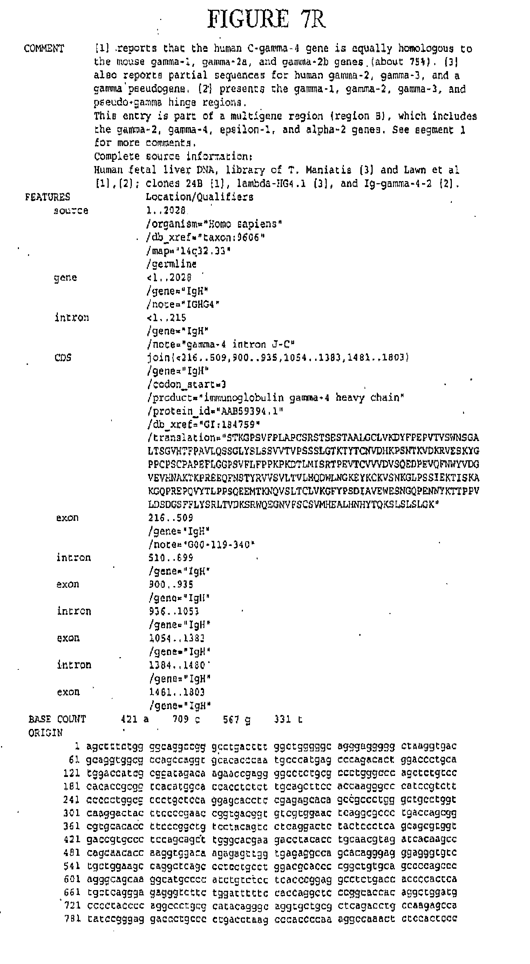

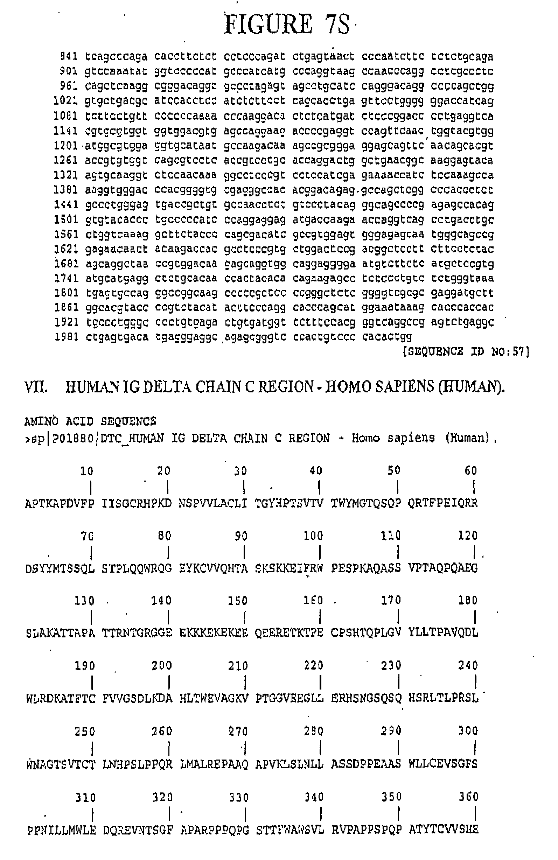

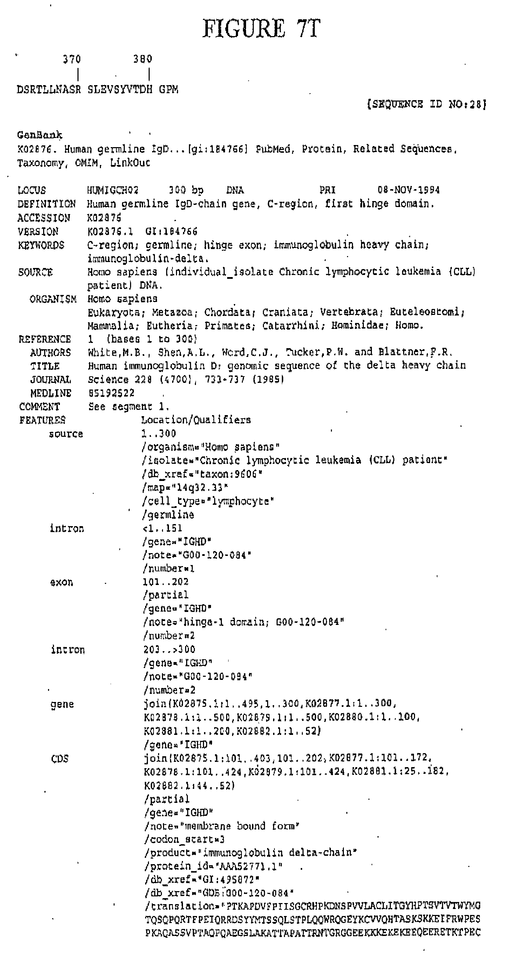

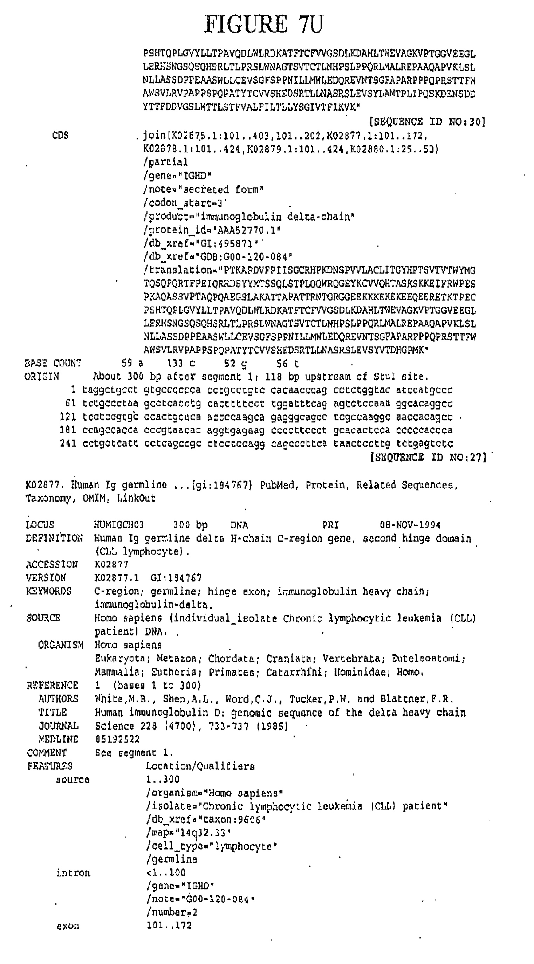

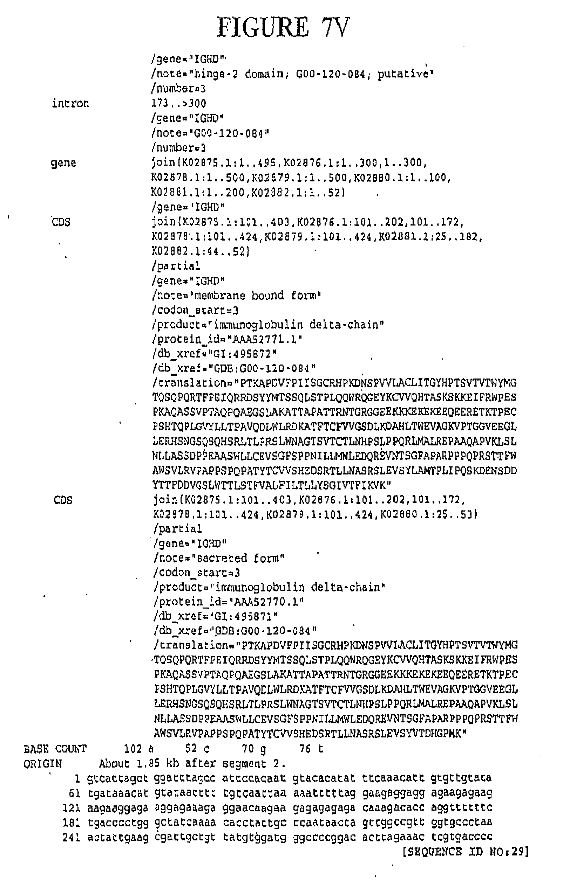

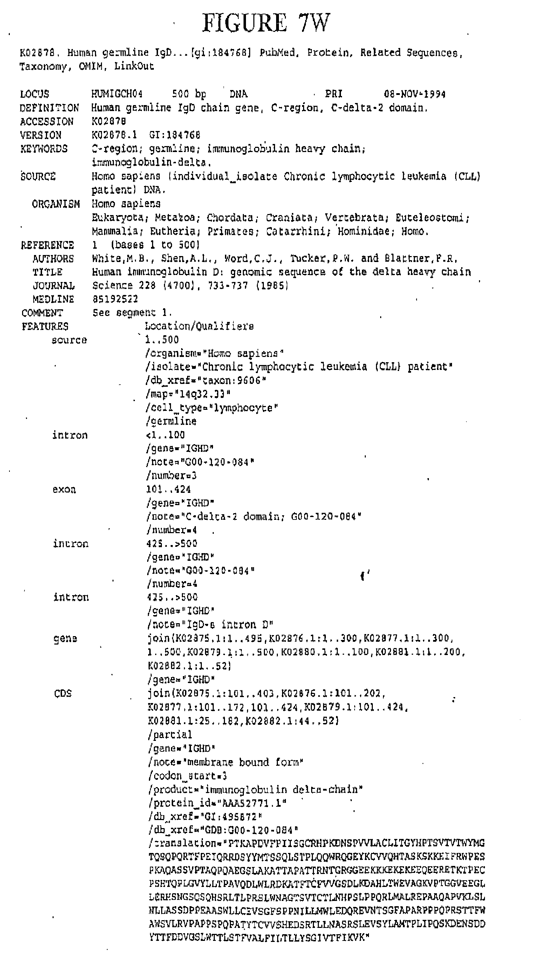

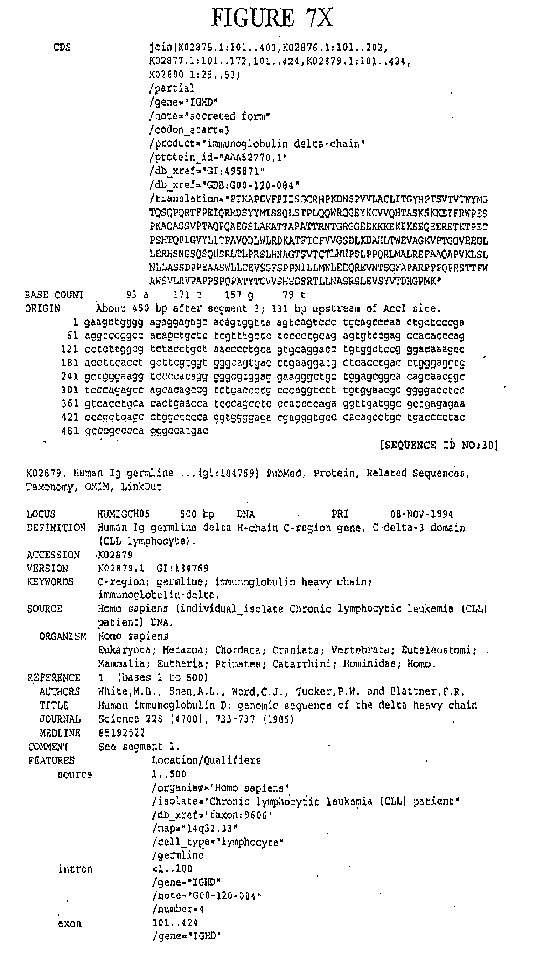

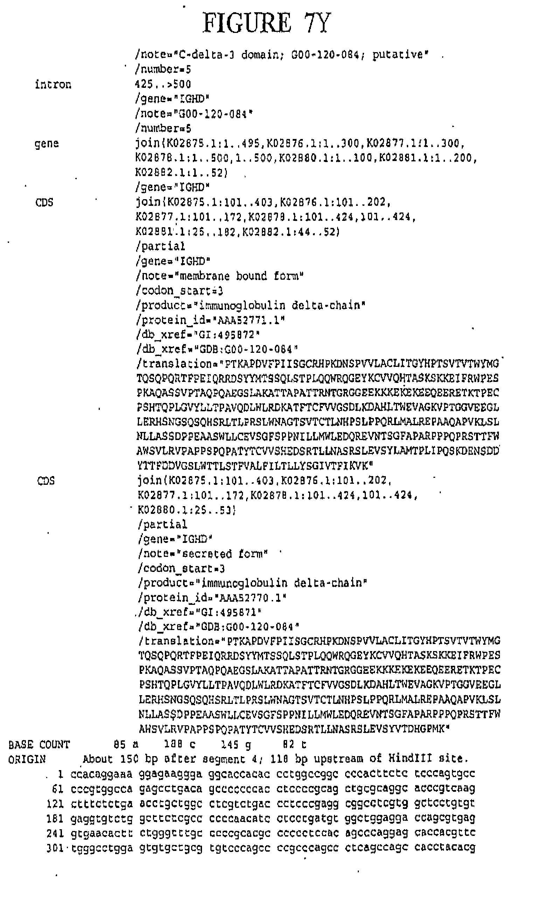

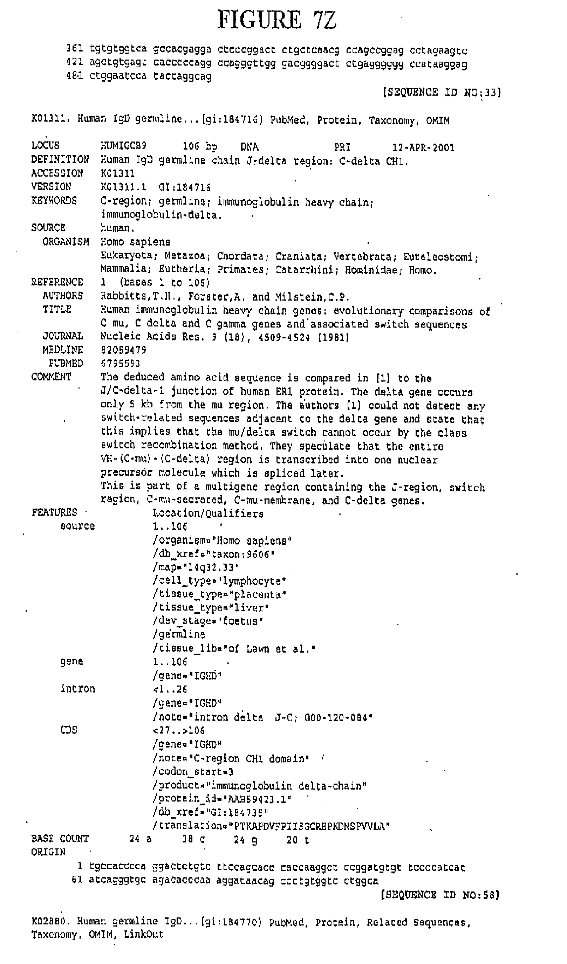

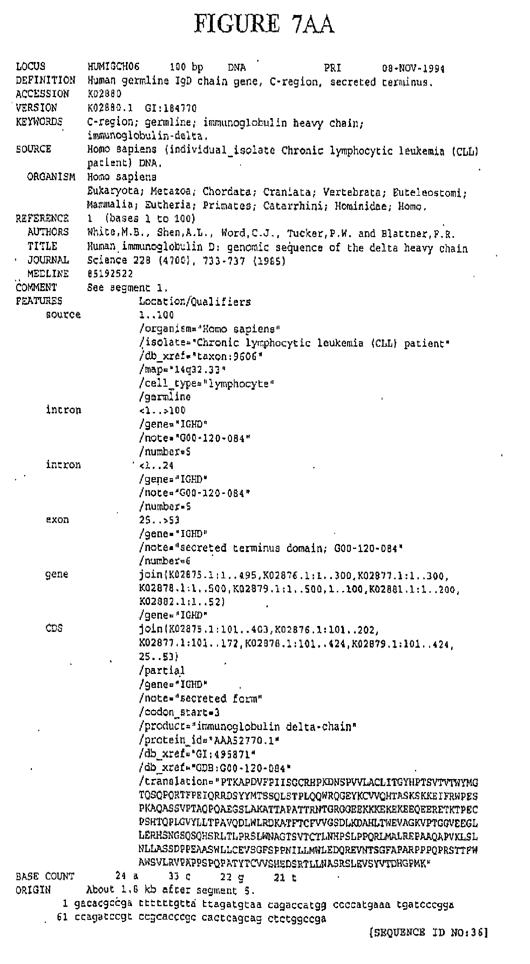

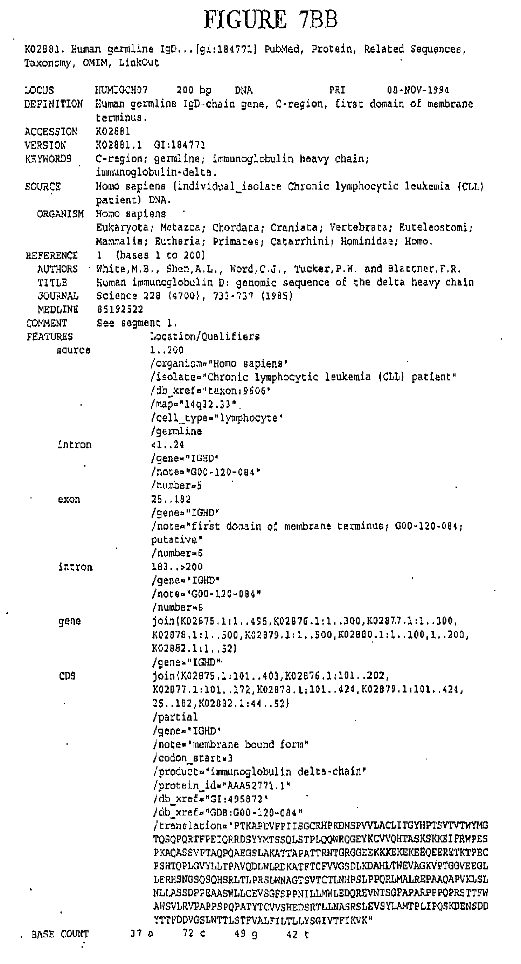

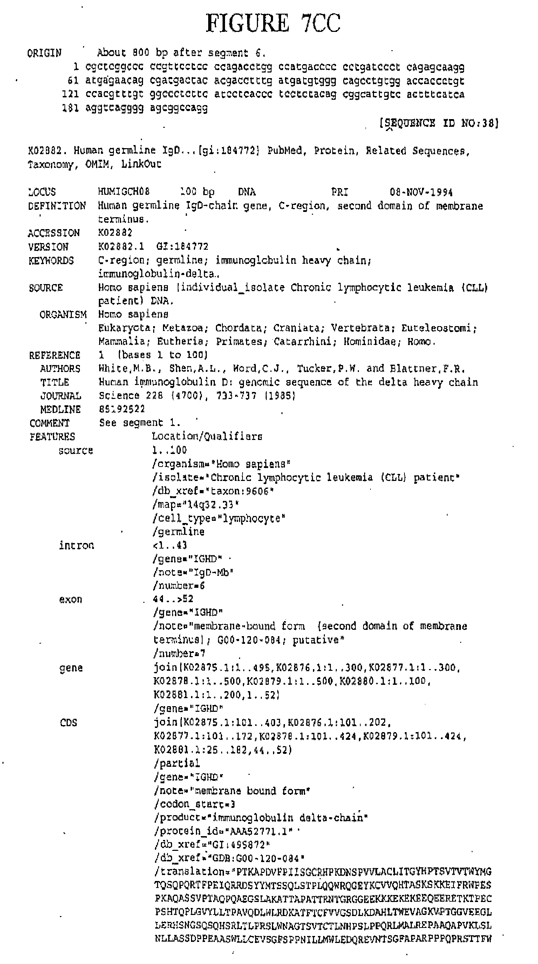

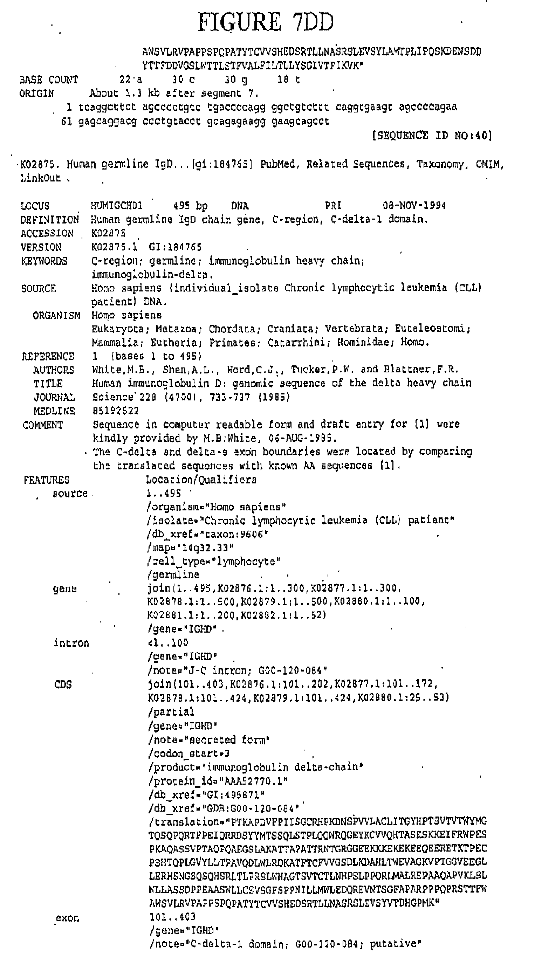

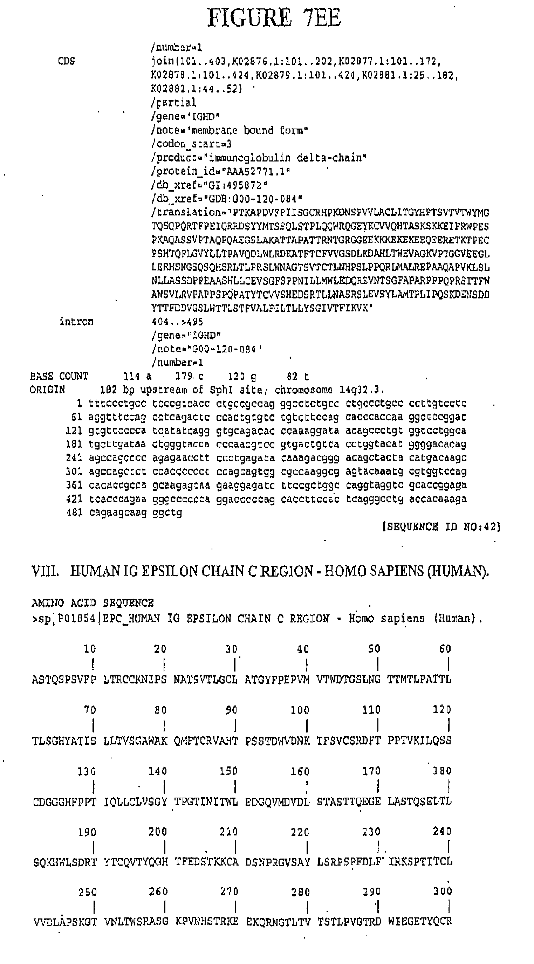

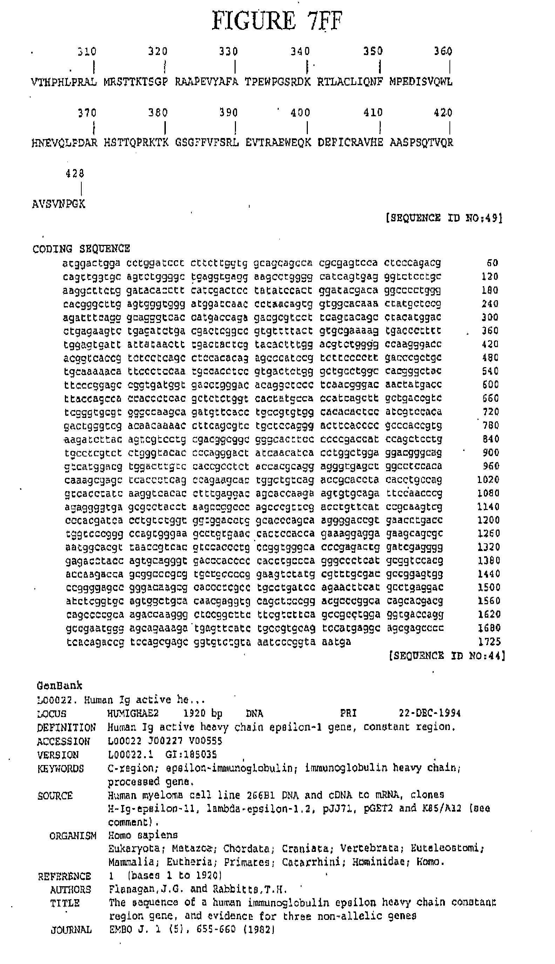

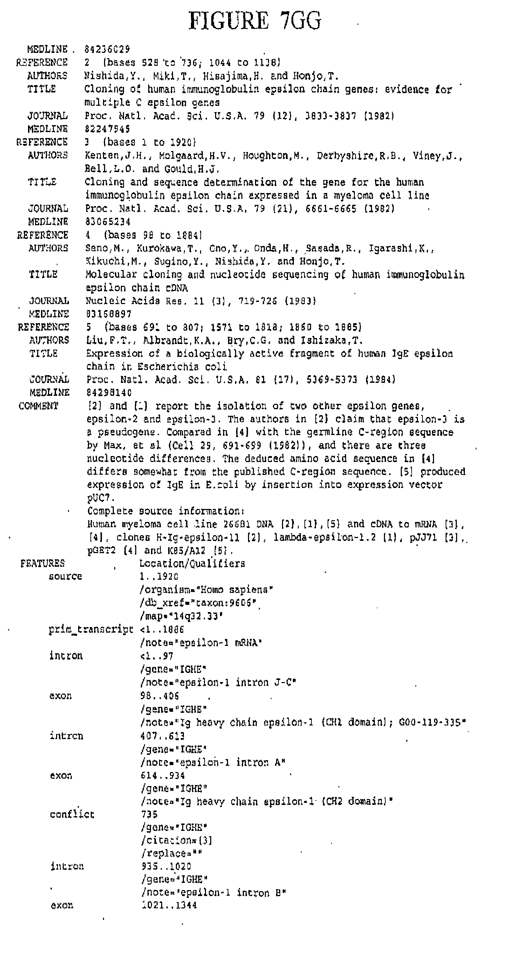

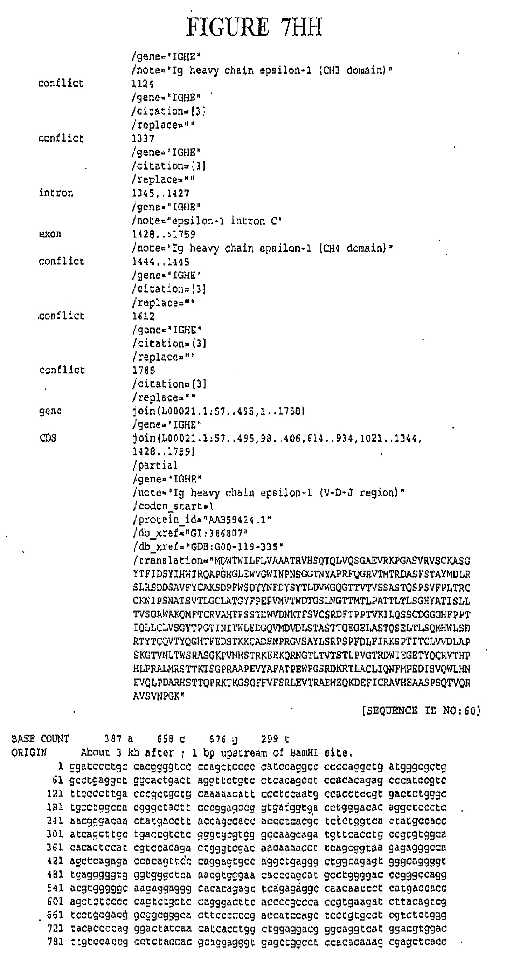

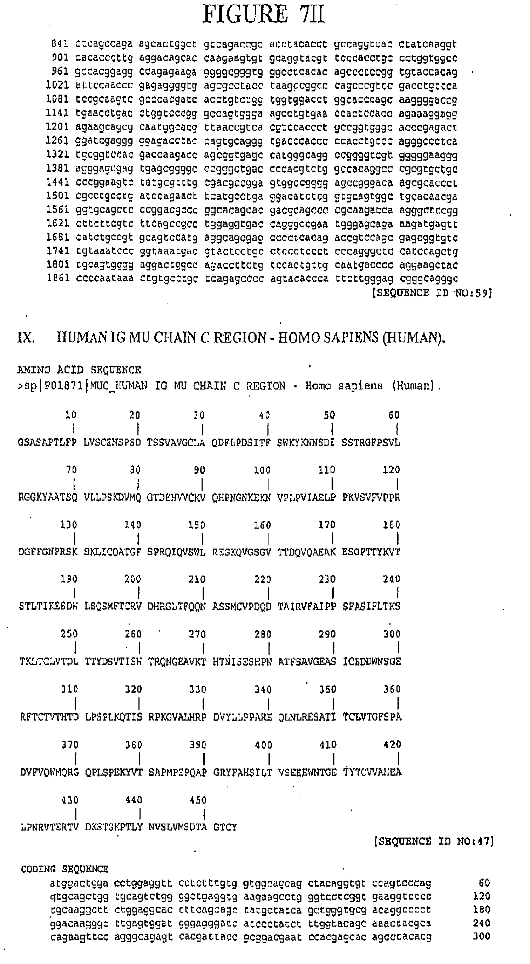

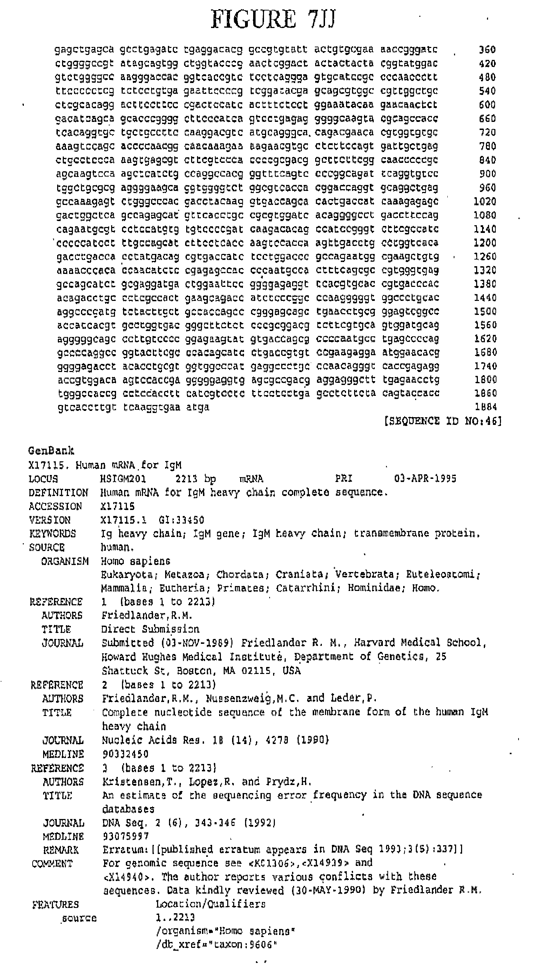

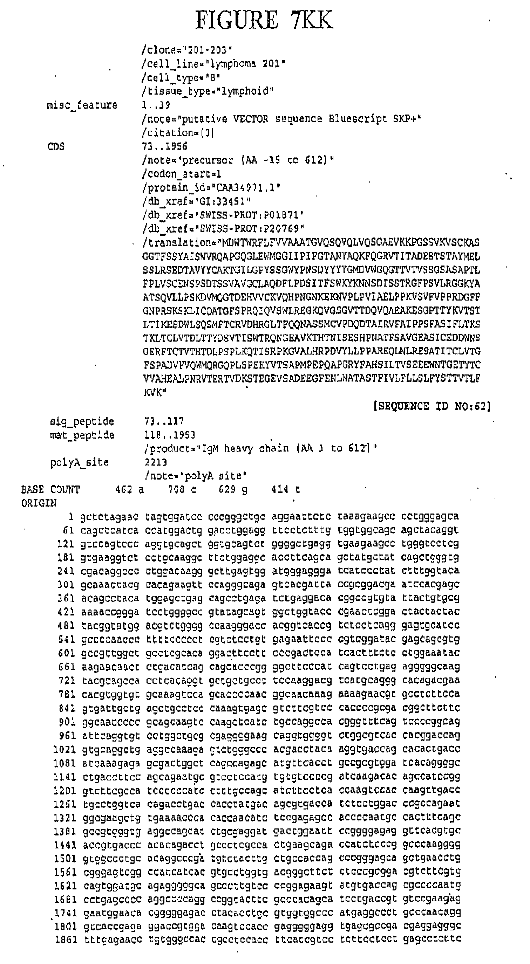

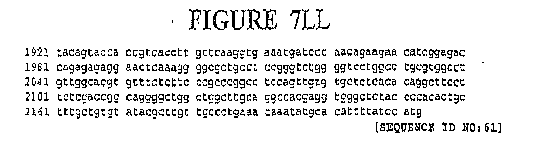

One of skill in the art will be able to identify immunoglobulin heavy chain constant region sequences. For example, a number of immunoglobulin DNA and protein sequences are available through GenBank. Table 2 shows the GenBank Accession numbers of immunoglobulin heavy chain genes and the proteins encoded by the genes. The sequences listed in Table 2 are shown in Fig. 7.

**Table 2**

| **GENBANK ACCESSION NO.** | **HUMAN IMMUNOGLOBULIN SEQUENCE NAME** | **SEQ ID NO.** |
|---|---|---|
| J00220 | Ig_{α1} Heavy Chain Constant Region Coding Sequence | 15, 52 |
| J00220 | Ig_{α1} Heavy Chain Constant Region Amino Acid Sequence | 16 |
| J00221 | IgA₂ Heavy Chain Constant Region Coding Sequence | 17, 53 |
| J00221 | IgA₂ Chain Constant Region Amino Acid Sequence | 18 |
| J00228 | Ig_{γ1} Heavy Chain Constant Region Coding Sequence | 19, 54 |
| J00228 | Ig_{γ1} Heavy Chain Constant Region Amino Acid Sequence | 20 |
| J00230 | IgG₂ Heavy Chain Constant Region Coding Sequence | 21, 55 |
| V00554 | | |
| J00230 | IgG₂ Heavy Chain Constant Region Amino Acid Sequence | 22 |
| V00554 | | |
| X03604 | IgG₃ Heavy Chain Constant Region Coding Sequence | 23, 57 |
| M12958 | | |
| X03604 | IgG₃ Heavy Chain Constant Region Amino Acid Sequence | 24 |
| M12958 | | |
| K01316 | IgG₄ Heavy Chain Constant Region Coding Sequence | 25 |
| K01316 | IgG₄ Heavy Chain Constant Region Amino Acid Sequence | 26 |
| K02876 | IgD Heavy Chain Constant Region Coding Sequence | 27 |
| K02876 | IgD Heavy Chain Constant Region Amino Acid Sequence | 28, 30, 32 |
| K02877 | IgD Heavy Chain Constant Region Coding Sequence | 29 |
| K02877 | IgD Heavy Chain Constant Region Amino Acid Sequence | 28, 30, 32 |
| K02878 | Germline IgD Heavy Chain Coding Sequence | 31 |
| K02878 | Germline IgD Heavy Chain Amino Acid Sequence | 28, 30, 32 |
| K02879 | Germline IgD Heavy Chain C-δ-3 Domain Coding Sequence | 33 |
| K02879 | Germline IgD Heavy Chain C-δ-3 Amino Acid Sequence | 28, 30, 32 |
| K01311 | Germline IgD Heavy Chain J-δ Region: C-δ CH1 Coding Sequence | 58 |
| K01311 | Germline IgD Heavy Chain J-δ Region: C-δ CH1 Amino Acid Sequence | 28, 30, 32 |
| K02880 | Germline IgD Heavy Chain Gene, C-Region, Secreted Terminus Coding Sequence | 36 |
| K02880 | Germline IgD Heavy Chain Gene, C-Region, Secreted Terminus Amino Acid Sequence | 28, 30, 32 |
| K02881 | Germline IgD-Heavy Chain Gene, C-Region, First Domain of Membrane Terminus Coding Sequence | 38 |
| K02881 | Germline IgD-Heavy Chain Gene, C-Region, First Domain of Membrane Terminus Amino Acid Sequence | 28, 30, 32 |
| K02882 | Germline IgD Heavy Chain Coding Sequence | 40 |
| K02882 | Germline IgD Heavy Chain Amino Acid Sequence | 28, 30, 32 |
| K02875 | Germline IgD Heavy Chain Gene, C-Region, C-δ-1 Domain Coding Sequence | 42 |
| K02875 | Germline IgD Heavy Chain Gene, C-Region, C-δ-1 Domain Amino Acid Sequence | 28, 30, 32 |
| L00022 | IgE Heavy Chain Constant Region Coding Sequence | 59 |
| J00227 | | |
| V00555 | | |
| L00022 | IgE Heavy Chain Constant Region Amino Acid Sequence | 60 |
| J00227 | | |
| V00555 | | |
| X17115 | IgM Heavy Chain Complete Sequence Coding Sequence | 61 |
| X17115 | IgM Heavy Chain Complete Sequence Amino Acid Sequence | 62 |

The ICAM-1 immunoadhesins of the present invention may, in addition to the chimeric ICAM-1 molecule, contain immunoglobulin light chains, or immunoglobulin J chain bound to the immunoglobulin derived heavy chains. In preferred embodiments, the immunoadhesin of the present invention comprises two or four chimeric ICAM-1 molecules and an immunoglobulin J chain bound to at least one of the chimeric ICAM-1 molecules. The J chain is described and known in the art. See, for example, M. Koshland, The Immunoglobulin Helper: The J Chain, in Immunoglobulin Genes, Academic Press, London, pg. 345, (1989) and Matsuuchi et al., Proc. Natl. Acad. Sci. U.S.A., 83:456-460 (1986). The sequence of the immunoglobulin J chain is available on various databases in the United States.

The immunoadhesin may have a secretory component associated with the chimeric ICAM-1 molecule. This association may occur by hydrogen bonds, disulfide bonds, covalent bonds, ionic interactions or combinations of these various bonds. Typically, chimeric ICAM-1 molecules are held together by disulfide bonds between the molecules. The interaction of the chimeric ICAM-1 molecules may be non-covalent or disulfide bonding.

The present invention contemplates the use of secretory component from a number of different species, including human, rat, rabbit, bovine and the like. The nucleotide sequences for these molecules are well known in the art. For example, U.S. Patent 6,046,037 contains many of the sequences and this patent is incorporated herein by reference. The immunoadhesins of the present invention containing the secretory component, the chimeric ICAM-1 molecule and J chain are typically bonded together by one of the following: hydrogen bonds, disulfide bonds, covalent bonds, ionic interactions or combinations of these bonds.

The present invention also contemplates immunoadhesins which comprise more than one receptor protein molecule, e.g., ICAM-1. ICAM-1 immunoadhesins, for example, may contain chimeric ICAM-1 molecules that are monomeric units and not disulfide bonded to other chimeric ICAM-1 molecules. In preferred embodiments, the immunoadhesin does contain chimeric ICAM-1 molecules that are in association with other chimeric ICAM-1 molecules to form dimers and other multivalent molecules. Typically the chimeric ICAM-1 molecule is present as a dimer because of the association of the immunoglobulin portion of the chimeric molecule. The immunoglobulin portion of the chimeric ICAM-1 molecule allows the association of two chimeric ICAM-1 molecules to form a dimeric molecule having two active binding portions made up of the rhinovirus receptor protein portion. In preferred embodiments, dimerization occurs via the disulfide bonding regions that normally occur between the immunoglobulin domains as part of a naturally-occurring immunoglobulin molecule and the native immunoglobulin protein. One of skill in the art will understand that these disulfide bonds that are normally present in the native immunoglobulin molecule can be modified, moved and removed while still maintaining the ability to form a dimer of the chimeric ICAM-1 molecules.

In other preferred embodiments, the immunoadhesin contains multimeric forms of the chimeric ICAM-1 molecule due to the association of J chain with the immunoglobulin portion of the chimeric ICAM molecule. The association of J chain with the dimer of two chimeric ICAM-1 molecules allows the formation of tetrameric forms of the immunoadhesin. In a preferred embodiment, the immunoglobulin portion of the chimeric ICAM-1 molecule is derived from the IgA molecule, and the addition of J chain allows the formation of a tetrameric complex containing four chimeric ICAM-1 molecules and four binding sites. In other preferred embodiments, the immunoglobulin heavy-chain portion of the chimeric molecule is derived from IgM and multivalent complexes containing ten or twelve molecules may be formed. In other preferred embodiments, in which the chimeric ICAM-1 molecule uses a chimeric immunoglobulin heavy-chain, the chimeric ICAM-1 molecule may form dimers or other higher order multivalent complexes through the domains from either IgA or IgM that are responsible for J chain binding. In other chimeric immunoglobulin molecules the portions of the immunoglobulin responsible for the disulfide bonding between the two immunoglobulin heavy-chains and/or the disulfide bonding between an immunoglobulin light-chain and heavy-chain may be placed in the chimeric immunoglobulin molecule to allow the formation of dimers or other high order multivalent complexes.

Various aspects and embodiments contemplate a chimeric ICAM-1 molecule in which the immunoglobulin domains comprising the heavy chain are derived from different isotypes of either heavy or light chain immunoglobulins. One skilled in the art will understand that using molecular techniques, these domains can be substituted for a similar domain and thus produce an immunoglobulin that is a hybrid between two different immunoglobulin molecules. These chimeric immunoglobulins allow immunoadhesins containing secretory component to be constructed that contain a variety of different and desirable properties that are conferred by different immunoglobulin domains.

Also contemplated are chimeric ICAM-1 molecules in which the portion of the chimeric molecule derived from immunoglobulin, heavy or light chain may contain less than an entire domain derived from a different immunoglobulin molecule. The same molecular techniques may be employed to produce such chimeric ICAM-1 molecules.

In preferred embodiments, the chimeric ICAM-1 molecules contain at least the CH1, CH2, CH3, domain of mouse or human IgA1, IgA2 or IgM. Other preferred embodiments of the present invention contain immunoglobulin domains that include at least the Cµl, Cµ2, Cµ3, or Cµ4 domains of IgM.

Preferred chimeric ICAM-1 molecules contain domains from two different isotypes of human immunoglobulin. Preferred chimeric ICAM-1 molecules that include immunoglobulins that contain immunoglobulin domains including at least the CH1, CH2, or CH3 of human IgG, IgG1, IgG2, IgG3, IgG4, IgA1, IgA2, IgE, or IgD. Other preferred immunoglobulins for use as part of chimeric ICAM-1 molecules include immunoglobulins that contain domains from at least the CH1, CH2, CH3, or CH4 domain of IgM or IgE. The present invention also contemplates chimeric ICAM-1 molecules that contain immunoglobulin domains derived from at least two different isotypes of mammalian immunoglobulins. Generally, any of the mammalian immunoglobulins can be used in the preferred embodiments, such as the following isotypes: any isotype of IgG, any isotype of IgA, IgE, IgD or IgM. Chimeric ICAM-1 molecules derived from a species such as human, mouse or other mammals are contemplated. In preferred embodiments, the chimeric ICAM-1 molecule contains the portion of IgA or IgM responsible for the association of J chain with the IgA and IgM. Thus, by using a chimeric immunoglobulin in the chimeric ICAM-1 molecule, the J chain may associate with a chimeric immunoglobulin that is predominantly of an isotype that does not bind J chain or secretory component.

The present invention also contemplates chimeric molecules that contain immunoglobulin domains derived from two different isotypes of rodent or primate immunoglobulin. The isotypes of rodent or primate immunoglobulin are well known in the art. The chimeric molecules of the present invention may contain immunoglobulin derived heavy chains that include at least one of the following immunoglobulin domains: the CH1, CH2, or CH3 domains of a mouse IgG, IgG1, IgG2a, IgG2b, IgG3, IgA, IgE, or IgD; the CH1, CH2, CH3 or CH4 domain of mouse IgE or IgM; the CH1 domain of a human IgG, IgG1, IgG2, IgG3, IgG4, IgA1, IgA2, or IgD; the CH1, CH2, CH3, CH4 domain of human IgM or IgE; the CH1, CH2, or CH3 domain of an isotype of mammalian IgG, an isotype of IgA, IgE, or IgD; the CH1, CH2, CH3 or CH4 domain of a mammalian IgE or IgM; the CH1, CH2 , or CH3 domain of an isotype of rodent IgG, IgA, IgE, or IgD; the CH1, CH2, CH3 or CH4 domain of a rodent IgE or IgM; the CH1, CH2, or CH3 domain of an isotype of animal IgG, an isotype of IgA, IgE, or IgD; and the CH1, CH2, CH3, or CH4 domain of an animal IgE or IgM. The present invention also contemplates the replacement or addition of protein domains derived from molecules that are members of the immunoglobulin superfamily into the chimeric molecules, e.g., chimeric ICAM-1. The molecules that belong to the immunoglobulin superfamily have amino acid residue sequence and nucleic acid sequence homology to immunoglobulins. The molecules that are part of the immunoglobulin superfamily can be identified by amino acid or nucleic acid sequence homology. See, for example, p. 361 of Immunoglobulin Genes, Academic Press (1989).

In preferred embodiments of the present invention, the immunoadhesin is expressed by methods that generate an immunoadhesin having plant-specific glycosylation. It is well-known in the art that glycosylation is a major modification of proteins in plant cells (Lerouge et al., Plant Molecular Biology 38:31-48, 1998). Glycosylation of proteins also occurs in other cell types, including mammalian and insect cells. The glycosylation process starts in the endoplasmic reticulum by the co-translational transfer of a precursor oligosaccharide to specific residues of the nascent polypeptide chain. Processing of this oligosaccharide into different types of glycans, which differ in the types of residues present and the nature of the linkages between the residues, occurs in the secretory pathway as the glycoprotein moves from the endoplasmic reticulum to its final destination. One of skill in the art will understand that at the end of their maturation, proteins expressed in plants or plant cells have a different pattern of glycosylation than do proteins expressed in other types of cells, including mammalian cells and insect cells. Detailed studies characterizing plant-specific glycosylation and comparing it with glycosylation in other cell types have been performed, for example, in studies described by Cabanes-Macheteau et al., Glycobiology 9(4):365-372 (1999), and Altmann, Glycoconjugate J. 14:643-646 (1997). These groups and others have shown that plant-specific glycosylation generates glycans that have xylose linked β(1,2) to mannose, but xylose is not linked β(1,2) to mannose as a result of glycosylation in mammalian and insect cells. Plant-specific glycosylation results in a fucose linked α(1,3) to the proximal GlcNAc, while glycosylation in mammalian cells results in a fucose linked α(1,6) to the proximal GlcNAc. Furthermore, plant-specific glycosylation does not result in the addition of a sialic acid to the terminus of the protein glycan, whereas in glycosylation in mammalian cells, sialic acid is added.

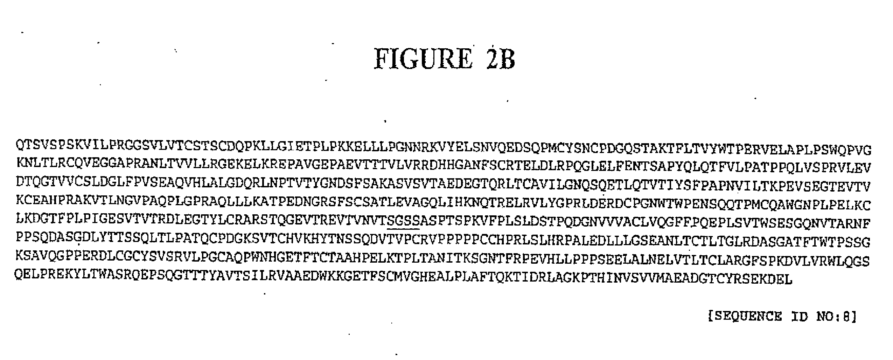

The immunoadhesin of the present invention that is glycosylated in a plant-specific manner can contain a chimeric molecule, e.g., chimeric ICAM-1, that includes any combination of extracellular domains, e.g., domains 1, 2, 3, 4, and 5 of the ICAM-1 molecule. FIG. 2B shows the amino acid sequence of the chimeric ICAM-1/IgA2 molecule (SEQ ID NO: 8) of the present invention, that contains all five domains of ICAM-1. The bolded N's represent asparagine residues to which oligosaccharide moieties are linked during glycosylation in plant cells, as well as mammalian and insect cells. One of skill in the art will know that the glycosylation sites are the tripeptide Asn-X-Ser/Thr where X can be any amino acid except proline and aspartic acid (Kornfeld and Kornfeld, Annu Rev Biochem 54:631-664, 1985). It will therefore be known to one of skill in the art that which amino acids of the protein having plant-specific glycosylation would depend on which domains of ICAM-1 are present. Because the sequence and domain boundaries of ICAM-1 are known (see Staunton et. al., Cell 52:925-933, 1988), it would be evident to one of skill in the art how to determine the plant-specific glycosylation sites on any potential combination of any of the five ICAM-1 domains.

In other preferred ICAM-1 aspects and embodiments of the present invention, the immunoadhesin having plant-specific glycosylation and containing a chimeric ICAM-1 molecule having any combination of ICAM-1 extracellular domains 1, 2, 3,4 and 5 further comprises a J chain and secretory component associated with said chimeric ICAM-1 molecule. As was true with respect to the chimeric ICAM-1 molecule, one of skill in the art will be able to identify the sites for plant-specific glycosylation in the J chain and secretory component sequences. The same principle applies for immunoahesins of the invention that contain chimeric receptor proteins other than chimeric ICAM -1.

The present invention contemplates immunoadhesins having plant-specific glycosylation, that contain a chimeric molecule, e.g., a chimeric ICAM-1 molecule, in which the immunoglobulin heavy chain is selected from the group of IgA (SEQ ID NOS:15-18 and 52-53), IgA1 (SEQ ID NOS:15-16 and 52), IgA2 (SEQ ID NO:17 and 53), IgG1 (SEQ ID NOS:19-20 and 54), IgG2 (SEQ ID NOS:21-22 and 55), IgG3 (SEQ ID NOS:23-24 and 56), IgG4 (SEQ ID NOS:25-26 and 57), IgM (SEQ ID NOS:46-47 and 61-62), IgD (SEQ ID NOS:27-33, 35-36, 38,40, and 42), IgE (SEQ ID NOS:44-45 and 59-60), and a chimeric immunoglobulin heavy chain. One of skill in the art will know that which of these immunoglobulin heavy chain sequences, or which combination of immunoglobulin heavy chain sequences are combined in a chimeric immunoglobulin heavy chain, will have an effect on the number and location of glycosylation sites in the chimeric molecule of the immunoadhesin. As was true with respect to the chimeric molecule, one of skill in the art will be able to identify the sites for plant-specific glycosylation in the immunoglobulin heavy chain sequences, including the various chimeric immunoglobulin heavy chain sequences that can be constructed.

Also provided herein are immunoadhesin functional derivatives. By "functional derivative" is meant a "chemical derivative," "fragment," or "variant," of the polypeptide or nucleic acid of the invention which retains at least a portion of the function of the protein, for example reactivity with an antibody specific for the protein, enzymatic activity or binding activity, which permits its utility in accordance with the present invention. It is well known in the art that due to the degeneracy of the genetic code numerous different nucleic acid sequences can code for the same amino acid sequence. It is also well known in the art that conservative changes in amino acid can be made to arrive at a protein or polypeptide that retains the functionality of the original. In both cases, all permutations are intended to be covered by this disclosure.

The derivatives may also be engineered according to routine methods to include an affinity purification tag such that large quantities and/or relatively pure or isolated quantities of immunoadhesin may be produced. Many different versions of tag exist that can be incorporated into one or more components of the immunoadhesin, preferably not destroying the desired binding activity of the immunoadhesin in the absence of tag. Such tags can be engineered as expressible encoded nucleic acid sequence fused with nucleic acid sequences encoding the immunoadhesins of the invention. The tags may further be engineered to be removable, e.g., with a commercially available enzyme.

Further, it is possible to delete codons or to substitute one or more codons with codons other than degenerate codons to produce a structurally modified polypeptide, but one which has substantially the same utility activity as the polypeptide produced by the unmodified nucleic acid molecule. As recognized in the art, the two polypeptides can be functionally equivalent, as are the two nucleic acid molecules that give rise to their production, even though the differences between the nucleic acid molecules are not related to the degeneracy of the genetic code.

Manipulations of this sort, and post-production chemical derivatization may be implemented, e.g., to improve stability, solubility, absorption, biological or therapeutic effect, and/or biological half-life. Moieties capable of mediating such effects are disclosed, for example, in Remington's Pharmaceutical Sciences, 18th ed., Mack Publishing Co., Easton, PA (1990). A functional derivative intended to be within the scope of the present invention is a "variant" polypeptide which either lacks one or more amino acids or contains additional or substituted amino acids relative to the native polypeptide. The variant may be derived from a naturally occurring complex component by appropriately modifying the protein DNA coding sequence to add, remove, and/or to modify codons for one or more amino acids at one or more sites of the C-terminus, N-terminus, and/or within the native sequence. It is understood that such variants having added, substituted and/or additional amino acids retain one or more characterizing portions of the native protein, as described above.

A functional derivative of a protein with deleted, inserted and/or substituted amino acid residues may be prepared using standard techniques well-known to those of ordinary skill in the art. For example, the modified components of the functional derivatives may be produced using site-directed mutagenesis techniques (as exemplified by Adelman et. al., 1983, DNA 2:183) wherein nucleotides in the DNA coding sequence are modified such that a modified coding sequence is produced, and thereafter expressing this recombinant DNA in a prokaryotic or eukaryotic host cell, using techniques such as those described above. Alternatively, proteins with amino acid deletions, insertions and/or substitutions may be conveniently prepared by direct chemical synthesis, using methods well-known in the art. The functional derivatives of the proteins typically exhibit the same qualitative biological activity as the native proteins.

In addition, the immunoadhesins of the invention may be not just modified receptor protein/Ig immunoadhesins, but may also embrace other native receptor protein family members, isotypes, and/or other homologous amino acid sequences, e.g., human, primate, rodent, canine, feline, bovine, avian, etc. Furthermore, the Ig type used in the immunoadhesins can vary, e.g., may assume a different Ig family member identity, within without a given species. ICAMs and Igs, for example, are diverse and have well-known sequences that one of ordinary skill can exploit to create different immunoadhesins having more or less different utility in a given organism to undergo treatment. An illustrative, nonexhaustive list of examples of molecules having ICAM-1 homology that can be used to create other immunoadhesins include those in the following table.

**Table 3**

| **ACCESSION NO.** | **ICAM NAME** | | **SPECIES** |
|---|---|---|---|
| NP 000192 | Intercellular Adhesion Molecule-1 (CD54) | [SEQ ID NO:63] | Homo sapiens |
| AAH03097 | Intercellular Adhesion Molecule ICAM-2 | [SEQ ID NO:64] | Homo sapiens |
| NP 002153 | Intercellular Adhesion Molecule 3 Precursor | [SEQ ID NO:65] | Homo sapiens |
| BAB20325 | TCAM-1 | [SEQ ID NO:66] | Homo sapiens |
| NP 003250 | Intercellular Adhesion Molecule 5 (Telencephalin) | [SEQ ID NO:67] | Homo sapiens |
| NM 007164 | Mucosal Vascular Address in Cell Adhesion Molecule (MADCAM1) | [SEQ ID NO:68] | Homo sapiens |
| NM 001078 | Vascular Cell Adhesion Molecule 1 (VCAM1) | [SEQ ID NO:69] | Homo sapiens |
| AAA37875 | MALA-2 | [SEQ ID NO:70] | Mus musculus |
| AAA37876 | Intercellular Adhesion Molecule-1 Precursor | [SEQ ID NO:71] | Mus musculus |
| AAG30280 | Intracellular Adhesion Molecule | [SEQ ID NO:72] | Cricetulus griseus |
| AAB39264 | Intercellular Adhesion Molecule-3 | [SEQ ID NO:73] | Bos taurus |
| AAF80287 | Intercellular Adhesion Molecule-1 Precursor | [SEQ ID NO:74] | Sus scrofa |
| AAA18478 | Telecephalin | [SEQ ID NO:75] | Oryctolagus cuniculus |
| NP 032345 | Intercellular Adhesion Molecule 5, telencephalin | | Mus musculus |
| | | [SEQ ID NO:76] | |
| BAB41106 | Cell adhesion molecule TCAM-1 | | Mus musculus |
| | | [SEQ ID NO:77] | |
| NP 067705 | Testicular Cell Adhesion Molecule | | Rattus norvegicus |
| | | [SEQ ID NO:78] | |
| AAG35584 | Nectin-Like Protein 1 | | Mus musculus |
| | | [SEQ ID NO:79] | |
| AAC18956 | CD22 Protein | | Homo sapiens |
| | | [SEQ ID NO:80] | |
| AAA35415 | Intercellular Adhesion Molecule 1 | | Pan troglodytes |
| | | [SEQ ID NO:81] | |
| AAA83206 | 89 kDa Protein | | Mus musculus |
| | | [SEQ ID NO:82] | |
| AAA92551 | Intercellular Adhesion Molecule-1 | | Canis familiaris |
| | | [SEQ ID NO:83] | |
| AAB06749 | Intercellular Adhesion Molecule-1 | | Bos taurus |
| | | [SEQ ID NO:84] | |
| AAD13617 | Intercellular Adhesion Molecule-1 Precursor | | Ovis aries |
| | | [SEQ ID NO:85] | |
| NP 037099 | Intercellular Adhesion Molecule-1 | | Rattus norvegicus |
| | | [SEQ ID NO:86] | |
| AAE22202 | ICAM-4 | | Rattus norvegicus |
| | | [SEQ ID NO:87] | |
| AAA60392 | cell surface glycoprotein | | Homo sapiens |
| | | [SEQ ID NO:88] | |
| AAF91086 | Nephrin | | Rattus norvegicus |
| | | [SEQ ID NO:89] | |
| AAF91087 | Nephrin | | Mus musculus |
| | | [SEQ ID NO:90] | |

Likewise, numerous heavy chain constant regions of different Ig molecules, both in humans and other species, are known that can be substituted in for those specific Ig regions of the chimeras described herein.

### C. Vectors, Cells and Plants Containing Immunoadhesins

The present invention also contemplates expression and cloning vectors, cells and plants containing the immunoadhesins of the present invention. Technology for isolating the genes encoding the various portions of the immunoadhesions are well-known to one of skill in the art and can be applied to insert the various required genes into expression vectors and cloning vectors such as those vectors can be introduced into cells and into transgenic plants.

The present invention contemplates a method of assembling an immunoadhesin comprising the steps of: introducing into an organism a DNA segment encoding a chimeric receptor protein molecule (e.g., an ICAM-1 molecule), immunoglobulin J chain, and introducing into the same organism a DNA encoding a secretory component. The preferred secretory component contains at least a segment of the amino acid residues 1 to residue about 606 of the human polyimmunoglobulin receptor (pIgR) amino acid residue sequence or analogous amino acid residues from other species (Mostov, Ann Dev. Immu. 12:63-84 1994).

The present invention contemplates eukaryotic cells, including plant cells, containing immunoadhesins of the present invention. The present invention also contemplates plant cells that contain nucleotide sequences encoding the various components of the immunoadhesin of the present invention. One skilled in the art will understand that the nucleotide sequences that encode the secretory component protection protein and the chimeric receptor protein molecule and J chain will typically be operably linked to a promoter and present as part of an expression vector or cassette. Typically, if the eukaryotic cell used is a plant cell then the promoter used will be a promoter that is able to operate in a plant cell. After the chimeric receptor protein genes, secretory component genes and J chain genes are isolated, they are typically operatively linked to a transcriptional promoter in an expression vector. The present invention also contemplates expression vectors containing a nucleotide sequence encoding a chimeric receptor protein molecule which has been operatively linked to a regulatory sequence for expression. These expression vectors place the nucleotide sequence to be expressed in a particular cell 3' of a promoter sequence which causes the nucleotide sequence to be transcribed and expressed. The expression vector may also contain various enhancer sequences which improve the efficiency of this transcription. In addition, such sequences as terminators, polyadenylation (poly A) sites and other 3' end processing signals may be included to enhance the amount of nucleotide sequence transcribed within a particular cell.

Expression of the components in the organism of choice can be derived from an independently replicating plasmid, or from a permanent component of the chromosome, or from any piece of DNA which may transiently give rise to transcripts encoding the components. Organisms suitable for transformation can be either prokaryotic or eukaryotic. Introduction of the components of the complex can be by direct DNA transformation, by biolistic delivery into the organism, or mediated by another organism as for example by the action of recombinant Agrobacterium on plant cells. Expression of proteins in transgenic organisms usually requires co-introduction of an appropriate promoter element and polyadenylation signal. In one embodiment of the invention, the promoter element potentially results in the constitutive expression of the components in all of the cells of a plant. Constitutive expression occurring in most or all of the cells will ensure that precursors can occupy the same cellular endomembrane system as might be required for assembly to occur.

Expression vectors compatible with the host cells, preferably those compatible with plant cells are used to express the genes of the present invention. Typical expression vectors useful for expression of genes in plants are well known in the art and include vectors derived from the tumor-inducing (Ti) plasmid of Agrobacterium tumefaciens described by Rogers et al., Meth. in Enzymol., 153:253-277 (1987). However, several other expression vector systems are known to function in plants. See for example, Verma et al., PCT Publication No. WO87/00551; and Cocking and Davey, Science, 236:1259-1262 (1987).

The expression vectors described above contain expression control elements including the promoter. The genes to be expressed are operatively linked to the expression vector to allow the promoter sequence to direct RNA polymerase binding and synthesis of the desired polypeptide coding gene. Useful in expressing the genes are promoters which are inducible, viral, synthetic, constitutive, and regulated. The choice of which expression vector is used and ultimately to which promoter a nucleotide sequence encoding part of the immunoadhesin of the present invention is operatively linked depends directly, as is well known in the art, on the functional properties desired, e.g. the location and timing of protein expression, and the host cell to be transformed, these being limitations inherent in the art of constructing recombinant DNA molecules. However, an expression vector useful in practicing the present invention is at least capable of directing the replication, and preferably also the expression of the polypeptide coding gene included in the DNA segment to which it is operatively linked.

In preferred embodiments, the expression vector used to express the genes includes a selection marker that is effective in a plant cell, preferably a drug resistance selection marker. A preferred drug resistance marker is the gene whose expression results in kanamycin resistance, i.e., the chimeric gene containing the nopaline synthase promoter, Tn5 neomycin phosphotransferase II and nopaline synthase 3' nontranslated region described by Rogers et al., in Methods For Plant Molecular Biology, a Weissbach and H. Weissbach, eds., Academic Press Inc., San Diego, Calif. (1988). A useful plant expression vector is commercially available from Pharmacia, Piscataway, N.J. Expression vectors and promoters for expressing foreign proteins in plants have been described in U.S. Pat. Nos. 5,188,642; 5,349,124; 5,352,605, and 5,034,322 which are hereby incorporated by reference.

A variety of methods have been developed to operatively link DNA to vectors via complementary cohesive termini. For instance, complementary homopolymer tracks can be added to the DNA segment to be inserted into the vector DNA. The vector and DNA segment are then joined by hydrogen bonding between the complementary homopolymeric tails to form recombinant DNA molecules. Alternatively, synthetic linkers containing one or more restriction endonuclease sites can be used to join the DNA segment to the expression vector. The synthetic linkers are attached to blunt-ended DNA segments by incubating the blunt-ended DNA segments with a large excess of synthetic linker molecules in the presence of an enzyme that is able to catalyze the ligation of blunt-ended DNA molecules, such as bacteriophase T4 DNA ligase. Thus, the products of the reaction are DNA segments carrying synthetic linker sequences at their ends. These DNA segments are then cleaved with the appropriate restriction endonuclease and ligated into an expression vector that has been cleaved with an enzyme that produces termini compatible with those of the synthetic linker. Synthetic linkers containing a variety of restriction endonuclease sites are commercially available from a number of sources including New England BioLabs, Beverly, Mass.

The nucleotide sequences encoding the secretory component, J chain, and the chimeric receptor protein molecules, e.g., ICAM-1, of the present invention are introduced into the same plant cell either directly or by introducing each of the components into a plant cell and regenerating a plant and cross-hybridizing the various components to produce the final plant cell containing all the required components.

Any method may be used to introduce the nucleotide sequences encoding the components of the immunoadhesins of the present invention into a eukaryotic cell. For example, methods for introducing genes into plants include Agrobacterium-mediated plant transformation, protoplast transformation, gene transfer into pollen, injection into reproductive organs and injection into immature embryos. Each of these methods has distinct advantages and disadvantages. Thus, one particular method of introducing genes into a particular eukaryotic cell or plant species may not necessarily be the most effective for another eukaryotic cell or plant species.

Agrobacterium tumefaciens-mediated transfer is a widely applicable system for introducing genes into plant cells because the DNA can be introduced into whole plant tissues, bypassing the need for regeneration of an intact plant from a protoplast. The use of Agrobacterium-mediated expression vectors to introduce DNA into plant cells is well known in the art. See, for example, the methods described by Fraley et al., Biotechnology, 3:629 (1985) and Rogers et al., Methods in Enzymology, 153:253-277 (1987). Further, the integration of the Ti-DNA is a relatively precise process resulting in few rearrangements. The region of DNA to be transferred is defined by the border sequences and intervening DNA is usually inserted into the plant genome as described by Spielmann et al., Mol. Gen. Genet., 205:34 (1986) and Jorgensen et al., Mol. Gen. Genet., 207:471 (1987). Modem Agrobacterium transformation vectors are capable of replication in Escherichia coli as well as Agrobacterium, allowing for convenient manipulations as described by Klee et al., in Plant DNA Infectious Agents, T. Hohn and J. Schell, eds., Springer-Verlag, New York, pp. 179-203 (1985). Further recent technological advances in vectors for Agrobacterium-mediated gene transfer have improved the arrangement of genes and restriction sites in the vectors to facilitate construction of vectors capable of expressing various polypeptide coding genes. The vectors described by Rogers et al., Methods in Enzymology, 153:253 (1987), have convenient multi-linker regions flanked by a promoter and a polyadenylation site for direct expression of inserted polypeptide coding genes and are suitable for present purposes.

In those plant species where Agrobacterium-mediated transformation is efficient, it is the method of choice because of the facile and defined nature of the gene transfer. Agrobacterium-mediated transformation of leaf disks and other tissues appears to be limited to plant species that Agrobacterium tumefaciens naturally infects. Thus, Agrobacterium-mediated transformation is most efficient in dicotyledonous plants.

Few monocots appear to be natural hosts for Agrobacterium, although transgenic plants have been produced in asparagus using Agrobacterium vectors as described by Bytebier et al., Proc. Natl. Acad. Sci. U.S.A., 84:5345 (1987). Therefore, commercially important cereal grains such as rice, corn, and wheat must be transformed using alternative methods. Transformation of plant protoplasts can be achieved using methods based on calcium phosphate precipitation, polyethylene glycol treatment, electroporation, and combinations of these treatments. See, for example, Potrykus et al., Mol. Gen. Genet., 199:183 (1985); Lorz et al., Mol. Gen. Genet., 199:178 (1985); Fromm et al., Nature, 319:791 (1986); Uchimiya et al., Mol. Gen. Genet., 204:204 (1986); Callis et al., Genes and Development, 1:1183 (1987); and Marcotte et al., Nature, 335:454 (1988).

Application of these methods to different plant species depends upon the ability to regenerate that particular plant species from protoplasts. Illustrative methods for the regeneration of cereals from protoplasts are described in Fujimura et al., Plant Tissue Culture Letters, 2:74 (1985); Toriyama et al., Theor Appl. Genet., 73:16 (1986); Yamada et al., Plant Cell Rep., 4:85 (1986); Abdullah et al., Biotechnology, 4:1087 (1986).

To transform plant species that cannot be successfully regenerated from protoplasts, other ways to introduce DNA into intact cells or tissues can be utilized. For example, regeneration of cereals from immature embryos or explants can be effected as described by Vasil, Biotechnology, 6:397 (1988). In addition, "particle gun" or high-velocity microprojectile technology can be utilized. Using such technology, DNA is carried through the cell wall and into the cytoplasm on the surface of small (0.525 µm) metal particles that have been accelerated to speeds of one to several hundred meters per second as described in Klein et al., Nature, 327:70 (1987); Klein et al., Proc. Natl. Acad. Sci. U.S.A., 85:8502 (1988); and McCabe et al., Biotechnology, 6:923 (1988). The metal particles penetrate through several layers of cells and thus allow the transformation of cells within tissue explants. Metal particles have been used to successfully transform corn cells and to produce fertile, stably transformed tobacco and soybean plants. Transformation of tissue explants eliminates the need for passage through a protoplast stage and thus speeds the production of transgenic plants.

DNA can also be introduced into plants by direct DNA transfer into pollen as described by Zhou et al., Methods in Enzymology, 101:433 (1983); D. Hess, Intern Rev. Cytol., 107:367 (1987); Luo et al., Plant Mol. Biol. Reporter, 6:165 (1988). Expression of polypeptide coding genes can be obtained by injection of the DNA into reproductive organs of a plant as described by Pena et al., Nature, 325:274 (1987). DNA can also be injected directly into the cells of immature embryos and the rehydration of desiccated embryos as described by Neuhaus et al., Theor. Appl. Genet., 75:30 (1987); and Benbrook et al., in Proceedings Bio Expo 1986, Butterworth, Stoneham, Mass., pp. 27-54 (1986).

The regeneration of plants from either single plant protoplasts or various explants is well known in the art. See, for example, Methods for Plant Molecular Biology, A. Weissbach and H. Weissbach, eds., Academic Press, Inc., San Diego, Calif. (1988). This regeneration and growth process includes the steps of selection of transformant cells and shoots, rooting the transformant shoots and growth of the plantlets in soil.

The regeneration of plants containing the foreign gene introduced by Agrobacterium tumefaciens from leaf explants can be achieved as described by Horsch et al., Science, 227:1229-1231 (1985). In this procedure, transformants are grown in the presence of a selection agent and in a medium that induces the regeneration of shoots in the plant species being transformed as described by Fraley et al., Proc. Natl. Acad. Sci. U.S.A., 80:4803 (1983). This procedure typically produces shoots within two to four weeks and these transformant shoots are then transferred to an appropriate root-inducing medium containing the selective agent and an antibiotic to prevent bacterial growth. Transformant shoots that rooted in the presence of the selective agent to form plantlets are then transplanted to soil to allow the production of roots. These procedures will vary depending upon the particular plant species employed, such variations being well known in the art.

The immunoadhesins of the present invention may be produced in any plant cell including plant cells derived from plants that are dicotyledonous or monocotyledonous, solanaceous, alfalfa, legumes, or tobacco.

Transgenic plants of the present invention can be produced from any sexually crossable plant species that can be transformed using any method known to those skilled in the art. Useful plant species are dicotyledons including tobacco, tomato, the legumes, alfalfa, oaks, and maples; monocotyledons including grasses, corn, grains, oats, wheat, and barley; and lower plants including gymnosperms, conifers, horsetails, club mosses, liverworts, hornworts, mosses, algaes, gametophytes, sporophytes or pteridophytes.

The present invention also contemplates expressing the immunoadhesins within eukaryotic cells including mammalian cells. One of skill in the art will understand the various systems available for expression of the immunoadhesin in mammalian cells and can readily modify those systems to express the immunoadhesins and chimeric protein receptor molecules, e.g., ICAM-1 molecules, in those cells. In preferred ICAM embodiments, the chimeric ICAM-1, J chain and secretory component molecules of the present invention are placed in a vector pCDM8 which has been previously described by Aruffo, et al., Proc. Natl. Acad. Sci. U.S.A., 84:8573-8577 (1987). The use of the PCDM8 construct is by no means unique and numerous other eukaryotic expression systems systems are available that do not utilize the cog cell expression system and that may be used with the chimeric ICAM-1 and other molecules of the immunoadhesin.

### D. Compositions Containing Immunoadhesins

The present invention also contemplates compositions containing an immunoadhesin of the present invention together with plant macromolecules or material. Typically these plant macromolecules or plant materials are derived from any plant useful in the present invention. The plant macromolecules are present together with an immunoadhesin of the present invention for example, in a plant cell, in an extract of a plant cell, or in a plant. Typical plant macromolecules associated with the immunoadhesin of the present invention in a composition are ribulose bisphosphate carboxylase, light harvesting complex pigments (LHCP), secondary metabolites or chlorophyll. The compositions of the present invention have plant material or plant macromolecules in a concentration of between 0.01 % and 99% mass excluding water. Other compositions include compositions having the immunoadhesins of the present invention present at a concentration of between 1% and 99% mass excluding water. Other compositions include immunoadhesins at a concentration of 50% to 90% mass excluding water.

The compositions of the present invention may contain plant macromolecules at a concentration of between 0.1 % and 5% mass excluding water. Typically the mass present in the composition will consist of plant macromolecules and immunoadhesins of the present invention. When the immunoadhesins of the present invention are present at a higher or lower concentration the concentration of plant macromolecules present in the composition will vary inversely. In other embodiments the composition of plant macromolecules are present in a concentration of between 0.12% and 1% mass excluding water.

The present invention contemplates a composition of matter comprising all or part of the following: a chimeric protein receptor molecule (e.g., an ICAM-1 molecule), a J chain or a secretory component. These components form a complex and are associated as was previously described. Typically, the composition also contains molecules derived from a plant. This composition may also be obtained after an extraction process yielding functional immunoadhesin and plant-derived molecules.

The extraction method comprises the steps of applying a force to a plant containing the complex whereby the apoplastic compartment of the plant is ruptured releasing said complex. The force involves shearing as the primary method of releasing the apoplastic liquid.

The whole plant or plant extract contains an admixture of immunoadhesin and various other macromolecules of the plant. Among the macromolecules contained in the admixture is ribulose bisphosphate carboxylase (RuBisCo) or fragments of RuBisCo. Another macromolecule is LHCP. Another molecule is chlorophyll.

Other useful methods for preparing compositions containing immunoadhesins include extraction with various solvents and application of vacuum to the plant material. The compositions of the present invention may contain plant macromolecules in a concentration of between about 0.1% and 5% mass excluding water.

The present invention also contemplates therapeutic compositions which may be used in the treatment of a patient or animal. Administration of the therapeutic composition can be before or after extraction from the plant or other transgenic organism. Once extracted the immunoadhesins may also be further purified by conventional techniques such as size exclusion, ion exchange, or affinity chromatography. Plant molecules may be co-administered with the complex.

The present invention also contemplates that the relative proportion of plant-derived molecules and animal-derived molecules can vary. Quantities of specific plant proteins, such as RuBisCo or chlorophyll may be as little as 0.01 % of the mass or as much as 99.9% of the mass of the extract, excluding water.

The present invention also contemplates the direct use of the therapeutic plant extract containing immunoadhesins without any further purification of the specific therapeutic component. Administration may be by topical application, oral ingestion, nasal spray or any other method appropriate for delivering the antibody to the mucosal target pathogen.

### E. Pharmaceutical Compositions, Formulations, And Routes Of Administration

The immunoadhesins described herein can be administered to a patient, preferably in the form of a suitable pharmaceutical composition. Such composition may include components in addition to, or in lieu of, those described above. The composition preferably exhibits either or both of a therapeutic and prophylactic property when administered. The preparation of such compositions can be done according to routine methodologies in the art, and may assume any of a variety of forms, e.g., liquid solutions, suspensions or emulsifications, and solid forms suitable for inclusion in a liquid prior to ingestion. Techniques for the formulation and administration of polypeptides and proteins may be found in Remington's Pharmaceutical Sciences, Mack Publishing Co., Easton, PA, latest edition. Using these procedures, one of ordinary skill can utilize the immunoadhesins of the invention to achieve success without undue experimentation.

### 1. Administration Routes

Suitable routes of administration for the invention include, e.g., oral, nasal, inhalation, intraocular, phanyngeal, bronchial, transmucosal, or intestinal administration. Alternatively, one may administer the compound in a local manner, e.g., via injection or other application of the compound to a preferred site of action.

### 2. Formulations

The pharmaceutical compositions of the present invention may be manufactured in a manner that is itself known, e.g., by means of conventional mixing, dissolving, granulating, dragee-making, levigating, emulsifying, encapsulating, entrapping or lyophilizing processes. One or more physiologically acceptable carriers comprising excipients and/or other auxiliaries can be used to facilitate processing of the active compounds into pharmaceutical preparations. Proper formulation is dependent upon the particular route of administration chosen.

For injection, the agents of the invention may be formulated in aqueous solutions, preferably in physiologically compatible buffers such as Hanks's solution, Ringer's solution, or physiological saline buffer. For transmucosal administration, penetrants appropriate to the barrier to be permeated are used in the formulation. Such penetrants are generally known in the art.

For oral administration, the compounds can be formulated readily by combining the active compounds with pharmaceutically acceptable carriers well known in the art. Such carriers enable the compounds of the invention to be formulated as tablets, pills, dragees, capsules, liquids, gels, syrups, slurries, suspensions and the like, for oral ingestion by a patient to be treated. Suitable carriers include excipients such as, e.g., fillers such as sugars, including lactose, sucrose, mannitol, and/or sorbitol; cellulose preparations such as, e.g., maize starch, wheat starch, rice starch, potato starch, gelatin, gum tragacanth, methyl cellulose, hydroxypropylmethyl- cellulose, sodium carboxymethylcellulose, and/or polyvinylpyrrolidone (PVP). If desired, disintegrating agents may be added, such as the cross-linked polyvinyl pyrrolidone, agar, or alginic acid or a salt thereof such as sodium alginate.

Dragee cores are provided with suitable coatings. For this purpose, concentrated sugar solutions may be used, which may optionally contain gum arabic, talc, polyvinyl pyrrolidone, carbopol gel, polyethylene glycol, and/or titanium dioxide, lacquer solutions, and suitable organic solvents or solvent mixtures. Dyestuffs or pigments may be added to the tablets or dragee coatings for identification or to characterize different combinations of active compound doses.

Pharmaceutical preparations which can be used orally include push-fit capsules made of gelatin, as well as soft, sealed capsules made of gelatin and a plasticizer, such as glycerol or sorbitol. The push-fit capsules can contain the active ingredients in admixture with filler such as lactose, binders such as starches, and/or lubricants such as talc or magnesium stearate and, optionally, stabilizers. In soft capsules, the active compounds may be dissolved or suspended in suitable liquids, such as fatty oils, liquid paraffin, or liquid polyethylene glycols. In addition, stabilizers may be added. All formulations for oral administration should be in dosages suitable for such administration.

For buccal administration, the compositions may take the form of tablets or lozenges formulated in conventional manner.

For administration by inhalation, the compounds for use according to the present invention may be conveniently delivered in the form of an aerosol spray presentation from pressurized packs or a nebuliser, with the use of a suitable propellant, e.g., dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of e.g. gelatin for use in an inhaler or insufflator may be formulated containing a powder mix of the compound and a suitable powder base such as lactose or starch.

Alternatively, the active ingredient may be in powder form for constitution with a suitable vehicle, e.g., sterile pyrogen-free water, before use.

In addition, the compounds may also be formulated as a depot preparation. Such long acting formulations may be administered by implantation (for example subcutaneously or intramuscularly) or by intramuscular injection. Thus, for example, the compounds may be formulated with suitable polymeric or hydrophobic materials (for example as an emulsion in an acceptable oil) or ion exchange resins, or as sparingly soluble derivatives, for example, as a sparingly soluble salt.

Additionally, the compounds may be delivered using a sustained-release system, such as semipermeable matrices of solid hydrophobic polymers containing the therapeutic agent. Various sustained-release materials have been established and are well known by those skilled in the art. Sustained-release capsules may, depending on their chemical nature, release the compounds for a few weeks up to over 100 days. Depending on the chemical nature and the biological stability of the therapeutic reagent, additional strategies for protein stabilization may be employed.

The pharmaceutical compositions also may comprise suitable solid or gel phase carriers or excipients. Examples of such carriers or excipients include but are not limited to calcium carbonate, calcium phosphate, various sugars, starches, cellulose derivatives, gelatin, and polymers such as polyethylene glycols.

Pharmaceutically compatible salts may be formed with many acids, including but not limited to hydrochloric, sulfuric, acetic, lactic, tartaric, malic, succinic, citric, etc. Salts tend to be more soluble in aqueous or other protonic solvents that are the corresponding free base forms. In solutions, manipulation of pH is also routinely employed for optimizing desired properties.

### 3. Determining Effective Dosages and Dosage Regimens

Pharmaceutical compositions suitable for use in the present invention include compositions where the active ingredients are contained in an amount effective to achieve an intended purpose, e.g., a therapeutic and/or prophylactic use. A pharmaceutically effective amount means an amount of compound effective to prevent, alleviate or ameliorate symptoms of disease or prolong the survival of the subject being treated. Determination of a pharmaceutically effective amount is well within the capability of those skilled in the art, and will typically assume an amount of between about 0.5 µg/kg/day and about 500g/kg/day, with individual dosages typically comprising between about 1 nanogram and several grams of immunoadhesin.

For any compound used in the methods of the invention, the therapeutically effective dose can be estimated initially from cell culture assays. For example, varying dosages can be administered to different animals or cell cultures and compared for effect. In this way, one can identify a desired concentration range, and prepare and administer such amount accordingly. Optimization is routine for one of ordinary skill in the art.

The person of skill, in addition to considering pharmaceutical efficacy, also considers toxicity according to standard pharmaceutical procedures in cell cultures or experimental animals, e.g., for determining the LD50 (the dose lethal to 50% of the population) and the ED₅₀ (the dose therapeutically effective in 50% of the population). The dose ratio between toxic and therapeutic effects is the therapeutic index and it can be expressed as the ratio between LD₅₀ and ED₅₀. Compounds which exhibit high therapeutic indices are preferred. The data obtained from these cell culture assays and animal studies can be used in formulating a range of dosage for use in humans. The dosage of such compounds lies preferably within a range of concentrations that include the ED₅₀ with little or no toxicity. The dosage may vary within this range depending upon the dosage form employed and the route of administration utilized. The exact formulation, route of administration and dosage can be chosen by the individual physician in view of the patient's condition. (See e.g., Fingl et al., 1975, in "The Pharmacological Basis of Therapeutics," Ch. 1 p.1).

Dosage amount and frequency may be adjusted to provide mucosal levels of immunadhesin sufficient to maintain or provide a pharmaceutical effect, e.g., therapeutic and/or prophylactic. The minimal effective concentration (MEC) will vary for each immunadhesin and immunoadhesin formulation, but can be estimated from in vitro and/or in vivo data. Dosages necessary to achieve MEC will depend on individual characteristics and route of administration. However, assays as described herein can be used to determine mucosal concentrations, which can then be further optimized in amount and precise formulation.

Dosage intervals can also be determined using MEC value. Compounds can be administered using a regimen which maintains mucosal levels above the MEC for 10-90% of the time, 30-90% of the time, or, most preferably, 50-90% of the time.

The compositions may, if desired, be presented in a pack or dispenser device which may contain one or more unit dosage forms containing the active ingredient. The pack may for example comprise metal or plastic foil, such as a blister pack. The pack or dispenser device may be accompanied by instructions for administration. The pack or dispenser may also be accompanied with a notice associated with the container in form prescribed by a governmental agency regulating the manufacture, use, or sale of pharmaceuticals, which notice is reflective of approval by the agency of the form of the immunoadhesin for human or veterinary administration. Such notice, for example, may be the labeling approved by the U.S. Food and Drug Administration for prescription drugs, or the approved product insert. Compositions comprising a compound of the invention formulated in a compatible pharmaceutical carrier may also be prepared, placed in an appropriate container, and labeled for treatment of an indicated condition, e.g. treatment or prophylaxis of a disease mediated by host organism/patient protein receptor molecules.

### F. Methods of Treatment and Prevention of ICAM-mediated Afflictions

A patient in need of therapeutic and/or prophylactic immunoadhesin chimeras of the invention, e.g., to counter rhinovirus infection and/or symptoms such as occur with colds, can be administered a pharmaceutically effective amount of desired immunoadhesin, preferably as part of a pharmaceutical composition determined, produced, and administered as described above. These formulations and delivery modalities can vary widely. Described following are preliminary procedures that can be used to deduce effective amounts and toxicity, and which can then be conveniently used to determine treatment and prophylaxis parameters and regimens, both in humans and other animals. These procedures are illustrative only and are not intended to be limiting of the invention. Further, these procedures are routine for one of ordinary skill in the art.

### 1. Ability of the Immunoadhesin to Reduce Rhinovirus Infectivity in Humans: Dose Escalation Tolerance Study

Immunoadhesins of the invention may be tested, e.g., using randomized controlled trials to determine the effect of administration, e.g., intranasal, of immunoadhesin on infection. Other administration routes can be used. Various assays exist that can be used to monitor effect, e.g., IL-8 response assays assays that evaluate illness symptoms, e.g., cold symptoms caused by rhinovirus infection. These studies can evaluate the extent to which an immunoadhesin taken by a patient subjects can prevent or treat rhinovirus infection. For example, healthy or unhealthy subjects can be administered the immunoadhesin and evalutated over a time course, e.g., in tandem with rhinovirus inoculation and/or illness progression. The clinical protocols used may be based on protocols previously used in evaluation of a recombinant soluble ICAM-1 molecule for efficacy against rhinovirus infection, or modifications thereto (Turner, et. al., JAMA 281:1797-804, 1999).

Male and female subjects of any species, age, health, or disease state can be evaluated The subjects may exhibit a serum neutralizing antibody titer in advance of study, which titer may fluctuate in response to infection and immunoadhesin administration.

The immunoadhesin of the present invention may be formulated as a buffered saline with varying amounts of immunoadhesin within and administered at various intervals to a patient. Single ascending dose and multiple ascending dose studies can be used to evaluate the safety of the immunoadhesin. In each case, one or more subjects may be evaluated at each dosage level, some receiving the immunoadhesin, and one or more optionally receiving placebo. In either study, multiple dosage levels may be evaluated. Dosage levels can vary, but are typically in the nanogram to gram range.

Dosages may be administered over seconds, minutes, hours, weeks, and months, and evaluated for toxicity and/or pharmaceutical effect.

Safety and toxicity may be assessed, e.g., by visual examination of the nasal mucosa for signs of irritation or inflammation. Blood safety evaluations can also be employed according to routine methods and using sensitive assays such as ELISA to determine various blood components, including circulating immunoadhesin and rhinovirus quantities. Naval lavage testing may similarly be done according to routine methodologies.

Routine statistical analyses and calculations may be employed to determine efficacy and toxicity predicted over time courses for single patients and/or for populations of patient-recipients..

Challenge studies as well known in the art can be used to demonstrate that treatment protects against clinical colds and/or reduces cold symptoms after viral challenge, and using commercially available starting materials such virus, cells, and animals. See, e.g., Gwaltney, et. al., Prog. Med. Virol. 39:256-263, 1992.

The following examples illustrate various aspects and embodiments of the disclosed invention. These examples in no way limit the scope of the claimed invention.

### EXAMPLES

### 1. Construction of ICAM-1 Immunoadhesin Expression Cassettes

A cassette encoding ICAM-1 extracellular domains D1 through D5 was prepared by PCR cloning. Specifically, a fragment containing all five extracellular Ig-like domains of ICAM-1 was amplified from plasmid pCDIC1-SD/IgA (Martin, et al. J. Virol. 67:3561-8, 1993) using the following oligonucleotide primers:
5'-
   TCTGTTCCCAGGAACTAGTTTGGCACAGACATCTGTGTCCCCCTCAAAAGTC-3' (SEQ ID NO: 6)
5'-CATACCGGGGACTAGTCACATTCACGGTCACCTCGCGG-3' (SEQ ID NO: 7)

These two primers were designed to introduce SpeI sites at the 5' and 3' ends of the PCR fragment (underlined nucleotides). PCR was performed with Pfu polymerase (Stratagene) to reduce accumulation of errors. The PCR fragment was cloned into the vector PCRScript (Stratagene), and sequenced before fusing to the human IgA2 cassettes (with and without SEKDEL [SEQ ID NO:4] at the carboxy-terminus).

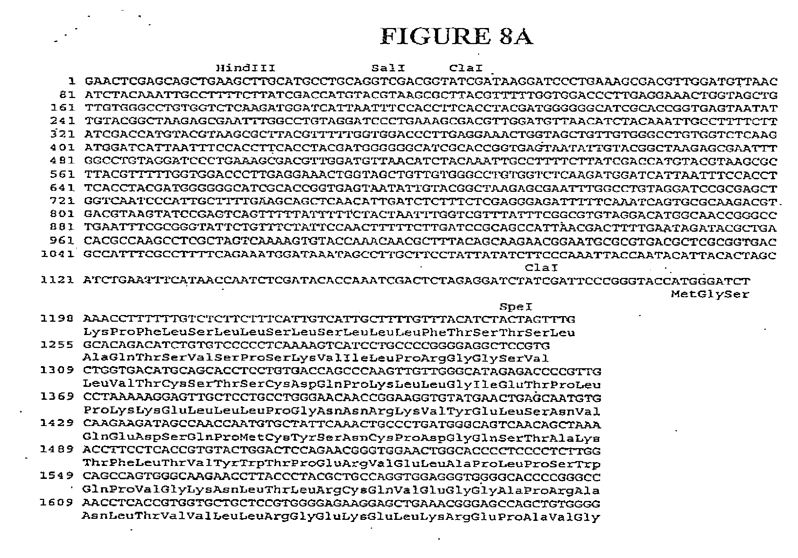

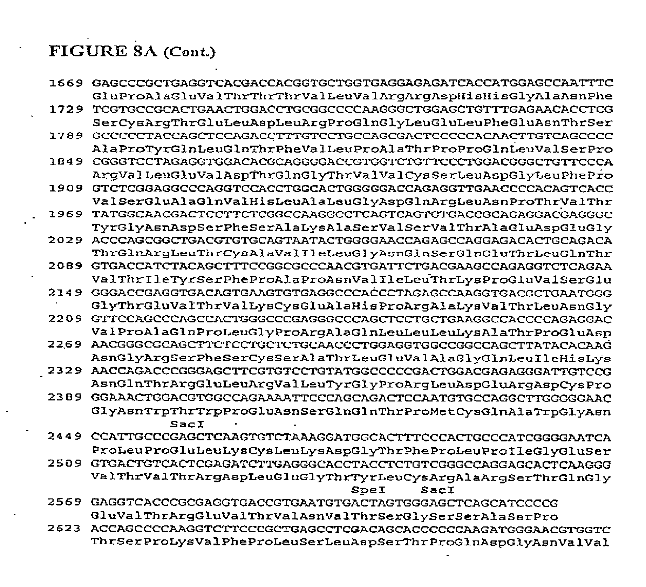

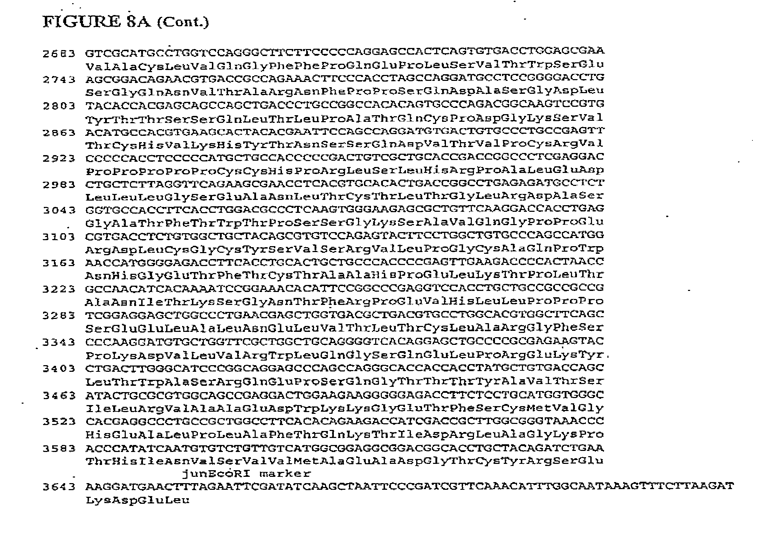

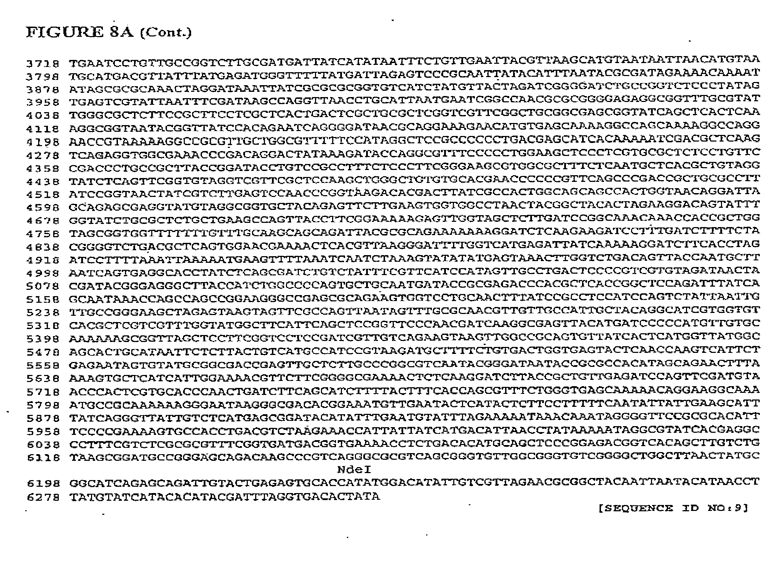

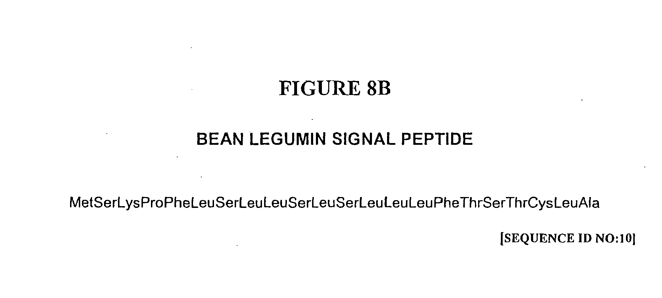

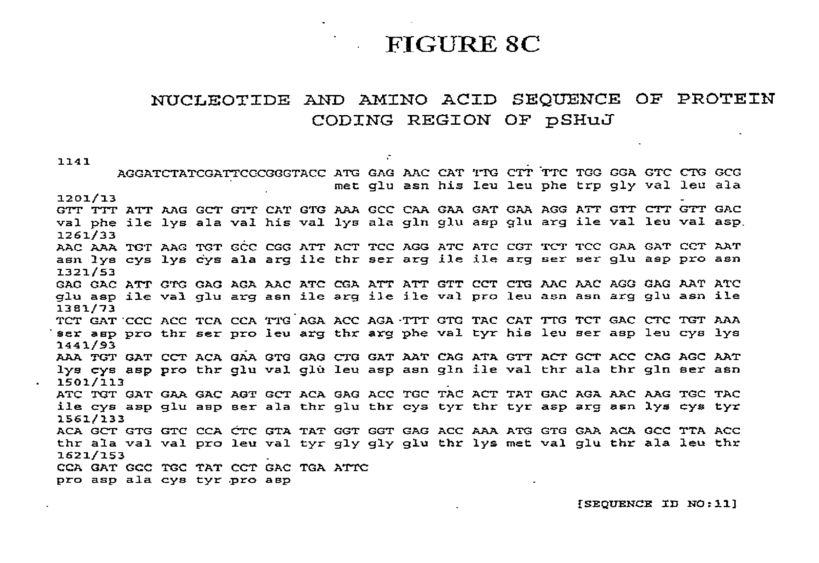

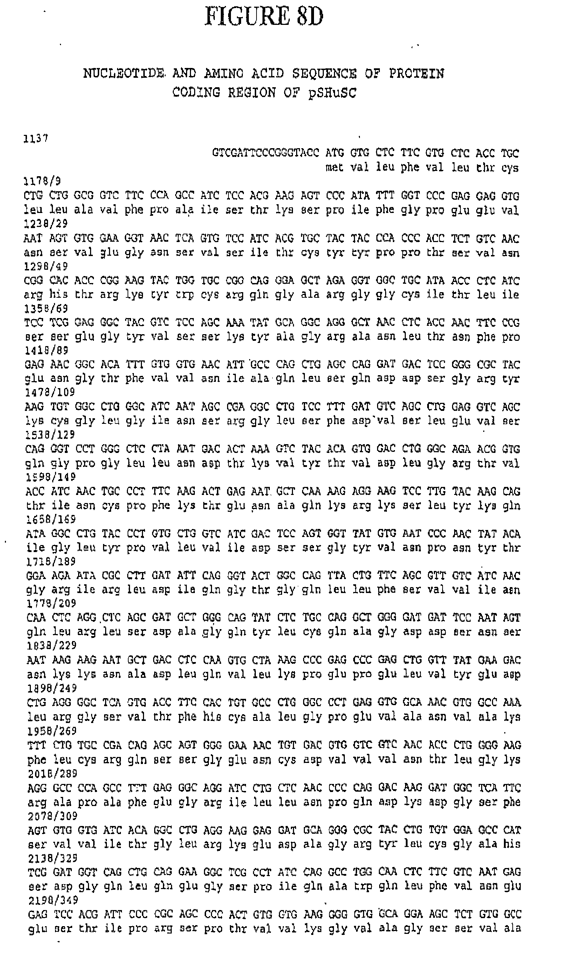

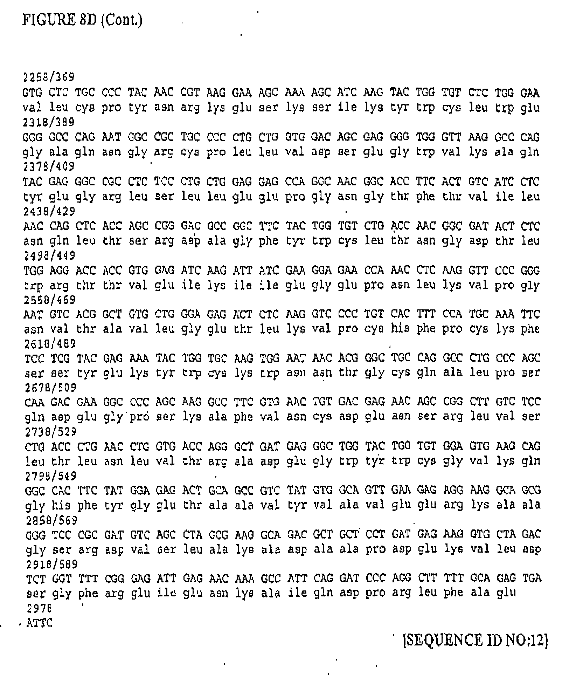

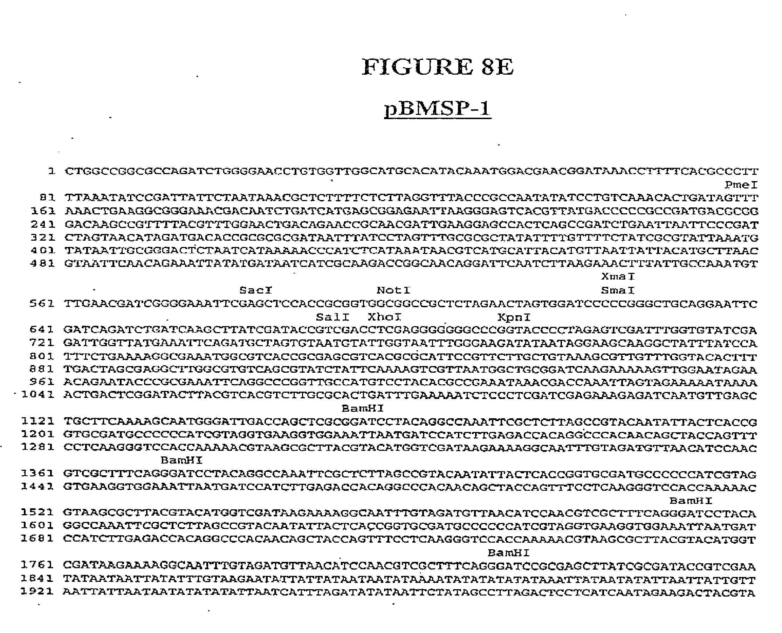

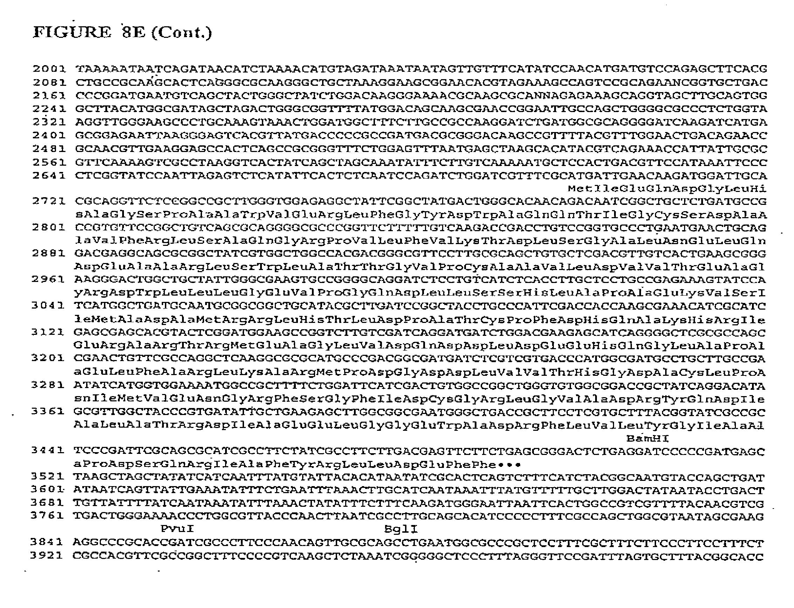

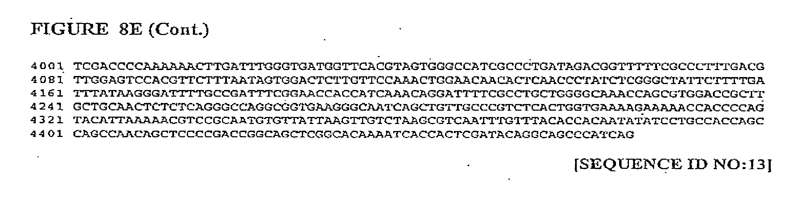

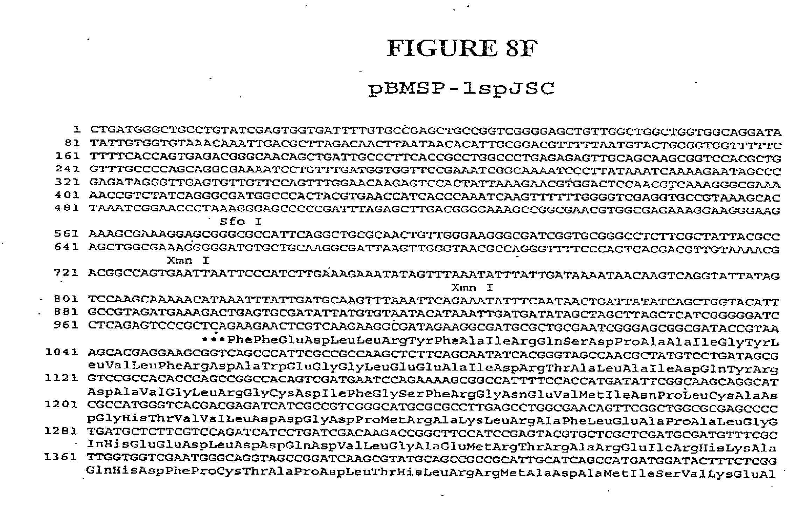

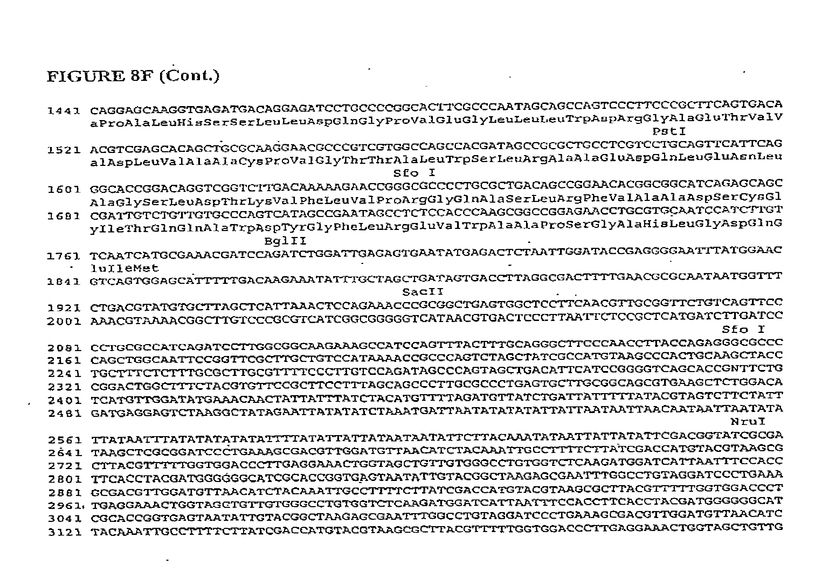

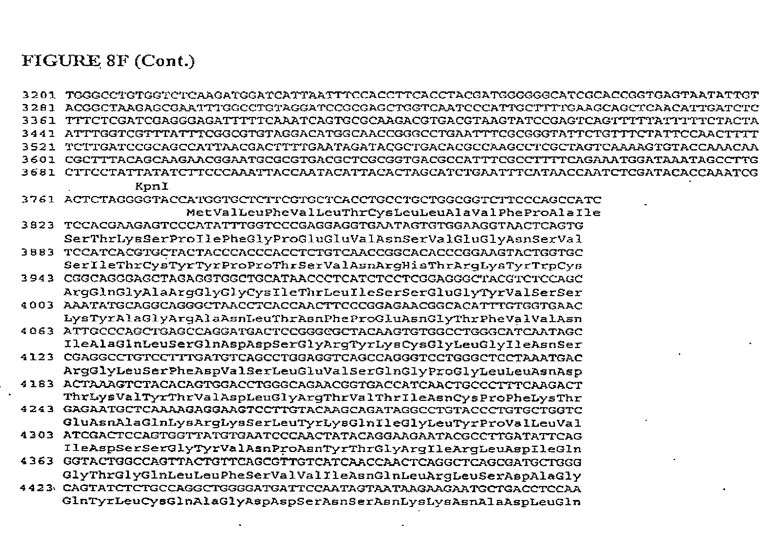

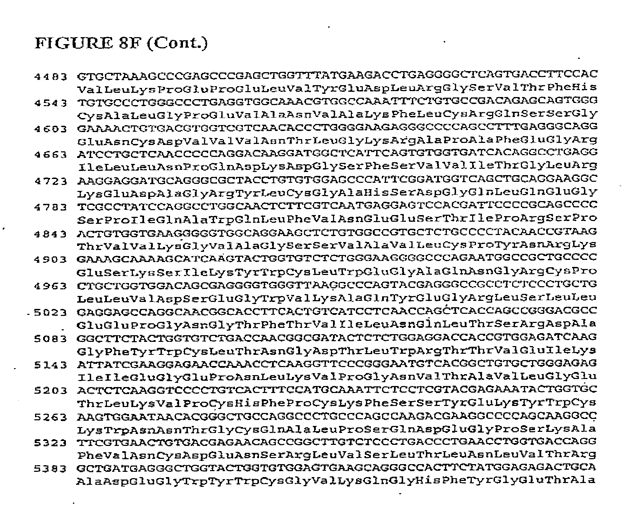

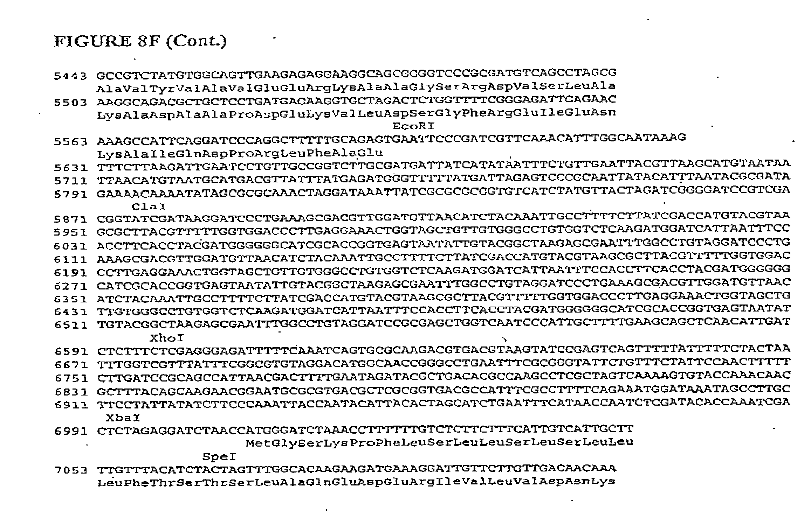

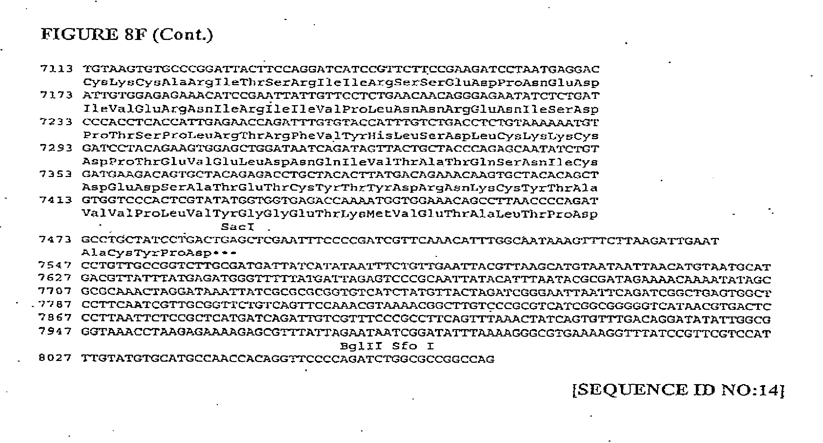

Constructs for the expression in plants of human J chain and secretory component, as well as a human IgA2 heavy chain, were developed. A heavy chain expression cassette vector was made and called pSSpHuA2 (See FIG. 1). It contains sequence encoding a bean legumin signal peptide (Baumlein et al., Nucleic Acids Res. 14 (6), 2707-2720, 1986). The sequence of bean legumin is provided as GenBank Accession No. X03677, and the sequence of the bean legumin signal peptide is SEQ ID NO: 10 (also see Fig. 8) and the IgA2m(2) constant region with SpeI and SacI sites in between, and the SuperMas promoter for driving the expression of a signal peptide and the constant regions of the human IgA2m(2) heavy-chain.

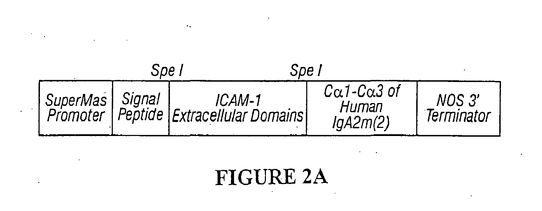

The amplified DNAs encoding the first five domains of human ICAM-1, and the Fc region of human IgA2m(2) were fused in a plant-expression cassette to make a chimeric ICAM-1 molecule expression construct, shown in FIG. 2A. This was done by cloning the fragment encoding the five extracellular domains of ICAM-1 into vector pSSPHuA2 to generate pSSPICAMHuA2. The convenient restriction sites (5' SpeI and 3' Spe I) allowed the amplified fragment to be inserted between the signal peptide and the Cα1 domain. In the resulting construct, expression of the chimeric ICAM-1 molecule is under the control of the constitutive promoter "superMAS" (Ni et. al., 1995) and the nos 3' terminator region.

The resulting chimeric ICAM-1 molecule construct contains no variable region. Upon translation of the mRNA, signal peptide cleavage is predicted to deposit the ICAM-1-heavy chain fusion into the plant cell's endoplasmic reticulum (ER). DNA encoding an ER retention signal (RSEKDEL, SEQ ID NO: 5) was appended to the 3' end of the heavy-chain in order to boost the expression level of the construct. The amino acid sequence SEKDEL (SEQ ID NO: 4) is the consensus signal sequence for retention of proteins in the endoplasmic reticulum in plant cells. This sequence has been shown to enhance accumulation levels of antibodies in plants (Schouten et al., Plant Molecular Biology 30:781-793,1996). The amino acid sequence of the chimeric ICAM-1 molecule construct is shown in FIG. 2B. The DNA sequence and translational frame of the construct was verified before it was used for particle bombardment.

It has been shown recently that assembly of J chain with IgA takes place in the Golgi apparatus (Yoo et al., J. Biol. Chem. 274:33771-33777, 1999), and so constructions of heavy chain without SEKDEL (SEQ ID NO: 4) have been made as well. The ICAM-1 fragment was cloned into an expression cassette containing the IgA2m(2) constant region without SEKDEL (SEQ ID NO: 4).

### 2. Expression of Assembled ICAM-1-Immunoadhesin in Plants

### A. Immunoadhesin Expression Vectors

The plasmid pSSPICAMHuA2 [SEQ ID NO:9 and FIG. 8A] is 6313 bp in length. Nucleotides 49-1165 represent the Superpromoter (Ni et al., Plant Journal 7:661-676, 1995). Nucleotides 1166-3662 comprise a sequence encoding a human ICAM-1/human IgA2m(2) constant hybrid with linker sequences. A consensus Kozak sequence (Kozak, Cell 44(2):283-92, 1986) is included (nt 1186-1192) to enhance translation initiation, as well as the signal peptide from V. faba legumin (nt 1189-1257; Bäumlein et al., Nucleic Acids Reg. 14(6):2707-2720 (1986). The sequence of the human IgA2m(2) constant region (nt 3663-3633) has been previously published (Chintalacharuvu, et al., J. 1mm. 152: 5299-5304, 1994). A sequence encoding the endoplasmic reticulum retention signal SEKDEL [SEQ ID NO:4] is appended to the end of the heavy Chain (nt 3634-3654). Nucleotides 3663-3933 derive from the nopaline synthase 3' end (transcription termination and polyadenlyation signal; Depicker et al., 1982). The remainder of the plasmid derives from the vector pSP72 (Promega).

The plasmid pSHuJ (FIG. 8C) is 4283 bp in length. Nucleotides 14-1136 represent the Superpromoter (Ni et al., Plant Journal 7:661-676, 1995) and nucleotides 1137-1648 are shown in FIG. 8 [SEQ ID NO:11] and comprise a sequence encoding the human J Chain including the native signal peptide (Max and Korsmeyer, J 1mm. 152:5299-5304, 1985) along with linker sequences. A consensus Kozak sequence (Kozak, Cell 44(2):283-92, 1986) is included (nt 1162-1168) to enhance translation initiation. Nucleotides 1649-1902 derive from the nopaline synthase 3' end (transcription termination and polyadenlyation signal; Depicker et al., J Mol Appl Genet 1(6):561-73, 1982). The remainder of the plasmid derives from the vector pSP72 (Promega).

The plasmid pSHuSC (FIG. 8D) is 5650 bp in length. Nucleotides 13-1136 are derived from the Superpromoter (Ni et al., Plant Journal 7:661-676, 1995), and nucleotides 1137-2981 comprise a sequence encoding the human Secretory Component including the native signal peptide (Krajci, et al., Biochem. and Biophys. Res. Comm 158:783, 1994) along with linker sequences [SEQ ID NO:12]. A consensus Kozak sequence (Kozak, Cell 44(2):283-92, 1986) is included (nt 1151-1157) to enhance translation initiation. Nucleotides 2982-3236 derive from the nopaline synthase 3' end, providing a transcription termination and polyadenlyation signal, described in Depicker et al., J Mol Appl Genet 1(6):561-73 (1982). The remainder of the plasmid derives from the vector pSP72 (Promega).

The plasmid pBMSP-1 [SEQ ID NO:13 and FIG. 8E] is derived from pGPTV-KAN. Becker et al., in Plant Molecular Biology 20, 1195-1197, (1992), describe new plant binary vectors with selectable markers located proximal to the left T-DNA border, and the sequences outside of the left and right borders. Nucleotides 18-187 of pBMSP-1 represent the right T-DNA border, and nucleotides 1811-775 represent the superMAS promoter. Nucleotides 2393-2663 represent the NOS promoter (Depicker et al., J Mol Appl Genet 1(6):561-73, 1982), nucleotides 2698-3492 encode the NPTII gene (conferring resistance to kanamycin), and nucleotides 3511-3733 are the polyadenylation signal from A. tumefaciens gene 7 (Gielen et al., Embo J 3:835-46, 1984). Nucleotides 1768-976 encode the NPTII gene, and nucleotides 4317-4464 represent the left T-DNA border.

The plasmid pBMSP-1spJSC [SEQ ID NO:14 and FIG. 8F] is a derivative of pBMSP, containing both J and SC under control of superpromoter. In this plasmid, nucleotides 1-149 represent the left T-DNA border. Nucleotides 955-733 are the polyadenylation signal from A. tumefaciens gene, nucleotides 1768-976 encode the NPTII gene (conferring resistance to kanamycin), and nucleotides 2073-1803 represent the NOS promoter. Nucleotides 2635-3768 represent the superMAS promoter, nucleotides 3774-5595 encode the Human Secretory component, and nucleotides 5603-5857 represent the NOS polyadenylation signal. Nucleotides 5880-6991 represent the superMAS promoter, nucleotides 7007-7490 encode the Human Joining Chain, and nucleotides 7504-7757 represent the NOS polyadenylation signal. Nucleotides 7886-8057 represent the right T-DNA border.

The plasmid pGPTV-HPT, encoding the enzyme conferring hygromycin resistance, is available commercially from the Max-Planck-Institut für Züchtungsforschung (Germany). It is described by Becker in Plant Molecular Biology 20, 1195-1197 (1992).

### B. Plant Transformation and ICAM-1 Immunoadhesin Expression in Plants

The expression cassettes described above were used to produce the assembled immunoadhesin in plants. Plasmids pSSPICAMHuA2, pSHuJ, pSHuSC and pBMSP-1 were co-bombarded into tobacco leaf tissue (N. tabacum cultivar Xanthi) and transformed microcalli were selected on nutrient agar in the presence of kanamycin. Individual microcalli, indicative of independent transformation events, were dissected from the parent tissue and propagated on nutrient agar with kanamycin.

The callus tissues were screened for transgene expression. Callus #7132 was shown to express a chimeric ICAM-1 immunoadhesin and J chain by immunoblotting and PCR (data not shown). This callus did not possess DNA encoding the SC. The callus grew well in culture and, upon accumulation of sufficient mass, #7132 was bombarded again, this time with two of the plasmids described above, PBMSP-1 SpJSC, containing expression cassettes for both the J chain and SC and pGPTV-HPT, containing an expression cassette for the hpt I gene which confers hygromycin resistance. After a period of selection and growth on nutrient agar, several independent transformants were identified, by immunoblotting, that expressed the chimeric ICAM-1 molecule, the J chain and SC in several states of assembly.

FIG. 3 illustrates the expression of the chimeric ICAM-1 molecule in independently transformed tobacco calli. FIG. 3A shows immunoblots of non-reducing SDS-polyacrylamide gels on which samples containing different transformed tobacco calli (C) and aqueous extracts (Aq) were run and probed for the presence of human ICAM. The solubility of the immunoadhesin assured us that extraction could be easily performed, and the similarity of signals leads us to believe in the reproducibility of expression. FIG. 3B shows immunoblots of nonreducing SDS-polyacrylamide gels containing various fractions of partially purified immunoadhesin from callus Rhi107-11. The blots were probed with antibodies against human ICAM (~ICAM), human IgA heavy chain (~α), human secretory component (~SC) and human J chain (~J). Secondary, enzyme-conjugated antibodies were employed as necessary to label immuno-positive bands with alkaline phosphatase. The specificity of immuno-blotting was further verified by a failure to detect immuno-positive bands in extracts of non-expressing calli (not shown). The expected MW for a dimerized chimeric ICAM-1 molecule, without glycosylation, is 173,318; this form is likely present in the band migrating just below the 250kD marker since it is immuno-positive for ICAM-1 and heavy-chain. This band is also immuno-positive for SC (total expected MW of ~248 kD) but not for J chain which is somewhat unexpected given the canonical pathway for SIgA assembly, which involves 2 cell types (in mammalian) and requires the presence of J chain prior to assembly of SC. A tetrameric immunoadhesin, containing a single molecule of J chain and a single molecule of SC, has an expected MW of ~440 kD, prior to glycosylation. Several species with molecular weights well in excess of 200 kD, immuno-positive with all four probes, are readily apparent.

Bombardment with DNA-coated microprojectiles is used to produce stable transformants in both plants and animals (reviewed by Sanford et al., Meth. Enz. 217:483-509,1993). Particle-mediated transformation with the vectors encoding the immunoadhesin of the present invention was performed using the PDS-1000/He particle acceleration device, manufactured by Bio-Rad. The PDS-1000/He particle acceleration device system uses Helium pressure to accelerate DNA-coated microparticles toward target cells. The physical nature of the technique makes it extremely versatile and easy to use. We have successfully transformed tobacco with all four components of a secretory IgA simultaneously.

The basic biolistic procedure was performed as follows: A stock suspension of microprojectiles was prepared by mixing 60 mg of particles in 1 ml of absolute ethanol. This suspension was vortexed and 25-50 µl was removed and added to a sterile microcentrifuge tube. After microcentrifuging for 30 seconds the ethanol was removed and the pellet resuspended in 1 ml sterile water and centrifuged for 5 minutes. The water was then removed and the pellet resuspended in 25-50 µl of DNA solution containing a mixture of plasmid DNAs, usually, but not always in equimolar amounts. The amount of plasmid added varied between 0.5 ng and 1 µg per preparation. The following were added sequentially: 220 µl of sterile water, 250 µl of 2.5M CaCl2, and 50 µl of 0.1M spermidine. This mixture was vortexed for at least 10 min and then centrifuged for 5 min. The supernatant was removed and the DNA/microprojectile precipitated in 600 µl of absolute ethanol, mixed and centrifuged 1min. The ethanol was removed and the pellet resuspended in 36 µl of ethanol. Ten µl of the suspension was applied as evenly as possible onto the center of a macrocarrier sheet made of Kapton (DuPont) and the ethanol was evaporated. The macrocarrier sheet and a rupture disk were placed in the unit. A petri dish containing surface-sterilized tobacco leaves was placed below the stopping screen. The chamber was evacuated to 28-29mm Hg and the target was bombarded once. The protocol has been optimized for tobacco, but is optimized for other plants as well by varying parameters such as He pressure, quantity of coated particles, distance between the macrocarrier and the stopping screen and flying distance from the stopping screen to the tissue.

Expression cassettes for chimeric ICAM-1 molecules were also assembled in binary vectors for use in Agrobacterium-mediated transformation. An Agrobacterium binary vector designed for expression of both human J chain and human secretory component, as well as kanamycin resistance, was introduced into A. tumefaciens strain LBA4404. The chimeric ICAM/IgA molecule in another binary vector was also used to transform LBA4404. Overnight cultures of both strains were used for simultaneous "co-cultivation" with leaf pieces of tobacco, according to a standard protocol (Horsch et al., Science 227:1229-1231, 1985).

A standard protocol for regeneration of both bombarded and Agrobacterium-transformed tobacco leaf disks was used (Horsch et al., Science 227:1229-1231, 1985). Because transformed plants, regenerated from bombarded tissue, frequently undergo gene-silencing upon maturation, transgenic tobacco plants were prepared via Agrobacterium mediated transformation, which gives a higher yield of expressing, mature plants.

### 3. Purification of Assembled ICAM-1 Immunoadhesin

The immunoadhesin expressed according to Examples 3 was purified. Calli were grown in large amounts to facilitate the development of extraction procedures. A partial purification schedule provided a stable concentrate, available in a variety of buffer conditions, for investigation of subsequent chromatographic techniques for the further purification of the immunoadhesin (See FIG. 3). Calli were extracted in a juicer, which crushes tissue between two stainless-steel gears, while bathed in a buffer containing sodium citrate (0.6 M, pH 7.4) and urea (final concentration of 2 M). The juice (~1 ml/g fresh weight) was precipitated, after coarse filtration through cheesecloth, with 0.67 volumes of saturated ammonium sulfate. A green pellet was collected after centrifugation and thoroughly extracted, in a small volume of 50 mM sodium citrate (pH 6.6), with a Dounce homogenizer. After additional centrifugation, a clear brown supernatant was collected and partially purified, during buffer exchange in a de-salting mode, by passage through a Sephadex G-100 column. The desalting/buffer exchange step has allowed preparation of a partially purified concentrate (~0.2 ml/ g fresh weight callus) in a desirable buffer; the G-100 column was eluted with 0.25 X phosphate buffered saline. This eluate appeared to be stable for at least 10 days at 2-8°C.

### 4. The ICAM-1_Immunoadhesin Inhibits Human Rhinovirus Infectivity

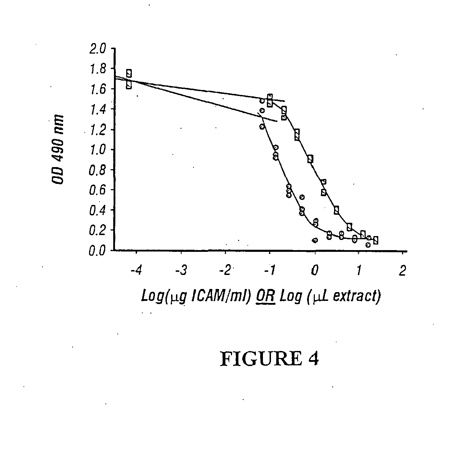

The infectivity of cells by human rhinovirus was demonstrated to be inhibited by the immunoadhesin prepared according to Example 3. Callus extract prepared according to Example 3 successfully competed for binding of an anti-ICAM monoclonal antibody to soluble ICAM-1. FIG. 4 shows the data from an enzyme-linked immunosorbent assay (ELISA). For the assay, 96-well plates were coated with 0.25 µg soluble ICAM-1/ml. The squares represent the increasing concentrations of sICAM and the circles represent the increasing amounts of callus extract (sterile filtered fraction from G-100) used to compete with the adhered ICAM for a constant amount of a mouse (anti-human ICAM) antibody. After washing the wells, adherent mouse antibody was detected with an anti-mouse antibody conjugated to horseradish peroxidase. Adherent enzyme activity was measured at 490 nm, with ortho-phenylene diamine as a substrate. The data (squares, sICAM; circles, Extract) are well described by sigmoids of the form OD490 = y =y0 + a/[1+e^-{(x-x0)/b}], where a = y max, y0 = y min, b = the slope of the rapidly changing portion of the curve and x0 = the value of x at the 50% response level. Relative to soluble ICAM-1, the immunoadhesin extract tested here contains the equivalent of ~250 µg ICAM/ml; this is an overestimate due to expected avidity effects of the dimeric and tetrameric assemblies of the ICAM-1-heavy-chain fusions. Thus, this ELISA demonstrated that the immunoadhesin competes with soluble ICAM-1 for binding to an anti-ICAM mAb.

The competitive ELISA allows for quantitative assessment of the recovery of activity by comparing the normalized amounts of various fractions required to give a 50% response. Upon purification, the titer of a immunoadhesin preparation may be expressed as a reciprocal dilution, or the number of milliliters to which a milligram of immunoadhesin must be diluted in order to give a 50 % response. This ELISA will facilitate the development of a purification process for the immunoadhesin.

A cytopathic effect assay (CPE) demonstrated the specific ability of the partially purified immunoadhesin to inhibit the infectivity of human cells by human rhinovirus (FIG. 5). Rhinovirus serotype HRV-39 was pre-incubated with human ICAM-1, an ICAM/IgA fusion (gift of Dr. Tim Springer), or with extracts from calli either expressing our ICAM-1/SIgA immunoadhesin or another, different, antibody before plating each of the mixtures with HeLa S3 cells at 33°C. After 3 days, viable cells were fixed and stained with a methanolic solution of Crystal Violet; the optical density at 570 nm provides a proportional measure of cell viability.

Two extracts derived from Rhi107-11, containing the immunoadhesin, clearly inhibited the virus' ability to infect and kill HeLa S3 cells (FIG. 5A, right side-up and upside-down triangles). Because the extracts were only partially purified, we also assayed a similarly prepared extract that contained a human IgA2m(2) directed against Doxorubicin, a chemotherapeutic agent. That extract, containing a similar immunoglobulin with an unrelated binding specificity, was unable to inhibit the infectivity of the rhinovirus and demonstrates that expression of the ICAM-1-heavy-chain fusion confers specificity to the inhibition. The CPE assay demonstrated, as expected, the differential ability of souluble ICAM-1 and an (IC1-5/IgA; Martin, et al., 1993) to inhibit viral infectivity (FIG. 5B). The insert in Figure 5B is the scale expansion in the range of the IC₅₀ for soluble ICAM-1 (1.35 µg/ml) and for the ICI-5/IgA (0.12 µg/ml; 11.3 fold less).

### 5. Production and Purification of Immunoadhesins for Clinical and Toxicological Studies

Production of sufficient immunoadhesin for the proposed clinical and toxicological needs is performed by making transgenic tobacco plants. The transgenic plants which express the immunoadhesin (without an ER retention signal) are generated by Agrobacterium-mediated transformation. The absence of an ER retention signal is anticipated to enhance assembly since the nascent SIgA is processed through the entire Golgi apparatus, including, in particular, the trans-Golgi, where SC is covalently linked to dIgA as suggested by pulse-chase experiments (Chintalacharuvu & Morrison, Immunotechnology 4:165-174, 1999). Because Agrobacterium-mediated transformation is much more likely to generate plants with consistent levels of transgene expression, it is likely that progeny of these plants will be used for the production of clinical grade immunoadhesin.

In order to maximize expression levels, and create a true-breeding line, it is desirable to create homozygous plants. The highest producing plants (generation T0) can self-fertilize in the greenhouse before seed is collected. One quarter of the T1 plants are expected to be homozygous. These are grown in the greenhouse and seed samples from several plants are separately germinated on medium containing kanamycin. All the progeny (T2) from homozygous positive plants are expected to be green. Some of the progeny of heterozygous plants are expected to be white or yellowish. Homozygosity is confirmed by back-crossing to wild-type and immunoblotting extracts of the progeny.

Harvesting and processing may be continuously meshed during a production campaign, especially since multiple harvests may be obtained from a single planting, i.e. plants cut to soil level for one harvest are regrown for subsequent harvests. In developing a sense of scale for the production of immunoadhesin it is necessary to decide on the required amount of finished immunoadhesin, account for expression levels (mg immunoadhesin present/ kg fresh weight tobacco), know the growth rate of the plants and the expected weight of the average plant, and the overall yield of the purification schedule (set at 20%). Setting the overall need at 3 g of finished immunoadhesin requires preparing for 4 harvests, each with an expected yield of 1 g of finished immunoadhesin.

Given these targets and parameters, the necessary number of plants and hence the space requirements for plant growth is determined. FIG. 6 shows an evaluation of the production necessities for making 1 gram of finished Immunoadhesin. In this diagram, the number of plants needed for 1 g of immunoadhesin, at 20% yield, at expected levels of expression and plant weight is illustrated. At different levels of immunoadhesin expression (mg/kg fresh weight) and overall recovery (set at 20%), the weight of each plant, and so the total number of plants, may be determined for a specified production target (1 g/harvest) within a window (dotted square) of reasonable possibilities. The number of required plants decreases, inversely, with the number of specified growth and re-growth periods. The expected biomass production, a function of time and growth conditions, influences the time to harvest and the time between harvests. These growth periods can be adjusted to the realities of the purification schedule by staggering planting and harvesting dates. For example, 1 g of finished immunoadhesin from plants with a reasonable expression level, of 100 mg of immunoadhesin/kg fresh weight, require 250 plants when harvested at a weight of 200 g/plant (~80 days post germination). At this scale, these plants require about 10 m² of growing space and are harvested twice over 150 days.

Processing 50+ kg of biomass at a time requires several moderately large-scale operations which all have counter-parts in the food-processing industry. These include bulk materials handling, size reduction, juicing and filtration. A Vincent Press and a Durco filtration system are used to efficiently process these quantities. The juicing step employs a proven and simple buffer of sodium citrate and urea. These components buffer the extract, help prevent the oxidation of phenolics and their association with proteins (Gegenheimer, Methods in Enzymology 182:174-193, 1990; Loomis, Methods in Enzymology, 31:528-544, 1974; Van Sumere, et al., The Chemistry and Biochemistry of Plant Proteins, 1975.) and ensure the solubility of the immunoadhesin during a subsequent acid precipitation.

Filtration of acid-insoluble lipid and protein (~90% of the total) is followed by tangential flow ultrafiltration to concentrate the immunoadhesin and to remove small proteins, especially phenolics. Diafiltration enhances the removal of small molecules and exchanges the buffer in preparation for short-term storage and subsequent chromatography. Either SP-Sepharose (binding at pH 5.0 or below) or Q-Sepharose (binding at pH 5.5 or above) are among the ion-exchanges that can be used for filtering immunoadhesin. They are readily available, scalable, robust and have high capacities. In particular, they are available for expanded-bed formats, which reduce the stringency of prior filtration steps. Cation-exchange chromatography, which can be more selective than anion-exchange chromatography, is used first. The immunoadhesin is purified from the several species of protein potentially present, to the point where at least 95% of the protein is in the form of ICAM-1/IgA, ICAM-1/dIgA or ICAM-1/SIgA, as the presence of di- and tetra-valent ICAM-1 domains are critical for potent anti-viral activity. Purified immunoadhesin is then tested for acceptable levels of endotoxin, alkaloids such as nicotine and for bio-burden. In addition, potency levels (defined by ELISA and CPE assays), protein concentration, pH and appearance are monitored. Subsequently, the stability of the clinical lots of immunoadhesin is determined, to ensure that patients receive fully potent immunoadhesin. Even partially purified extracts have been found to be stable for 10 days when refrigerated. The titer and potency of clinically formulated immunoadhesin (in phosphate-buffered saline), when stored at -20°C, 2-8°C, and at 37°C, over a period of 3 to 6 months, is also tested.

### 6. The Immunoadhesins Have Plant-Specific Glycosylation

The immunoadhesins produced are analyzed to determine the pattern of glycosylation present. Cabanes-Macheteau et al.,Glycobiology 9(4):365-372 (1999), demonstrated the presence of several glycosyl moieties, typical of plants, on a plant-expressed antibody construct. Their methods are used to demonstrate that the immunoadhesins produced according to Example 1, 2 and 3 have a plant-specific glycosylation pattern. We anticipate that this diversity will also be a source of variability for immunoadhesin. Since crude extracts have been shown to have anti-viral activity in vitro (data not shown), glycosylation, as such, does not appear to affect potency. N-linked glycosylation (FIG. 2 shows that there are fifteen potential sites on the chimeric ICAM-1 molecule alone) probably contributes to the diversity of bands seen in immuno-blots. Immunoadhesin preparations are digested with N-Glycosidase A, before blotting, showing that the difference in banding patterns collapse into fewer, discrete bands. In this way, glycoforms are initially characterized with reducing and non-reducing polyacrylamide gels. In addition, digested and mock-digested fractions are tested in the CPE assay and competition ELISA, demonstrating the effect of N-linked glycosylation on potency and titer in vitro.

### 7. The ICAM-1 Immunoadhesin Inactivates Human Rhinovirus

The immunoadhesin prepared according to Examples 1, 2 and 3 is assayed for its ability and to inactivate HRV by binding to the virus, blocking virus entry, and inducing the formation of empty virus capsids. To measure binding of the immunoadhesin to HRV, the immunoadhesin is incubated with [³H]leucine-labeled HRV-39 for 30 min and then added to HeLa cells for 1 hr. After washing, cells and bound virus are detached with Triton X-100 and [³H] measured in a scintillation counter.

Inactivation of HRV-39 by incubation with the immunoadhesin is compared with HRV inactivation by sICAM-1. HRV-39 is not directly inactivated to a significant extent (<0.5 log10 reduction in infectivity) by incubation with monomeric sICAM-1, while incubation with IC1-5D/IgA reduced infectivity approximately 1.0 log10 (Arruda, et al., Antimicrob. Agents Chemother. 36:1186-1191, 1992; Crump, et al., Antimicrob. Agents Chemother. 38:1425-7, 1994). In order to test the ability of the immunoadhesin to inactivate HRV-39, 106 50% tissue culture infective doses (TCID₅₀) of HRV-39 are incubated in medium containing a concentration of sICAM-1 or immunoadhesin equal to ten times the IC50 of each molecule for that virus, or in plain medium, for 1 hr at 33°C on a rocker platform. Each virus-immunoadhesin or virus-medium mixture are then diluted serially in ten-fold dilutions, and the titer determined on HeLa cells in 96-well plates.

The effect of the immunoadhesin on HRV attachment to host cells is tested by inoculating HeLa cells with HRV-39 at a MOI of 0.3 in the presence or absence of the immunoadhesin. Absorbance proceeds for one hour at 4°C, the cells are washed, and media is replaced plus or minus the immunoadhesin. Cells are incubated for ten hours at 33°C (to allow one round of replication), and virus are harvested by freeze/thawing the cells. The virus is titered on HeLa cells.

ICAM-IgA (ICl-5D/IgA) is more efficient than Sicam-1 at inducing conformational changes in HRV, leading to the formation of empty, non-infectious viral particles (Martin, et al. J. Virol. 67:3561-8, 1993). To examine the ability of the immunoadhesin produced according to Examples 1, 2 and 3 to induce conformational changes in HRV, causing release of viral RNA, purified immunoadhesin is incubated with [³H]leucine-labeled HRV-39 for 30 min and then the virus is overlayed onto a 5 to 30% sucrose gradient. Following centrifugation for 90 min at 40,000 rpm, fractions are collected, [³H] measured, and fractions assessed for infectivity. (Intact HRV sediments at 149S on a sucrose gradient while empty capsids lacking RNA sediments at 75S (Martin, et al. J. Virol. 67:3561-8, 1993)). Due to its increased valence, we expect the ICAM/SIgA immunoadhesin is more efficient at inducing empty non-infectious particles than ICAM-IgA.

The inhibitory effect of purified immunoadhesin on a panel of both major and minor (that do not use ICAM-1 as a receptor) HRV serotypes will be examined using the CPE assay. The ability of ICAM-1 to inhibit HRV infection varies among viral isolates. It has been shown (Crump, et al., Antimicrob. Agents Chemother. 38:1425-7, 1994) that the EC₅₀ for sICAM-1 varies from 0.6 µg/ml to >32 µg/ml when tested on a panel of HRV major receptor serotypes assay using HeLa cells. Our panel includes nine major serotypes (HRV-3, -13, -14, -16, -23, -39, -68, -73, and -80) and the minor receptor serotype HRV-1A.

### 8. Clinical Studies Demonstrating the Ability of the ICAM-1 Immunoadhesin to Reduce Infectivity in Humans: Dose Escalation Tolerance Study

The immunoadhesin of the present invention is tested in two randomized controlled trials to determine the effect of intranasal administration of the immunoadhesin on infection, IL-8 response, and illness in experimental rhinovirus colds. These two studies evaluate the immunoadhesin taken by subjects before or after rhinovirus inoculation. The clinical protocols used here are based on protocols previously used by in evaluation of a recombinant soluble ICAM-1 molecule for efficacy against rhinovirus infection (Turner, et al., JAMA 281:1797-804, 1999).

### A. Subjects

Subjects are recruited from university communities at the University of Virginia, Charlottesville. Subjects are required to be in good health, non-smokers, and between the ages of 18 and 60 years. Subjects are excluded if they have a history of allergic disease or nonallergic rhinitis, abnormal nasal anatomy or mucosa, or a respiratory tract infection in the previous 2 weeks. Pregnant or lactating women or women not taking medically approved birth control are also excluded. In the experimental virus challenge study (Phase I/II, see below), subjects are required to be susceptible to the study virus as evidenced by a serum neutralizing antibody titer of 1:4 or less to the virus, determined within 90 days of the start of the trial.

### B. Study Medication

The immunoadhesin of the present invention is formulated as a phosphate-buffered saline (PBS) spray solution containing 2.6 mg/ml. The placebo consists of PBS and is identical in appearance to the active preparation. The solutions are administered using a medication bottle equipped with a metered nasal spray pump. The pump delivers 70 µl of solution containing 183 µg of the immunoadhesin with each spray. The medication is administered as two sprays per nostril, six times daily (at 3-hour intervals) for a total daily dose of 4.4 mg. This is the same dose, in mg protein/day, as was used for soluble ICAM-1 in the tremacamra study infection (Turner, et al., JAMA 281:1797-804, 1999). A mole of the immunoadhesin has about twice the mass as a mole of sICAM-1. However, given the differences in in vitro activity between sICAM-1 and ICAM/IgA fusions, the immunoadhesin is many fold more effective on a molar basis than sICAM-1. Thus, this amount is a conservative calculation of what is necessary. This amount is used, except in the event that the dose escalation study reveals problems at this dose.

### C. Study Design

Single ascending dose and multiple ascending dose studies are used to evaluate the safety of the immunoadhesin. In each case, three subjects are evaluated at each dosage level, two receiving the immunoadhesin and one receiving placebo. In the single ascending dose study, four dosage levels are evaluated. The lowest individual dose is half the anticipated dose to be used in the challenge study, and the highest individual dose is twice the anticipated challenge study dose. The dosage levels are as follows: one spray in each nostril (366 µg total), two sprays in each nostril (732 µg total), three sprays in each nostril (1098 µg total), four sprays in each nostril (1464 µg total).

The same dosage levels are used in the multiple ascending dose study. Subjects receive doses every three hours (six times per day) for five days. In both studies subjects are evaluated at each dosage level, staggering the start of each subsequent level until it is clear that there is no acute toxicity at the previous level. All subjects return for a single dose 21 days after the first dose, and then for a follow-up at six weeks (for determination of serum antibody against the immunoadhesin).

A separate group of twelve subjects is given one dose of two sprays in each nostril (732 µg total), and nasal lavage is done at 1, 2, 4, 8 and 16 hours (two subjects at each time point). Washings are assayed at Panorama Research by ELISA for the immunoadhesin in order to calculate its in vivo half-life. The total amount of the immunoadhesin to be used in the dose escalation and half-life determination studies (on a total of 28 subjects) will be approximately 270 mg.

### D. Safety Evaluations

In addition to routine adverse event recording, the safety of the immunoadhesin is assessed in three ways. First, prior to the first dose and after the last dose the investigators perform a visual examination of the nasal mucosa, in particular looking for signs of irritation or inflammation. Any visible changes are noted. Second, standard blood safety evaluations are done on samples collected prior to treatment and after the last dose on study days 1, 4, and 8 (and 21 in the multiple ascending dose study). Third, serum samples are saved, frozen, and used to determine if the immunoadhesin is able to pass through the nasal mucosa into the blood. This is accomplished in two ways. First, the presence the immunoadhesin in serum samples is measured by ELISA. In this assay, anti-human IgA antibodies adsorbed to microtiter plates capture any the immunoadhesin in the serum, which are detected by an anti-ICAM antibody. The sensitivity of the assay is determined using normal human serum samples spiked with known concentrations of the immunoadhesin. Alternatively, anti-ICAM antibodies can be adsorbed to plates to capture the immunoadhesin in the serum, that would be detected by anti-IgA. Second, the presence of an immune response to the immunoadhesin is assayed with an ELISA method that uses the immunoadhesin adsorbed to microtiter plates. Any anti-immunoadhesin antibodies in the serum bind, and are detected with anti-human IgG or anti-human IgM. Pre-treatment and post-treatment serum samples are compared, and any change in titer is considered evidence of uptake of the immunoadhesin. If there is any positive evidence of anti-immunoadhesin antibodies, additional assays will be done to distinguish between anti-ICAM-1 and anti-IgA activity.

Patients are screened for the development of an allergic reaction to the immunoadhesin. (In previous studies, there were no episodes of adverse reactions with soluble ICAM applied topically in the nose or plantibodies applied topically in the oral cavity.) Individuals exhibiting symptoms of nasal allergy are tested for anti-immunoadhesin-specific IgE antibodies in nasal lavage fluids using a sensitive two-step ELISA (R & D Systems).

### E. Statistical Analysis.

The sample size for these studies is based on previous studies using the rhinovirus challenge model. The sample size planned for the protection studies should be adequate to detect a reduction in the incidence of clinical colds from 75% in the placebo groups to 25% in the active treatment groups at 1-sided levels of α=.05 and 1-ß =.80. In addition, the sample size should be adequate to detect a change in the total symptom score of 5 units assuming an SD of 5.8 units.

### 9. Clinical Studies Demonstrating the Ability of the Immunoadhesin to Reduce Infectivity in Humans: Challenge Studies

Challenge studies are used to demonstrate that treatment with the immunoadhesin of the present invention protect against clinical colds or reduce cold symptoms after viral challenge.

### A. Challenge Virus.

The challenge virus used for this study is rhinovirus 39 (HRV-39). Rhinovirus type 39 is a major group of rhinovirus that requires ICAM-1 for attachment to cells. The challenge virus pool is safety-tested according to consensus guidelines (Gwaltney, et al., Prog. Med. Virol. 39:256-263, 1992). All subjects are inoculated with approximately 200 median tissue culture infective dose (TCID₅₀). The virus are administered as drops in two inocula of 250 µl per nostril given approximately 15 minutes apart while the subjects are supine.

**TABLE 4**

| | | | | Pre-inoculation stugy timetable | | | | | |
|---|---|---|---|---|---|---|---|---|---|
| | | | | Day | | | | | |
| | 0 | 1 | 2 | 3 | 4 | 5 | 6 | 7 - 14 | 21 |
| Medications | | 6 doses | 6 doses | 6 doses | 6 doses | 6 doses | | | |
| Inoculation | | hour 4 | | | | | | | |
| Symptom scores | | m/e | m/e | m/e | m/e | m/e | m/e | e | |
| Nasal lavage | | m | m | m | m | m | m | | |
| Serum sample | X | | | | | | | | X |
| | | | | | | | | | |
| | | | | | | | | | |

| | | | | Post-inoculafion study timetable | | | | | |
|---|---|---|---|---|---|---|---|---|---|
| | | | | Day | | | | | |
| | 0 | 1 | 2 | 3 | 4 | 5 | 6 | 7-14 | 21 |
| Medications | | 6 doses | 6 doses | 6 doses | 6 doses | 6 doses | | | |
| Inoculation | hour 0 | | | | | | | | |
| Symptom scores | | m/e | m/e | m/e | m/e | m/e | m/e | e | |
| Nasal lavage | | m | m | m | m | m | m | | |
| Serum sample | X | | | | | | | | X |
| Note: In both studies on days 1-5, doses are given at hours 0, 3, 6, 9, 12, and 15 | | | | | | | | | |
| m = morning | | | | | | | | | |
| e = evening | | | | | | | | | |

### B. Study Design

Two randomized rhinovirus challenge studies are performed (see Table 4). The same formulation of the immunoadhesin of the present invention is evaluated in pre-inoculation and post-inoculation studies. In both studies, medication is administered as six doses each day for five days. Subjects are randomly assigned to receive either the immunoadhesin or matching placebo at the time of enrollment into each study. The study is blinded and all clinical trial personnel, subjects, and employees of Panorama Research remain blinded until all data are collected.

In the pre-inoculation study, medications are started four hours (two doses) prior to viral challenge. The virus challenge is administered one hour after the second dose of the immunoadhesin (or placebo) and the four remaining doses of study medication for the first day are given as scheduled. In this study eighteen subjects receive the active treatment and eighteen subjects receive placebo.

In the post-inoculation study, medications begin 24 hours after virus challenge. This timepoint was chosen because it has been used in other studies of protection from virus challenge, and because cold symptoms are clearly present (Harris & Gwaltney, Clin. Infect. Dis. 23:1287-90, 1996). Virus challenge in this study is administered in the morning of study day 0 approximately 24 hours prior to the first dose of study medication on the morning of study day 1. In this study, 36 subjects receive the active treatment and 18 subjects receive placebo.

Subjects are isolated in individual hotel rooms from study day 0 (the day of virus challenge) to study day 6. On each of these days a symptom score and a nasal lavage for virus isolation are done in the morning prior to the first dose of medication and a second symptom score is done each evening. On study day 6, subjects are released from isolation but continue to record symptom scores each evening through day 14. The subjects return to the study site on study day 21, when a final serum sample for detection of anti-immunoadhesin antibodies will be collected. The total amount of immunoadhesin to be used in the two virus challenge studies (on a total of 54 subjects) is approximately 1200 mg.

### C. Viral Isolation

Virus shedding is detected by virus isolation in cell culture. Nasal wash specimens are collected by instillation of 5 ml of 0.9% saline into each nostril. This wash is then expelled into a plastic cup and kept chilled for one to two hours until it is processed for viral cultures. Immunoadhesin is removed from the specimens by treatment with anti-ICAM-1 antibody adsorbed to an agarose support (Affi-Gel 10, Bio-Rad Laboratories, Hercules, CA). A portion of each processed specimen is stored at -80°C, and another portion is inoculated into two tubes of HeLa-1 cells, a HeLa cell line enriched for the production of ICAM-1 Arruda, et al., J. Clin. Microb. 34:1277-1279, 1996). Rhinovirus are identified by the development of typical cytopathic effect. Subjects with a positive viral culture on any of the postchallenge study days are considered infected. Viral titers in the specimens stored at -80°C are determined by culturing serial ten-fold dilutions in microtiter plates of HeLa-1 cells.

Antibody to the challenge virus are detected by serum neutralizing titers done using standard methods Gwaltney, et al, Diagnostic Procedures for Viral Rickettsial and Chlamydial Infections, p. 579-614, American Public Health Association). Serum specimens for antibody testing are collected during screening, immediately prior to virus challenge (acute), and again 21 days later (convalescent). Subjects with at least a fourfold rise in antibody titer to the challenge virus when the convalescent serum sample is compared with the acute serum sample are considered infected.

### D. Evaluation of Illness Severity

Illness severity is assessed as previously described (Turner, et al., JAMA 281:1797-804, 1999). Symptom scores are recorded prior to virus challenge (baseline) and twice each day at approximately twelve-hour intervals for the next 6 days. On study days 7 through 14 each subject records his/her symptom score once per day in the evening. At each evaluation, subjects are asked to judge the maximum severity of the following eight symptoms in the interval since the last symptom evaluation: sneezing, rhinorrhea, nasal obstruction, sore throat, cough, headache, malaise, and chilliness. Each symptom is assigned a severity score of 0 to 3 corresponding to a report of symptom severity of absent, mild, moderate, or severe. If symptoms are present at baseline, the baseline symptom score will be subtracted from the reported symptom score. The higher of the two daily evaluations are taken as the daily symptom score for each symptom. The daily symptom scores for the eight individual symptoms are summed to yield the total daily symptom score. The total daily symptom scores for the first 5 days after virus challenge (study days 1-5) are summed and on the evening of study day 5, all subjects are asked, "Do you feel you have had a cold?" Subjects who had a total symptom score of at least 6 and either at least three days of rhinorrhea or the subjective impression that they had a cold are defined as having a clinical cold.

The weight of expelled nasal secretions is determined on days 1-7 by providing all subjects with packets of preweighed nasal tissues. After the tissues are used they are stored in an airtight plastic bag. Each morning the used tissues, together with any unused tissues from the original packet, are collected and weighed.

### E. IL-8 Assay.

Recent studies have suggested that the host inflammatory response, particularly interleukin 8 (IL-8), may play a role in the pathogenesis of common cold symptoms due to rhinovirus infection. Concentrations of IL-8 in nasal lavage are determined with a commercially available ELISA (R&D Systems, Minneapolis, Minn) as previously described (Turner, et al., JAMA 281:1797-804, 1999).

### F. Safety Evaluations

The same evaluations are done in the challenge study as in the dose escalation study described in Example 8.

### G. Statistical Analysis

Statistical analysis is performed similarly as to that described for the dose escalation study described in Example 8.

The foregoing examples and discussion, while predominantly addressed to ICAM-1 immunoadhesins, can be readily adapted by one of skill to achieve and implement the use of other types of immunoadhesins active against other types or subtypes of virus and bacterial pathogens. The following examples illustrate anti-bacterial immunoadhesin embodiments making use of the anthrax toxin receptor (ATR) as receptor protein.

### 10. Construction of ATR Immunoadhesin Expression Cassettes

A cassette encoding a portion of the extracellular domains of human anthrax toxin receptor (ATR) is prepared by PCR cloning. Specifically, a fragment of 523 bp, encoding amino acids 44-216 (the so-called von Willebrand factor type A domain) is amplified from plasmid ATR (Bradley et al., 2001), or from plasmid TEM8 (St Croix et al., 2000) using the following oligonucleotide primers:
5'-GACCTGTACTTCATTTTGGACAAATCAGG-3'
   (SEQ ID NO: 91)
5'-GAGCTCAAAATTGAGTGGATGATGCCTTGCAGAG-3'
   (SEQ ID NO: 92)

The second primer (SEQ ID NO: 92) is designed to introduce a Sac I site at the 3' end of the coding region of the ATR extracellular domain (solid underline). PCR is performed with Pfu polymerase (Stratagene) to reduce accumulation of errors. A second fragment of 124 bp, which includes a 5' untranslated region and a plant signal peptide, is amplified from plasmid δATG-TOPO#4 (which is a PCR clone of a plant-optimized 5' untranslated region and signal peptide in the Invitrogen cloning vector pCR4-TOPO), using the following oligonucleotide primers:
5'-GGTACCACTTCTCTCAATCCAACTTTC-3'
   (SEQ ID NO: 93)
5'-GTCCAAAATGAAGTACAGGTCAGCCAAACTAGTAGAGGTGAACAAAAGC-3'
   (SEQ ID NO: 94)

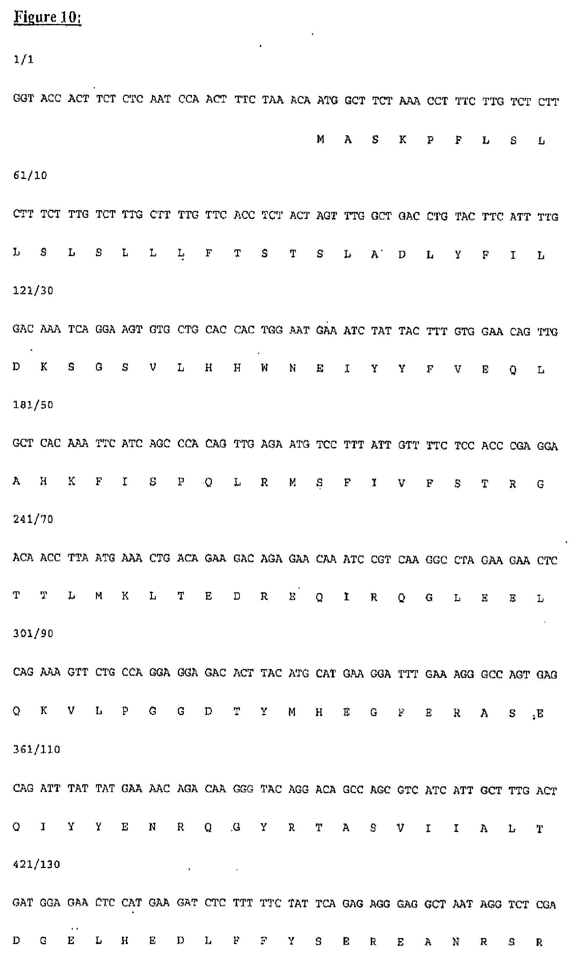

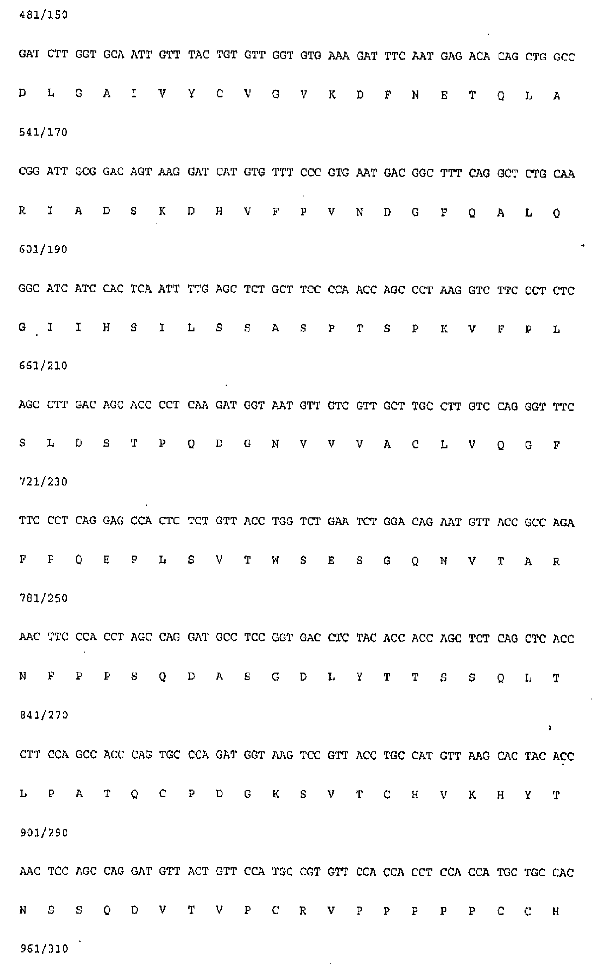

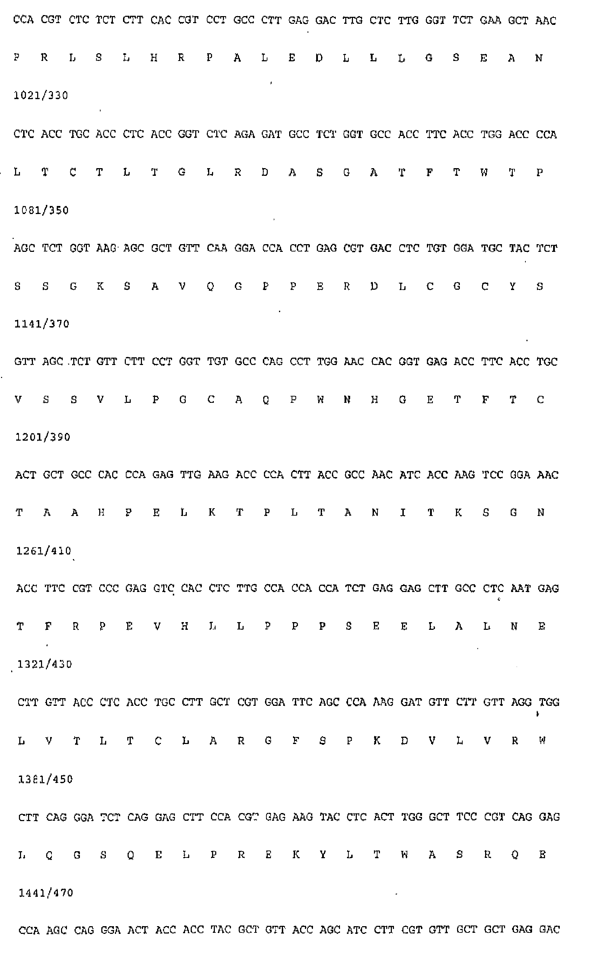

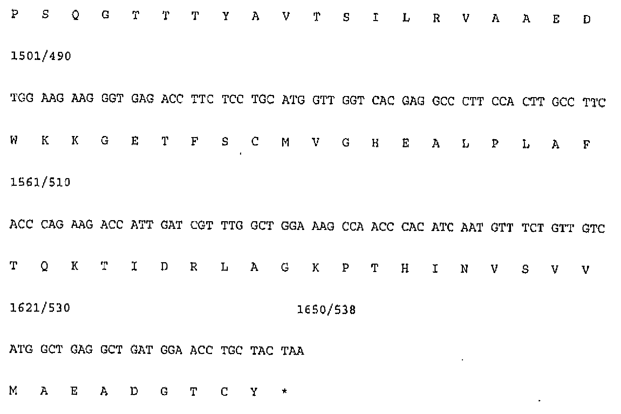

The first primer (SEQ ID NO: 93) is designed to introduce a Kpn I site at the 5' end of the PCR fragment (solid underline). The two PCR fragments have 20 nt of complementary sequence (dotted underlines). The two PCR fragments are mixed together, and a fragment of 626 bp is amplified using SEQ ID NO: 93 and SEQ ID NO: 92. The resulting PCR fragment is cloned into the vector PCRScript (Stratagene), and sequenced before cloning between Kpn I and Sac I sites in the vector pMSP-coICAM, resulting in plasmid pMSP-ATR-IgA2. This results in a genetic fusion of the extracellular domain of ATR and the constant region of human IgA2. This human IgA2 constant region has been synthesized to use codons optimal for expression in tobacco cells. The full nucleotide and amino acid sequence of the ATR-IgA2 fusion (the immunoadhesin) is shown in Figure 10. In the resulting construct, expression of the chimeric ATR-IgA2 molecule is under the control of the constitutive promoter "superMAS" (Ni et al., 1995) and the ags 3' terminator region.

The entire expression cassette (promoter + ATR-IgA2 + terminator) is removed from pMSP-ATR-IgA2 with the restriction enzyme *Asc* I, and cloned into the binary *Agrobacterium* Ti plasmid vector pGPTV-kan-ocs, resulting in plasmid pGPTV-kan-ocs-ATR-IgA2. The vector pGPTV-kan-ocs is derived from pGPTV-kan (Becker et al., 1992), which was modified in the following manner. The sequence between the Eco RI and Hind III sites of pGPTV-kan, including the entire *uid* A gene, was removed and replaced with the *ocs* 3' terminator region (MacDonald et al., 1991) oriented toward the npt II gene, plus the restriction sites for *Asc* I and *Sac I.* The purpose of this terminator adjacent to the right border of the T-DNA is to eliminate transcriptional interference with the transgene due to transcription originating in the plant DNA outside of the right border (Ingelbrecht et al., 1991).

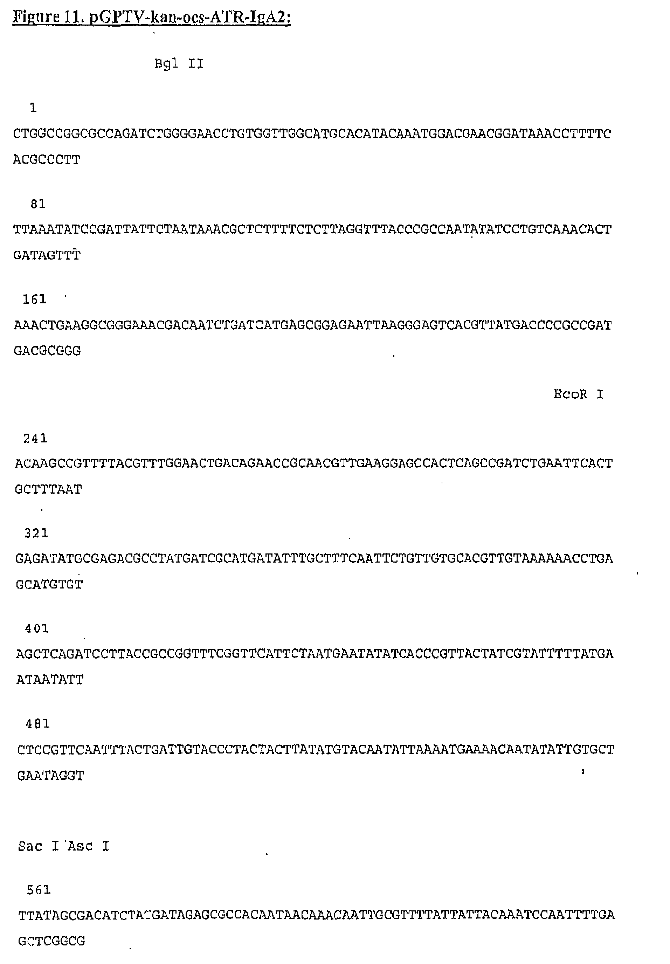

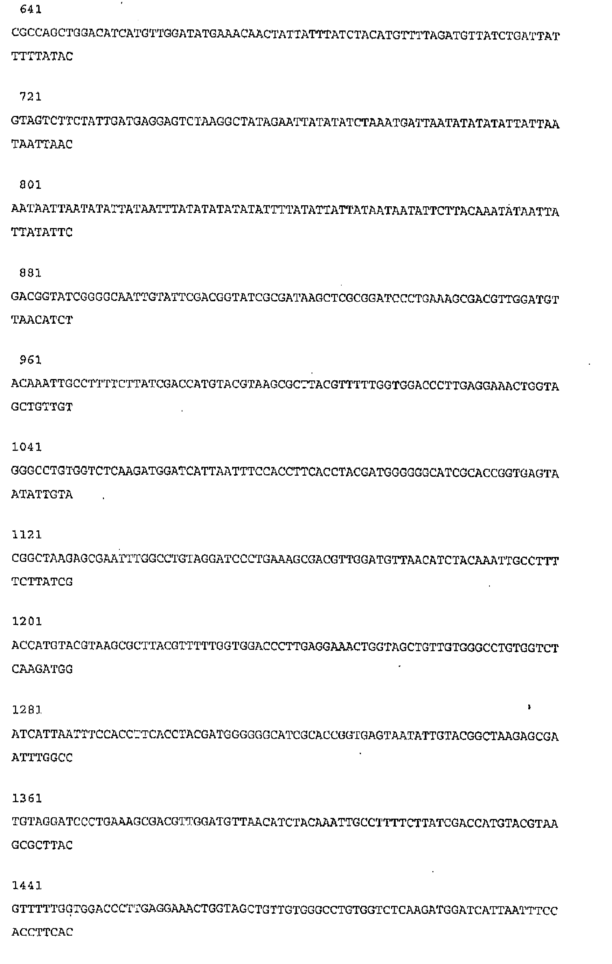

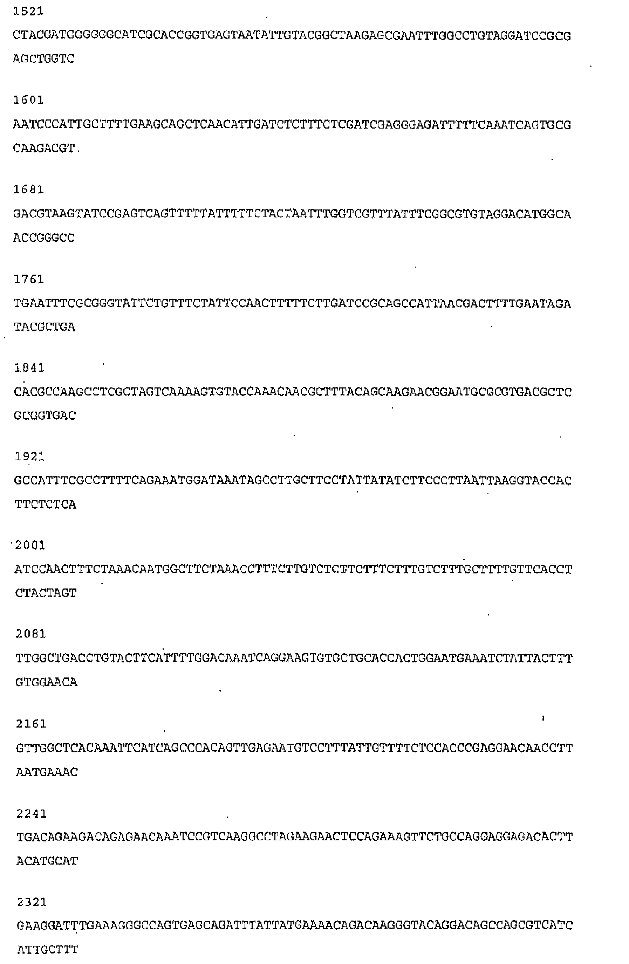

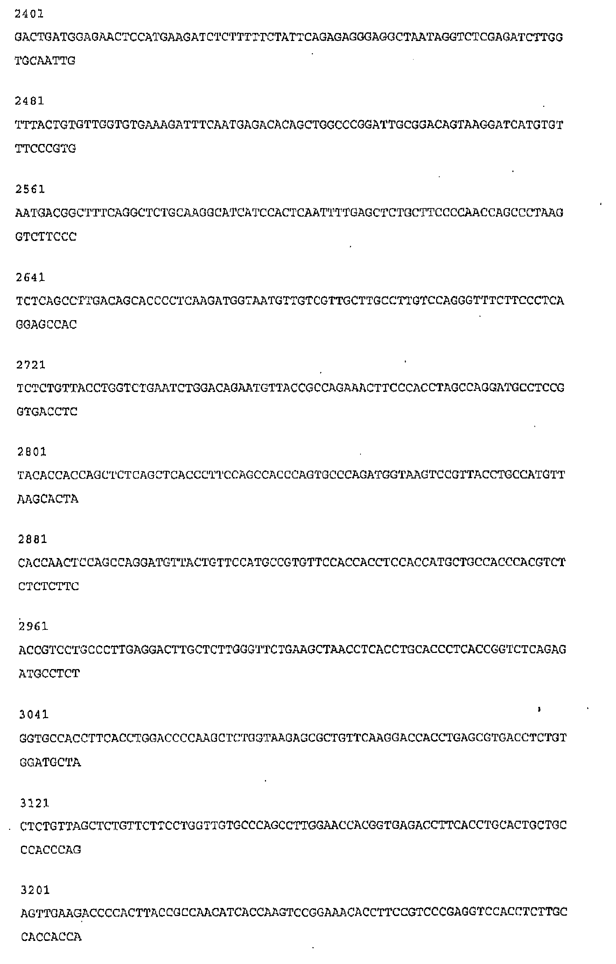

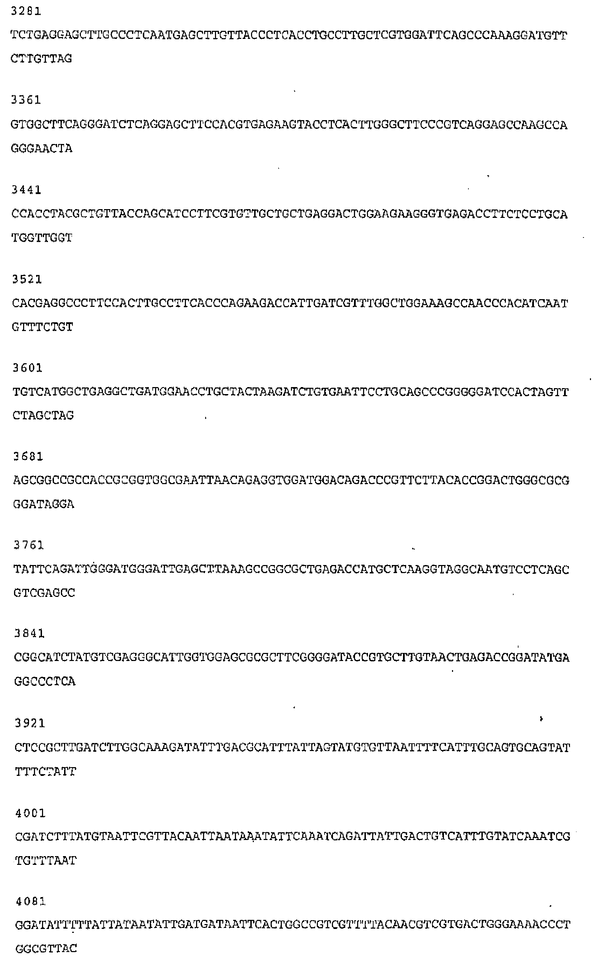

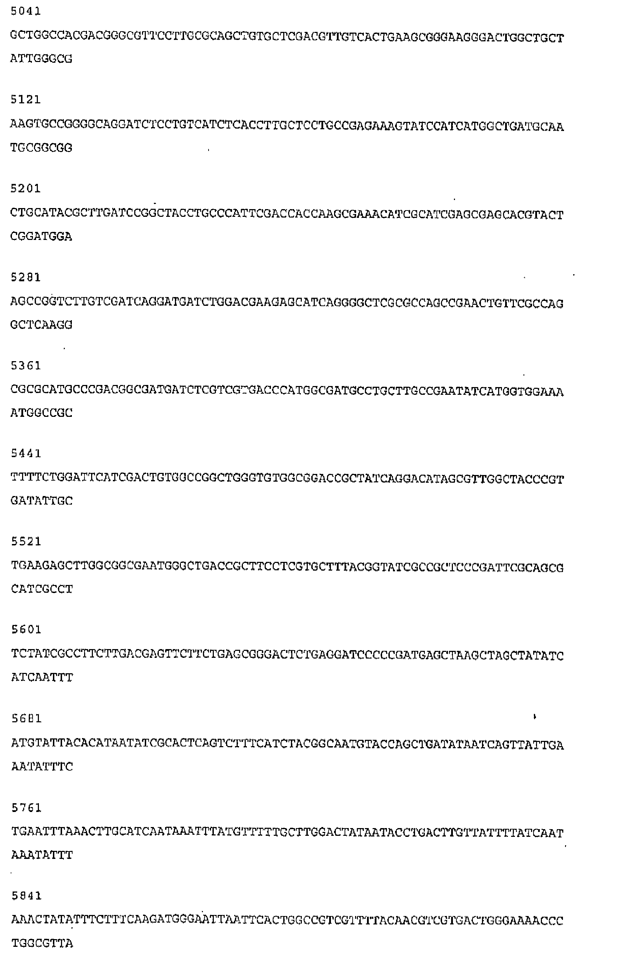

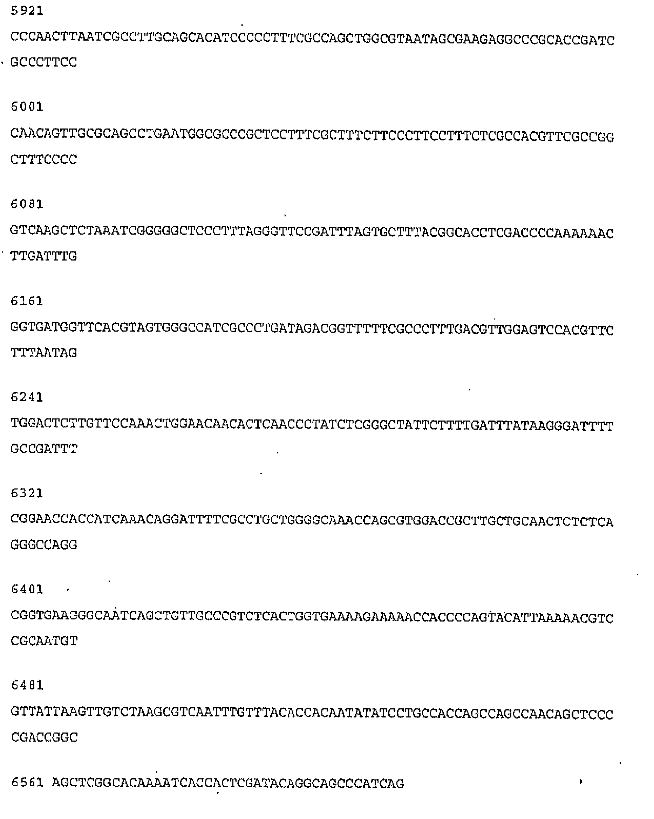

Sequence between the T-DNA borders of the plasmid pGPTV-kan-ocs-ATR-IgA2 is shown in Figure 11. Sequence outside the left and right borders are as described (Becker et al., 1992). Nucleotides 18-187 represent the right T-DNA border. Nucleotides 311-630 represent the ocs 3' terminator region. Nucleotides 927-1976 represent the superMAS promoter. Nucleotides 1990-2017 represent a 5' untranslated region from the *Nicotiana sylvestris psa*Db gene (Yamamoto et al., 1995). The context around the initiation ATG (nucleotides 2012-2026) was designed to match that found in highly expressed plant genes (Sawant et al., 1999). Nucleotides 2018-2086 comprise a sequence encoding a modified version of the signal peptide of *Vicia faba* legumin (Bäumlein et al., 1986). Nucleotides 2087-2605 comprise a sequence encoding the von Willebrand factor type A domain of ATR (Bradley et al., 2001). Nucleotides 2606-3631 comprise a sequence encoding the human IgA2m(2) constant region (Chintalacharuvu et al., 1994). Nucleotides 3794-4108 derive from the agropine synthase *(ags)* terminator. Nucleotides 4530-4800 represent the NOS promoter (Depicker et al., 1982). Nucleotides 4835-5626 encode the *npt* II gene (conferring resistance to kanamycin). Nucleotides 5648-5870 are the polyadenylation signal from *A. tumefactions* gene 7 (Gielen et al., 1984). Nucleotides 6454-6602 represent the left T-DNA border.

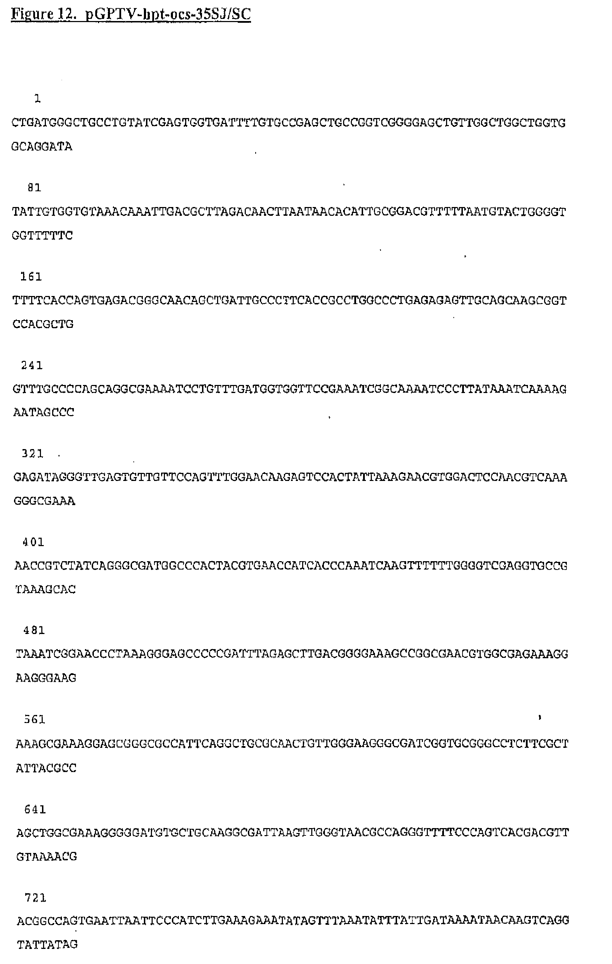

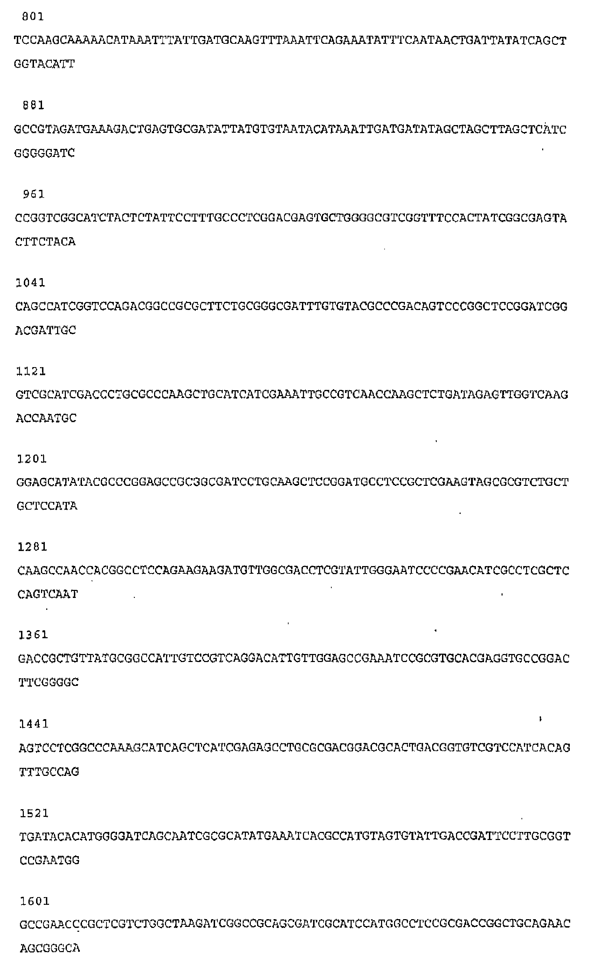

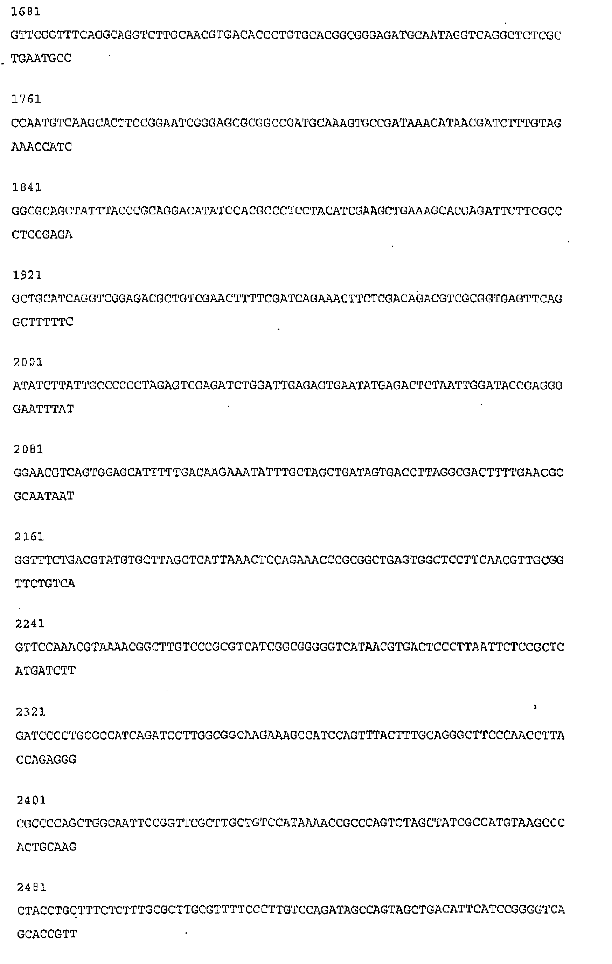

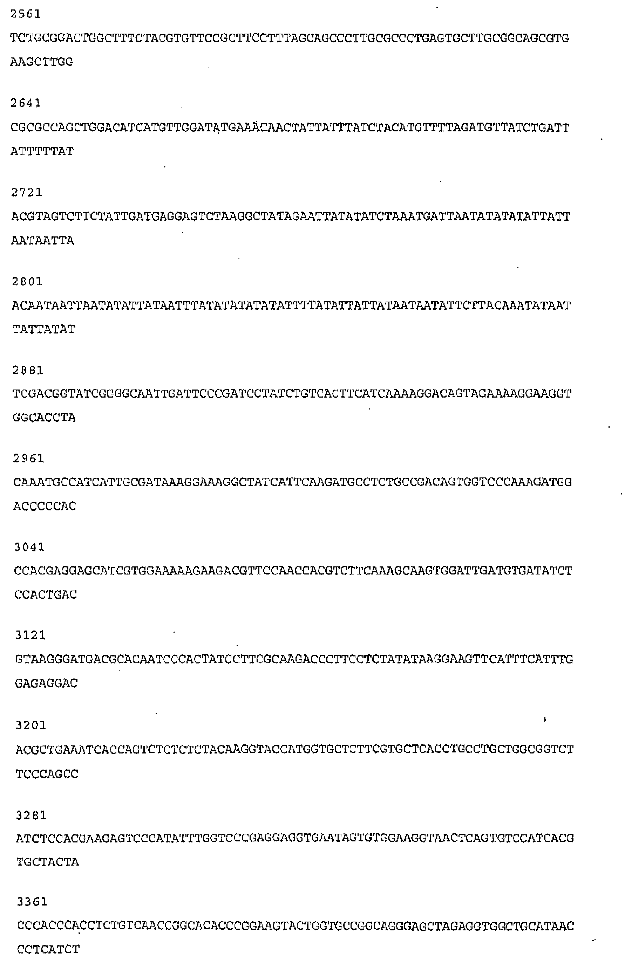

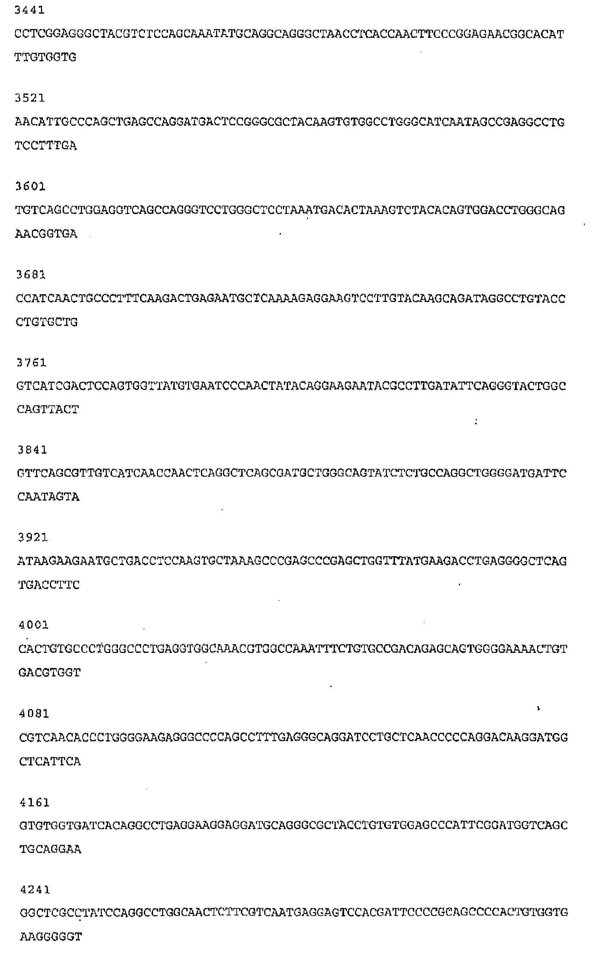

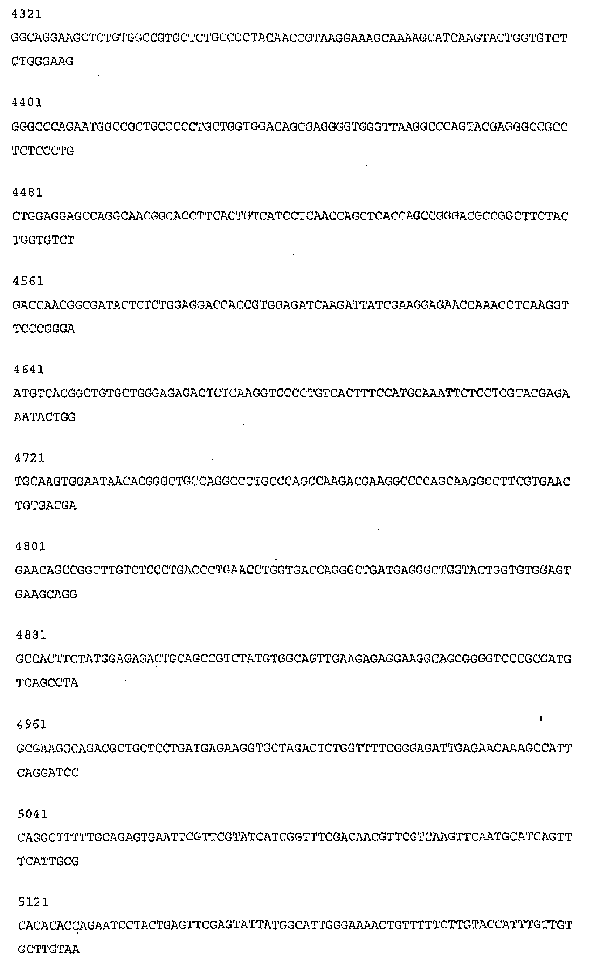

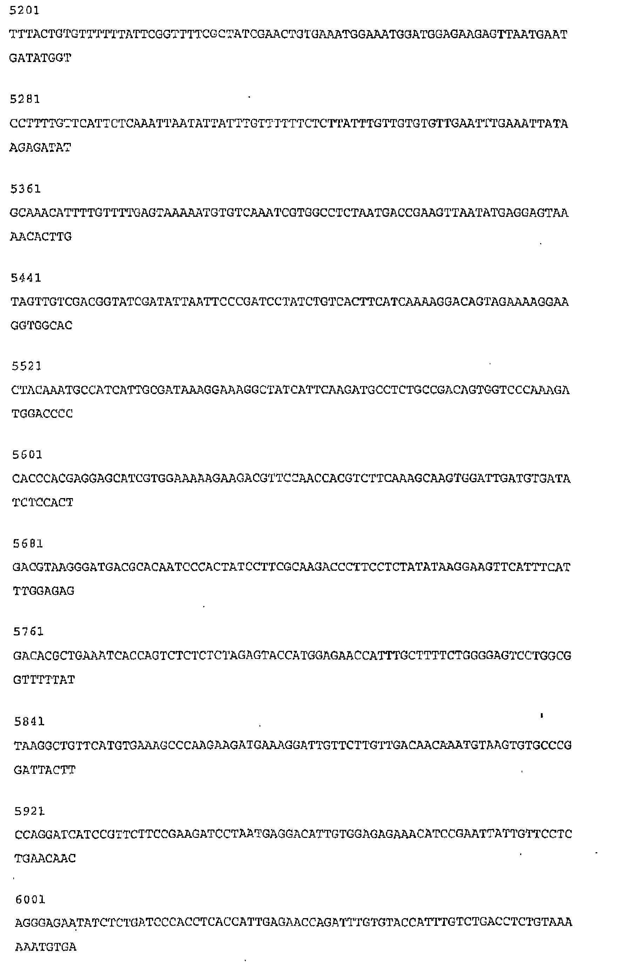

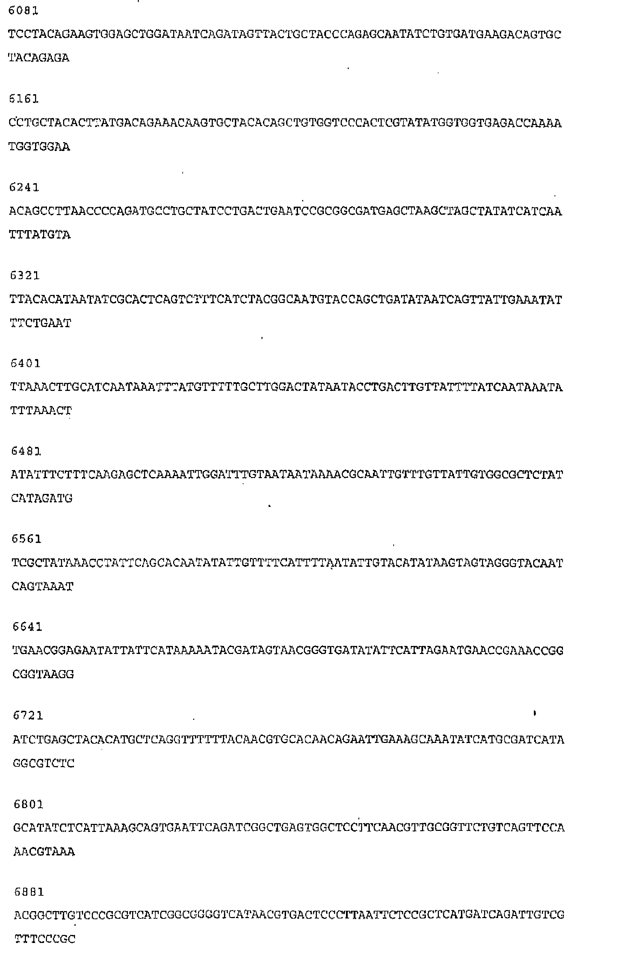

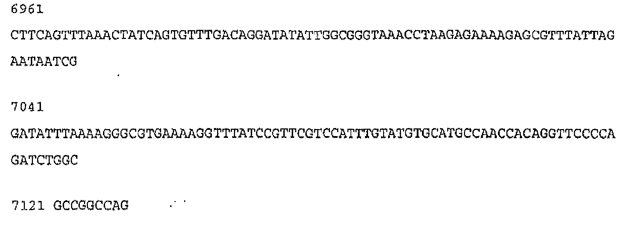

A construct for the expression in plants of human J chain and secretory component has also been developed. This construct, pGPTV-hpt-ocs-35SJ/SC, is based on the vector pGPTV-hpt-ocs, derived from pGPTV-hpt in the same manner as described for pGPTV-kan-ocs above. Sequence between the T-DNA borders of the plasmid pGPTV-hpt-ocs-35SJ/SC is shown in Figure 12. Sequence outside the left and right borders are as described (Becker et al., 1992). Nucleotides 1-149 represent the left T-DNA border. Nucleotides 733-955 (complement) represent the polyadenylation signal from A. *tumefactions* gene 7 (Gielen et al., 1984). Nucleotides 980-2002 (complement) represent the hpt gene (conferring resistance to hygromycin). Nucleotides 2049-2318 (complement) represent the NOS promoter (Depicker et al., 1982). Nucleotides 2898-3230 represent the cauliflower mosaic virus (CaMV) 35S promoter driving expression of the human secretory component gene including it's native signal peptide (nucleotides 3236-5056), and nucleotides 5060-5445 represent the polyadenylation signal from the pea rbcS-E9 gene (Mogen et al., 1992). Nucleotides 5457-5788 represent a second copy of the CaMV 35S promoter driving expression of the human Joining (J) chain gene including it's native signal peptide (nucleotides 5797-6273), and nucleotides 6281-6494 represent the gene 7 terminator. Nucleotides 6501-6819 (complement) represent the *ocs* 3' terminator region. Nucleotides 6944-7113 represent the right T-DNA border.

### 11. Plant Transformation and ATR Immunoadhesin Expression in Plants

The expression cassettes described above are used to produce the assembled immunoadhesin in plants, *via Agrobacterium-mediated* transformation. Plasmids pGPTV-hpt-ocs-35SJ/SC and pGPTV-kan-ocs-ATR-IgA2 are introduced separately into A *tumefaciens* strain LBA4404. Overnight cultures of both strains are used for simultaneous "co-cultivation" with leaf pieces of tobacco, according to a standard protocol (Horsch et al., 1985). Transformed plant tissue is selected on regeneration medium containing both kanamycin (100 µg/mL) and hygromycin (25 µg/mL).

Plantlets that regenerate in the presence of antibiotic are screened for transgene expression. This is accomplished by preparing extracts of leaf tissue in phosphate buffered saline (PBS) and spotting clarified extracts on nitrocellulose paper. These "dot" blots are probed with alkaline-phosphatase-conjugated antisera specific for human IgA, J chain or secretory component. Plants that test positive on this first screen are subjected for further screens involving western blotting and PCR. The ATR-IgA2 immunoadhesin is expected to have a subunit MW of 59 kDa. Due to natural dimerization of the heavy chain constant region, dimers of ~118 kDa are also expected to form. These dimers further dimerize within the plant cell in the presence of J chain, forming a molecule of ~252 kDa. With the addition of secretory component, a molecular species of ~320 kDa is observed.

The presence of a signal peptide in the chimeric heavy chain, J chain and secretory component constructs is important for assembly into a multimeric immunoadhesin. Upon translation of the mRNAs, signal peptide cleavage is predicted to deposit the each protein into the plant cell's endoplasmic reticulum (ER). Assembly into a multimeric immunoadhesin is expected to take place in the ER and golgi bodies, and the assembled molecule is then secreted from the cell.

### 12. Purification of Assembled ATR Immunoadhesin

Purification of Assembled ATR Immunoadhesin can be accomplished essentially as described for the ICAM-1 mmunoadhesin of Example 3, *supra.*

### 13. The ATR Immunoadhesin Inhibits Toxin Action on Mammalian Cells

The expression cassettes described above are used to produce the assembled immunoadhesin, which is purified from plant extracts. The purified immunoadhesin is used to protect CHO-K1 cells from being killed in a simple bioassay. CHO-K1 cells have the receptor to which PA binds on their cell surfaces, but they are not sensitive to the toxin. They are killed when challenged with PA and LF_{N}-DTA, a fusion protein composed of the N-terminal 255 amino acids of LF linked to the catalytic A chain of diptheria toxin. This recombinant toxin exploits the same LF-PA-receptor interactions that are required for the binding and entry of the native LF and OF proteins. To test the protective effect of the immunoadhesin, CHO-K1 cells are mixed with an increasing amount of ATR-IgA2 in the presence of a constant (toxic) amount of PA and LF_{N}-DTA, and the subsequent effect on protein synthesis is measured. ATR-IgA2 is an effective inhibitor of toxin action, inhibiting toxin action at a lower molar concentration than soluble ATR.

### 14. The ATR Immunoadhesin Inhibits Toxin Action in Human Subjects

The purified immunoadhesin is prepared in a pharmaceutically acceptable buffer and is administered to human subjects infected with Anthrax. The route of administration may be either as an inhaled aerosol or as an injection. Subjects in late stages of infection who would normally die are protected from toxin action by the immunoadhesin.

### 15. Construction of an Alternative ATR Immunoadhesin Expression Cassette

A cassette encoding the entire extracellular portion of human ATR (amino acids 24-320) is prepared by PCR cloning. Specifically, a fragment of 878 bp is amplified from plasmid ATR (Bradley et al., 2001), or from plasmid TEM8 (St Croix et al., 2000) using the following oligonucleotide primers:
5'-GGGGGACGCAGGGAGGATGGGGGTCCAG-3'
   (SEQ ID NO: 95)
5'-GAGCTCCCGTCAGAACAGTGTGTGGTGGTG-3'
   (SEQ ID NO: 96)

The second primer (SEQ ID NO: 96) is designed to introduce a Sac I site at the 3' end of the coding region of the ATR extracellular domain (solid underline). PCR is performed with *Pfu* polymerase (Stratagene) to reduce accumulation of errors. A second fragment of 121 bp, which includes a 5' untranslated region and a plant signal peptide, is amplified from plasmid δATG-TOPO#4, using the following oligonucleotide primers:
5'-GGTACCACTTCTCTCAATCCAACTTTC-3'
   (SEQ ID NO: 93)
5'-ATCCTCCCTGCGTCCCCCAGCCAAACTAGTAGAGGTGAACAAAAGC-3'
   (SEQ ID NO: 97)

The first primer (SEQ ID NO: 93) is designed to introduce a *Kpn* I site at the 5' end of the PCR fragment (solid underline). The two PCR fragments have 20 nt of complementary sequence (dotted underlines). The two PCR fragments are mixed together, and a fragment of 981 bp is amplified using SEQ ID NO: 93 and SEQ ID NO: 96. The resulting PCR fragment is cloned into a plant expression cassette to form a genetic fusion with human IgA2 in the same manner as the partial ATR extracellular domain (Example 1).

An alternate construction using this same method would amplify amino acids 41-227.

The foregoing examples are not limiting and merely representative of various aspects and embodiments of the present invention. All documents cited are indicative of the levels of skill in the art to which the invention pertains. The disclosure of each document is incorporated by reference herein to the same extent as if each had been incorporated by reference in its entirety individually, although none of the documents is admitted to be prior art.

One skilled in the art will readily appreciate that the present invention is well adapted to carry out the objects and obtain the ends and advantages mentioned, as well as those inherent therein. The methods and compositions described illustrate preferred embodiments, are exemplary, and are not intended as limitations on the scope of the invention. Certain modifications and other uses will occur to those skilled in the art, and are encompassed within the spirit of the invention, as defined by the scope of the claims.

It will be readily apparent to one skilled in the art that varying substitutions and modifications may be made to the invention without departing from the scope and spirit of the invention. Thus, such additional embodiments are within the scope of the invention and the following claims.

The invention illustratively described herein suitably may be practiced in the absence of any element or elements, limitation or limitations which is not specifically disclosed herein.. The terms and expressions which have been employed are used as terms of description and not of limitation, and there is no intention in the use of such terms and expressions of excluding any equivalents of the features shown and described, or portions thereof. It is recognized that various modifications are possible within the scope of the invention claimed. Thus, it should be understood that although the present invention has been specifically disclosed by preferred embodiments, optional features, modifications and variations of the concepts herein disclosed may be resorted to by those skilled in the art, and that such modifications and variations are considered to be within the scope of this invention as defined by the description and the appended claims.

In addition, where features or aspects of the invention are described in terms of Markush groups or other grouping of alternatives, those skilled in the art will recognize that the invention is also thereby described in terms of any individual member or subgroup of members of the Markush group or other group, and exclusions of individual members as appropriate.

Other embodiments are within the following claims.

## Claims

1. An immunoadhesin comprising a chimeric non-viral toxin receptor protein, said non-viral toxin receptor comprising:
a non-viral toxin receptor protein linked to at least a portion of an immunoglobulin heavy chain; and J chain and secretory component associated with said chimeric non-viral toxin receptor protein.

2. The immunoadhesin of claim 1 wherein said non-viral toxin receptor protein is an Anthrax toxin receptor protein comprised of:
the extracellular domain of Anthrax toxin receptor or any portion thereof.

3. The immunoadhesin of claim 1 wherein said immunoglobulin heavy chain is selected from the group consisting of:
IgA, IgA1, IgA2, IgM, and chimeric immunoglobulin heavy chains.

4. The immunoadhesin of claim 2 comprising at least one additional chimeric Anthrax toxin receptor protein.

5. The immunoadhesin of claim 2 wherein said Anthrax toxin receptor protein is comprised of any portion of the extracellular domain of Anthrax toxin receptor protein; and said immunoglobulin heavy chain comprises at least a portion of an IgA2 heavy chain.

6. The immunoadhesin of claim 1 expressed in transgenic plants.

7. The immunoadhesin of claim 1 expressed in monocotyledonous plants.

8. The immunoadhesin of claim 1 expressed in dicotyledonous plants.

9. The immunoadhesin of claim 1 wherein all proteins are human.

10. The immunoadhesin of claim 1 expressed in heterologous cells derived from plants, vertebrates, or invertebrates.

11. The immunoadhesin of claim 1 expressed in mammalian cells.

12. The immunoadhesin of claim 1 expressed in hairy root cultures.

13. The immunoadhesin of claim 1 expressed in plant cells in tissue culture.

14. An immunoadhesin comprising a chimeric bacterial or viral non-viral toxin receptor protein, said non-viral toxin receptor protein comprising: a non-viral toxin receptor protein linked to at least a portion of an immunoglobulin heavy chain, wherein said immunoadhesin has plant-specific glycosylation is expressed in a plant.

15. The immunoadhesin of claim 14 wherein said non-viral toxin receptor protein is an Anthrax toxin receptor protein.

16. The immunoadhesin of claim 15 wherein said immunoadhesin further comprises a J chain and secretory component associated with said chimeric Anthrax toxin receptor protein.

17. The immunoadhesin of claim 15 wherein said Anthrax toxin receptor protein is comprised of the extracellular domain of Anthrax toxin receptor or any portion thereof.

18. The immunoadhesin of claim 14 wherein said immunoglobulin heavy chain is selected from the group of IgA, IgA₁, IgA₂, IgG₁, IgG₂, IgG₃, IgG₄, IgD, IgE, IgM, and a chimeric immunoglobulin heavy chain.

19. The immunoadhesin of claim 14 or 15 comprising at least one additional chimeric non-viral toxin receptor protein.

20. The immunoadhesin of claim 14 or 15 wherein said non-viral toxin receptor protein is comprised of any portion of the extracellular domain of said non-viral toxin receptor protein; and said immunoglobulin heavy chain comprises at least a portion of an IgA₂ heavy chain.

21. The immunoadhesin of claim 14 wherein all proteins are human or associated with a human host during infection and/or pathagenesis pathogenesis.

22. The immunoadhesin of claim 14 expressed in heterologous cells derived from plants, vertebrates, or invertebrates.

23. The immunoadhesin of claim 14 expressed in hairy root cultures.

24. The immunoadhesin of claim 14 expressed in plant cells in tissue culture.

25. The immunoadhesin of claim 14 expressed in transgenic plants.

26. The immunoadhesin of claim 14 expressed in monocotyledonous plants.

27. The immunoadhesin of claim 14 expressed in dicotyledonous plants.

28. A composition comprising an immunoadhesin and plant material, wherein said immunoadhesin comprises a chimeric non-viral toxin receptor protein, said chimeric non-viral toxin receptor protein linked to at least a portion of an immunoglobulin heavy chain.

29. The composition of claim 28 further comprising a J chain and secretory component with said chimeric non-viral toxin receptor protein.

30. The composition of claim 28 wherein said chimeric non-viral toxin receptor protein is comprised of any portion of the extracellular domain of said non-viral toxin receptor protein; and said immunoadhesin has plant-specific glycosylation is expressed in a plant.

31. The composition of claim 28 wherein said immunoglobulin heavy chain is selected from the group consisting of IgA, IgA₁, IgA₂, IgG₁, IgG₂, IgG₃, IgG₄, IgD, IgE, IgM, and a chimeric immunoglobulin heavy chain.

32. The composition of claim 28 comprising at least one additional chimeric non-viral toxin receptor protein.

33. The composition of claim 28 wherein said non-viral toxin receptor protein is comprised of any portion of the extracellular domain of said non-viral toxin receptor protein; and said immunoglobulin heavy chain comprises at least a portion of an IgA₂ heavy chain.

34. The composition of claim 28-33 wherein said non-viral toxin receptor protein is an Anthrax toxin receptor protein.

35. A method for reducing the binding of a viral or bacterial non-viral antigen to a host cell, said method comprising: contacting said non-viral antigen with an immunoadhesin of claim 1, 14 or [[27]] 28, and wherein said immunoadhesin binds to said non-viral antigen and reduces the toxic activity thereof.

36. A method for reducing mortality and morbidity of a viral or bacterial non-viral pathogen, said method comprising: contacting an antigen of said viral or bacterial non-viral pathogen with an immunoadhesin of claim 1, 14 or 28, and wherein said immunoadhesin binds to said antigen and reduces the toxic activity thereof.

37. A method for reducing mortality and morbidity due to a bacterial or viral non-viral toxin in a human subject, said method comprising: administering to said subject an effective amount of an immunoadhesin of claim 1, 14 or 28, and wherein said immunoadhesin binds to said non-viral toxin and reduces the toxic activity thereof.

38. The method of any of claims 35-37 wherein said non-viral antigen or toxin is an anthrax PA toxin.

39. A pharmaceutical composition comprising the immunoadhesin of claim 1, 14 or 28 in a pharmaceutically acceptable buffer.

40. An expression vector comprising a gene encoding a chimeric non-viral toxin receptor protein operatively linked to a plant promoter, said chimeric non-viral toxin receptor protein linked to at least a portion of an immunoglobulin heavy chain.

41. The expression vector claim 40 wherein said non-viral toxin receptor protein is an anthrax toxin receptor protein.
